(19)

Europäisches
Patentamt
European
Patent Office
Office européen
des brevets

(11)     **EP 2 389 573 B1**

(12)     **EUROPEAN PATENT SPECIFICATION**

(45) Date of publication and mention
of the grant of the patent:
**20.01.2016 Bulletin 2016/03**

(51) Int Cl.:
***G01N 21/21*** *(2006.01)*      ***A61B 5/00*** *(2006.01)*
***G06F 19/00*** *(2011.01)*

(21) Application number: **10741554.9**

(22) Date of filing: **20.01.2010**

(86) International application number:
**PCT/US2010/021529**

(87) International publication number:
**WO 2010/093503 (19.08.2010 Gazette 2010/33)**

(54) **SKIN ANALYSIS METHODS**

HAUTANALYSEVERFAHREN

PROCÉDÉS D'ANALYSE DE LA PEAU

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **06.02.2009 US 150331 P
20.01.2009 US 145756 P
02.02.2009 US 149027 P
20.08.2009 US 235362 P
05.02.2009 US 150053 P
23.10.2009 US 254214 P
15.04.2009 US 169316 P
02.02.2009 US 149025 P
04.11.2009 US 612297
05.02.2009 US 150010 P**

(43) Date of publication of application:
**30.11.2011 Bulletin 2011/48**

(73) Proprietor: **Myskin, Inc.**
**Jersey City, NJ 07310 (US)**

(72) Inventors:
• **BANDIC, Jadran**
**Pancevo 26000 (RS)**
• **MEHENDALE, Rahul**
**Jersey City, NJ 07310 (US)**
• **MARINKOVICH, Sava**
**Chicago, IL 60659 (US)**
• **KORUGA, Djuro**
**Belgrade 11152 (RS)**

(74) Representative: **Moreland, David et al**
**Marks & Clerk LLP**
**Aurora**
**120 Bothwell Street**
**Glasgow G2 7JS (GB)**

(56) References cited:
**WO-A1-2009/089292      WO-A2-2008/086311
US-A- 5 016 173      US-A1- 2002 084 417
US-A1- 2004 097 812      US-A1- 2006 227 137
US-A1- 2007 178 067      US-A1- 2007 249 913
US-A1- 2008 194 928      US-A1- 2008 294 012
US-B1- 6 993 167      US-B2- 6 622 033**

## Description

## BACKGROUND OF THE INVENTION

**Field of the Invention**

The invention relates to methods and apparatus for enabling the collection of dermal and non-dermal images using a non-invasive imaging device, the development of a skin state based at least in part on analysis of such images, and the monitoring of the skin state by, at least, a collection and analysis of subsequent images. The invention further pertains to the field of skin care devices and systems capable of facilitating skin care decisions, more specifically the field of devices for skin condition assessment, skin care regimen recommendation, and skin care regimen effectiveness tracking.

The present invention also relates to an image processing technique. More particularly, the present invention relates to determining a skin photo type of a captured image in a Red Green Blue (RGB) color imaging system and is also applicable in classification of other skin characteristics (e.g. elasticity, melanin, oil concentration etc.), melanoma, skin related tumors and skin related disorders.

**Description of the Related Art**

Skin is the largest organ of the integumentary system which further includes skin's accessory structures, such as hair, nails, scales, feathers, sweat glands and their products. Skin comprises multiple layers of epithelial tissues that guard underlying muscles and organs. Since skin is subject to constant attack from various external and internal factors, it can be afflicted by numerous ailments. Thus, it is critical to monitor skin health and the effect of any treatments, skin care products, or cosmetics applied to the skin.

Certain skin care decision systems involving conventional photography are also known as "clinical imaging." Some of the known methods involve illuminating the skin surface with white light while the digital image is acquired by the camera. The effectiveness of clinical imaging is, however, compromised by specular (mirror-like) reflection of the skin.

The existing processes of making skin care decisions for both medical and cosmetic purposes are generally based on scattered information collected from multiple sources in different time periods. The existing processes are cumbersome and inefficient, resulting in delayed and sub-optimal skin-care decisions. Examples of skin-care decisions that can be significantly enhanced by the principles of the invention include diagnosis of skin conditions, selection of skin-care products and regimens, and tracking the effectiveness of skin-care products and regimens over a period of time. A skin-care regimen includes both selection of appropriate skin-care products and the entire procedure of applying the selected skin-care products, including the dosage, timing, methodology, and frequency of application of the skin-care products.

While various methods exist for determining and monitoring skin health, most require access to a dermatologist or a dermatological facility, thus, there may be difficulty, inconvenience, and prohibitively high cost in accessing the necessary resources. There is a need for a simple solution for skin health determination and monitoring that may be operable by an untrained or trained user, and, in the absence of an in-person consultation, where dermal images may be submitted to an expert, an analysis facility, or for automated analysis.

Skin characteristics are typically determined using a Fitzpatrick classification. Skin phototype is categorized according to a conventional Fitzpatrick Skin Typing Test questionnaire (skin type scale), which ranges from very fair (skin type I, for example) to very dark (skin type VI, for example). Conventionally, various image processing techniques are disclosed for determining skin characteristics using captured skin images. In a conventional digital image processing technique it is often useful to detect multiple features in the captured skin image, such as skin color. This information is used, for example, to adjust the skin colors in the image to be comfortable to perceive. The location of skin color is also used in face detection and face recognition algorithms, automatic image retrieval algorithms, and red-eye correction algorithms.

Less work has been done on objective measurement of human skin coloration to enable its color classification. Classification of a person's skin coloration would be useful, for example, in the medical field for quantification of skin erythema, lesions, ultra-violet radiation effects, and other skin coloration phenomena. In the field of computer graphics images of people could be rendered more accurately in video-conferencing and their appearance could be improved or altered. While the science of digital skin imaging analysis has identified various skin responses that are useful indicators of various skin condition parameters, it would still be desirable to identify and use additional specific responses of the skin that are indicative of skin conditions and skin characteristics.

Cosmetic appearance is one of the top priorities to most of the humans in the modern world. Techniques or systems existing in the known art analyze the skin conditions and suggest suitable products to improve the cosmetic appearance of a human being.

There is a need for a minimal error and speed efficient method and system to determine the phototype of skin.

WO 2009/089292 (MySkin Inc.) falls under Art. 54(3) EPC and relates to a method for estimation of skin type and skin features to create a unique spectral signature,

## SUMMARY OF THE INVENTION

According to an aspect of the present invention there is provided a system and method according to the appended claims. Real-time analysis of digitally captured skin characteristics facilitates timely skin condition assessment, skin regimen recommendation, and skin regimen effectiveness tracking.

The problem of generating a skin condition assessment in real-time is solved by having a skin condition analysis module capable of doing real-time analysis of digital skin data, acquired partly using diffused reflectance spectroscopy and/ or detecting the red-green-blue components of re-emitted white light.

In an arrangement, a skin care device may include an electromagnetic radiation source capable of directing incident electromagnetic radiation to a location on the skin of a user, a radiation detector for measuring various parameters of radiation re-emitted from the location, and a skin condition analysis module coupled to the detector, the analysis module capable of generating a skin condition assessment in real-time, based partly on at least one of RGB analysis and diffused reflectance analysis of the radiation parameters. In the device, the incident electromagnetic radiation may include radiation in at least one of the visible, near-infrared, and near-ultraviolet spectrum. The incident radiation may include white light. In the device, the radiation parameters may include at least the degree of polarization of the re-emitted radiation. In the device, the radiation source may be a set of light emitting diodes. In the device, the skin condition assessment may also be partly based on analysis of a photographic image of a skin region surrounding the location. In the device, the device may be a miniature device. Miniature may mean that no dimension of the detector exceeds six inches. The device may further comprise a memory module for storing the skin condition assessment. The device may further comprise a user interface. The user interface may be operated using voice commands. In the device, skin assessment data of locations may be overlaid on an image of a larger skin region and displayed on the display surface. The device may further comprise an access restriction module used for restricting access to authorized users only. The access restriction module may be based on biometric access control. The device may be capable of generating alerts about abnormal skin conditions in real-time. The device may further comprise a skin care regimen recommendation module that generates a displayable skin care regimen recommendation. The skin care regimen recommendation may be based at least partly on determination of a skin profile of the user and use of skin care regimen recommendations of persons with a similar profile. The skin care regimen recommendation module may be linked to a product database. The product database may include products available in a point-of-sale location. The availability of a specific product recommended by the skin care regimen recommendation module may be indicated by an audio-visual signal. The device may further comprise a skin care regimen effectiveness module that generates a displayable skin care regimen effectiveness report. The device may further comprise a communication module for communicating with a remote computer. The communication may occur wirelessly. The communication may occur over an internet. The remote computer may be operable by a physician. The device may be wand-shaped. The device may be wearable by the user.

In an arrangement, the skin care device may include an electromagnetic radiation source capable of directing incident electromagnetic radiation to a location on the skin of a user, a detector for measuring various parameters of radiation re-emitted from the location, a skin condition analysis module coupled to the detector, the analysis module capable of generating a skin condition assessment in real-time, based partly on at least one of RGB analysis and diffused reflectance analysis of the radiation parameters, and a display panel for reflecting the image of the user. In the device, the display panel may be touch-sensitive such that touching the location in a skin region image displayed in the display panel triggers display of a magnified image of the location. The device may further comprise a camera. The camera may be integral with the display panel. The camera may be wirelessly linked to the display panel. In the device, the display panel may be a mirror. In the device, a stored image of the user is used to automatically identify the person. The device may further comprise a user interface for controlling the skin care device. The user interface may be operated using voice commands. The device may further comprise a skin care regimen recommendation module capable of generating a displayable skin care regimen recommendation. The skin care regimen recommendation may be based at least partly on determination of a skin profile of the user and use of skin care regimen recommendations of persons with a similar profile. The device may further comprise a skin care regimen effectiveness module capable of generating a displayable skin care regimen effectiveness report.

In arrangement, an imaging device permits a user to take high magnification pictures of the skin in the vicinity of an area of concern and submit those pictures, optionally along with textual and data responses, for medical, non-medical, and cosmetic analysis, diagnosis and treatment recommendation and follow-up.

In an arrangement, a method and system of a non-invasive imaging device may comprise an illumination source comprising an incident light source to direct light upon skin; and a detector for detecting the degree of polarization of light reflected from the skin. In the method and system, the illumination source may be positioned to direct light at a selected angle alpha. Varying alpha may vary the depth of the measurement of the layers in the skin. Each depth may have a specific angle which produces a full polarized reflection. In the method and system, the incident light source may be an unpolarized light source. The unpolarized light may be white light, multiple selected wavelengths, or a single wavelength. The method and system may further comprise a sensor for capturing an image of the reflected or re-emitted light. The method and system may further comprise an optical facility for detecting reflected or re-emitted light from the skin. The method may determine both reflected or re-emitted light, and newly emitted light, through the process of absorption and re-emission. The method and system may further comprise a communication facility for transmitting the detected information. The method and system may further comprise a storage facility for storing information collected by the device.

In an arrangement, a method and system for determining a skin state may comprise illuminating skin with an incident light source, detecting the degree of polarization of light reflected from the skin, and determining a skin state based on an aspect of the polarization of the reflected or re-emitted light. In the method and system, the incident light may be directed at a selected angle alpha. Varying alpha may vary the depth of the measurement of the layers in the skin. Each depth may have a specific angle which produces a full polarized reflection. In the method and system, the incident light source may be an unpolarized light source. The unpolarized light may be white light, multiple selected wavelengths, or a single wavelength. In the method of claim, the aspect of the polarization may be at least one of an orientation, an amplitude, a phase, an angle, a shape, a degree, an amount, and the like. In the method and system, determining may be done using an algorithm. The algorithm may involve artificial neural networks, non-linear regression, genetic algorithms, fuzzy logic, fractal and multi-fractal analysis, and the like. The methods and systems may further comprise filtering the reflected or re-emitted light to obtain polarized light of at least one wavelength defined by the filter output. The algorithmic analysis may be performed on the filtered image. In the method and system, determining may involve creating an image from the difference between the reflected diffusion light and the reflected polarized light. In the method and system, determining may involve comparing the aspect of the polarization of the reflected or re-emitted light to a calibration signal. In the method and system, determining may further comprise considering at least one of user input and a visual analysis.

In an arrangement, a non-invasive imaging device may comprise an illumination source comprising an incident light source to direct light upon an area of concern and a detector for detecting the degree of polarization of light reflected from the area of concern. In the method and system, the illumination source may be positioned to direct light at a selected angle alpha. Varying alpha may vary the depth of the measurement of the layers in the skin. Each depth may have a specific angle which produces a full polarized reflection. In the method and system, the incident light source may be an unpolarized light source. The unpolarized light may be white light, multiple selected wavelengths, or a single wavelength. The method and system may further comprise a sensor for capturing an image of the reflected or re-emitted light. The method and system may further comprise an optical facility for detecting reflected or re-emitted light from the skin. The method and system may further comprise a communication facility for transmitting the detected information. The method and system may further comprise a storage facility for storing information collected by the device.

In an arrangement, a method of determining moisture levels in the skin may comprise emitting incident light towards a skin structure, detecting a degree of polarization of the light induced by the skin structure, and determining a moisture level based on the amount of polarized and reflected or re-emitted light. The method and system may further comprise combining the assessment of moisture level with skin color measurements to determine luminosity. In the method and system, the incident light may be unpolarized light. The unpolarized light may be white light, light of multiple selected wavelengths, or of a single wavelength, or one or more monochromatic lights. In the method and system, determining may involve use of an algorithm. In the method and system, determining a moisture level may be based on the ratio of polarized and reflected or re-emitted light.

In an arrangement, a method and system of determining elasticity of the skin may comprise emitting incident light towards a skin structure, detecting an aspect of polarization of the light reflected by the skin structure, correlating the aspect of polarization with a concentration of elastin, and determining elasticity level based on the elastin status. In the method and system, determining may involve use of an algorithm. In the method and system, the incident light may be unpolarized light. The unpolarized light may be white light, light of multiple selected wavelengths, or a single wavelength of light.

In an arrangement, a method and system of determining firmness of the skin may comprise emitting incident light towards a skin structure, detecting an aspect of polarization of the light reflected by the skin structure, correlating the aspect of polarization with the status of at least one of an elastin, a collagen, and an activity of a sebaceous gland, and determining firmness based on the concentration of at least one of elastin and collagen and sebaceous gland activity. In the method and system, the sebaceous gland activity may be indicated by at least one of a number of glands, percent of glands open/closed, and level of clog/ fill. In the method and system, correlating may involve use of an algorithm.

In an arrangement, a method and system for obtaining dermal biophysical properties may comprise performing a spectral analysis of image data acquired from the degree of polarization of reflections and absorption and re-emission of incident light from skin structures, wherein the property is at least one of a structure, form, status, number, size, state, and stage of at least one of a: melanocyte, melanin, hemoglobin, porphyrin, tryptophan, NADH, FAD, keratin, carotene, collagen, elastin, sebum, sebaceous gland activity, pore (sweat and sebaceous), moisture level, elasticity, luminosity, firmness, fine line, wrinkle count and stage, pore size, percent of open pores, skin elasticity, skin tension line, spot, skin color, psoriasis, allergy, red area, general skin disorder or infection, tumor, sunburn, rash, scratch, pimple, acne, strias, insect bite, itch, bleeding, injury, inflammation, photodamage, pigmentation, tone, tattoo, percent burn/ burn classification, mole (naevi, nevus), aspect of a skin lesion (structure, color, dimensions/asymmetry), melanoma, automated follow-up of pigmented skin lesions, dermally observed disorder, cutaneous lesion, cellulite, boil, blistering disease, congenital dermal syndrome, (sub)-cutaneous mycoses, melasma, vascular condition, rosacea, spider vein, texture, skin ulcer, wound healing, post-operative tracking, melanocytic lesion, non-melanocytic lesion, basal cell carcinoma, seborrhoic keratosis, sebum (oiliness), nail- and/or hair-related concern, and the like.

In an arrangement, a system and method may comprise providing an interface that includes a social networking domain or rating-and-ranking system and at least one of a skin state determination facility and a recommendation engine, and enabling users, either all or a selected few, of the interface to perform a skin state determination within the interface. In the method and system, the skin state determination facility may comprise capturing images with a non-invasive imaging device comprising an illumination source comprising an incident light source to direct light upon skin, and a detector for detecting the degree of polarization of light reflected from the skin, and determining a skin state based on an aspect of the polarization of the reflected or re-emitted light. The method and system may further comprise receiving product and regimen recommendations from the recommendation engine based on what other users with similar skin states are using as well as data regarding ingredients, effectiveness, safety, and the like. The method and system may further comprise comparing skin states, products, regimens, and recommended products or regimens with peers within the social networking domain of the interface. Comparing may comprise an analysis of similarity based on the spectral analysis of the degree of polarization of reflected or re-emitted light from users' skin. In the method and system, the interface may comprise a regimen tracker. The regimen tracker may be populated using a drag-and-drop or click-to-add functionality. In the method and system, the interface may comprise a rating facility or a product information facility. The product information facility may enable a user to obtain product information by search. Search may be a search of product identifiers, product ratings, drag-and-drop items, images, barcode scans, skin states, and profiles.

In an arrangement, a method and system for determining a skin state may comprise obtaining the answers to a series of subjective questions regarding the skin, obtaining an objective skin analysis using a dermal imaging device, and combining the subjective and objective results algorithmically to obtain a skin state.

In an arrangement, a system and method for providing recommendations for skin care based on a skin state and a skin care goal may comprise obtaining a skin state of an individual, categorizing the individual by skin state, and recommending products and regimens that are effective for other individuals of the category in achieving the skin care goal. In the method and system, the system may be operable over a network. In the method and system, the skin state may be determined based on analysis of the degree of polarization of light reflected from the skin of the individual.

In an arrangement, a method for tracking the effectiveness of a skin care product or regimen may comprise obtaining a baseline skin state assessment, recommending a monitoring interval based on at least one of the skin care goal, product, and regimen, obtaining a second skin state assessment, comparing the second assessment to the baseline assessment to determine progress towards a skin care goal, and optionally, optimizing the regimen or product in order to improve a skin state. In the method and system, the skin assessment may be based on analysis of the degree of polarization of light reflected from the skin of the individual.

In an arrangement, a personalized skin condition analysis system and related methods may comprise an imaging device, comprising an illumination source comprising an incident light source to direct light upon skin, and a detector for detecting the degree of polarization of light reflected from the skin, and a user interface for controlling the device. In the methods and system, the device may be adapted to interact with a physical interface to download image data to update a record of at least one of a practitioner, a spa, a salon, cosmetic sales, a cosmetics manufacturer, a clinical trials database, and a third party database. In the method and system, the illumination source may be positioned to direct light at a selected angle alpha. Varying alpha may vary the depth of the measurement of the layers in the skin. Each depth may have a specific angle which produces a full polarized reflection. In the method and system, the incident light source may be an unpolarized light source. The unpolarized light may be white light, multiple selected wavelengths, or a single wavelength. The method and system may further comprise a sensor for capturing an image of the reflected or re-emitted light. The method and system may further comprise an optical facility for detecting reflected or re-emitted light from the skin. The method and system may further comprise a communication facility for transmitting the detected information. The method and system may further comprise a storage facility for storing information collected by the device.

In an arrangement, a non-invasive imaging device may comprise an illumination source comprising an incident light source to direct light upon skin; and a detector for detecting a characteristic of the light reflected from the skin. In the device, the illumination source may be positioned to direct light at a selected angle alpha. Varying alpha may vary the depth of the measurement of the layers in the skin. Each depth may have a specific angle which produces a full polarized reflection. In the device, the incident light source may be a polarized light source or unpolarized light source.

The unpolarized light may be at least one of white light, light of a single wavelength, and light of multiple single wavelengths. The device may further comprise a sensor for capturing an image of the reflected or re-emitted light. The device may further comprise an optical facility for detecting reflected or re-emitted light from the skin. The device may further comprise a communication facility for transmitting the detected information. The device may further comprise a storage facility for storing information collected by the device. In the device, the reflected or re-emitted light may be at least one of polarized light and unpolarized light.

In an arrangement, a method and system for determining a skin state may comprise illuminating skin with an incident light source; detecting a characteristic of the light reflected from the skin; and determining a skin state based on at least one characteristic of the reflected or re-emitted light. In the method and system, the incident light may be directed at a selected angle alpha. Varying alpha may vary the depth of the measurement of the layers in the skin. Each depth may have a specific angle which produces a full polarized reflection. In the method and system, the incident light may be unpolarized or polarized light. The unpolarized light may be at least one of white light, light of a single wavelength, and light of multiple single wavelengths. In the method and system, the reflected or re-emitted light may be at least one of polarized light and unpolarized light. In the method and system, the characteristic may be at least one of light source, light intensity, wavelength of light, angle of light, electrical and magnetic properties of the light, and polarization state of the light. An aspect of the polarization may be at least one of an orientation, an amplitude, a phase, an angle, a shape, a degree, and an amount. In the method and system, determining may be done using an algorithm. The algorithm may involve artificial neural networks, non-linear regression, genetic algorithms, fuzzy logic, or fractal and multi-fractal analysis. The method and system may further comprise filtering the reflected or re-emitted light to obtain light of a wavelength defined by the filter output. The analysis may be performed on the filtered image. In the method and system, determining may involve creating an image of the difference between reflected diffusion light and reflected polarized light. In the method and system, determining may involve comparing the aspect of the polarization of the reflected or re-emitted light to a calibration signal. In the method and system, determining may further comprise considering at least one of user input and a visual analysis.

In an arrangement, a non-invasive imaging device may comprise an illumination source comprising an incident light source to direct light upon an area of concern; and a detector for detecting a characteristic of the light reflected from the area of concern. In the device, the illumination source may be positioned to direct light at a selected angle alpha. Varying alpha may vary the depth of the measurement of the layers in the skin. Each depth may have a specific angle which produces a full polarized reflection. In the device, the incident light source may be a polarized light source or unpolarized light source. The unpolarized light may be at least one of white light, light of a single wavelength, and light of multiple single wavelengths. The device may further comprise a sensor for capturing an image of the reflected or re-emitted light. The device may further comprise an optical facility for detecting reflected or re-emitted light from the skin. The device may further comprise a communication facility for transmitting the detected information. The device may further comprise a storage facility for storing information collected by the device. In the device, the reflected or re-emitted light may be at least one of polarized light and unpolarized light.

In an arrangement, a system and method may be used to determine healthy and melanoctyic skin. First, you take the first reflected spectrum and/or reflected spectrum of the melanocytic "skin malformation (SM)" and subtract the reflected spectrum of the healthy skin (SN). The second step is taking the resulting spectral plots (SM-SN) and subtracting the reflected spectrum from an adequate comparing screen, which represents the spectral plot of a the light source. The resulting plot gives the specific changes as related to selected screen. For differentiation between melanoma, other malignant or benign nevus and healthy skin can be accomplished by comparing the various maxima, minima and zero positions in resulting plot.

In an arrangement, a system and method may comprise capturing an image of a material illuminated with incident non-angled white light and angled white light, generating a normalized red and blue color channel histogram for each image, correlating the normalized red and blue color channel histograms to a wavelength scale to obtain red and blue color channel spectral plots, and convoluting the spectral plots by subtracting the spectral plot for angled light from the spectral plot for non-angled light for each color channel to generate red and blue normalized, composite color channel spectral plots, and subtracting the normalized, composite blue channel spectral plot from the normalized, composite red channel spectral plot to generate a spectral signature for the material. In the system and method, the illumination source may be positioned to direct light at a selected angle alpha. Varying alpha varies the depth of the measurement in the material. In the system and method, the unit scale on the spectral signature may be a difference of wavelength. In the system and method, the material is inorganic and/ or organic matter. In the system and method, the spectral signature may be analyzed for at least one of number of peaks and troughs, amplitude and shape of peaks and intermediate structures and patterns. In the system and method, the spectral signature may be analyzed for metal composition, identification, purity, and strength. In the system and method, the spectral signature may be analyzed for water quality, composition, and purity. In the system and method, elements of the spectral signature may be tagged and tracked over time in order to track changes in the characteristics of the material. In the system and method, the spectral signature may be analyzed to measure, track or monitor a skin state. In the system and method, the spectral signature may be useful for the counterfeit analysis of money. In the system and method, the spectral signature may be analyzed for at least one of sweat gland activity and anti-perspirant effectiveness. In the system and method, the spectral signature may be analyzed for Mad Cow disease. In the system, the spectral signature may be analyzed for food, all epidermal diseases, melanoma and skin cancers, rheumatoid diseases, and all diseases that show on the skin. In the system and method, the spectral signature may be useful for monitoring post-operative cosmetic concerns. In the system and method, the spectral signature may be useful for predicting and monitoring secretion from the mammary glands of lactating women. In the system and method, the spectral signature may be fed into a recommendation engine to provide feedback and modifications to aspects of a regimen. In the system and method, the wavelength position of ideal blue in Maxwell's color triangle is aligned with the wavelength position of ideal red in Maxwell's color triangle when convoluting the composite spectral plots to obtain the spectral signature.

A method and a system are disclosed for determining skin characteristics and cosmetic features. A minimal error output is generated. In accordance with exemplary embodiments of the present invention, according to a first arrangement, a method for determining skin characteristics and cosmetic features using color analysis may include a step of analyzing color of skin images in a pixel by pixel manner in a Red Green Blue (RGB) color system for an acquired digital image. The step of analyzing color of skin images in a pixel by pixel manner in a RGB color system for an acquired digital image may include analyzing a picture of a part of a person's skin by generating a table of most frequent colors appearing in the picture.

According to the first arrangement, a method for determining skin characteristics and cosmetic features using color analysis includes a step of generating a sample of most frequent standard RGB (sRGB) colors responsive to analyzing color of skin images in a pixel by pixel manner in the RGB color system for the acquired digital image after converting colors obtained in a device dependent RGB color system into a device independent standard RGB color system (sRGB). The step of generating a sample of most frequent sRGB colors responsive to analyzing color of skin images in the sRGB color system for the acquired digital image may include preserving a plurality of sRGB color values.

In this embodiment of the invention, the sRGB color system may be used for image analysis. Determination of other skin characteristics, melanoma, skin related tumors and skin related disorders require image analysis based on other color systems such as YIQ, YCbCr, L*a*b*, L*u*v* and HSL/HSV. The enhancement of the current algorithm may include at least one of these color systems and its/their correlation with presented sRGB analysis.

According to the first arrangement, a method for determining skin characteristics and cosmetic features using color analysis includes a step of modeling the R, G and B component color distribution with Gaussian probabilistic distribution with estimated parameters (expected value and standard deviation) of the generated sRGB color sample for the acquired digital image further including approximating colors of the generated sRGB color samples by a Gaussian normal distribution. In accordance with an exemplary embodiment of the present invention the step of approximating colors of the generated sRGB color samples by a Gaussian normal distribution comprises approximating colors of the generated sRGB color samples by a superposition of a plurality of Gaussian normal distributions.

According to the first arrangement, a method for determining skin characteristics and cosmetic features using color analysis includes a step of generating a phototype of the skin through a decision tree unit responsive to the estimated distribution model parameters colors. The phototype of the skin may be generated according to a corrected Fitzpatrick classification. In accordance with an exemplary embodiment of the present invention, the step of generating a phototype of the skin according to a corrected Fitzpatrick classification includes generating the phototype of the skin according to a skin type scale which ranges from very fair skin to very dark skin. This method may be measured both on the most exposed region and relate to the current level of phototype based on the level of tan of the skin.

According to a second arrangement, a system for skin phototype determination using photograph analysis may be disclosed. The system may include an image capturing device for capturing digital images of a skin. The image capturing device may include a digital camera unit.

According to the second arrangement, the system for skin phototype determination using photograph analysis may include an analyzer coupled to the image capturing device for performing a pixel by pixel analysis of a picture of a part of a person's skin. The analyzer may include a quantization device for generating a look-up table of most frequent colors appearing on the picture of the part of the person's skin.

According to the second arrangement, the system for skin phototype determination using photograph analysis may include a sampling device coupled to the image capturing device for generating standard Red Green Blue (sRGB) color samples for the captured digital image of the skin.

According to the second arrangement, the system for skin phototype determination using photograph analysis may include an approximating device coupled to the sampling device for approximating the color distribution parameters on the generated sRGB color samples using the estimates of expected value and standard deviation for the captured digital image of the skin. The approximating device may include at least one Gaussian normal distribution unit.

According to the second arrangement, the system for skin phototype determination using photograph analysis may include a decision tree unit coupled to the approximating device for generating a phototype of the skin using Red and Blue components of the approximated colors. The decision tree unit may include a Fitzpatrick scaling unit for categorizing a skin phototype in accordance with a skin type scale which ranges from very fair skin to very dark skin.

According to the second arrangement, an exemplary embodiment of the present invention discloses a scaled Gaussian normal distribution unit for approximating colors on the generated sRGB color samples using estimates of expected value and standard deviation for the captured digital image of the skin.

According to the second arrangement, the system for skin phototype determination using photograph analysis may include a subsystem for determination of cosmetic features for a human element and a veterinary element. The cosmetic features may further include features pertaining to hair, nail and skin.

In another arrangement the system may include a sampling device for generating standard Red Green Blue color samples of the captured digital image of the skin, the generated samples of standard Red Green Blue are in the range of values between 0 and 255 and they are preserved for further processing.

In another arrangement the system may include an approximating device coupled to the sampling device for approximating the color distribution parameters on the generated sRGB color samples in the range of values between 0 and 255 by Gaussian normal distribution using the estimates of expected value and standard deviation for the captured digital image of the skin.

In another arrangement the system may further include a decision tree unit coupled to the approximating device for generating a phototype of the skin using standard Red and Blue components of the approximated colors, the decision tree unit with an algorithm equates estimates of expected values and standard deviation for the captured image of the skin to the Fitzpatrick notation of skin analysis for determination of skin phototype.

In another arrangement the system may automatically adjust lighting intensity and wavelengths and angles in order to assess various factors of the skin.

In yet another arrangement of the system skin phototype may be determined using photograph analysis for use in cosmetics and surgical industry.

In an arrangement, a skin care device may include an electromagnetic radiation source capable of directing incident electromagnetic radiation to a location on the skin of a user, a radiation detector for measuring various parameters of radiation re-emitted from the location, and a skin condition analysis module coupled to the detector, the analysis module capable of generating a skin condition assessment in real-time, based partly on at least one of RGB analysis and diffused reflectance analysis of the radiation parameters. In the device, incident electromagnetic radiation may include radiation in at least one of the visible, near-infrared, and near-ultraviolet spectrum. The incident radiation may be white light. In the device, the radiation parameters include at least the degree of polarization of the re-emitted radiation. In the device, the radiation source may be a set of light emitting diodes. In the device, the skin condition assessment may be also partly based on analysis of a photographic image of a skin region surrounding the location. In the device, the device may be a miniature device. Miniature may mean that no dimension of the detector exceeds six inches. The device may further include a memory module for storing the skin condition assessment. The device may further include a user interface. The device may further include a display surface. The skin assessment data of locations may be overlaid on an image of a larger skin region and displayed on the display surface. The device may further include an access restriction module used for restricting access to authorized users only. The access restriction module may be based on biometric access control. The device may be capable of generating alerts about abnormal skin conditions in real-time. The user interface may be operated using voice and/or eye movement commands. The device may further include a skin care regimen recommendation module that generates a displayable skin care regimen recommendation. The skin care regimen recommendation may be based at least partly on determination of a skin profile of the user and use of skin care regimen recommendations of persons with a similar profile. The skin care regimen recommendation module may be linked to a product database. The product database may include products available in a point-of-sale location. The availability of a specific product recommended by the skin care regimen recommendation module may be indicated by an audio-visual signal. The device may further include a skin care regimen effectiveness module that generates a displayable skin care regimen effectiveness report. The device may further include a communication module for communicating with a remote computer. The communication may occur wirelessly. The communication may occur over an internet. The remote computer may be operable by a physician. The device may be wand-shaped. The device may be wearable by the user.

In an arrangement, the device an electromagnetic radiation source capable of directing incident electromagnetic radiation to a location on the skin of a user, a detector for measuring various parameters of radiation re-emitted from the location, a skin condition analysis module coupled to the detector, the analysis module capable of generating a skin condition assessment in real-time, based partly on at least one of RGB analysis and diffused reflectance analysis of the radiation parameters, and a display panel for reflecting the image of the user. In the device, the display panel may be touch-sensitive such that touching the location in a skin region image displayed in the display panel triggers display of a magnified image of the location. The skin care device may further include a camera. The camera may be integral with the display panel. The camera may be wirelessly linked to the display panel. In the device, the display panel may be a mirror. In the device, a stored image of the user may be used to automatically identify the person. The device may further include a user interface for controlling the skin care device. The user interface may be operated using voice and/or eye movement commands. The device may further include a skin care regimen recommendation module capable of generating a displayable skin care regimen recommendation. The skin care regimen recommendation may be based at least partly on determination of a skin profile of the user and use of skin care regimen recommendations of persons with a similar profile. The device may further include a skin care regimen effectiveness module capable of generating a displayable skin care regimen effectiveness report.

In an arrangement, a system and method for moving information objects available on a website to a receptacle to communicate with a plurality of people in a controlled access community network may include enabling movement of a plurality of information objects from a predetermined website to a web based network responsive to a regimen of a person, a routine of a person, a purpose of use of an information object of the plurality of information objects and a degree of affinity of a first person towards a second person, initiating at least one customized action from the actions including a drop down movement; a drag and drop movement for populating data; and a pop-up movement in a Graphical User Interface (GUI) responsive to enabling movement of a plurality of information objects from a predetermined healthcare website, and enabling transportation of the plurality of information objects across a plurality of websites. In the system and method, the plurality of information objects may pertain to a questionnaire on at least one of a human skin condition, product information, an article, a blog posting, an image, a video, an individual message, a forum posting, and a veterinary skin condition. In the system and method, the plurality of information objects pertains to a questionnaire on human cosmetic parameters and veterinary cosmetic parameters. The questionnaire on human cosmetic parameters and veterinary cosmetic parameters may include questions on at least one of a human nail and a veterinary nail. The questionnaire on human cosmetic parameters and veterinary cosmetic parameters may include questions on at least one of a human hair and a veterinary hair. In the system and method, the purpose of use of the information object may pertain to controlling at least one of cleansing, protection, repair, moisturizing, elasticity, firmness, glow, luminosity, anti-inflammatory properties, anti-itch properties, anti-wrinkle properties, firming, exfoliating, anti-redness properties, oil controlling, anti-aging properties and shine of a human skin. In the system and method, the degree of affinity of a first person towards a second person comprises at least one of a relationship of friendship between the first person and the second person; a genetic similarity between the first person and the second person; a similarity of lifestyle between the first person and the second person; a climatic similarity between a first residential environment and a second residential environment; and a skin type similarity between the first person and the second person. In the system and method, the step of enabling transportation of the plurality of information objects across a plurality of websites may include a sub-step of dragging an item of user interest off a website of the plurality of websites in a predetermined format and transferring through an electronic signal to affiliates of a user accessing the website. The affiliates of the user may be friends and relatives of the user or associated experts. In the system and method, the step of enabling movement of a plurality of information objects from a predetermined website to a web based network may include a sub-step of enabling drop down menus on the Graphical User Interface (GUI) responsive to a plurality of end user convenience and requirement parameters. In the system and device, the plurality of people in a web based network includes a plurality of people in an online friendship network. In the system and device, the plurality of people in a web based network includes a plurality of people in an online social network.

In an arrangement, an interface including a social networking domain and at least one skin health assessment and recommendation unit for enabling users of the interface to perform a skin health assessment within the interface and to receive product and regimen recommendations from a recommendation engine based on a predetermined usage of health assessment and maintenance data may include a regimen tracker populated using a drag and drop facility, a rating unit for rating a plurality of healthcare facilities, and a product information unit for enabling a user to obtain product information by conducting a web based search of a plurality of web based drag and drop products, web based images and bar code scans. In the interface, the regimen tracker includes a diet tracking unit. In the interface, the plurality of healthcare facilities comprises at least one of skin cleansing, skin protection, skin moisture control, skin repair, skin elasticity, skin luminosity, skin firmness, skin wrinkles, pone size on skin, spots on skin , glow on skin, hair color, hair type, age and life stage further including marriage, pregnancy, dating and social life. In the interface, the product information comprises at least one of a product type, a product function, a product format, a product appropriateness level, a regimen information, product articles, product blogs, product safety, product toxicity, a product effectiveness index, a product cost information, and a product timeliness information. In the interface, the interface is a multiple language and customized interface for: web based applications; mobile phone applications; touch screen applications; and personal digital assistant applications. In the interface, the interface is seamlessly coupled with a dermal imaging device for customized web based access, control and maintenance of spectral analysis of image data acquired from a degree of polarization of reflections and re-emission of incident light from skin structures. The degree of polarization of reflections and/or re-emissions of incident light from skin structures is derived from at least one of a Red Green Blue (RGB) color analysis of a plurality of digital images; and an analysis from spectroscopic data image analysis.

In an arrangement, a system and method for determining a health state may include obtaining the answers to a series of subjective questions regarding health conditions, obtaining an objective health assessment report through a dermal imaging device, and generating a combination of answers to the series of subjective questions and the objective health assessment report to thereby generate a health state output and a real skin type output. In the system and method, a real skin type output is generated based on biophysical properties generated by at least one of a person seeking skin health monitoring, a spa, and a cosmetic advisor. In the system and method, the objective health assessment report may include an objective skin health assessment report on at least one of systemic hydration, skin hydration, skin firmness, skin wrinkles, pore size on skin, spots on skin, glow on skin, melanocyte, melanin, hemoglobin, porphyrin, triptofan, NADH, FAH, keratin, carotene, collagen, elastin, sebum, sebaceous gland activity, sweat pore, sebaceous pore, moisture level, elasticity, luminosity, firmness, fine line, wrinkle count, pore size, percent of open pores, skin elasticity, skin tension line, spots, viscosity, epidermal, dermal sebum levels, skin color, psoriasis, allergy, red area, general skin disorder, infection, tumor, sunburn, rash, scratch, pimple, acne, insect bite, itch, bleeding, injury, inflammation, photodamage, pigmentation, tone, tattoo, percent burn, burn classification, mole, aspect of a skin lesion, melanoma, dermally observed disorder, cutaneous lesion, cellulite, strias, current tan level, boil, blistering disease, congenital dermal syndrome, cutaneous mycoses, melasma, vascular condition, rosacea, spider vein, texture, skin ulcer, wound healing, post-operative tracking, melanocytic lesion, nonmelanocytic lesion, basal cell carcinoma, seborrhoic keratosis, sebum hair color, hair type, nail condition, and age and life stage further including marriage, pregnancy, dating and social life. In the system and method, the objective health assessment report is sent to an end user through at least one of email, SMS, MMS, mobile phone, a graphical user interface (GUI) of an internet connected device, and a touch screen enabled personal digital assistant. The system and method may further include obtaining health assessment and maintenance data from a physiologically polarized light data. The step of obtaining health assessment and maintenance data from a physiologically polarized light data comprises obtaining health assessment and maintenance data from a Red Green Blue (RGB) color analysis device, wherein the data comprise at least one of a white light data, a blue light data, and an ultra violet light data. The step may further comprise obtaining at least one of the white light data, the blue light data, and the ultra violet light data by reading and recording conditions of at least one of the dermis and epidermis. Obtaining health assessment and maintenance data from a physiologically polarized light data comprises obtaining data pertaining to age, geography and demography for a person subjected to health monitoring.

In an arrangement, a web-enabled health tracking method and system may include a camera comprising a photo guide unit for generating notes for each photograph captured, an interface coupled between the camera and a web-enabled computing system for uploading the photograph captured by the camera, a graphical user interface unit included in the web-enabled computing system for generating a frequently asked questionnaire unit further comprising a self answer guide module, a scoring module coupled to the frequently asked questionnaire unit, a comparison module coupled to the scoring module for comparing: a color parameter; a symmetry parameter; and a border parameter, an automation unit coupled to the graphical user interface for enabling a time-based synchronization of the frequently asked questionnaire unit, the scoring module, and the comparison module, and a learning unit coupled to the automation unit for activating: a user training module, an article module coupled to the user training module, a blogging unit coupled to the user training module and the article module, and a report unit including an email unit for emailing health related information. In the system and method, the camera comprises a tracking unit for tracking at least one of skin spots over time, laser treatment effectiveness, cellulite content in skin, current tan level, condition of veins and capillaries, botox treatment effectiveness, anti-aging treatment effectiveness, anti-acne treatment effectiveness, and a pictorial history of skin to be given to the doctor. The skin spots over time include at least one of blemishes, scars, rashes, lesions, and moles. In the system and method, the web-enabled computing system for uploading the photograph captured by the camera further includes a walkthrough module for walking through features of a skin health record of a first time user of the system, a personal skin photo album for reviewing pictorial history of a regular user of the system, and a product quality menu for tracking product expiration dates. In the system and method, the interface for uploading the photograph further includes a reminder unit for next photo for a regular user of the system; and a cosmetic status unit coupled to the reminder unit for displaying a current usage of a cosmetic for the regular user of the system. The current usage comprises a usage of at least one of a moisturizer, an antiseptic, a toner, a laser, and a botox. The system and method may further include a photo review unit for date based reviewing of at least one of a condition of a predetermined body part, a current usage status of a cosmetic, and a recommended usage list of cosmetics. In the system and method, the report unit may further include a secure transmission unit for sending a health assessment report to a medical practitioner, an affinity unit for discussing health assessment data with a friend, and a printing unit for printing health assessment data.

In an arrangement, a mobile device-based health assessment system and method may include a photograph capturing device for capturing a skin image of a mobile device user, a transmission unit coupled with the photograph capturing device for uploading the captured skin image to a network location, a global positioning device coupled to the photograph capturing device for determining a location of the photograph capturing device, and a weather estimation device coupled to the photograph capturing device to determine a weather condition at a location of the mobile device user to thereby obtain a remote diagnosis report. In the system and method, the photograph capturing device further comprises at least one of a skin photograph assessment unit, a nail photograph assessment unit, and a hair photograph assessment unit. In the system and method, the global positioning device comprises a location tracker for answering user raised questions pertaining to geographical positioning of the user. In the system and method, the location tracker includes a database pertaining to weather intensive cosmetics. The system and method may further include a phone number tracker for enabling a mobile device user to contact health assessment and cosmetic outlets.

In an arrangement, a system and method for estimation of skin type and skin features to create a unique spectral signature may include convoluting data from a first image captured in incident diffuse white light, wherein the data relate to reflected and/or re-emitted polarized or white light, convoluting data from a second image captured in incident polarized light, wherein the data relate to reflected and/or re-emitted polarized light, comparing extreme positions of at least two unique convolutions generated by convoluting data from the first image and the second image, and determining a distance between minimum and maximum intensity positions in convoluted red minus blue spectral plots from the at least two unique convolutions for generating a numerical skin type output. In the system and method, the physiological white light comprises three spectral intervals including a width less than 100 nanometer. The three spectral intervals pertain to red, green, and blue (RGB) colors. The three spectral intervals provide a natural white light sensation to a human eye. In the system and method, the step of comparing extreme positions of at least two unique convolutions comprises comparing a component (R-B)(W-P) for the reflected and/or re-emitted polarized light, and a component (R-B)W for the white light. The two unique convolutions in white light and polarized light further include a White Red component (WR), a White Blue component (WB), a reflected and/or re-emitted Polarized Blue component (PB) and a reflected and/or re-emitted Polarized Red component (PR). The two unique convolutions are based on a numerical value difference correlating to medical standards. The system and method may further include a spectral convolution scheme wherein multiple combinations of subtraction of blue spectrum from red, in white light and polarized white light are determined, wherein the spectral interval is expressed in a wavelength scale interval of 100 nanometers to 300 nanometers.

In an arrangement, a system and method for creating a unique spectral signature of skin features may include a RGB (Red Green Blue) color channel spectral plot generated from digital images including single wavelength light matter interaction thereby generating skin type characterization output, skin moisture conductivity and skin elasticity in numerical and descriptive standards. In the system and method, the RGB (Red Green Blue) color channel spectral plots generated from digital images include multi-wavelength light matter interaction.

In an arrangement, a system and method to track and store movement parameters of an imaging device moving over a subject area may include the steps of capturing an image of the subject area at a plurality of locations, identifying a direction of movement of the imaging device using an image processing technique for at least one captured frame, recognizing the direction of movement of the imaging device by comparing each frame with at least three distinct features captured to thereby triangulate a location of the imaging device, and comparing data of the captured image with a predetermined image database to store the image of the subject area and to store placement parameters of the imaging device. In the system and method, the step of capturing the image of the subject area at a plurality of locations comprises a sub step of capturing a continuous video image of the subject area. In the system and method, the step of capturing the image of the subject area at a plurality of locations comprises a sub step of capturing a frame by frame sequence of images of the subject area. In the system and method, the step of identifying a direction of movement of the imaging device using an image processing technique comprises a sub-step of a frame by frame comparison of the captured image to identify movement parameters of the imaging device. In the system and method, the step of recognizing the direction of movement of the imaging device by comparing each frame with at least three distinct features captured to triangulate a location of the imaging device comprises a sub-step of capturing a direction of movement of the imaging device by comparing three or more distinct positions across different frames.

In an arrangement, an automated location tracking and data storage method and system for an imaging device may include an image capturing unit, a positioning unit coupled to the image capturing unit for positioning the imaging device on a subject area, and an image processing unit for enabling a frame by frame comparison of the captured image and for enabling the imaging device to capture three or more distinct points to triangulate a location of the imaging device to identify a direction of movement of the imaging device. In the system and method, the image capturing unit comprises a digital camera. In the system and method, the image capturing unit comprises at least one of a mobile device and a Personal Digital Assistant (PDA). In the system and method, the image processing unit comprises a comparison unit for comparing positions of three or more distinct points across different frames to capture direction of movement of the imaging device. The system and method may further include a sub-system for measuring lateral motion of the image capturing unit from a predetermined point to a new location on the subject area.

In an arrangement, a system and method for determining a surgical excision margin may include illuminating a melanocytic lesion skin with an incident light source, detecting a characteristic of the light reflected and/or re-emitted from the melanocytic lesion, and determining a border between the melanocytic lesion and surrounding healthy tissue based on at least one characteristic of the reflected and/or re-emitted light. In the system and method, the incident light is directed at a selected angle alpha. In the system and method, varying alpha varies the depth of the measurement of the layers in the melanocytic lesion. Each depth has a specific angle which produces a full polarized reflection. In the system and method, the incident light is unpolarized light. The unpolarized light is at least one of white light, light of a single wavelength, and light of multiple single wavelengths. In the system and method, the incident light is polarized light. In the system and method, the reflected and/or re-emitted light is at least one of polarized light and unpolarized light. In the system and method, the characteristic is at least one of light source, light intensity, wavelength of light, angle of light, electrical and magnetic properties of the light, and polarization state of the light. An aspect of the polarization is at least one of an orientation, an amplitude, a phase, an angle, a shape, a degree, and an amount. In the system and method, determining is done using an algorithm. The algorithm involves at least one of artificial neural networks, fuzzy logic, fractal and multi-fractal analysis, non-linear regression, a genetic algorithm, white light analysis and RGB color analysis. The system and method may further include filtering the reflected and/or re-emitted light to obtain light of a wavelength defined by the filter output. Algorithmic analysis is performed on the filtered image. In the system and method, determining involves creating an image of the difference between reflected diffusion light and reflected polarized light. In the system and method, determining involves comparing the aspect of the polarization of the reflected and/or re-emitted light to a calibration signal. In the system and method, determining further comprises considering at least one of user input and a visual analysis.

According to certain embodiments of the invention, there is disclosed a system comprising an antenna subsystem comprising a transmission unit for transmitting electromagnetic signals and a reception unit for capturing images of materials illuminated with polarized and unpolarized electromagnetic signals, a first signal processing subsystem for processing the captured images thereby facilitating generation of unique identifiers for the materials, a second signal processing subsystem for processing the electromagnetic signals and a combiner (heterodyne) for combining the processed electromagnetic signals and processed images to generate enhanced signals.

According to certain embodiments of the invention, there is disclosed a system comprising an antenna subsystem for transmitting electromagnetic signals and capturing images of materials illuminated with polarized and unpolarized electromagnetic signals, a first signal processing subsystem for processing the captured images and generating unique identifiers for the materials, a second signal processing subsystem for processing the electromagnetic signals transmitted from the antenna subsystem and a combiner subsystem for combining the processed electromagnetic signals and images to generate enhanced signals.

According to certain embodiments of the invention, there is disclosed a system for managing physiological state based on one or more physiological parameters comprising a host subsystem comprising an illumination module for directing polarized and unpolarized light (or electromagnetic signals) to physiological organs, a sensor module for capturing the polarized and unpolarized electromagnetic signals reflected from the physiological organs and a hydration management module for monitoring the hydration levels of the physiological organs and a remote computing subsystem for at least receiving the hydration levels, processing the hydration levels, displaying the hydration levels, transmitting the hydration levels back to the host computing subsystem, and any combination thereof.

According to certain embodiments of the invention, there is disclosed a system comprising an illumination subsystem for directing polarized and unpolarized electromagnetic signals to physiological organs, a sensor subsystem for capturing the polarized and unpolarized electromagnetic signals absorbed, reflected and re-emitted from the physiological organs and a host computing subsystem for facilitating determination of physiological conditions of the physiological organs based on the detection of at least one of a plurality of unique dermascopic structures using the captured electromagnetic signals.

In one arrangement a mirror or a reflecting surface is provided to facilitate a consumer to get personalized skin care assistance.

In another arrangement the system may be accessible from multiple locations and used for desired purpose.

In another arrangement of the system products are recommended for the consumer based on the properties of image like wrinkles, elasticity, luminosity etc.

In another arrangement of the system, procedures are recommended for the consumer based on the properties of the image like wrinkles, elasticity, luminosity etc and combined with historical trends and biographical information, and diet.

In another arrangement the system provides an automated technique for assessment of the skin.

In another arrangement the system may have a portable skin surface imaging attachment, to get more detailed skin assessment, or just use the portable skin imaging device as the source of the imaging.

In yet another arrangement the system is connected to a computer and a database to get accurate recommendations based on the assessment of the skin.

In yet another arrangement the system may automatically adjust lighting intensity and wavelengths and angles in order to assess various factors of the skin.

In yet another arrangement the system may have an interactive viewer on the surface so that a live consultation with an expert professional may be done real-time or remotely, such as teleconsultation.

In yet another arrangement the system allows for a user to managing all information through touch screen or gestural commands.

In certain arrangements there are disclosed methods, apparatuses and systems for analysis of water using OMF.

In accordance with certain arrangements, a system for distinguishing between a healthy biological skin tissue and an unhealthy biological skin tissue for enabling an excision proximate to the healthy biological skin tissue is disclosed.

The system for distinguishing between a healthy biological skin tissue and an unhealthy biological skin tissue (may also be referred to as unhealthy skin tissue) for enabling an excision proximate to the healthy biological skin tissue (may also be referred to as healthy skin tissue) comprises an imaging capturing device for identifying a healthy biological skin tissue, a diseased biological skin tissue, and tracking growth of the unhealthy biological skin tissue. The image capturing device may be real time digital camera device.

According to an arrangement, the system for distinguishing between a healthy biological skin tissue and an unhealthy biological skin tissue for enabling an excision proximate to the healthy biological tissue wherein the biological skin tissue may be a human skin tissue, a veterinary skin tissue, an agricultural product skin tissue and the like.

According to an arrangement, the system for distinguishing between a healthy biological skin tissue and an unhealthy biological skin tissue for enabling an excision proximate to the healthy biological tissue comprises an optical analysis device coupled to the image capturing device and the surgical intervention unit.

According to an arrangement, the system for distinguishing between a healthy biological skin tissue and an unhealthy biological skin tissue for enabling an excision proximate to the healthy biological skin tissue comprises an optical analysis device coupled to the image capturing device and the surgical intervention unit. The optical analysis device coupled to the image capturing device and the surgical intervention unit comprises a Red Green Blue (RGB) unit further comprising a sampler coupled to a pixel by pixel by analyzer of skin images for generating a sample of most frequent of a standard R G B (sRGB) color component.

According to an arrangement, the system for distinguishing between a healthy skin tissue and an unhealthy skin tissue for enabling an excision proximate to the healthy biological skin tissue comprises a Gaussian probabilistic distributer for modeling the standard sRGB component color distribution with estimated parameters on the generated sRGB color sample for the captured image.

According to an arrangement, the system for distinguishing between a healthy skin tissue and an unhealthy skin tissue for enabling an excision proximate to the healthy skin tissue comprises a phototype generator coupled to the Gaussian probabilistic distributer for generating a phototype of the healthy and unhealthy skin tissue through a decision tree unit.

According to an arrangement, the system for distinguishing between a healthy biological skin tissue and an unhealthy biological skin tissue for enabling an excision proximate to the healthy biological tissue comprises a white light unit further comprising a comparison unit for comparing extreme positions of at least two unique convolutions in white light and in polarized light responsive to convoluting data of a first skin image and a second skin image and an output unit for determining a distance between minimum and maximum intensity positions in convoluted red minus blue wavelength scale in the at least two unique convolutions for generating a numerical skin type output.

According to an arrangement, the system for distinguishing between a healthy biological skin tissue and an unhealthy biological skin tissue for enabling an excision proximate to the healthy biological skin tissue wherein the optical analysis device further comprises a skin biophysical analysis unit further including at least one of the following parameters a skin fairness parameter, a skin darkness parameter, systemic hydration, skin hydration, skin firmness, skin wrinkles, pore size on skin, spots on skin, glow on skin, melanocyte, melanin, hemoglobin, porphyrin, keratin, carotene, collagen, elastin, sebum, sebaceous gland activity, sweat pore, sebaceous pore, moisture level, elasticity, luminosity, firmness, fine line, wrinkle count, pore size, percent of open pores, skin elasticity, skin tension line, spots, viscosity, epidermal, dermal sebum levels, skin color, psoriasis, allergy, red area, general skin disorder, infection, tumor, sunburn, rash, scratch, pimple, acne, insect bite, itch, bleeding, injury, inflammation, photodamage, pigmentation, tone, tattoo, percent burn, burn classification, mole, aspect of a skin lesion, melanoma, dermally observed disorder, cutaneous lesion, cellulite, boil, blistering disease, congenital dermal syndrome, cutaneous mycoses, melasma, vascular condition, rosacea, spider vein, texture, skin ulcer, wound healing, post-operative tracking, melanocytic lesion, nonmelanocytic lesion, basal cell carcinoma and seborrhoic keratosis.

According to an arrangement, the system for distinguishing between a healthy biological skin tissue and an unhealthy biological skin tissue for enabling an excision proximate to the healthy biological skin tissue further comprises a converter for converting colors obtained in a device dependent RGB color system into a device independent standard RGB color system (sRGB) used for biological skin image analysis.

According to an arrangement, the system for distinguishing between a healthy biological skin tissue and an unhealthy biological skin tissue for enabling an excision proximate to the healthy biological skin tissue wherein the optical analysis device further comprising a diffused reflectance unit for generating a predetermined set of wavelengths for reflection intensity measurement of the spectral data, utilizing a plurality of reflection intensity values and a plurality of reflection intensity ratio values of the spectral data for classification of a skin type responsive to generating a predetermined set of wavelengths, normalizing the reflection intensity values of spectral data with respect to spectral source and spectral classification of the skin type, generating a skin photo type output by applying nonparametric regression analysis on measured spectral data responsive to normalizing the reflection intensity values of spectral data.

According to an arrangement, the system for distinguishing between a healthy biological skin tissue and an unhealthy biological skin tissue for enabling an excision proximate to the healthy biological skin tissue comprises a suspect skin tissue image generator coupled to the analysis device for imaging a site on a biological skin area, determining a border area on the site and determining the suspect skin tissue. The suspect skin tissue image generator comprises an image of an area to be exercised. The suspected skin tissue comprises a dead biological skin tissue and an unhealthy biological skin tissue. Further, the image of an area to be excised comprises a visible suspect skin tissue, a normal visible skin tissue surrounding the visible suspect tissue for excision, a border between the visible suspect tissue and the normal visible skin tissue, a healthy skin tissue surrounding both the visible suspect skin tissue and the normal visible skin tissue and an outlined area for a surgeon to cut a predetermined skin tissue portion including the visible suspect skin tissue, the normal visible skin tissue, the border and the healthy skin tissue. The visible suspect skin tissue is a coetaneous melanoma affected skin tissue.

According to an arrangement, the system for distinguishing between a healthy skin tissue and an unhealthy skin tissue for enabling an excision proximate to the healthy skin tissue further comprises a surgical intervention unit coupled to the image capturing device for enabling an automatic cutting of the unhealthy skin tissue. The surgical intervention unit comprises an ink marking unit for drawing borders between the healthy and diseased tissue to determine a surgical incision space. The surgical intervention unit tracks a growth of a healthy and an unhealthy area of the biological skin tissue for determining whether the tracked biological skin tissue is at least one of a stable tissue and a tissue to be excised.

In accordance with certain arrangements, a system and a method are disclosed for determining a predisposition of sebaceous pores and skin structures around a sebaceous gland, a level of acne, and a predisposition of a portion of skin to improve and worsen the acne. In accordance with an exemplary embodiment of the present invention, according to a first arrangement, a system for determining a predisposition of sebaceous pores and skin structures around a sebaceous gland, a level of acne, and a predisposition of a portion of skin to improve and worsen the acne may include an illuminator for illuminating a portion of a surface on the skin. The illuminator for illuminating a portion of a surface on the skin may include a white light source, a blue light source, an ultraviolet light source and the like.

According to some arrangements, the system for determining a predisposition of sebaceous pores and skin structures around a sebaceous gland, a level of acne, and a predisposition of a portion of skin to improve and worsen the acne may include an image capturing device coupled to the illuminator for imaging the portion of the surface on the skin. The image capturing device coupled to the illuminator for imaging the portion of the surface on the skin may include a digital imaging device.

According to some other arrangements, the system for determining a predisposition of sebaceous pores and skin structures around a sebaceous gland, a level of acne, and a predisposition of a portion of skin to improve and worsen the acne includes an image processor coupled to the image capturing device for processing the imaged portion of the surface on the skin and isolating sebaceous parameters pertaining to acne formation. The image processor may include a plurality of characteristic acne elements elimination unit for isolating sebaceous pore openings, sebaceous pore channel, sebaceous pore intersection, sebaceous gland intersection, blockage of sebaceous pore openings, contents of the sebaceous pore, determining age of sebum, whether the sebaceous gland is actively producing sebum, and a level of p-acne bacteria and isolating unhealthiness arising out of age of the sebaceous gland, inflammation around the gland, inflammation around the sebaceous pores, inflammation around the sebaceous gland, inflammation around hair follicles and level of p-acne bacteria.

In accordance with an exemplary embodiment of the present invention, the plurality of characteristic acne elements may be measured on at least one of discrete scale and a continuous scale. The continuous scale may include a plurality of acne improvement and worsening conditions further includes a predetermined number of acne status outcomes. The discrete scale may comprise at least one of following acne conditions of an acne condition unit closed, partially open and open for sebaceous pore opening; full, partially full and empty for sebaceous pore contents; blocked, partially blocked and clear for gland and hair connection; full, partially full and empty for sebaceous gland contents; active, partially active and inactive for sebaceous gland activity and high, medium, low and none for inflammation. According to an example of the present invention, the continuous scale may comprise at least one of the following acne conditions of an acne condition unit closed, partially open and open for sebaceous pore opening; full, partially full and empty for sebaceous pore contents; blocked, partially blocked and clear for gland and hair connection; full, partially full and empty for sebaceous gland contents; active, partially active and inactive for sebaceous gland activity and high, medium, low and none for inflammation. The acne condition unit may include a questionnaire unit for generating an acne status questionnaire.

According to an aspect, the system for determining a predisposition of sebaceous pores and skin structures around a sebaceous gland, a level of acne, and a predisposition of a portion of skin to improve and worsen the acne may include an optical assessment unit coupled to both the image capturing device and the image processor. In accordance with an exemplary embodiment of the present invention, the optical assessment unit may further include a color Red Green Blue (RGB) analysis device including a standard RGB (sRGB) color unit. According to an example of the present invention, the optical assessment unit may include a spectroscopic analysis unit.

According to an aspect, the system for determining a predisposition of sebaceous pores and skin structures around a sebaceous gland, a level of acne, and a predisposition of a portion of skin to improve and worsen the acne wherein the optical assessment unit is coupled to both the image capturing device and the image processor, the optical assessment unit may further include white light polarization device coupled to the RGB analysis device for comparing extreme positions of at least two unique convolutions in white light and in polarized light responsive to a convoluting data of a first captured image and a second captured image. The white light polarization device may further include an output generator determining a distance between minimum and maximum intensity positions in a convoluted red minus blue wavelength scale in the at least two unique convolutions to generate a numerical skin type output.

According to another aspect, the system for determining a predisposition of sebaceous pores and skin structures around a sebaceous gland, a level of acne, and a predisposition of a portion of skin to improve and worsen the acne may include a marking unit for outlining and marking areas on the surface on the skin to thereby enable surgical excision of the skin structure.

Yet, in accordance with some other arrangements, a method for determining a predisposition of sebaceous pores and skin structures around a sebaceous gland, a level of acne, and a predisposition of a portion of skin to improve and worsen the acne is disclosed. The method for determining a predisposition of sebaceous pores and skin structures around a sebaceous gland, a level of acne, and a predisposition of a portion of skin to improve and worsen the acne may include analyzing at least one of an individual level of pores, glands, inflammation, sebum, blockage and age of sebum over a surface area, an aggregate level of pores, glands, inflammation, sebum, blockage and age of sebum over a surface area and a correlation level of pores, glands, inflammation, sebum, blockage and age of sebum over a surface area. The correlation level may include at least one of a fuzzy logic, a non-linear regression, a genetic algorithm, and a neural network.

In accordance with some arrangements, a method for determining skin photo type by non-parametric classification of diffuse reflectance spectral data is disclosed. According to an aspect of the present invention a method for determining skin photo type by non-parametric classification of diffuse reflectance spectral data includes a step of generating a predetermined set of wave lengths for reflection intensity measurement of the spectral data. The step of generating a predetermined set of wave lengths for reflection intensity measurement of the spectral data comprises a sub step of generating a predetermined set of wavelengths for a plurality of incident spectral rays. The skin photo type may be of a human skin, a veterinary skin, an agricultural body and the like.

According to some other arrangements the method for determining skin photo type by non-parametric classification of diffuse reflectance spectral data is machine autonomous and is applicable to any diffused reflectance measurement system operating in the Ultra-Violet Spectroscopy spectral range. The non parametric classification of diffuse reflectance spectral data is free from potential errors due to human interpretation.

According to yet another arrangement, the method for determining skin photo type by non-parametric classification of diffuse reflectance spectral data includes a step of utilizing a plurality of reflection intensity values and plurality of reflection intensity ratio values of the spectral data for classification of skin type responsive to generate a predetermined set of wavelengths. The step of utilizing a plurality of reflection intensity values and plurality of reflection intensity ratio values of the spectral data for classification of skin type responsive to generate a predetermined set of wavelengths comprises a sub step of utilizing a plurality of differential reflection intensity values.

According to an arrangement the method for determining skin photo type by non-parametric classification of diffuse reflectance spectral data includes a step of normalizing the reflection intensity values of spectral data with respect to spectral source and spectral classification of skin source. The step of normalizing the reflection intensity values of spectral data with respect to spectral source and spectral classification of skin source comprises a sub step of making spectral data independent of making spectral data independent of measurement instrument.

According to another arrangemnt, the method for determining skin photo type by non-parametric classification of diffuse reflectance spectral data includes a step of generating a skin photo type output by applying non parametric regression analysis on measured spectral data responsive to normalizing the reflection intensity values of spectral data. The step of generating a skin photo type output by applying non parametric regression analysis on measured spectral data responsive to normalizing the reflection intensity values of spectral data comprises a sub step of using a plurality of intensity of reflection values, a plurality of differential reflection intensity values and a plurality of ratios of reflection intensity values for deriving a skin photo type from regression tree previously generated by applying nonparametric regression analysis on measured spectral data. According to an example of the present invention, intensities of reflection, difference in reflection intensities and ratios of reflection intensity values are used to derive skin photo type from regression tree previously generated by nonparametric regression analysis on the training data group collected from 120 human volunteers.

These and other systems, methods, objects, features, and advantages of the present invention will be apparent to those skilled in the art from the following detailed description of the preferred embodiment and the drawings..

## BRIEF DESCRIPTION OF THE FIGURES

The invention and the following detailed description of certain embodiments thereof may be understood by reference to the following figures:

Fig. 1 depicts a skin care system for skin health analysis and monitoring, and skin care assessment and recommendation.

Fig. 2 depicts a mechanism for light polarization by a skin structure.

Fig. 3 depicts a process for skin care examination.

Fig. 4A & B depict a front and back view of a dermal imaging device.

Fig. 5 depicts a skin health monitoring page of a skin care system.

Fig. 6 depicts an interactive modeling tool of a skin care system.

Fig. 7 depicts a recommendations page of a skin care system.

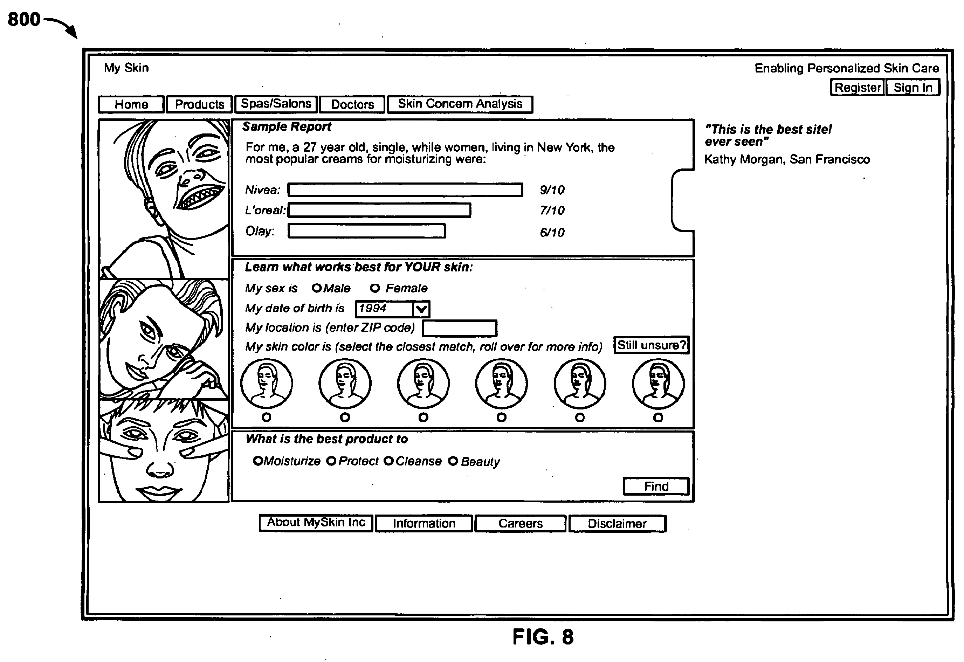

Fig. 8 depicts a user interface of a skin care system.

Fig. 9 depicts a welcome page of a skin care system.

Fig. 10 depicts a questionnaire page of a skin care system.

Fig. 11 depicts a skin image capture page of a skin care system.

Fig. 12 depicts a results page with bar graphs of a skin care system.

Fig. 13 depicts a results page with line graphs of a skin care system.

Fig. 14 depicts a summary screen of a skin care system.

Fig. 15 depicts an elasticity summary screen of a skin care system.

Fig. 16 depicts a summary screen of a skin care system.

Fig. 17 depicts an elasticity summary screen of a skin care system.

Fig. 18 depicts a map of a user interface for a skin care system.

Fig. 19 depicts a review page of a skin care system.

Fig. 20 depicts a review page of a skin care system.

Fig. 21 depicts a My Experience page of a skin care system.

Fig. 22 depicts a What Works page of a skin care system.

Fig. 23 depicts an Info For Me page of a skin care system.

Fig. 24 depicts an example of a skin care shelf portion of a user interface of a skin care system.

Fig. 25 depicts an example of a skin care shelf portion of a user interface of a skin care system.

Fig. 26 depicts a user interface of a skin care system.

Fig. 27 depicts a registration page of a skin care system.

Fig. 28 depicts a recommendation page of a skin care system.

Fig. 29 depicts a mobile content map for a mobile user interface of a skin care system.

Fig. 30 depicts a How Good Is This Product message flow.

Fig. 31 depicts a What Should I Look For? message flow

Fig. 32 depicts a Suncheck message flow.

Fig. 33 depicts an Alert message flow.

Fig. 34 depicts an Options message flow.

Fig. 35 depicts an algorithm and method for analyzing materials.

Fig. 36 depicts the reflection and capture of white light and reflected polarized light from a specimen based on varying angles.

Figs. 37A& B depict color coordinate systems that can be used in digital image processing.

Fig. 38 depicts a histogram of color density.

Fig. 39 depicts a normalized color channel histogram correlated to wavelength scale.

Figs. 40A and 40B depict overlaid, normalized color channel histograms.

Figs. 41A and 41B depict convolutions of individual color channel histograms.

Fig. 42 depicts the combination of the two convolutions of the two color channel histograms.

Fig. 43 depicts a mathematical modeling of a portion of Maxwell's color triangle.

Figs. 44A & B depict the resulting spectral signatures for light and dark skin.

Figs. 45A - C depict the resulting spectral signatures for pure and alloy metals.

Figs. 46A & B depict the resulting spectral signatures for different types of water.

Fig. 47 depicts a block diagram of a skin care device embodiment.

Fig. 48 depicts a wand-shaped skin care device embodiment.

Fig. 49 depicts a vertical display panel including skin care device.

Fig. 50 depicts an embodiment of a wearable skin care device.

Fig. 51 depicts positive and negative intensities on a waveform as a function of emission and absorption of specific wavelengths within skin tissue.

Fig. 52 depicts the comparison between spectral signatures of healthy skin and malignant skin around a reference wavelength.

Fig. 53 depicts malignant pigmented skin in white and physiologically polarized white light.

Fig. 54 depicts the comparison of convolutions between healthy, benign and malignant skin lesions.

Fig. 55 depicts a system for tracking and targeting an image.

Fig. 56 depicts a system for determining an excision margin.

Fig. 57 depicts a system for determining an excision margin.

Fig. 58 is a flowchart illustrating a process for RGB color analysis.

Fig. 59 is a diagram depicting a pixel view of an acquired digital image of a sample of person's skin.

Fig. 60 is a diagram depicting a pixel view of the acquired digital image of a sample of person's skin after quantization.

Fig. 61 is a diagram depicting a Histogram / Distribution of standard R, G and B colors on one of the taken photographs of a patient whose skin phototype is classified as type III by Fitzpatrick, and their Gaussian normal approximation / hull.

Fig. 62 is a diagram depicting a Histogram / Distribution of standard R, G and B colors on one of the patient's photographs whose skin phototype is classified as type VI by Fitzpatrick, and their Gaussian normal approximation / hull.

Fig. 63 is a flowchart illustrating an algorithm for determining the skin phototype according to the estimated values of mathematical expectation for R and B colors in a standard RGB color system.

Fig. 64 depicts an embodiment of a friend toolbar.

Fig. 65 depicts the auto-scroll feature of the friend toolbar.

Fig. 66 depicts the drag-and-drop share functionality of the friend toolbar.

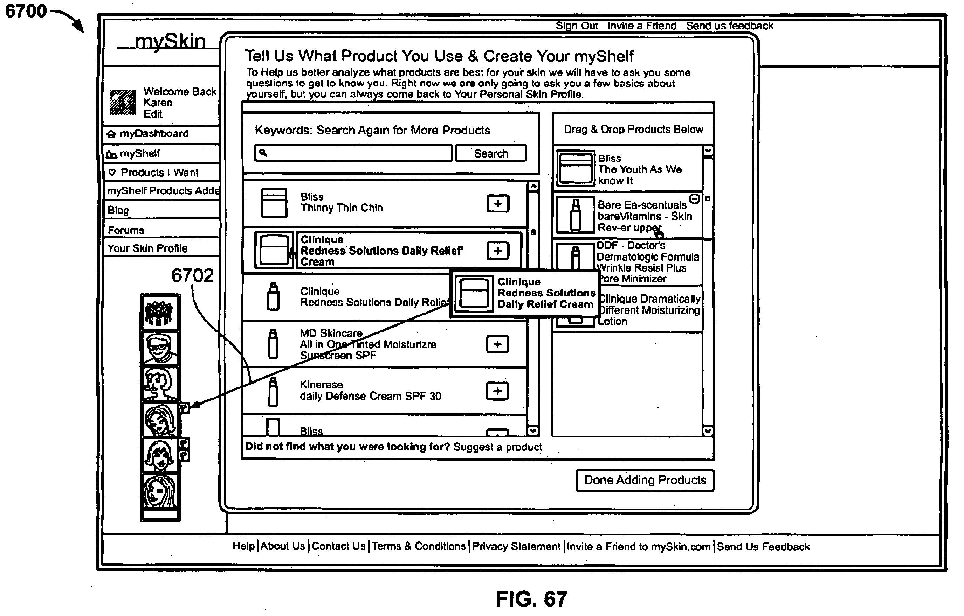

Fig. 67 depicts the drag-and-drop share functionality of the friend toolbar.

Fig. 68 depicts sharing skin data as a data object with friends.

Fig. 69 depicts posting skin care data as a data object on a blog or forum where users may discuss the data.

Fig. 70 depicts sharing skin data as a data object where the data object becomes part of the content that a user may wish to discuss.

FIG. 71 is a schematic view of a system for automated diagnosis of skin disorders by image processing detection of skin lesions or dermascopic structures, designed and implemented in accordance with at least some embodiments of the invention; and

FIG. 72 is an exploded diagrammatic representation of the host computing subsystem, of Fig. 1, comprising the skin disorder management module designed and implemented in accordance with at least some embodiments of the invention.

FIG. 73 is a block diagrammatic view of a system facilitating implementation of an Opto-Magnetic process based on light-matter interaction using digital imaging for detection of EPV and CMV viruses in blood plasma samples, designed and implemented in accordance with certain embodiments of the invention;

FIG. 74 is an exploded diagrammatic representation of the host computing subsystem, of the Fig. 1, comprising the Opto-Magnetic Fingerprint (or OMF) Generator module designed and implemented in accordance with at least some embodiments of the invention;

FIG. 75 depicts a flow diagram delineating at least one process implemented by the system configuration of FIGS. 1 and 2 thereby facilitating estimation of blood plasma type and properties (or characteristics) thereof and creation of a unique spectral signature;

FIGS. 76A and 76B depict a dual pair of typical digital images of samples, tested positive and negative for EBV and CMV, captured with diffuse white light (W) and reflected polarized light (P), in that order;

FIGS. 77A and 77B depict a first pair of plots of typical spectral data obtained on implementation of the OMF method for processing digital images of unique samples from a first set of two patients subjected to a first test case for confirmation of EBV, namely "Case I: EBV-IgM", designed and implemented in accordance with certain embodiments of the invention;

FIGS. 78A and 78B depict a second pair of plots of typical spectral data obtained on implementation of the OMF method for processing digital images of unique samples from a second set of two different patients subjected to a second test case for confirmation of EBV, namely "Case II: EBV-IgM", designed and implemented in accordance with certain embodiments of the invention;

FIGS. 79A and 79B depict a third pair of plots of typical spectral data obtained on implementation of the OMF method for processing digital images of unique samples from a third set of two different patients subjected to a third test case for confirmation of EBV, namely "Case III: EBV-IgG", designed and implemented in accordance with certain embodiments of the invention;

FIGS. 80A and 80B depict a fourth pair of plots of typical spectral data obtained on implementation of the OMF method for processing digital images of unique samples from a fourth set of two different patients subjected to a fourth test case for confirmation of EBV, namely "Case IV: EBV-IgG", designed and implemented in accordance with certain embodiments of the invention;

FIG. 81 is a block diagrammatic view of a system facilitating implementation of an Opto-Magnetic process based on light-matter interaction using digital imaging for Papanicolau Test Analysis of samples, designed and implemented in accordance with certain embodiments of the invention;

FIG. 82 is an exploded diagrammatic representation of the host computing subsystem, of Fig. 81, comprising the Opto-Magnetic Fingerprint (or OMF) Generator module designed and implemented in accordance with at least some embodiments;

FIG. 83 depicts a flow diagram delineating at least one process implemented by the system configuration of FIGS. 81 and 82 thereby facilitating estimation of Pap test sample type and properties (or characteristics) thereof and creation of a unique spectral signature;

FIGS. 84A-B, 85A-B and 86A-B depict a triple pair of typical digital images of samples (or Pap smear slides), categorized as Group I (or normal tissue state), captured with diffuse white light (W) and reflected polarized light (P), in that order;

FIG. 84C depicts a plot of typical spectral data (or OMF diagram) obtained on implementation of the OMF method on digital images of FIGS. 84A-B of the given, selected first sample (or Pap smear slide) categorized as Group I (or normal tissue state), in accordance with certain embodiments of the invention;

FIG. 85C depicts a plot of typical spectral data (or OMF diagram) obtained on implementation of the OMF method on digital images of FIGS. 85A-B of the given, selected second sample (or Pap smear slide) categorized as Group I (or normal tissue state), in accordance with certain embodiments of the invention;

FIG. 86C depicts a plot of typical spectral data (or OMF diagram) obtained on implementation of the OMF method on digital images of FIGS. 86A-B of the given, selected third sample (or Pap smear slide) categorized as Group I (or normal tissue state), in accordance with certain embodiments of the invention;

FIG. 87 depicts a plot of typical spectral data (or OMF diagram) obtained on implementation of the OMF method on digital images of a given, selected sample (or Pap smear slide) categorized as Group II (or non-typical inflammation), in accordance with certain embodiments of the invention;

FIG. 88 depicts a plot of typical spectral data (or OMF diagram) obtained on implementation of the OMF method on digital images of a given, selected sample (or Pap smear slide) categorized as Group III (dysplasia), in accordance with certain embodiments of the invention;

FIG. 89 depicts a plot of typical spectral data (or OMF diagram) obtained on implementation of the OMF method on digital images of a given, selected sample (or Pap smear slide) categorized as Group IV (carcinoma in situ), in accordance with certain embodiments of the invention;

FIG. 90 depicts a plot of typical spectral data (or OMF diagram) obtained on implementation of the OMF method on digital images of a given, selected sample (or Pap smear slide) categorized as Group V (suspicion to carcinoma), in accordance with certain embodiments of the invention;

FIG. 91 depicts a system for generating enhanced heterogeneous signals for use in non-invasive processing of materials utilizing an Opto-Magnetic Antenna (or OMA), designed and implemented in accordance with certain embodiments of the invention;

FIG. 92 is block diagrammatic view of at least one workable configuration for use in tandem with the system of FIG. 91;

FIG. 93 depicts a flow diagram delineating at least one process implemented by the system configuration of FIG. 92 thereby facilitating multi sensor high frequency imaging;

FIG. 94 is a schematic view of a wearable computing system for monitoring of one or more physiological parameters designed and implemented in accordance with at least some embodiments of the invention;

FIG. 95 is an exploded diagrammatic representation of the host computing subsystem, of Fig. 94, comprising the skin hydration management module designed and implemented in accordance with at least some embodiments of the invention;

FIG. 96 is a perspective view of the WHM of FIG. 94 designed and implemented as a handheld monitor for measurement of hydration status, in accordance with some other embodiments of the invention;

FIG. 97 is a diagram depicting an image of area to be excised;

FIG. 98 is a diagram depicting the process employed for automatically determining the area to be excised;

FIG. 99 is a diagram depicting a system for distinguishing between a healthy skin biological tissue and an unhealthy biological skin tissue for enabling an excision proximate to the healthy biological tissue;

FIG. 100 is a schematic diagram depicting a system for determining a predisposition of sebaceous pores and skin structures;

FIG. 102 is a diagram depicting reflectance of spectral rays (diffusely reflected spectral rays) in all directions from the surface of the skin.

Fig. 103 depicts Opto-magnetic diagrams for 18.2 MΩ water at -4.4 °C

Fig. 104 depicts Opto-magnetic diagrams for 18.2 MΩ water at 25 °C

DETAILED DESCRIPTION

Provided herein may be methods, systems, and a device for dermal and non-dermal imaging. Throughout this disclosure the phrase "such as" means "such as and without limitation". Throughout this disclosure the phrase "for example" means "for example and without limitation". Throughout this disclosure the phrase "in an example" means "in an example and without limitation". Throughout this disclosure, the term "product" refers to any medical, non-medical, cosmetic, skin, hair, or nail care product. Generally, any and all examples may be provided for the purpose of illustration and not limitation.

Real-time analysis of digitally captured skin-related and other information may facilitate real-time skin condition assessment, real-time skin regimen recommendation, and real-time evaluation of the effectiveness of a selected skin regimen. Real-time analysis of digitally captured data may be performed by using a skin care device embodying the principles of the invention disclosed herein. A skin care device embodying the principles of the invention may include, for example, an electromagnetic radiation source capable of directing incident electromagnetic radiation, a radiation detector for measuring various parameters of the re-emitted radiation, and a skin condition analysis module capable of generating a skin condition assessment in real-time.

The skin condition assessment may be cosmetic and/or medical in nature. By way of example, and in no way limiting the scope of the invention, the skin condition assessment may include any one of an acne condition assessment, a pore condition assessment, a wrinkle condition assessment, a skin elasticity assessment, a skin oiliness assessment, a skin moisture assessment, a skin luminosity assessment, a skin sebum assessment, a skin redness assessment, a skin inflammation assessment, a skin texture assessment, a skin color assessment or any combination of the listed assessments. For example, the pore condition assessment can help in determining whether the pores are clean, open and of optimal health.

Skin-condition data may be acquired, for example, by directing incident electromagnetic radiation to a location, such as a pin-point location, on the skin of a person and detecting the re-emitted radiation from the location by using a radiation detector. The effectiveness of generating high-quality, real-time skin condition assessments may be enhanced in some embodiments by using a skin condition analysis module that bases its analysis at least partly on diffused reflectance spectroscopy. The quality of real-time skin condition assessments may be further enhanced in other embodiments by using white light as the incident radiation and by detecting the red-green-blue components of the re-emitted light.

The term "digital image" refers to a representation of a two-dimensional image using ones and zeros (or binary digits or bits). The digital image may be of vector or raster type depending on whether or not the image resolution is fixed. However, without qualifications the term "digital image" usually refers to raster images.

The term "image processing", as used herein, refers to any form of signal processing for which the input is an image, such as photographs or frames of video. The output of image processing can be either an image or a set of characteristics or parameters related to the image. Most image-processing techniques involve treating the image as a two-dimensional signal and applying standard signal-processing techniques to it.

Image processing usually refers to digital image processing, but optical and analog image processing are also possible. The acquisition of images, i.e. producing the input image in the first place, is referred to as imaging.

The term "digital image processing", as used herein, refers to the use of computer algorithms to perform image processing on digital images. As a subfield of digital signal processing, digital image processing has many advantages over analog image processing. For example, digital image processing allows a much wider range of algorithms to be applied to the input data and can avoid problems, such as the build-up of noise and signal distortion during processing.

Likewise, the term "digital imaging or digital image acquisition" refers to creation of digital images, typically from a physical object. The term is often assumed to imply or include the processing, compression, storage, printing and display of such images.

Medical imaging refers to the techniques and processes used to create images of the human body (or parts thereof) for clinical purposes (medical procedures seeking to reveal, diagnose or examine disease) or medical science (including the study of normal anatomy and physiology).

As a discipline and in its widest sense, it is part of biological imaging and incorporates radiology (in the wider sense), radiological sciences, endoscopy, (medical) thermography, medical photography and microscopy (e.g. for human pathological investigations).

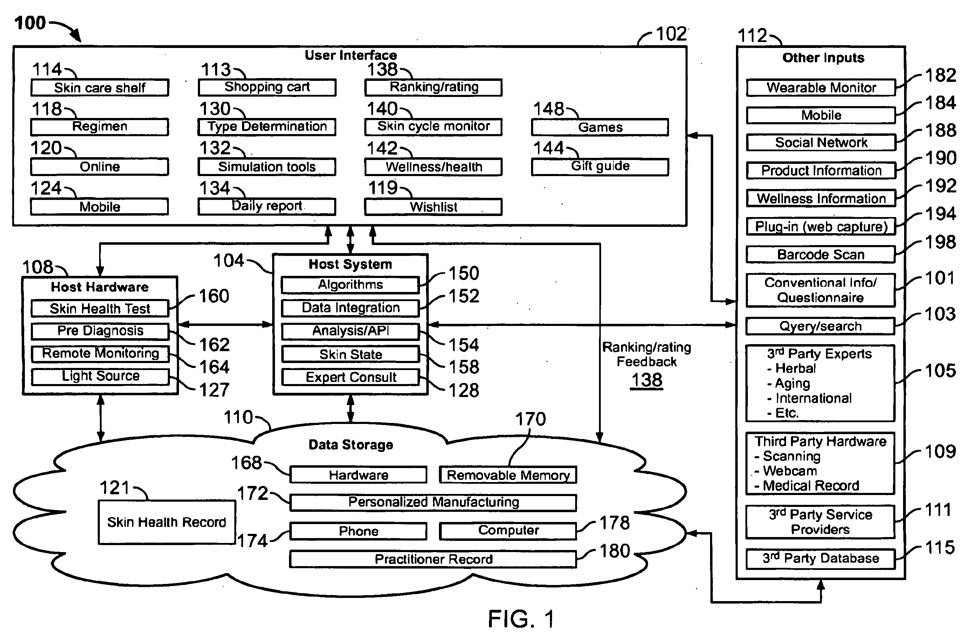

Referring to Fig. 1, a system for skin health analysis, monitoring, and recommendation may comprise host hardware 108, such as an imaging device 108, for capturing biophysical skin properties such as in a skin health test 160, performing pre-diagnosis 162, and performing remote monitoring 164 using a light source 127; a user interface 102 interfacing with the host hardware 108, an online platform 120, or a mobile platform 124 for capturing demographic information, additional anecdotal information on skin health, current skin care regimen 118, rankings and ratings 138 of current skin care products and regimen, populating a skin care shelf 114, and accessing a skin cycle monitor 140, health and/or wellness information 142, games 148, a gift guide 144, a wishlist 119, a Daily Report 134, simulation tools 132, a type determination engine 130, a shopping cart 113, and the like; a host system 104 for processing and analyzing captured information such as by employing an algorithm 150, obtaining an expert consultation 128, data integration 152, and analysis tools/API's 154 to define a skin state 158; other inputs 112 to the host system 104, which may be subject to ranking/ rating feedback 138, for providing additional granularity in identifying, monitoring, and adjusting a skin state 158, such as a wearable monitor 182, a mobile communications device 184, a social network 188, product information 190, wellness information 192, a plug-in (web capture) 194, a barcode scan 198, conventional information/ questionnaire answers 101, a query/search 103, third part experts 105, third party hardware 109, third part service providers 111, and the like; and data storage 110 for storing data from the host hardware 108, host system 104, user interface 102, and other inputs 112, such as hardware 168, removable memory 170, a wireless communication device 174, a computer 178, a practitioner record 180 such as a dermatologist, general physician, aesthetician, spa employee, salon employee, cosmetic salesperson, and the like, a personalized manufacturing record 172, and the like. While dermal embodiments are contemplated throughout this disclosure, except where context prohibits such embodiments should be understood to encompass non-dermal embodiments, such as and without limitation any hair, nail, agricultural, veterinary, internal, biological and non-biological embodiments.

An imaging device 108 may be used to capture images of skin structures to obtain biophysical skin properties such as in a skin health test 160, a pre-diagnosis 162, remote monitoring 164, and the like. The imaging device 108 may also be adapted to capture images of non-dermal structures, such as hair, nails, teeth, eyes, internal organs and structures, and the like. The imaging device 108 may use an internal or external light source 127 to provide a specific sequence of irradiation using unpolarized light, such as diffusion light, white light, monochromatic light, light of multiple single wavelengths, and the like, then polarized light in order to obtain data on skin structures. In embodiments, the incident light may be polarized or unpolarized and the reflected or re-emitted light may be polarized or unpolarized. The polarized light may result from the reflection on the skin and is not polarized from the light source. The capture and storage of the reflections enables the imaging and analysis of skin lesions, as well as all types of skin diseases, skin problems, and cosmetic concerns and indications. Analysis of polarized reflections may enable obtaining thermal, electrical, and magnetic properties of the imaged skin area. The images may be transmitted to an analysis facility 154, analyst, practitioner and the like, which may also include assessment with patient questionnaires, to determine a final analysis of skin health. The device 108 may also employ specific targeted wavelengths, such as in the red, green, and blue areas, to identify key features, based on spectroscopic and quantitative analysis of skin lesions. The device 108 may be used with diffused reflectance techniques, as well as with color imaging analysis based on indirect results from spectroscopic techniques (DR, SF, etc). In embodiment, the device 108 may be adapted to emit polarized light. The device 108 may be adapted to emit more than one type of light and may be able to switch among or combine various light sources 127. The skin health analysis may be compared with a previous user skin health analysis, other users' skin health analysis, other users' experience data, and ingredient, product, and regimen characteristics to provide a recommendation for and track the effectiveness of a product or regimen 108.

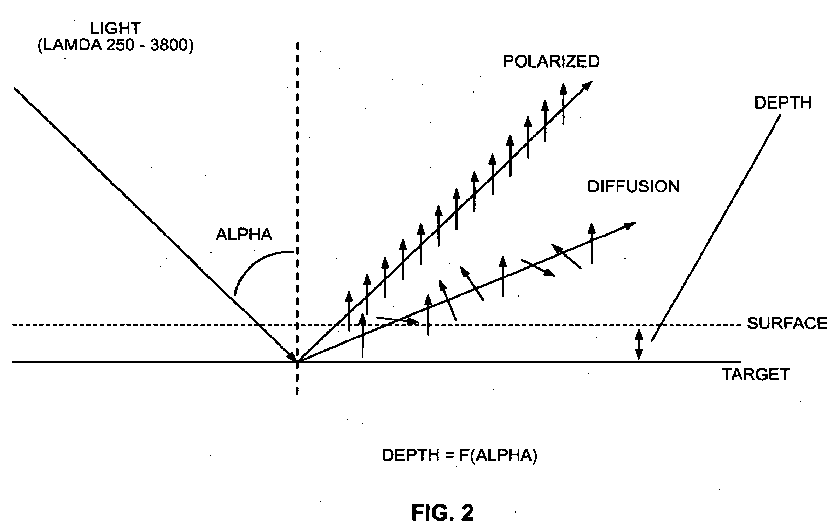

Referring now to Fig. 2, in an embodiment, the imaging device 108 may comprise an illumination source 127 to direct unpolarized light, diffusion light, white light, monochromatic light, light of multiple single wavelengths, polarized light, and the like, upon the skin at an angle alpha, a sensor for detecting reflected or re-emitted light from a skin structure, and an image storage device for storing and transmitting the captured images. A skin structure may be at least one of a cell, a molecule, a group of cells, a group of molecules, an epidermis and sublayers, a basement membrane, a dermis, a subcutis, a gland, a stratum, a follicle, a pore, a vascular component, and the like resident within the skin. In an embodiment, the light source may be white light for generating reflected or re-emitted light and diffuse emission, such as polarized light, to measure the electrical and magnetic components of the skin. White light may be emitted as a combination of wavelengths of light across the spectrum of visible light. Incident unpolarized light may be directed at its target at a defined angle 'alpha' from vertical. As the value of alpha changes, such as and without limitation over a range of 0 to 90 degrees from vertical, incident unpolarized light may interact with different structural elements of the skin since varying the angle of incidence affects the depth of penetration. The angle alpha may be changed by changing the position of the light source, either manually, through a remote control, through a user interface 102, and the like. The relationship between depth of penetration and alpha may be defined by the formula depth = f(alpha). For each skin structure which may correspond to a particular known depth within the skin, there may be a specific angle of incidence which produces a full polarized reflection. By analyzing the reflected or re-emitted light and/or diffuse emission, either polarized and/or diffusion, information on the underlying skin structures responsible for the reflection and/or re-emission may be obtained. The diffuse emission occurs because there is scattering and absorption that occurs from light bouncing around in the substructures. The polarization of the light may be due to classical / quantum effects of skin structures interacting water. That is, skin structures possess enough of a magnetic and electric field to be able to alter the polarization of light as it strikes the structures and to affect the wavelength of light as it strikes the structures. An aspect of the polarization of the reflected or re-emitted light, such as an orientation, an amplitude, a phase, an angle, a shape, a degree, an amount, and the like, may correlate with various measures associated with the particular skin structures targeted, and ultimately, a skin state 158. For example, a lesion present in a particular skin structure may cause the diffusion of a portion of the reflected or re-emitted light resulting in reflected or re-emitted light that is partially polarized and partially diffused. For example, collagen structures are one indicator of a biological difference between a benign and a malignant melanocytic skin lesion. The collagenous differences may affect the polarization state of reflected or re-emitted light, and the resultant images may indicate locations of tumor center and tumor periphery. Such images may aid a practitioner in visualizing excision margins, as will be further described herein. Because melanocytes are located at the lower part of the epidermis, the appropriate wavelength may be selected for this depth as well as for the chromophores within the various types of nevi.

If incident light is polarized, only the electrical properties of skin will be apparent but unpolarized incident light may reveal both the electrical and magnetic properties of skin. While using polarized light may generate improved induction of optical activity, the data sets generated may be of less value as compared to the data sets captured using incident unpolarized light, such as white light, a monochromatic light, light of multiple single wavelengths, and the like. By measuring the effects between 10E-34 and 10E-30 Js, one can make measurements at the border area of quantum and classical physics effects on the skin and as a difference of action of electrical and magnetic forces of valence electrons of skin's biomolecules.

In an embodiment, the wavelength and/or intensity of the incident light may be modified in order to measure the presence of specific molecules, such as collagen, elastin, cadherin, hemoglobin, and the like. Certain molecules possess the property of endogenous fluorescence. For example, if incident light is limited to a particular wavelength, such as 325 nm, collagen may be detected at an emission wavelength of 400 nm and 405 nm. Table 1 lists certain illustrative examples of excitation and emission maxima of biological molecules that exhibit endogenous fluorescence, such as amino acids, structural proteins, enzymes and coenzymes, vitamins and vitamin derivates, lipids, porphyrins, and the like. To detect the presence of specific molecules in the skin, a user may shine a light of a specified wavelength, such as and without limitation those shown in the excitation maxima column, onto the skin and collect reflected or re-emitted light to identify the presence of specific emission wavelengths in the reflections. It may be understood by one knowledgeable in the art that many different single wavelengths and combinations of wavelengths of light may be used to illuminate the skin.

| ENDOGENOUS FLUORESCENCE | | EXCITATION MAXIMA (NM) | EMISSION MAXIMA (NM) |
|---|---|---|---|
| **AMINO ACIDS** | | | |
| | TRYPTOPHAN | 280 | 350 |
| | TYROSINE | 275 | 300 |
| | PHENYLALANINE | 260 | 280 |
| **STRUCTURAL PROTEINS** | | | |
| | COLLAGEN | 325 | 400,405 |
| | ELASTIN | 290,325 | 340,400 |
| **ENZYMES AND COENZYMES** | | | |
| | FAD, FLAVINS | 450 | 535 |
| | NADH | 290, 351 | 440,460 |
| | NADPH | 336 | 464 |
| **VITAMINS** | | | |
| | VITAMIN A | 327 | 510 |
| | VITAMIN K | 335 | 480 |
| | VITAMIN D | 390 | 480 |
| **VITAMIN B6 COMPOUNDS** | | | |
| | PYRIDOXINE | 332,340 | 400 |
| | PYRIDOXAMINE | 335 | 400 |
| | PYRIDOXAL | 330 | 385 |
| | PYRIDOXIC ACID | 315 | 425 |

(continued)

| ENDOGENOUS FLUORESCENCE | | EXCITATION MAXIMA (NM) | EMISSION MAXIMA (NM) |
|---|---|---|---|
| | PYRIDOXAL 50-PHOSPHATE | 330 | 400 |
| | VITAMIN B12 | 275 | 305 |
| LIPIDS | | | |
| | PHOSPHOLIPIDS | 436 | 540, 560 |
| | LIPOFUSCIN | 340-395 | 540, 430-460 |
| | CEROID | 340-395 | 430-460, 540 |
| PORPHYRINS | | 400-450 | 630,690 |

FAD, flavin adenine dinucleotide; NADH, reduced nicotinamide adenine dinucleotide; AND(P)H, reduced nicotinamide adenine dinucleotide phosphate.

In an embodiment, light may be emitted at any wavelength, such as across the range from 280 nm to 3800 nm. Incident light may be blue, yellow, orange, red, or some other light.

Continuing to refer to Fig.1, in an embodiment, the light source may be integral to the device 108 or provided from an associated source. The light source may be a light-emitting or laser diode (LED) of any wavelength, such as and without limitation 280, 340, 360, 385, 405, 395, 400, or 480 nm incident excitation wavelengths, as well as infrared and near-infrared. Wavelengths in the ultraviolet and infrared ranges may also be emitted by the device 108. The light source may be diffusion light, white light, monochromatic light, light of multiple single wavelengths, incandescent, electroluminescent, fluorescent, halogen, ultraviolet, polarized light, collimated light, light provided by a wireless communications device, light provided by a fiber optic cable, and the like. In an embodiment, the light source may comprise a diffuser to provide diffuse incident light.

In an embodiment, a sensor for detecting reflected or re-emitted light from the skin may be embodied in optics resident in a CCD camera, CMOS-based imaging system, digital camera, webcam, camera embedded in a communications device such as a cell phone or iPhone, PDA (Personal Digital Assistant), a watch or other wearable device for continuous monitoring of the skin as in a sports-type indication, a third party device, a scanner, and the like. The sensor may be adapted to absorb any wavelength of light, such as near IR or visible wavelengths. The sensor may be adapted to automatically filter out particular wavelengths. The sensor may be adapted to image any size area, such as a small portion of the skin, the full face, a complete cutaneous examination, and the like. The sensor may be adapted to operate without any intervening fluids between the device 108 and the area of concern, or may be used with an oillike application or other reflective media to the area of concern. The sensor may be adapted to detect reflected or re-emitted light, from any distance from the area or when in contact with the area of concern, which may be used for subsequent visual and/or algorithmic analysis. The images generated from this reflected or re-emitted light may be considered both visual as well as spectroscopically resolved images or electromagnetic skin maps. The sensor may have an internal calibration scale that enables measuring the size of the region being imaged as well as the distance from the imaged area. In an embodiment, a lens may focus the reflected or re-emitted light from the detection optics onto a visible-NIR sensitive CCD, CMOS, or other sensory device. In an embodiment, the sensor may be adapted to acquire images at a high frame rate. In an embodiment, the device may possess a high magnification lens.

In an embodiment, the device 108 may store captured images for analysis and/or transmittal to an analysis facility 154. The analysis facility 154 may be a practitioner, an automated analysis tool, a practitioner employing analysis tools, and the like. Data storage 110 may occur manually when image capture is initiated, may occur automatically upon contact with the skin, may be remotely controlled, and the like. Data may be stored in an internal device memory 168 or may be stored externally in memory media 170 such as USB memory, an external hard drive, a mass storage device, and the like. The device may be able to connect externally, either through a wired connection or wirelessly, to a computer, such as a laptop, kiosk, desktop computer, central server, and the like. For example, the connection may be a direct USB connection. When the device 108 is connected to the computer, captured data may be downloaded or transmitted either automatically or upon manual initiation from the device 108 to the computer. For example, the device 108 may have a cradle in connection with a computer. When the device 108 is placed in the cradle, data may be transmitted or downloaded from the device 108. Additionally, the device 108 may receive software updates when connected to the computer, such as through the cradle. In embodiments, the device 108 may have no internal storage and may only be able to transmit or store data externally through a persistent hard-wired or wireless connection. Data transmittal and storage may be a fully automated process or,may be manually operated. Data may be transmitted over a wireless network connection, a cellular connection, a wired connection, a Bluetooth connection, and the like. Data transmittal from the device 108 may enable remote assessment techniques. In an embodiment, non-image data may also be stored and/or transmitted by the device 108 as described herein, such as voice responses, text responses, video data, and the like. The device 108 may have an internal microphone to record audio, a video camera to record video, a keyboard input to record text responses, and the like. In an embodiment, the device 108 may use externally available audio and video.

In an embodiment, data storage may be in a skin health record 121. The skin health record 121 may be an object or database or repository for an individual that contains information on key medical, non-medical, and cosmetic indications related to a user's skin. This may comprise images, graphics, icons, written history, personal demographic information, levels of cosmetic conditions such as moisture, elasticity, firmness, texture, color level, or non-medical conditions such as inflammation, and the like. A user may self-populate the record 121 with data from any device 108, 109 or input 112. The record 121 may contain a history of skin concerns, comments, a user blog, and the like. In an embodiment, the skin health record 121 may auto-populate upon acquisition of an image. For example, when a user submits their first image for analysis, a record 121 may be automatically created and populated with information, which may be edited, derived from the image and its analysis.

In an embodiment, data storage 110 may occur in a practitioner record 180. A practitioner record 180 may be a repository of key health characteristics including background demographic data, personal information, information on diet, skin health record 121 and the like. It may have embedded images, links to other image data files, tracking effectiveness of personal skin products, medical products, and OTC products and the like and their historical impact on key parameters. It may also capture community data or data of selected individuals who may be similar to the patient or user and may include rankings and comments and the like

In an embodiment, data storage 110 may be in a personalized manufacturing record 172. Based on the skin health measurement 160, product ingredients to obtain a desired effect to make the skin healthy may be selected. This ingredient selection may be achieved by analyzing and tracking the change of various skin health parameters through the application of various products and ingredients through using the device 108 and tracking the change of the skin health over time through a personalized manufacturing record 172. Once the selected product ingredients are identified, they may be mixed to create a product best suited for the individual's skin characteristics and/or desired goals (such as improved moisturization). Thus a personalized product may be developed for the user. Additionally, this same process could be used for creation of specific customized skin products and ingredients for medical and non-medical purposes and conditions.

In an embodiment, the form of the data captured may be compatible with any standard image processing and manipulation software and techniques, word processing software, slideshow presentation, spreadsheet applications, and the like. For example, the captured data may be in any suitable image format, such as jpeg, tiff, pict, png, bmp, gif, pdf, and the like. In an embodiment, multiple images may be captured as a movie or a movie may be constructed from combining multiple images.

In an embodiment, the device 108 may be powered by any suitable source, such as an electric power plug, a battery, solar power, USB power, and the like. A user may initiate power to the device 108 in order to begin acquiring images. Acquisition may commence automatically, may commence when the device 108 is placed against the skin, may commence when a trigger, such as a button, is actuated by a user, and the like.

The device 108 may have a display for viewing the area to be imaged. For example, a user may use the display with positioning tools to obtain exact images over time, such as a series of images taken over different days. The display may be integral to the device 108 or may be a separate display. For example, the device 108 may be connected to a monitor, such as that of a computer, using a wired connection or a wireless connection. In an embodiment, a user interface 102 to the device 108 may display a real time view of the imaging.

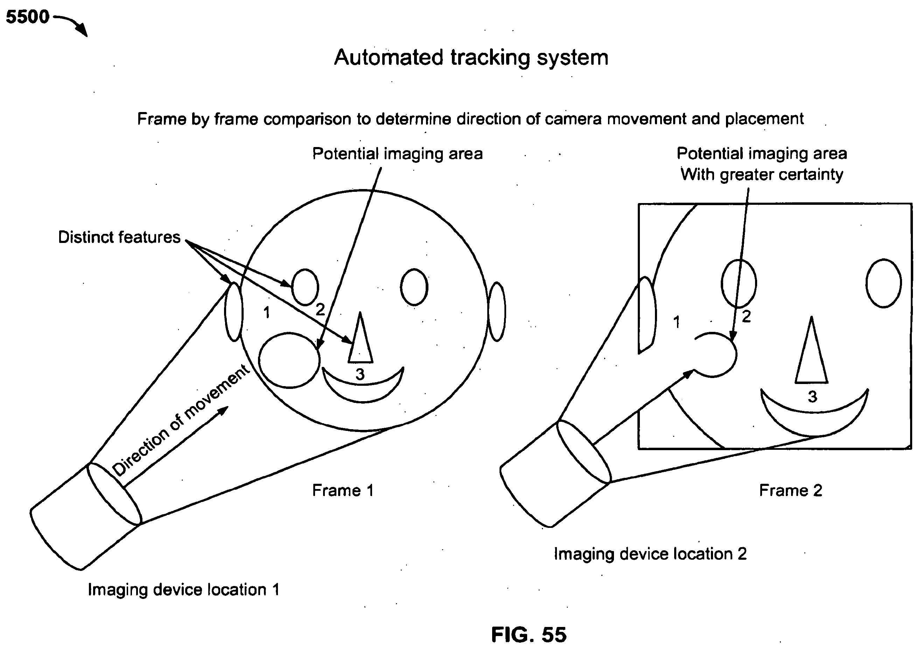

The positioning tools may enable tracking and targeting. Referring to Fig. 55, a method of tracking and targeting is depicted. The positioning tools may be used to track and store movement parameters of the imaging device 108 moving over a subject area. First, the device may capture an image of the subject area at a plurality of locations. Then, the device 108 may identify a direction of movement of the imaging device 108 using an image processing technique for at least one captured frame. The image processing technique may recognize the direction of movement of the imaging device by comparing each frame with at least three distinct features captured to thereby triangulate a location of the imaging device, as shown in Fig. 55. The data of the captured image may be compared with a predetermined image database to store the image of the subject area and to store placement parameters of the imaging device 108. If no entry exists in the database, a new entry may be made. The step of capturing the image of the subject area at a plurality of locations may include a sub-step of capturing a continuous video image of the subject area. The step of capturing the image of the subject area at a plurality of locations may include a sub-step of capturing a frame by frame sequence of images of the subject area. The step of identifying a direction of movement of the imaging device using an image processing technique may include a sub-step of a frame by frame comparison of the captured image to identify movement parameters of the imaging device. The step of recognizing the direction of movement of the imaging device by comparing each frame with at least three distinct features captured to triangulate a location of the imaging device may include a sub-step of capturing a direction of movement of the imaging device by comparing three or more distinct positions across different frames. The positioning tools may be an automated location tracking and data storage system for the imaging device 108, including an image capturing unit, a positioning unit coupled to the image capturing unit for positioning the imaging device on a subject area, and an image processing unit for enabling a frame by frame comparison of the captured image and for enabling the imaging device to capture three or more distinct points to triangulate a location of the imaging device to identify a direction of movement of the imaging device. The image capturing unit may include a digital camera. The image capturing unit may include at least one of a mobile device and a Personal Digital Assistant (PDA). The image processing unit may include a comparison unit for comparing positions of three or more distinct points across different frames to capture direction of movement of the imaging device. The automated location tracking and data storage system may further include a sub-system for measuring lateral motion of the image capturing unit from a predetermined point to a new location on the subject area.

In an embodiment, the device 108 may have security features in order to protect the privacy of user data. For example, the device 108 may have a unique MacID with encryption technology.

In an embodiment, the device 108 may be associated with peripherals or other functional attachments. For example, the device 108 may be associated with a blood pressure monitor or sensor, a heart rate monitor or sensor, and the like. For example, the device 108 may be used to perform a pre-diagnosis 162 of a skin lesion while also monitoring other endpoints such as blood pressure, heart rate, and the like in order to assess other aspects of health in addition to skin health.

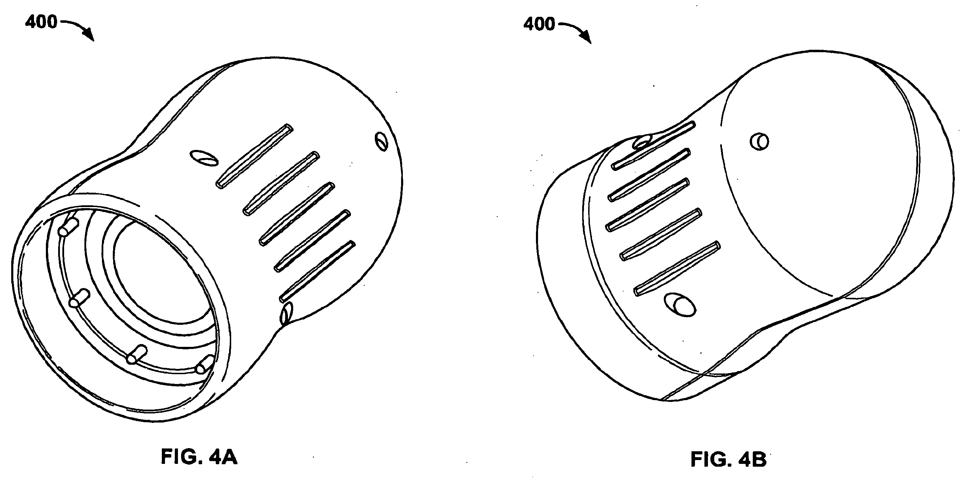

In an embodiment, the device 108 may be sized to permit a user to operate the device 108 in a handheld fashion. The device 108 may sized for portability. The device 108 may adapted for single-handed operation. For example, the device may be embodied as in Fig. 4 A & B, but it may have multiple other embodiments in any shape and/or size, such as a mirror, a large device adapted to image a large area, a PDA, a scanner, a mobile communication device, and the like. In Fig. 4A, the illumination source is visible as a ring of LED's around a central detection area. In both images, the size, handheld nature, and portability are clearly demonstrated. The ease of operation enables even an inexperienced user, such as a home user connected to a laptop, to employ the device 108. The device 108 may be a self-contained unit and not part of a larger camera system. In an embodiment, the device 108 may be designed for one handed ergonomic holding. In an embodiment, the device 108 may be used with or without application of reflective media. In an embodiment, the device 108 may be used to capture images at a distance, close-up, in direct contact, and the like. For example, software loaded on a computer interfaced with the device 108 may prompt for near distance and far distance image capture.

In an embodiment, the device 108 may also be a standalone, non-hand-held version, which may be used to take images or particular body components or materials.

In some embodiments of the skin care device, the device may be a miniature one, enabling portability and hand-held use. Some embodiments of the skin care device may be in the form of a hand-held and portable wand that can be conveniently moved across a skin region to be examined. Some other embodiments of the skin care device may be so miniaturized that no dimension of the skin care device exceeds six inches. Such skin care devices may be embedded in wearable accessories, for example, bracelets, necklaces, ear-rings, and the like. Some embodiments of the skin care device may have a convenient user interface and/ or a display surface. In some embodiments of the skin care device, the device may be coupled to or embedded in a vertical display panel, for example but not limited to, a mirror, an LCD screen, a plasma screen, and the like.

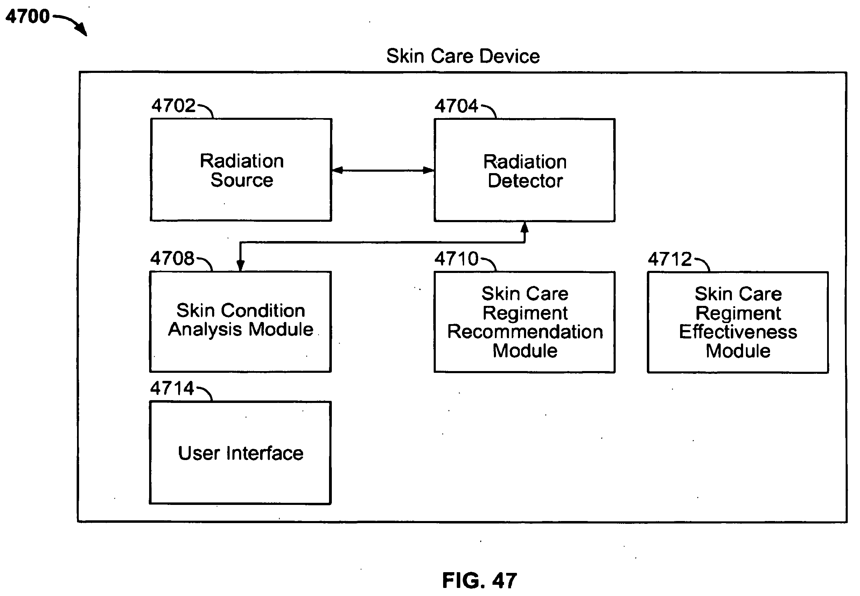

Referring to Fig. 47, an exemplary skin care device 4700 embodying the principles of the invention is shown in a block diagram. The skin care device 4700 may include an electromagnetic radiation source 4702, a radiation detector 4704, and a skin condition analysis module 4708.

The electromagnetic radiation source 4702 may be capable of directing incident electromagnetic radiation to one or more locations on the skin of a person. For example, and not by way of limitation, the radiation source 4702 may be a set of light emitting diodes (LEDs). In certain embodiments, the incident radiation emitted by the radiation source 4702 may include radiation in the visible, near-infrared (NIR) and near-ultraviolet (NUV) spectrum. In certain other embodiments, the incident radiation may include white light.

As depicted in Fig. 47, the electromagnetic radiation source 4702 may be coupled to the radiation detector 4704. The radiation detector 4704 may be capable of detecting the radiation re-emitted from the location and measuring various radiation parameters of the re-emitted radiation. As shown in the Fig. 47, the radiation detector 4704 may be coupled to the skin condition analysis module 4708. A variety of radiation parameters may be detected by the radiation detector, including, for example but not limited to, degree of polarization, intensity of the radiation at different wavelengths, and the like. The electromagnetic radiation sources, radiation detectors, and the skin condition analysis module have been previously described herein.

The skin condition analysis module 4708 may be capable of analyzing the radiation parameters of the reflected radiation and other information to generate a skin condition assessment. The skin condition analysis module 4708 may be adapted to generate the skin condition assessment in real-time. In some embodiments, the radiation detector 4704 measures diffused reflectance. In some other embodiments, the incident radiation may be white light and the radiation detector 4704 may measure the red, green, and blue components of the re-emitted light.

In certain embodiments, the skin condition assessment may also be partly based on analysis of a photographic image of the skin location.

As used in the specification and the appended claims, the term "diffused reflectance" may refer to radiation, sometimes loosely referred to as light, scattered in many directions from target samples. Diffused reflectance is the complement to specular, or mirrorlike, reflection. If a surface is completely non-specular, the reflected or re-emitted light will be evenly spread over the hemisphere surrounding the surface. Diffused reflectance stems from tiny irregularities on surfaces of targets and is the reflection of incident light from uneven or granular surfaces of targets such that incident light strikes the targets and is scattered over wide angles.

Some embodiments of the skin care device may have a memory module for storing the skin condition assessments and other data, such as with timestamps. Some embodiments of the skin care device may have a communication module for communicating the skin condition assessments and other data with timestamps to a remote computer. The communication of data may occur, for example, over a wire, wirelessly, using an internet, and the like. The skin condition assessments and other data may also be accessed in remote locations via mobile devices and/or computers. Such remote access may be particularly convenient for service providers, such as for example, dermatologists.

Some embodiments of the skin care device may have a user interface to enable a user to interact with the skin care device. The user interface may enable a user to give instructions to the device, for example, to analyze the available information to generate a real-time skin condition assessment of a skin location or a larger skin region. In some other embodiments, the user interface may be voice-operated providing the facility to give commands to the skin care device through speech commands. Other examples of user interfaces that may be used in the skin care device are graphical user interface (GUI), web-based user interface (WUI), command line interface, touch interface, and any combination of the above.

In certain embodiments, the user interface may also provide alerts to a user if any abnormal skin condition, such as for example, a clogged pore, is detected. The alerts may be in the form of a light signal, a beep, an email alert, an SMS alert, and the like. There may be other methods, such as a small electric tingle, a mark, a sound, and a light, a heat emitting signal, and the like, to alert users about skin conditions requiring user attention.

Some embodiments of the skin care device may have also have a display surface either for a more convenient and intuitive user interface and/or for viewing an image of a skin region and/or for viewing some useful skin-related information, for example, a skin condition assessment report, a skin regimen recommendation report, and/or a skin regimen effectiveness report. In some embodiments, the display surface and/or the user interface may be touch-sensitive to enable touch-control of the device.

In some embodiments, the skin condition assessment data of locations may be overlaid on an image of a larger skin region displayed on the display surface, providing a useful picture of the health of the entire skin region in a single view.

Some embodiments of the skin care device may also have an access restriction module restricting access to patient data to authorized users only. The access restriction module may be based on a user name and password feature and/or biometric access control, for example, fingerprint recognition, facial recognition, retina recognition, and the like.

In some embodiments, the skin condition analysis module 4708 may have access to user information like age, gender, ethnic group, and the like, and such information may be used to build a user profile and used in analysis of the skin condition.

The skin care device 4700 may be used in a user's home, a user's bathroom, a cosmetic store, a provider's office, a mobile location, and the like. The skin care device 4700 may be used at any time of the day, such as before going to bed, before or after using a cleanser on the skin, and the like.

The skin care device 4700 may have a skin care regimen recommendation module 4710 capable of generating a displayable skin care regimen recommendation. The skin care regimen recommendation may include information not only about the most appropriate skin-care products, but also information about the best way of applying the product, the timing, amount, and frequency of application, and the like. The skin care regimen recommendation module 4710 may be linked to the skin condition analysis module 4708 so that the skin care regimen recommendation is personalized to the skin condition of each person. The skin care regimen recommendation may be generated in real-time based on skin condition assessments generated by the skin condition analysis module 4708, product information, and other relevant information analyzed using algorithms, as described herein. In some embodiments, the skin care regimen recommendations generated by the skin care regimen recommendation module 4710 may be displayed to the user in real-time, for example, on a display surface attached with the skin care device 4700.

In some embodiments, it may be possible to print the skin care regimen recommendations generated by the skin care regimen recommendation module 4710.

In some embodiments, the skin care regimen recommendations generated by the skin care regimen recommendation module 4710 are based at least partly on determination of a skin profile, or skin state 158, of the user and use of skin care regimen recommendations of persons with a similar profile.

In some other embodiments, the skin care regimen recommendation module 4710 is coupled to a skin-care product database 190. If the products recommended by the skin care regimen recommendation module 4710 are available in the product database 190, the user may be informed and given an option to purchase the product immediately. In some embodiments, the user may operate the skin care device 4700 in a point-of-sale location, for example, a retail store, and the availability of a product recommended by the skin care regimen recommendation module 4710 may be indicated by an audio-visual signal, such as for example by lighting up the shelf in which the product is located.

A user practicing a specific skin care regimen, for example, use of a skin-care product in a prescribed manner, may be interested in tracking the effectiveness of the skin care regimen over a period of time. The skin care device 4700 may have a skin care regimen effectiveness module 4712. The skin care regimen effectiveness module 4712 may be coupled with the skin condition analysis module 4708. The skin condition of the user may be tracked at different points of time using the skin care device 4700 and may be displayed to the user on a display surface. The device could also help track changes by various activities - exercise, food, smoking, work, and the like.

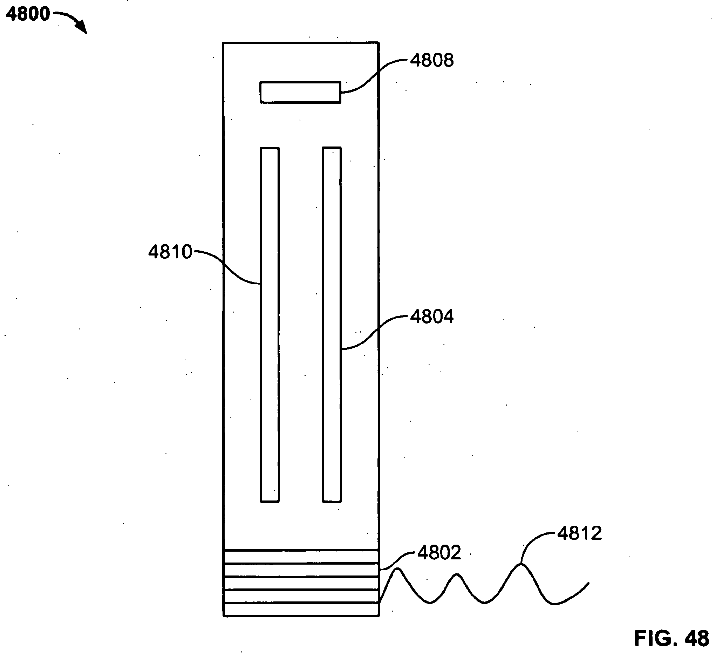

Fig. 48 shows an embodiment of a skin care device 4700 in which the skin care device is wand-shaped. For example, a user may switch on the wand-shaped device 4800 and move the device over her face. The wand-shaped device may have a grip 4802, a radiation detector 4808, an indicator 4804 that may provide an indication such as with light, warmth, sound, and the like, an LED light 4810, and a power source 4812.

The wand-shaped device 4800 is functionally similar to the skin care device 4700 described earlier. The wand-shaped device 4800 may comprise an electromagnetic radiation source, a radiation detector, and a skin condition analysis module. The wand-shaped device 4800 may be miniature, hand-held, and portable.

In some embodiments of the wand-shaped device, the electromagnetic radiation source may be one or more LEDs. Each of the LEDs may have unique predetermined frequencies. In some embodiments, the one or more LEDs may be arranged in a line to form a light strip.

In some embodiments, the wand-shaped device 4800 may be powered via a USB coupled to an external power source or through built-in batteries, or other similar power source.

As the wand is moved over the skin, light is emitted from the radiation source 4702. Then, the radiation detector 4704 detects re-emitted light and sends information back to the skin condition analysis module 4708. The module 4708 employs an algorithm for skin condition analysis.

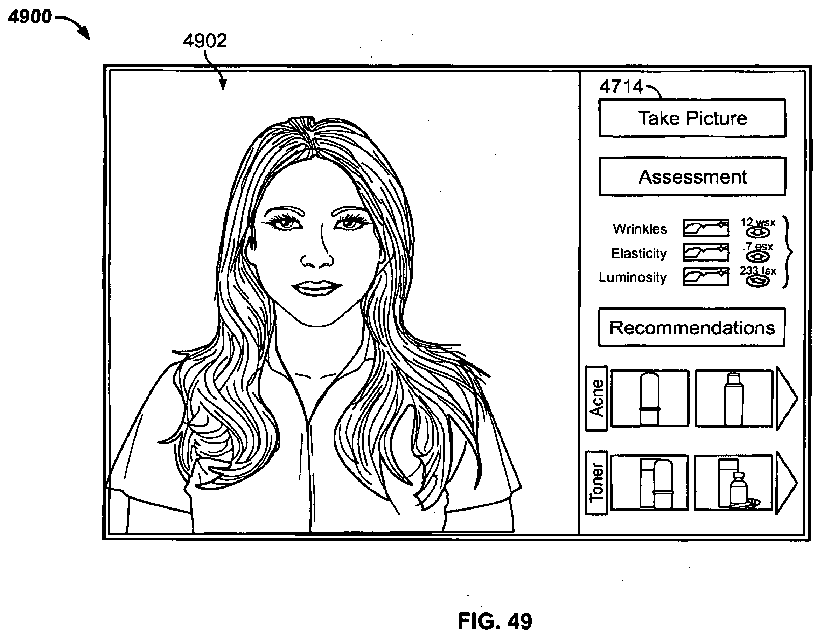

Fig. 49 shows another embodiment of a vertical panel-including skin care device 4900, in which the skin care device comprises an electromagnetic radiation source 4702, a radiation detector 4704, a skin condition analysis module 4708, a user interface 4714, and a vertical display panel 4902.

The vertical display panel 4902 may have the user interface 4714 on the sides of the vertical display panel 4902. In some embodiments, the display panel may be touch-sensitive and in such cases, the vertical panel itself may be part of the user interface. An image of a skin region may be displayed in the display panel. A user may touch a location on an image and this may trigger display of a magnified image either on the display panel or on another screen. A menu bar may show up in the user interface 4714, and the user may be able to view various reports, for example, a skin condition assessment report, a skin regimen recommendation report, a skin regimen effectiveness tracking report, and the like.

The user interface 4714 may enable a user to give instructions to the device, for example, to analyze the available information to generate a real-time skin condition assessment of a skin location or a larger skin region. In some other embodiments, the user interface may be voice-operated providing the facility to give commands to the skin care device 4900 through normal speech commands. Other examples of user interfaces that may be used in the skin care device 4900 are graphical user interface (GUI), web-based user interface (WUI), command line interface, touch interface, and any combination of the above.

The basic functioning of the vertical panel-including skin care device 4900 is similar in many respects to the skin care device 4700. The electromagnetic radiation source 4702 is capable of directing incident electromagnetic radiation to one or more locations on the skin of a person. For example, and not by way of limitation, the radiation source 4702 may be a set of light emitting diodes (LEDs). In certain embodiments, the incident radiation emitted by the radiation source 4702 may include radiation in the visible, near-infrared (NIR) and near-ultraviolet (NUV) spectrum. In certain other embodiments, the incident radiation may include white light.

As depicted in Fig. 49, the electromagnetic radiation source 4702 may be coupled to the radiation detector 4704. A variety of radiation parameters may be detected by the radiation detector 4704, including, for example but not limited to, degree of polarization, intensity of the radiation at different wave-lengths, and the like.

In certain embodiments of the vertical panel-including skin care device, the skin condition assessment may also be partly based on analysis of a photographic image of the skin location.

Some embodiments of the vertical panel-including skin care device may have a memory module for storing the skin condition assessments and other data, such as with timestamps.

Some embodiments of the vertical panel-including skin care device may have a communication module for communicating the skin condition assessments and other data with timestamps to a remote computer. The communication of data may occur, for example but not limited to, over a wire, wirelessly, using an internet, and the like. The skin condition assessments and other data may also be accessed in remote locations via mobile devices and/or computers. Such remote access may be particularly convenient for service providers, such as for example, dermatologists.

In certain embodiments, the user interface 4714 may also provide alerts to a user if any abnormal skin condition (for example, a clogged pore) is detected. The alerts may be in the form of a light signal, a beep, an email alert, an SMS alert, etc. There may be other methods e.g. a small electric tingle, a mark, a sound, and a light, a heat emitting signal, etc. to alert users about skin conditions requiring user attention.

In some embodiments, the skin condition assessment data of locations may be overlaid on an image of a larger skin region displayed on the vertical display panel 4902, providing a useful picture of the health of the entire skin region in a single view.

Some embodiments of the vertical panel-including skin care device may also have an access restriction module restricting access to private information to authorized users only. The access restriction module may be based on a user name and password feature and/or biometric access control, for example, fingerprint recognition, facial recognition, retina recognition, and the like.

In some embodiments, the skin condition analysis module 4708 may have access to user information like age, gender, ethnic group, and the like, and such information may be used to build a user profile and used in analysis of the skin condition.

The vertical panel-including skin care device 4900 may be used in a consumer's home, a consumer's bathroom, a cosmetic store, a provider's office and/or a mobile location. The vertical panel-including skin care device 4900 may be used at any time of the day, such as before going to bed, before or after using a cleanser on the skin.

In some embodiments of the vertical panel-including skin care device, the device may include or be coupled with a skin care regimen recommendation module capable of generating a displayable skin care regimen recommendation.

In some other embodiments of the vertical panel-including skin care device, the device may include or be coupled with a skin care regimen effectiveness module capable of generating a displayable skin care regimen effectiveness report.

In some embodiments of the vertical panel-including skin care device, the vertical display panel is a mirror.

In some embodiments of the vertical panel-including skin care device, the vertical display panel is an LCD panel or a plasma screen.

In some embodiments of the skin care device, the device also includes or is coupled with a camera for taking photographic images of a skin region.

In certain embodiments of the skin care device, the camera is integrally attached to the display surface or display panel. In certain other embodiments, the camera is either wired to the display surface or display panel. In other embodiments, the camera is wirelessly coupled to the display surface or display panel.

In certain embodiments of the vertical panel-including skin care device, the user interface 4714 may have one or more buttons (not shown explicitly) for doing a skin scan and/or analysis. The buttons may be of different types, for example push buttons, hard wired buttons, or a combination of both. The user may touch a button on the display panel for doing a skin scan, while she may touch another button for directing the machine to do a skin analysis.

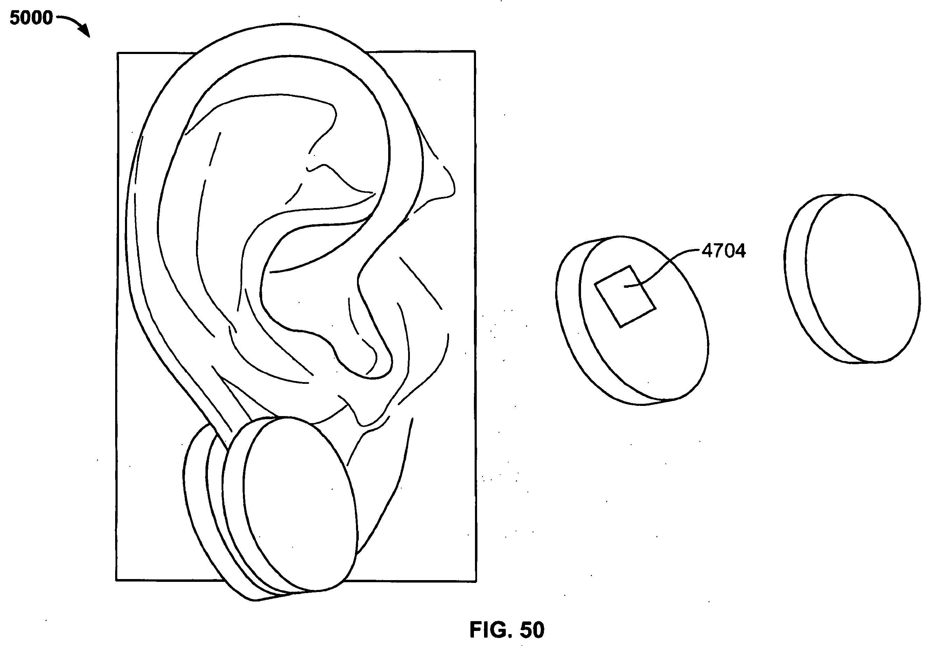

Fig. 50 shows an embodiment of a wearable skin care device 5000, in which the device is in the form of a wearable device. The wearable device can be worn by a user in the form of necklace, ear-rings, bracelets, a patch, or as a sensor attached to a strap, and the like. Such wearable devices can be persistent, personalized skin care monitors.

The wearable skincare device 5000 is functionally similar to the skin care device 4700 described earlier. Similar to the skin care device 4700, the wearable skincare device 5000 comprises an electromagnetic radiation source, a radiation detector, and a skin condition analysis module. Preferably, the wearable skincare device 5000 is miniature, hand-held, and portable, and no dimension of the device exceeds six inches.

In some embodiments of the wearable skincare device, the electromagnetic radiation source may be one or more LEDs. Each of the LEDs may have unique predetermined frequencies. In some embodiments, the one or more LEDs may be arranged in a line to form a light strip.

In some embodiments, the wearable skincare device 5000 may be powered via a USB coupled to an external power source or through built-in batteries, motion power, solar power, or other similar power source

Embodiments of the wearable skincare device may also have sensors for measuring various body and environmental parameters. Examples of body parameters that could be measured by the wearable skincare device are body temperature, hemoglobin antioxidant level, etc. Examples of environmental parameters that could be measured by the wearable skincare device are air cleanliness, humidity, temperature, UV index, external air quality, smoke index, and the like.

In an embodiment, the device 108 may be adapted for use as a component of a minimally invasive medical device associated with laparoscopy, cytoscopy, ureteroscopy, arthroscopy, endoscopy, dermoscopy, gynecology, urology, dentistry, natural orifice insertion analysis such as through ears, mouth, anus, nose, and external breast cancer analysis through the skin, and the like. For example, the system may be able to process the data and to appear on a video monitor or other display in a surgical suite or other medical setting. A medical professional may be able to select a viewing mode, such as still image capture or video capture, and may be able to manually adjust the parameters of the light source, sensor and display to assist in observation, identification, and monitoring with the device 108. In an embodiment, the system may be pre-programmed with various protocols for the various types of medical procedures and tissues types that a medical professional may encounter such that the system may automatically handle the device 108 based on the medical professional's indication of the type of procedure and tissue being examined.

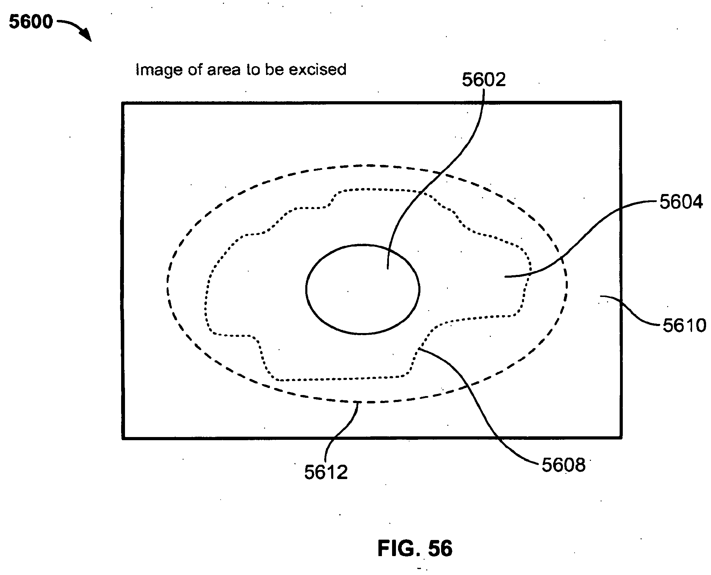

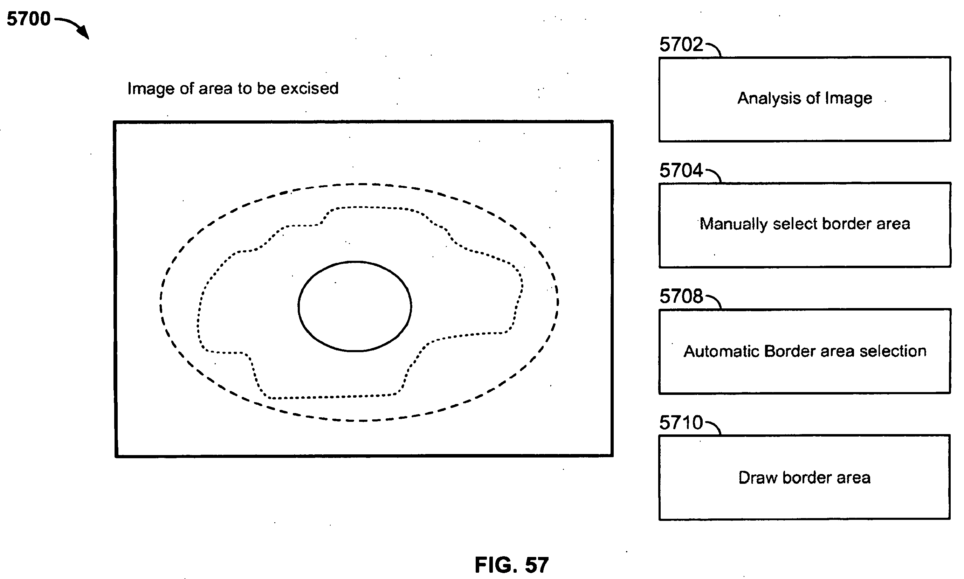

For example, the device 108 may be used as part of a system and method for distinguishing between healthy and suspect tissue in real or near-real time on a patient. The imaging device 108 allows a surgeon or other practitioner to precisely determine the border area around a surgical intervention for primary cutaneous melanoma, skin cancers, and other skin diseases that require excision around the skin. Generally, the surgical excision of suspect tissue, such as cutaneous melanoma, may be determined either by a surgeon's experience or through a Breslow scale and punch biopsy that determines the thickness of a melanoma and hence generally agreed-to border areas. The device 108 allows an automatic determination of the excision margin for primary cutaneous melanoma based on the optical characteristics of the surrounding skin. By precisely defining where there is healthy tissue and where there is suspect tissue, a surgeon could leave a larger amount of healthy tissue around a site, decrease recurrence and decrease micrometastasis in surrounding skin while enabling minimal surgical morbidity and improved cosmetic appearance. The device 108 and associated algorithms 150 and analysis techniques, such as the convolution technique and RGB color analysis discussed later herein, embodied in software, may be employed to image a particular site, and determine border area, suspect tissue, either before surgery, in pre-surgery, or during surgery. The software could also show post surgical analysis of affected skin tissue. Using the device 108 allows more precise determination of the border area instead of relying on subjective experience or fixed tables as noted in medical journals and other published works. The advantage of this method is better isolated suspect tissue and retaining a greater degree of healthier tissue. Referring now to Fig. 56, a melanocytic lesion is displayed. The visible melanoma 5602 or suspect tissue is surrounded by normal looking skin, but which may contain unhealthy /diseased tissue that must be excised 5604 (pseudo-normal skin 5604). The device 108 may be able to visualize the border between healthy and non healthy tissue 5608, thereby allowing the surgeon to spare healthy tissue 5610 that should remain intact. The device 108 may perform an estimation and provide an outlined area 5612 indicating where the surgeon should cut the tissue. In Fig. 57, an embodiment of a user interface for visualizing a melanocytic lesion is displayed along with access to tools for analyzing an image of the lesion 5702, manually selecting a border 5704, automatically selecting a border 5708, drawing a border area 5710, and the like.

In an embodiment, the device 108 may enable a skin health test 160. The imaging device 108 may be used to perform a skin health test 160 to learn the characteristics of the skin and to obtain a diagnosis. The hardware device may capture an image and enable analysis of the image. The imaging components within the device 108 may enable measuring various skin health characteristics like color, age, damage, collagen, elastin, pores and types, keratin, and the like. The skin health test 160 may be performed in the home, in a spa, clinic, hospital, from a mobile phone at any location, and the like. The skin health test 160 may be used in conjunction with specific background information through questionnaires, image upload, genetic testing, DNA samples, and lifestyle habits to determine a skin state 158. The test 160 would respond with specific information related to the biophysical health of the skin, a portion of which would be physical and genetic disposition to certain medical or non-medical or cosmetic problems or conditions.

In an embodiment, the device 108 may enable a pre-diagnosis 162. This is a system of pre-diagnosis where a practitioner (such as the user, a dermatologist, medical practitioner, aesthetician, and the like) may receive or request from a user to take an image and/questionnaire of a skin concern or the like and receive a pre-diagnosis based on algorithmic analysis of pre-existing conditions. The user may submit a questionnaire and image with a pre-diagnosis of conditions prior to going to see a practitioner and allow a follow-up. Images captured by the device may be submitted to obtain a preliminary diagnosis to enable effectively referring the case to the best practitioner. The pre-diagnosis 162 may be performed by software algorithms on the images, manual analysis, a combination thereof, and the like. The pre-diagnosis 162 may include the preliminary assessment as well as indicate the time required and the steps required for the final diagnosis or assessment. This pre-diagnosis 162 feature may enable effective scheduling of the practitioner. The pre-diagnosis 162 could also help screen for particular skin issues as well as identify users with certain issues.

In an embodiment, the device 108 may enable remote monitoring 164: The user may use the device in the privacy of their home, work, or any other location to perform remote monitoring 164 and submit images to track progress of their skin's health or medical conditions. A practitioner may be able to remotely guide changes in treatment or guide on prevention factors. Remote diagnosis may greatly increase efficiency of progress monitoring since users will not have to make a physician trip to the provider, and the provider could conveniently select a time during the day to observe the patients change. The monitored data may be viewed as a recording or in real time.

In an aspect of the invention, the imaging device 108 may illuminate an area of concern at a known angle of incidence with unpolarized light. To obtain a spectral diagram based on the magnetic properties of the area only, the reflected polarized light, which possesses the electrical properties of the area of concern, may be subtracted from any reflected diffusion light, which possesses electromagnetic properties of the area of concern. The distribution of pixels in the image corresponding to the diffusion light and reflected polarized light may be determined and indicated by any conventional means. For a known image sensor, a one-to-one mapping of pixel image distribution between the diffusion light image, corresponding to an electromagnetic signal, and reflected polarized light, corresponding to an electrical signal image, may be made with a distribution of the intensity of the spectroscopic data for the same area. A magnetic gradient image of the area may be made by equipment such as an AFM-MMR (Atomic Force Microscopy in Magnetic Mode Regime) and from the one-to-one correspondence, a skin state 158 may be based on the gradient image, diffusion light image, and reflected polarized light image.

In an embodiment, the device 108 may be an imaging device 108 for performing digital spectroscopic imaging of the skin. Incident unpolarized light may be delivered, either vertically or on an angle alpha from vertical, from an unpolarized light source associated with the device 108, such as a white light, diffuse light, monochromatic light, light of multiple single wavelengths, and the like, to a target skin structure. White light, which possesses both electrical and magnetic properties, when incident onto a skin structure at a particular angle interacts with the structure's components and leads to the reflected or re-emitted light having a polarized light component. In embodiments, the incident light may be polarized. Unpolarized light reflected by skin structures may become polarized, at least in part. The reflected or re-emitted light, either polarized or diffusion light, may be captured by the device 108. Such multispectral skin imaging may be used to develop an electromagnetic skin topography. By measuring aspects of the polarization of the reflected or re-emitted light such as an orientation, an amplitude, a phase, an angle, a shape, a degree, and an amount, and the wavelength of the reflected or re-emitted light, the biophysical properties of skin structures may be obtained. A skin state 158 may be determined from the aggregate biophysical data obtained from one or more skin structures as well as a visual analysis of the captured images and any additional data obtained from the user anecdotally. For example, the skin state 158 may encompass data on moisture, wrinkles, pores, elasticity, luminosity, and any of a number of measures, as described herein. By varying alpha, the angle of incident white light, the depth of penetration of the light to skin structures may be varied. Each depth within the skin corresponds to different skin structures. For each skin structure or depth, there may be a specific angle which produces a full polarized reflection. For example, a certain angle of incidence may be used to obtain data for skin structures within the epidermis, however, the angle of incidence may need to be changed in order to obtain data on skin structures within the subcutis which resides at a different depth within the skin. The angle of incidence may be modified to penetrate the skin anywhere from a few microns up to a few centimeters, thus enabling the capture of reflections from other non-dermal structures. For example, the device 108 may be used as a non-invasive imaging tool, such as to image tumors, breast cancer, melanoma, and the like. In an embodiment, the area to be imaged may be any biological tissue that may have normal or pathologic variations in its structure, such as variations in the tissue's birefringent properties. For example, scars, keloids, hypertrophic scars, and stria all have organizations of collagen fibers that are different from normal skin. Since collagen is a primary determinant of cutaneous wound repair, it may be of interest to monitor changes in collagen structure and concentration. For example, the stage of healing may be determined by the size of collagen bundles which may increase as healing progresses, by the organization of collagen structures at the molecular or small-fibril level which may increase as healing progresses, by the return or increase of birefringence, and the like. Since collagen structures are polarization-sensitive, changes that occur in the structures may be monitored using a polarization-based technique during scar formation, the healing process, and treatment of scars, as has been and will be further described herein.

Being able to measure the electrical and magnetic properties of various skin structures may enable the differentiation between healthy and non-healthy skin structures. Normal or healthy skin structures exhibit a unique conformation that differs from the conformation exhibited by equivalent structures when unhealthy or abnormal. These conformational changes can be detected by differences in an aspect of the light reflected off of skin, re-emitted light, or amount of absorption in the skin, such as an aspect of the polarization of the reflected or re-emitted light. The aspect of polarization may be the wavelength of the light, an orientation, an amplitude, a phase, an angle, a shape, a degree, an amount of polarization of the light, and the like. According to Maxwell's equations, light can be described as comprising an electric field and a magnetic field which can be described as two vectors, E and B, which behave as waves. The vectors are perpendicular to the propagation direction of the light, and they are orthogonal to each other. Furthermore, given the electric field E, B can be determined via Maxwell's equations, and vice versa. Thus, by measuring the electrical component of the light reflected, re-emitted, or absorbed by the skin structures, the magnetic component or the degree of polarization/polarization state may be determined. Alternatively, the light may spread to other wavelengths that can be measured. By comparing those electrical and magnetic readings from the polarized component of reflected or re-emitted light and non-polarized white light to that of normal or healthy skin structures incident with light at the same or similar angles, changes may be detected in the skin structure and its molecular or structural conformation. Based on the amount or other aspect of both electrical and magnetic determination, specific defects such as cancer, skin diseases, cosmetic indications and the like, may be detected, since each range of measurements may correspond to a particular defective conformation. If any other molecules, cell, or structure are now incident with the same type of light at the same angle, the strength of certain wavelengths of the reflected component may enable the measurement of the intensity of the difference in conformation states of the measured component. The polarization state of the reflected or re-emitted light may be described by a number of parameters. The polarization state may be described in terms of the polarization ellipse, specifically its orientation and elongation. Parameters which may be used to describe the polarization state may include the azimuth angle ($\psi$) which is the angle between the major semi-axis of the ellipse and the x-axis, the ellipticity ($\varepsilon$) which is the ratio of the two semi-axes, the ellipticity angle which is the arctangent of the ellipticity, the eccentricity, the amplitude and phase of oscillations in two components of the electric field vector in the plane of polarization, and the like. For example, an ellipticity of zero corresponds to linear polarization and an ellipticity of 1 corresponds to circular polarization. The polarization of the reflected or re-emitted light may be at least one of elliptical, linear, circular, left-circular, right-circular and any potential combinations thereof.

In an embodiment, determining a skin state 158 may comprise processing and analyzing 154 the reflected or re-emitted light to obtain images for visual and spectroscopic analysis. Analysis 154 may be facilitated by examining the wavelength and other characteristics of the reflected or re-emitted light. For example, if the incident light is white light, the reflected or re-emitted light may be filtered to examine a collection of wavelengths or a single wavelength and, ultimately, a specific skin structure fluorescence. In another example, monochromatic or semi-monochromatic light, such as provided by an LED may be used to excite targeted fluorophores and chromophores. In this example, fluorescence of deeper layers may be extracted. The reflected or re-emitted light in this example may also be filtered to isolate a specific fluorescence. In another example, varying the wavelength of the illuminating light may enable detection of biophysical properties from various depths within the skin. In addition, certain chromophores, such as the various forms of hemoglobin found in blood, have specific absorption bands; thus processing of data created with different color light may yield information about chromophore distribution that may be polarization-sensitive. The wavelength dependence may be obtained in several ways: 1) illuminate sequentially with light of a single wavelength or multiple single wavelengths and collect each resultant image separately; or 2) illuminate with white light and examine the reflected or re-emitted light for individual wavelengths or a collection of individual wavelengths either during detection or during processing. Algorithms 150 may be used to obtain information from data obtained by either method by processing and analyzing one or more wavelengths of light to form a spectroscopic, polarization-based image. In an embodiment, the combination of both techniques may enable the elimination of the reflection from the surface of the skin.

In an embodiment, filtering may be employed to filter out a range of wavelengths, such as those belonging to the ultraviolet, infrared, near infrared, visible, and the like. The filter may be a digital or an analog filter. For example, captured images may be processed by software that may be able to employ digital filter techniques to process the images for analysis. For example, using software, any digital filter parameter may be selected such as a particular cutoff wavelength, a set of single wavelengths, a sampling interval, and the like. For example and without limitation, a digital filter may be used to isolate reflections of 405, 458, 488, 532, 580, and 633 nm wavelengths. In another example, an analog filter may be employed to filter the images as they are captured, such as a filter that is integral to the optics of the device 108, or as they are stored, transmitted, manipulated, processed, and the like, such as with an external analog filter. Filtering the images may result in obtaining images of underlying structures and/or a specific pattern of polarization. Filtering the images may result in the separation of the electrical and magnetic components of the reflected or re-emitted light. Filtered images may be subjected to algorithmic analysis. Filtering may eliminate reflections due to skin surface reflections by isolating specific wavelengths of light. For example, sebaceous glands may appear as bright spots in an image when only a certain wavelength of light is isolated for analysis, while isolation of a different wavelength of light enables the visualization of all the pores in the imaged area. Thus, the fluorescence from deeper layers may be isolated. Image processing may be used to count and measure changes in the sebaceous glands and pores, including count, size, activity of gland, quantity of sebum/other materials inside the sebaceous gland, quantity of sebum/other materials inside the pore, age of the contents within the gland, age of contents within the pore, amount of inflammatory processes surrounding the gland, and the like. Multiple images from different image sources may be combined for the analysis. The analysis results in function, diagnosis, prognosis of skin health, such as disposition to acne, oiliness, shine, viscosity, and the like. The analysis may be combined with color image processing (RGB analysis, for example) to determine other skin characteristics.

In an aspect of the invention, a host system 104 may comprise algorithms 150, data integration 152, analysis tools/ API's 154, a skin state 158, an expert consult 128, and the like. The skin state 158 may be a data object or characterization of skin based on tests 160, prediagnoses 162, and monitoring 164 performed by a device 108, user input, expert consult 128, other inputs 112, analysis 154, algorithms 150, and the like. The skin state 158 along with all of the underlying data and user information may be stored in a skin health record 121. In an embodiment, the host system 104 may comprise server architecture. The host system may be technology agnostic. The host system 104 may comprise one or more cloud computing, service-oriented architecture, distributed objects, and the like.

In an embodiment, expert consult 128 may provide analysis, recommendations, assessment advice, and the like. The skin image data collected as well as the pre-diagnosis, in addition with any other allied data such as physician's diagnosis, insurance, blood analysis, and the like may be referred to an expert either by the user or a practitioner, or by other users to obtain an analysis, recommendation or assessment advice. Experts could be located in geographically distant locations, and may have very different skills. For example, the skin image data and analysis may be shared at the request of another user with an herbal specialist in India, or the user may request the image data to be shared with an aging expert in France to learn of best suited skin care treatment from their experience. The expert's consultation analysis may be maintained on the host system 104 as part of the skin history record 121 and may be accessed by the user at their convenience, or shared with other users.

In an embodiment, the system 104 may be a home-based, in clinical or medical settings, at spas and salons, at a cosmetics counter and in cosmetics sales, and the like to perform skin analysis discretely and accurately in a low cost, rapid, and secure fashion. In embodiments, the device 108 may integrate with a user interface 102, online platform 129, mobile platform 124 and the like to perform analysis 154, skin state 158 record keeping, obtain referrals/analysis from a remote practitioner or algorithm 150, and the like. The home-based system 104 may allow a practitioner, who may be any qualified or unqualified person to give advice, to analyze cosmetic or non-cosmetic conditions that may be captured by an imaging device 108 or third party device 109 and give advice and recommendations on products, regimen, diet, lifestyle and the like based on inputs from questionnaires, uploaded images, and the like. The system may consist of a starter website that may be customizable for a personal business where the practitioner could organize clients' cosmetic skin health, track their regimens, recommend products, be their online advisor, and the like. This would leverage the analysis and device platform to allow a practitioner to analyze comments, images, questions, and/or concerns and the like and give advice, consultation on lifestyle improvement and tracking. A spa/ salon based system may enable personalized skin assets. For example, the spa may own the device, the device may capture images to feed a large scale display adapted to present a skin condition, and then a practitioner may be able to simulate the effect of treatment. Users may compare a skin state 158 with peers or other spa goers and generate recommendations based on what worked for them or what they bought. Desired improvements may be correlated to ingredients and most effective products / regimens 118 for the users' skin. The regimen 118 may be a feature that enables users to learn what product sequence would work best for their skin, based on a hardware-led personalized skin care assessment 122 and / or type determination 130 for the skin and product experience sharing via ranking and rating 138 and / or comments regarding product effectiveness and experience (e.g. smell, taste, feel, texture, color, etc.) collection. The regimen 118 may be a dynamic recommendation based on users' collective inputs as well as experts' inputs on products that would best suit the user's individual needs.

The spa / salon based system 104 may generate product/ service recommendations based on a skin state 158, offer one-click shopping based on recommendations and enable SKU tracking, offer wellness packages such as through a contractual relationship, provide the ability to port regimen from spa to spa, from home to spa, and the like, enable optimization of regimens/ advising such as helping practitioners tailor the length of a procedure, enable development of targeted therapies, enable clear, visual communication to clients, generate effectiveness of products/services reports, and the like. Reports may be based on or comprise correlation with other users, feedback on regimen 118, modifications of a regimen 118, skin cycle monitoring, and the like. A medical practitioner based system, such as a dermatologist, general physician, metabolist, and the like, may enable pre-diagnosis, may link to the practitioner's scheduling system, may enable pre-pricing of services, may enable follow-up tracking, and the like. A cosmetic sales or retail based system 104 may enable integration with inventory of product enabling clearing of inventory. A handheld/portable device 108 may be used at a makeup counter, in a drugstore, at a home or trade makeup show/party, and the like. Users may purchase peripherals/accessories for the device, such as a holster, charger, and the like. Users may pay-per-scan or may have a subscription scanning service and the like. The system 104 may be based in health clubs, gyms, resorts, and the like. A cosmetics manufacturing / testing based system may enable skin state-based product design, targeting skin care samples to particular consumers, and the like. The system 104 may be veterinarian based to monitor veterinary dermal- and non-dermal concerns. The system 104 may be based in a hospital, ER, military setting, and the like to enable rapid assessment of medical conditions, triaging urgent skin care, and the like. The system 104 may be agriculturally based to enable application to fruits, vegetables, and other such agricultural products. The system 104 may be used in a battlefield scenario or in an austere environment, such as in space flight, air flight, underwater, submarine, and the like, to enable wound management, battlefield diagnosis and triage, and the like. The system 104 may be research based to enable comparing any materials and their specific composition. Based on using the reading of the electrical property of the light, a user may be able to determine a similarity or difference between imaged material.

In an embodiment, determining a skin state 158 may comprise employing an analysis 154. In an embodiment, the acquired data may be analyzed by a practitioner, such as a physician, dermatologist, spa employee, clinical trial practitioner, aesthetician, cosmetologist, nutritionist, cosmetic salesperson, and the like. The practitioner may analyze the data upon acquisition, visually, with the assistance of an algorithm 150, expert consult 128, database 115, and the like. In an embodiment, the practitioner may be remote from the location of data acquisition. In an embodiment, an algorithm 150 may be used to process and analyze 154 the reflected or re-emitted light to obtain spectroscopically resolved images, either automatically or under the control of a user, practitioner, and the like. For example, to obtain a spectroscopic image of the magnetic properties of the area only, an algorithm 150 may be used to generate an image of an area of concern using the difference between the reflected polarized light, which possesses the electrical properties of the area, and the reflected diffusion light, which possesses the electromagnetic properties of the area of concern. Algorithms 150 may be rules-based software and processes to 1) analyze imaging evidence to obtain skin health, 2) correlate skin health with ingredients, medicaments, and/or products that may be best suited for the determined skin health, 3) correlate skin health with peers in a skin health community, and 4) recommend and design personalized products based on skin health and/or other like users usage experience, 5) observe measurable changes in skin health, and the like. Algorithms 150 may be automated. Algorithms 150 may be used to analyze 154 medical concerns, such as degree of suspicion of cancer, rash analysis, and the like. Algorithms 150 may be used to analyze 154 non-medical concerns, such as the effectiveness of a medical, non-medical, or cosmetic regimen 118, a pimple avoidance regimen 118, a sun-protection effectiveness, an itch prevention cream, and the like. Algorithms 150 may be useful for correlating desired improvements with ingredients and most effective products for improving or maintaining the user's skin health. The algorithm 150 may utilize a calibration scale to determine the skin structures imaged based on the angle of incidence, wavelength and intensity of the light source, an aspect of the reflected or re-emitted light, filter parameters, and the like. Algorithms 150 may be useful for determining a dermascopic effect, a luminescence effect, a spectroscopic effect, and the like. For all algorithms 150, there may be an input, an output, and functional parameters to modulate the algorithm 150. In an embodiment, analysis 154 may comprise examining at least one of: physical data and/or an image of the material using diffusion white light; physical data and/or an image of material using light of a single wavelength or multiple single wavelengths; physical data and/or an image of the material using polarized, reflected or re-emitted light of a certain angle; physical data and/or an image of the material generated using the difference between diffusion white light and polarized reflected or re-emitted light of a certain angle; physical data and/or an image of the material generated using the difference between light of a single or multiple wavelengths and polarized, reflected or re-emitted light of a certain angle; and the like. Algorithms 150 may be used with data and images generated by the device 108 or third party hardware 109. Algorithms 150 may be used with data and mages captured using any image capture device or technique, employing any kind of incident light, such as unpolarized light, polarized light, monochromatic light, diffuse light, white light, multiple single wavelength light, and the like. In embodiments, any captured data or image may be subjected to algorithmic analysis, as described herein.

In an embodiment, the algorithm 150 may be based on artificial neural networks, non-linear regression, or fuzzy logic. For example, the algorithm 150 may be used in skin lesion diagnosis based on a probabilistic framework for classification. Two kinds of data may be inputs to the neural network or to non-linear regression: numerical data such as intensity, size, numbers, and the like, and descriptive data such as white, gray, dark, and the like. Fuzzy logic may directly encode structured descriptive data in a numerical framework. Based on associative memories, learning algorithms 150, and adaptive control system behavior, neural and fuzzy machine intelligence may enable correspondence between input data taken from collected images and a biophysical skin state 158.

In an embodiment, the algorithm 150 may be based on fractal and multi-fractal analysis of images based on biophysical and spatio-temporal data. Both digital image data and spectroscopic data of skin may be analyzed using Hausdorff dimensions (fractal property) and Kolmogorov's entropy (K-entropy). Then, spectroscopic data may be divided into spatio-temporal cells and analyzed as multi-fractal objects, yielding information about a level of functional disharmony of skin structures (epidermal and dermal). Structural data of these two analyses can be correlated to determinate a one-to-one correspondence between them. Once fractal correlations between digital image data and spectroscopic data of skin are established, it may be possible to obtain information about a functional state of skin structures through multi-fractal analysis of digital image data.

In an embodiment, an algorithm 150 may be for the analysis 154 of data integrity. For example, an algorithm 150 may be able to determine if the image has been captured in high enough detail to render subsequent analyses reliable.

In an embodiment, an algorithm 150 may be useful for the analysis of skin characteristics, obtaining the biophysical properties of the skin, and determining a skin state 158. The skin state 158 may capture a combination of underlying skin structure with time-based variance. Some variation may be predictable but some may be based on a transient condition like infection, sunburn, hormonal imbalance, and the like. The algorithm 150 may be able to measure aspects such as the structure, form, concentration, number, size, state, stage, and the like of melanocytes/ melanin, hemoglobin, porphyrin, keratin, carotene, collagen, elastin, sebum, sebaceous gland activity, pores (sweat and sebaceous), wrinkles, moisture, elasticity, luminosity, all forms of the aforementioned, such as derivatives, salts, complexes, and the like. The algorithm 150 may be used to make a quantitative assessment of clinical, medical, non-medical, and cosmetic indications, such as moisture level, firmness, fine lines, wrinkle count and stage, pore size, percent of open pores, skin elasticity, skin tension lines, spots, skin color, psoriasis, allergies, red areas, general skin disorders and infections, or other skin related concerns for the user such as tumors, sunburns, rashes, scratches, pimples, acne, insect bites, itches, bleeding, injury, inflammation, photodamage, pigmentation, tone, tattoos, percent burn/ burn classification, moles (naevi, nevus), aspects of skin lesions (structure, color, dimensions/asymmetry), melanoma, dermally observed disorders and cutaneous lesions, cellulite, boils, blistering diseases, management of congenital dermal syndromes, (sub)-cutaneous mycoses, melasma, vascular conditions, rosacea, spider veins, texture, skin ulcers, wound healing, post-operative tracking, melanocytic lesions, non-melanocytic lesions, basal cell carcinoma, seborrhoic keratosis, sebum (oiliness), nail- and/or hair-related concerns, and the like. The algorithm 150 may also be useful for the analysis of and obtaining the physical properties and composition of hair, nails, biological substances, gaseous substances, food, wine, water, liquid, metal, non-metals, plastics, polymers, and the like. Either manually or as determined by an algorithm 150, a targeted wavelength or wavelengths may be employed for specific endpoint measurements.

Either a specific wavelength or multiple wavelengths may be chosen for the incident light or a specific wavelength or wavelengths may be isolated by filtering, as described herein. An algorithm 150 may determine the presence, absence, structure, form, and the like of particular skin structures based on the properties of the reflected or re-emitted light. For example, an algorithm 150 may detect which axes/ angle the light is polarized on and compare this to signature emission spectra of individual proteins/ underlying skin structures. Each skin structure may have a unique signature pattern based on the electrical and magnetic contributions of molecule(s) present in the skin structure. The algorithms 150 may identify, analyze and separate the electrical and magnetic components of the unique polarization signal, as described herein. The signals may correlate with the aggregate conformation state of molecules in the skin structure. By comparing this signal to a standard calibration signal, aspects of the underlying skin structures may be determined. The standard calibration signal may be provided by a catalog of skin structures/ molecules and their specific wavelength of observation. The catalog may be developed by the technique described herein or any other spectroscopic technique. For example, to determine moisture levels in the skin, an algorithm 150 may determine a ratio of the reflected polarized light and reflected diffusion light and correlate the ratio with a moisture level. Ideally, close to 100% polarized light may be generated from reflections, however if a portion of the reflected or re-emitted light is diffusion light, such as 95% polarized, 5% diffusion, the amount of diffused light may be correlated with a level of moisture. Incident unpolarized light may interact with a skin structure and lead to varying amounts of polarization of the reflected or refracted light. This polarized reflected or refracted light strength may be measured. This polarization may be as much as 100 percent, however, the reflected polarized strength may even be less than 100% in some cases. The incident angle and the imaged material would help determine the maximum strength possible for the polarization of the reflected or re-emitted light. It should be understood that there may be a maximum amount of polarization with a maximum of 100% for a particular incident angle, but any amount of polarization ranging from 0 to 100% polarized may be expected from the light reflected by any skin structure. The underlying cause for the differences in reflection may be due to the ratio of the captured and free water in the skin. To determine elasticity, an algorithm 150 may determine the concentration of elastin per area of concern. To determine luminosity, an algorithm 150 may combine moisture levels and skin color into a single, objective assessment. Objective measures may be correlated with an expert grading scale or other external measure. To determine firmness/ tightness, an algorithm 150 may combine an assessment of collagen and elastin concentrations in an area of concern along with the activity of sebaceous glands (as measured by number of glands, percent open/closed, level of clog/ fill). The algorithm 150 may be able to overlay varying wavelengths and intensities and spectroscopic techniques, such as reflectance, excitation/emission, and the like. The algorithm 150 may be able to process and analyze 154 images collected by the device 108 or any other imaging device using unpolarized light, polarized light, or a combination thereof. The algorithm 150 may be able to process and analyze 154 many different types of images, such as thermoelectromagnetic (TEM) images or electromagnetic (EM) images, images collected with incident polarized light, traditional dermoscopy images, spectroscopically resolved images, conventional images, harmonized light images, and the like. The algorithm 150 may be able to calculate a variance measurement of skin state 158 over time. Determining a skin state 158 may also include, in addition to the processing and analysis of images of the skin for various measures and endpoints as described herein, a visual analysis of the images, user entered information, and third party information, such as lifestyle, smoking history, exercise habits, diet, allergies, and the like. For example, a user may enter anecdotal information, such as medication they may be taking, recent overexposure to sun, stage in a menstrual cycle, and the like.

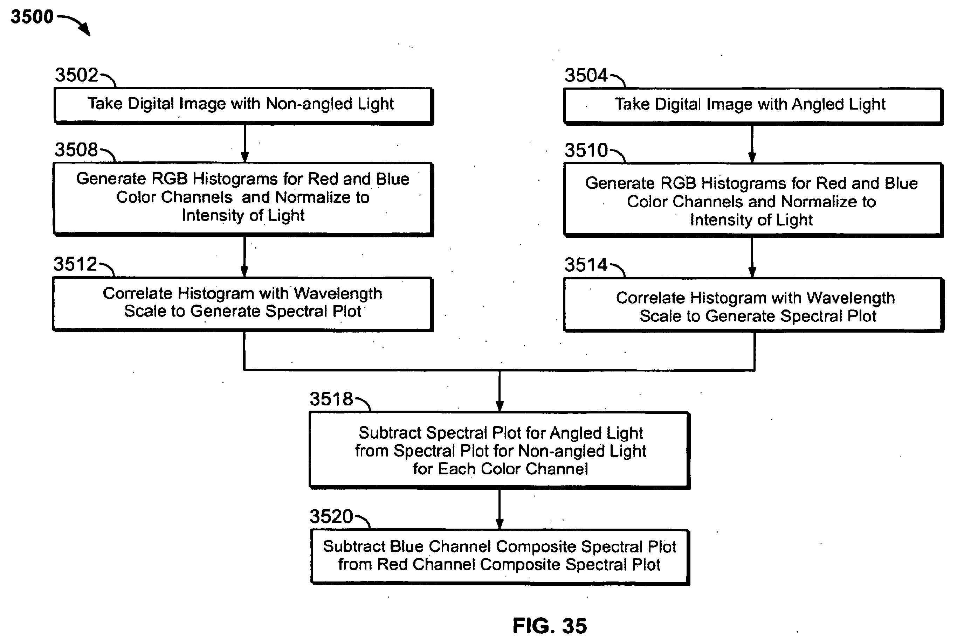

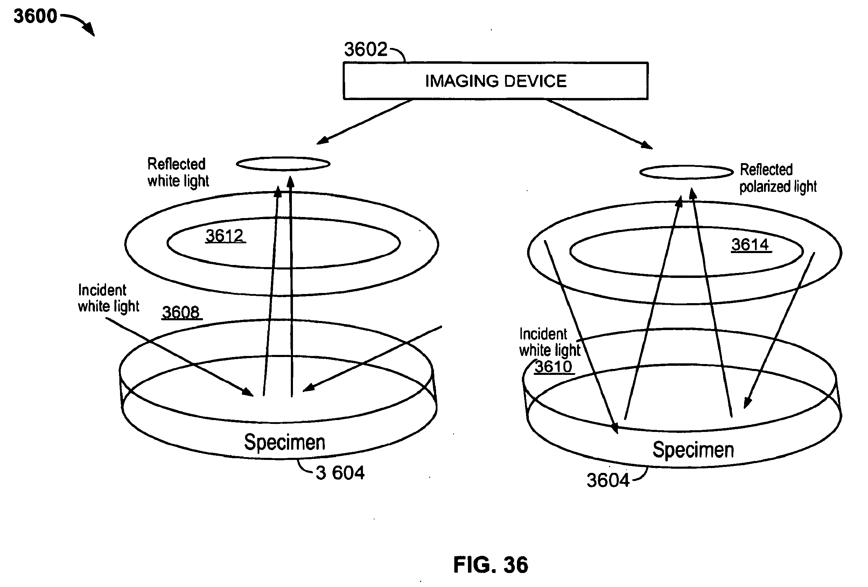

Referring to Fig. 35, in an embodiment, an algorithm 150 may comprise spectral convolution of digital images taken with: 1) "angled white light", or white light incident on an angle sufficient to produce a polarized reflection; and 2) "non-angled white light", or white light incident on an angle that produces substantially no polarized reflections. While the foregoing discussion will focus on skin as the primary specimen, it should be understood that any specimen, such as material characterized by covalence effects, ionic effects, and hydrogen bond effects, including skin, hair, biological materials, foodstuffs, liquid, wine, metallic materials, non-metallic materials, and the like may be specimens for the algorithm 150. Briefly, a digital image of a specimen is captured with non-angled light 3502 and angled light 3504, blue and red color channel histograms are generated for each image 3508, 3510 and are normalized to the relative intensity of the light, and the color channel histograms are correlated to a wavelength scale 3512, 2514. The spectral convolution proceeds in two steps. The first step involves subtracting, for each of the red and blue color channels, the color channel histogram for angled light from the color channel histogram for non-angled light 3518. Two composite histograms are generated, the blue color channel composite histogram and the red color channel composite histogram. The second step of the spectral convolution involves subtracting the blue channel composite histogram from the red channel composite histogram 3520. Continuing to refer to Fig. 35 throughout the discussion of Figs. 36 through 43, the various steps of the algorithm will now be described in greater detail.

Referring now to Fig. 36, a specimen 3604, which may be any suitable material for imaging as described previously, may be illuminated with non-angled white light 3608 and angled white light 3610. As described previously herein, varying the angle of incidence affects the depth of penetration of the light to various skin structures. For each skin structure which may correspond to a particular known depth within the skin, there may be an angle of incidence which produces a polarized reflection. By analyzing the reflected or re-emitted light, either polarized 3614 and/or diffusion 3612, captured by an imaging device 3602, information on the underlying skin structures responsible for the reflection may be obtained. The term "angled white light" 3610 refers to incident white light that is directed towards the specimen at an angle sufficient to produce a polarized reflection. The term "non-angled white light" refers to incident white light that is not directed at a specific angle towards the specimen and is diffuse. In this case, the non-angled white light may produce reflected white light, polarized light, or a combination thereof. In an embodiment, reflected polarized light generated by non-angled white light may be of a different characteristic than polarized light generated by angled white light.

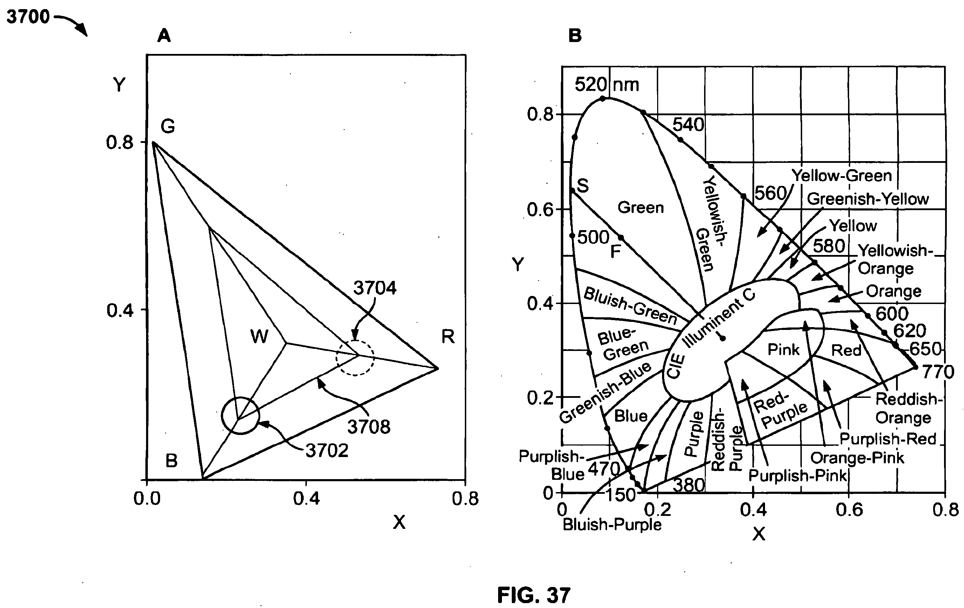

Referring now to Fig. 37, Maxwell's color triangle, in Fig. 37B, may facilitate an understanding of the nature of white light. Maxwell's color triangle depicts the complete visible color spectrum, with reference to specific wavelengths. In order to establish a mathematical coordinate system for the RGB color space, a simplified version is used with straight lines, shown in Fig. 37A. Each of the vertices of the outer triangle corresponds to an ideal color, either ideal green, red, or blue going clockwise from the top. Along the sides of a Maxwell triangle mixing of two of the three color components occurs with every possible proportion. As one travels from the side towards the center, the third primary color becomes increasingly important. Near the center at the "equal energy" point, E, a true white is seen, with radial axes extending to each of the three vertices. Mixing of the full intensity of red, green, and blue gives this true white. Thus, every point on the triangle is a result of a mixture of at least one of red, green, and blue, including the point representing white light. For example, the solid circle 3702 represents a point in color that is between pure/dark blue and pure white. Similarly, the dashed circle 3704 represents a point in color that is between pure/dark red and pure white. Using digital photos of white paper, the coordinate system may be validated, as represented by the internal triangle 3708. The internal triangle 3708 validates the system when the sides are parallel to the limits of the color space lines of the original coordinate system. If they are not parallel, then the coordinate system is not valid.

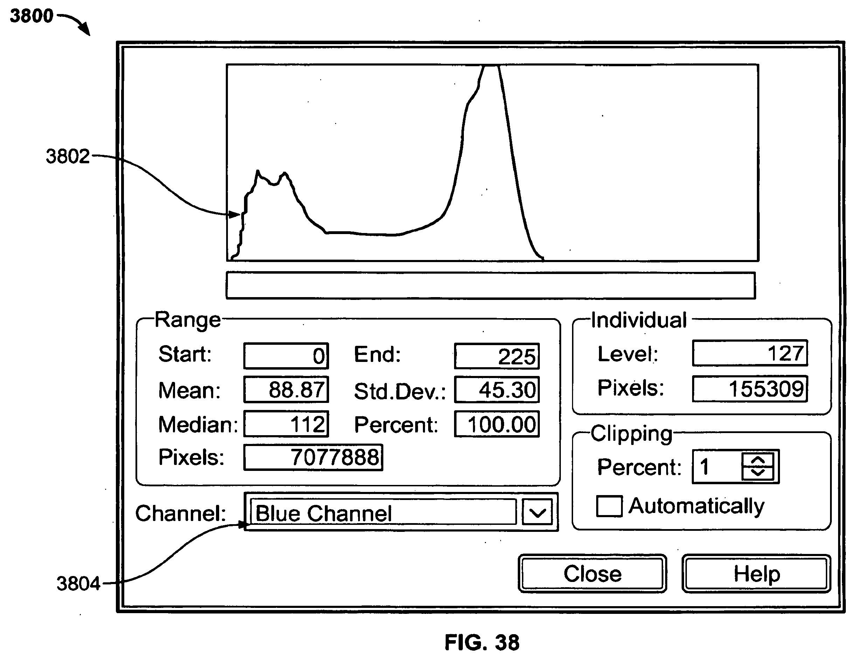

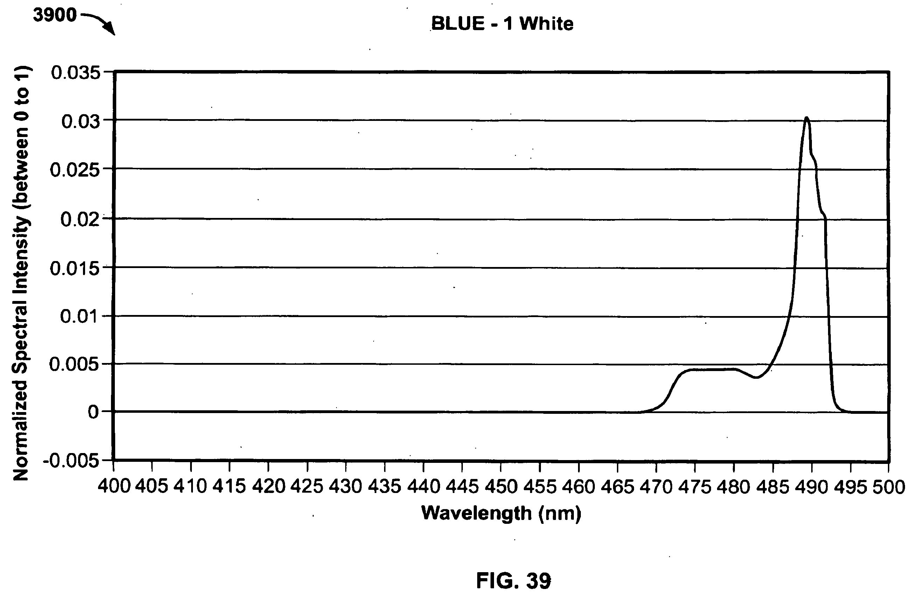

Referring now to Fig. 38, an RGB histogram for each color channel is generated for each of the images. An RGB digital image has three color channels: red, green, and blue. Each of these channels may be examined and analyzed separately. A blue color channel histogram is generated for the image taken with non-angled white light and another blue color channel histogram is generated for the image taken with angled white light. Similarly, a red color channel histogram is generated for the image taken with non-angled white light and another red color channel histogram is generated for the image taken with angled white light. For example, an automated system may be used to generate the histograms for each color channel, as shown in Fig. 38. By simply specifying which channel 3804 a user may wish to examine, a histogram 3802 may be generated for that channel. The histogram may be normalized to the relative intensity of the light. Normalizing the histograms to the intensity of incident light is important to be able to process the histograms generated from different images. Referring now to Fig. 39, the RGB color channel histograms are then correlated to a specific wavelength scale to generate RGB color channel spectral plots.

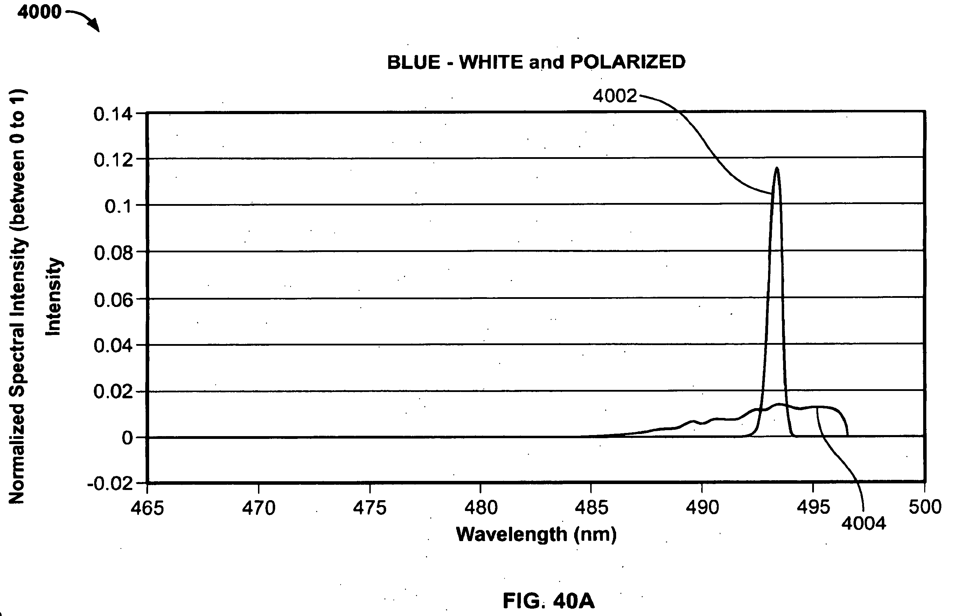

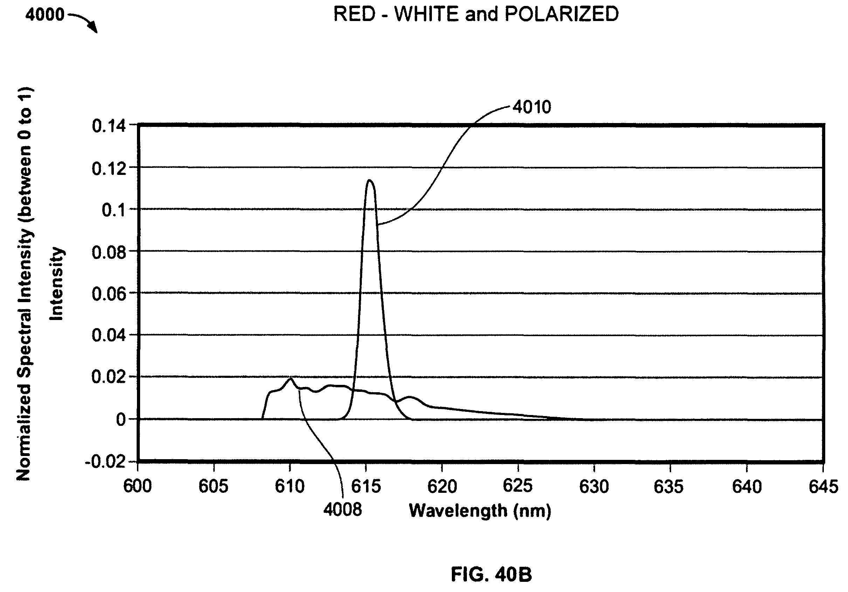

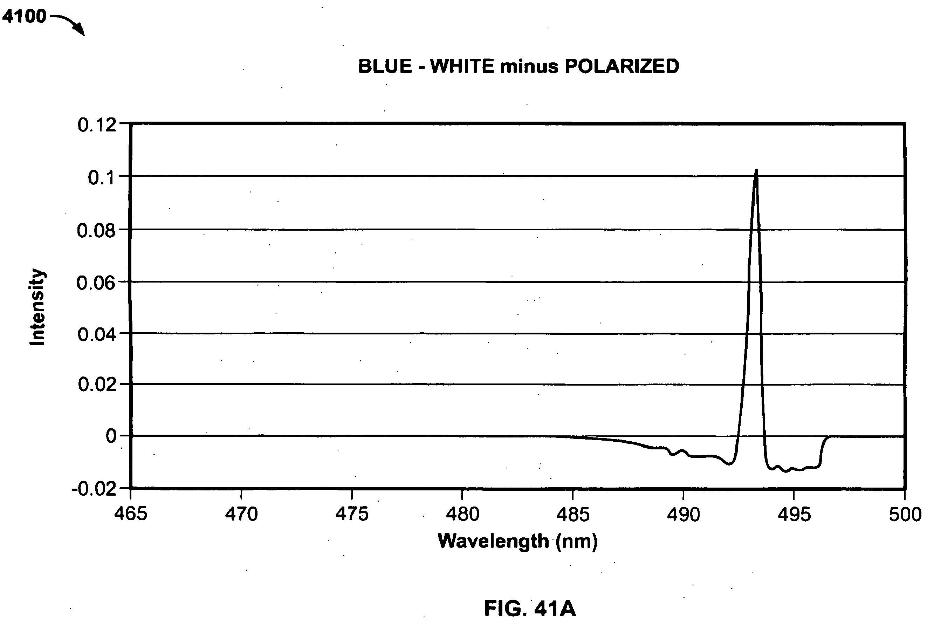

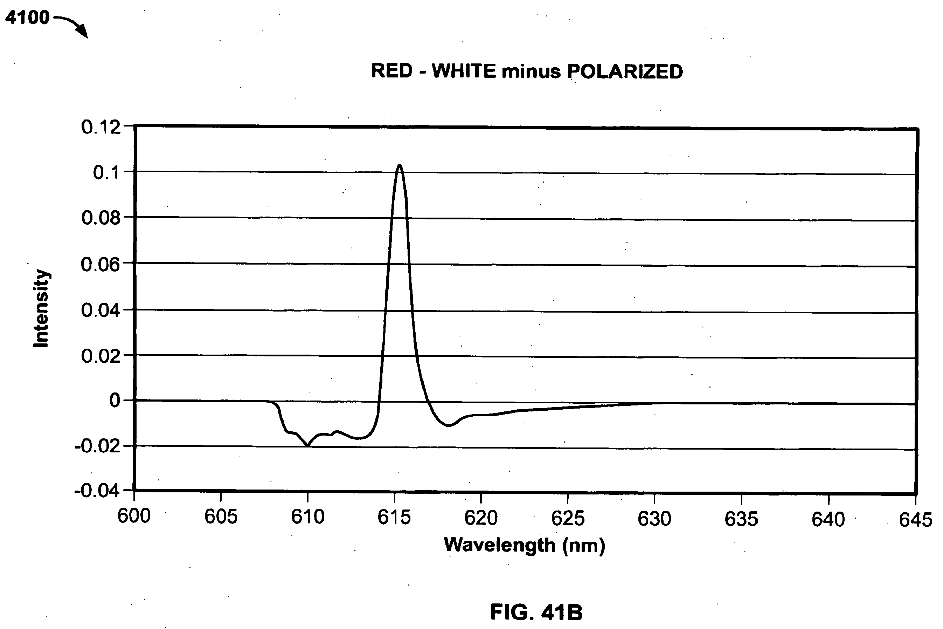

Referring now to Fig. 40, the data from the pair of images are then combined mathematically in two steps. In the first step, the blue color channel spectral plot generated from the image taken with angled white light 4004 is subtracted from the blue color channel spectral plot generated from the image taken with non-angled white light 4002 to generate a blue color channel composite spectral plot. The two spectral plots 4002, 4004 are shown first overlaid in Fig. 40A and then subtracted in Fig. 41A. Similarly, the red color channel spectral plot generated from the image taken with angled white light 4008 is subtracted from the red color channel spectral plot generated from the image taken with non-angled white light 4010 to generate a red color channel composite spectral plot. The two spectral plots 4008, 4010 are shown first overlaid in Fig. 40B and then subtracted in Fig. 41B. Subtraction may be facilitated by aligning the spectral plots by wavelength and mathematically subtracted the normalized intensities at each wavelength. For example, if the intensity is 0.005 at 470 nm for the blue channel spectral plot from angled white light and the intensity at the same wavelength of the blue channel spectral plot from non-angled white light is 0.003, the resultant spectral plot would comprise an intensity of -0.002 at 470 nm. The specific intensities and wavelengths in the spectral plots reflect the specific properties of the underlying material and the angle at which the material was exposed to light.

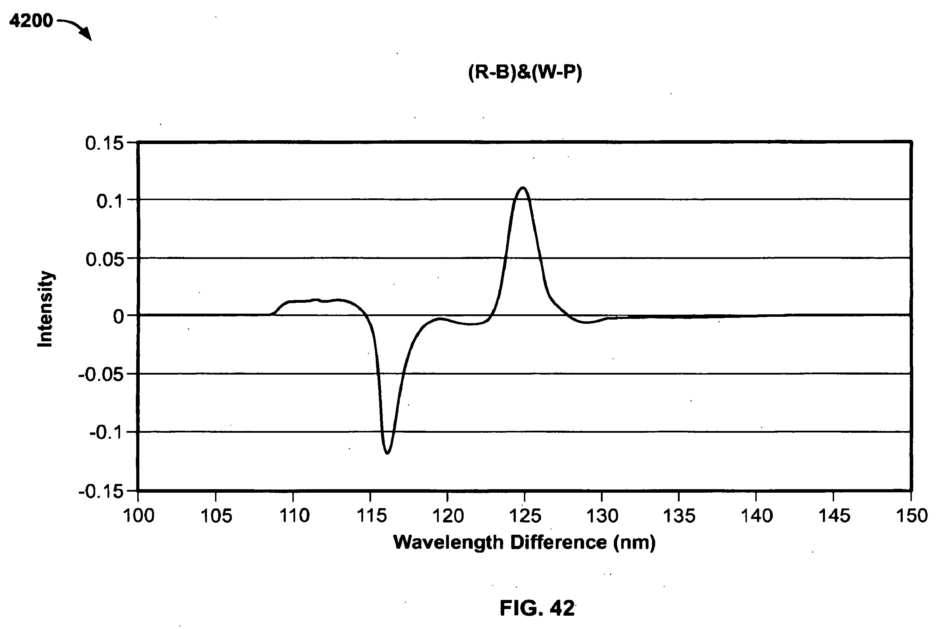

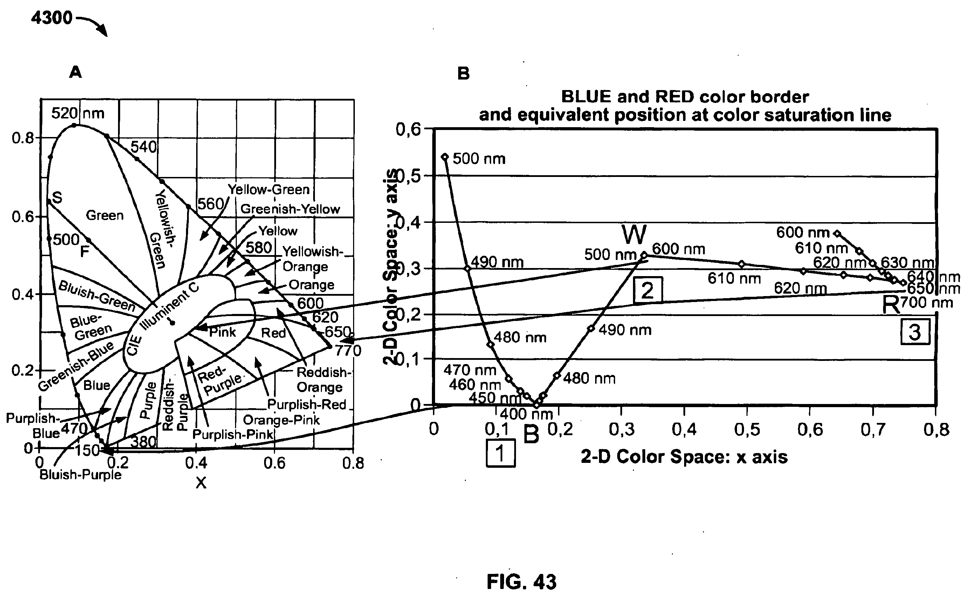

Referring now to Fig. 42, the two color channel composite, normalized spectral plots are then combined to create a unique spectral signature of the specimen. The normalized, composite blue channel spectral plot is subtracted from the normalized, composite red channel spectral plot. The scale is determined as a difference in wavelengths between the red and blue color images, starting from the darkest point in both colors. This scale is based on the mathematical coordinate system for Maxwell's color triangle. For example, and referring to Fig. 43, the lower part of Maxwell's color triangle is shown plotted out in Fig. 43B, with arrows indicating the correspondence in the plot with the position on the color triangle shown in Fig. 43A. Position 1 in the plot corresponds to ideal blue in Maxwell's color triangle, position 2 corresponds to true white, and position 3 corresponds to ideal red. Points 1 and 3 are aligned when convoluting the composite spectral plots to obtain the spectral signature, hence the unit scale on the convoluted histogram is a difference of wavelength (e.g. 500-400nm to 700-400nm).

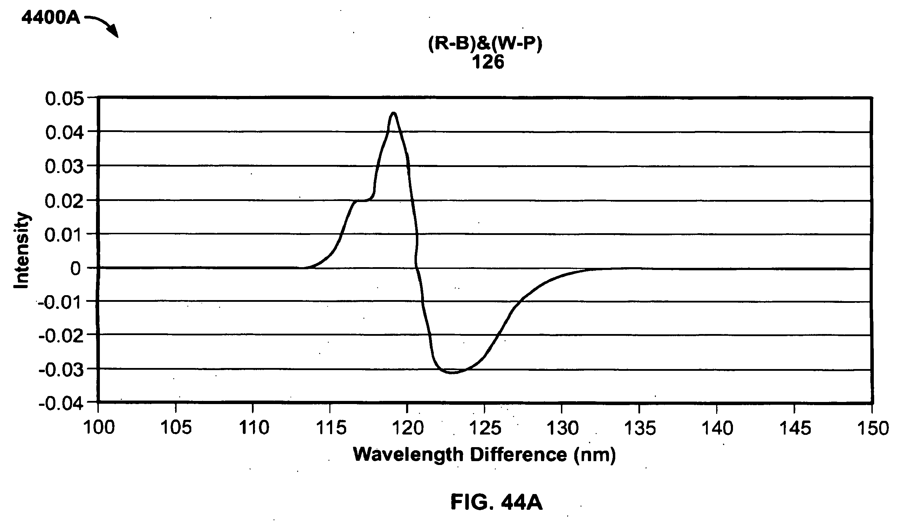

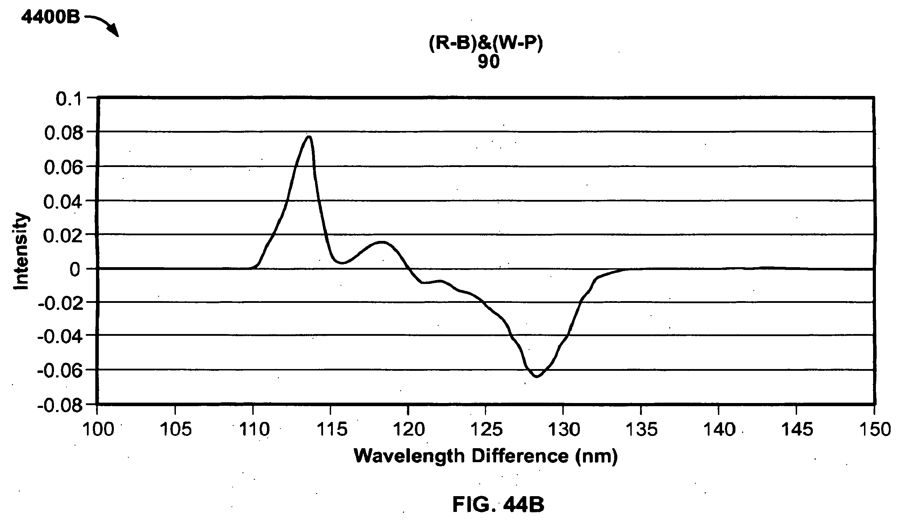

The spectral signature obtained may be analyzed for a number of characteristics, such as number of peaks and troughs, amplitude and shape of peaks and intermediate structures and patterns, and the like. Various mathematical, visual, and algorithm processing techniques may be used to process and analyze the spectral signatures. The spectral signatures obtained for various specimens may be unique, for example, the spectral signature in Fig. 44A is for light skin while the spectral signature in Fig. 44B is for dark skin.

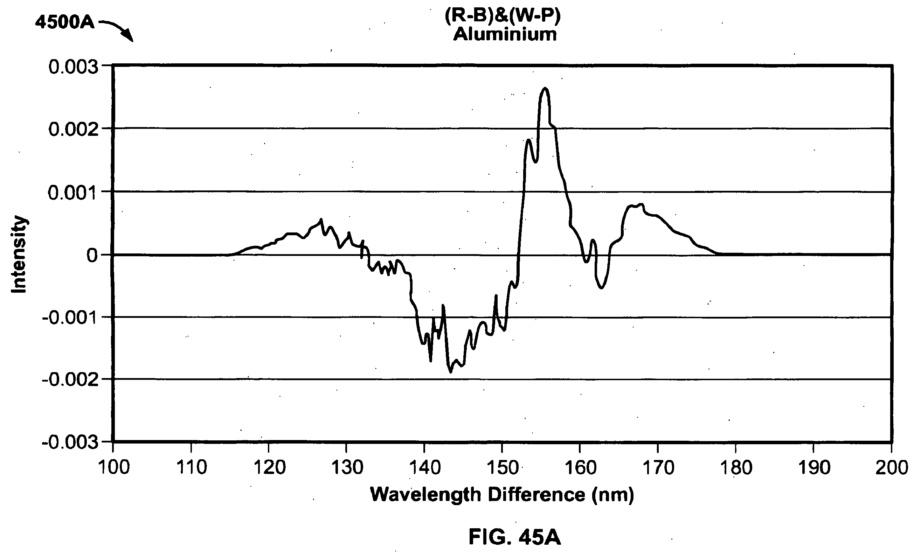

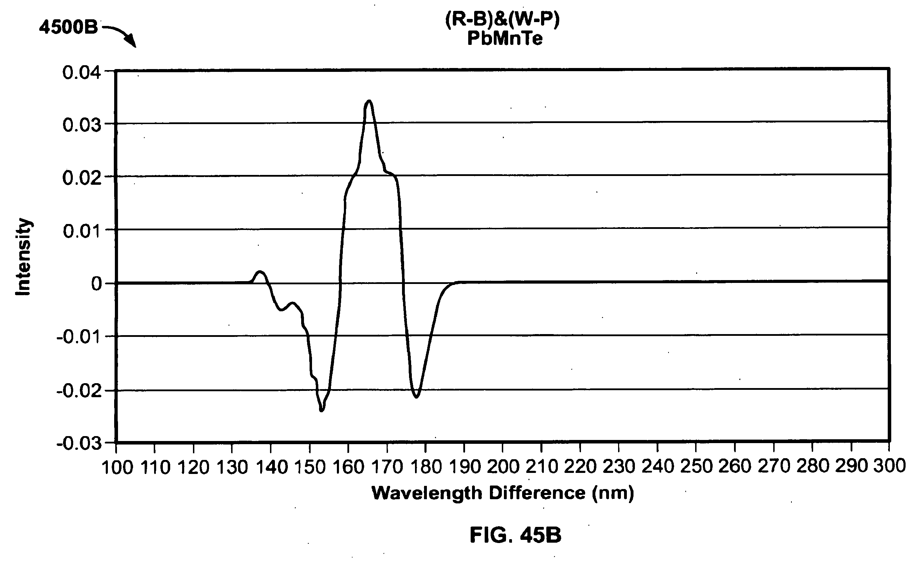

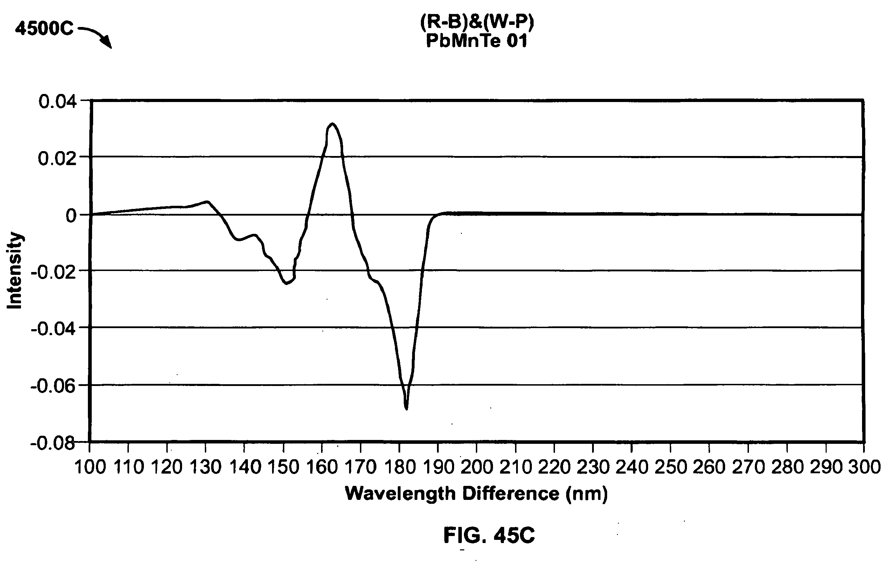

In an embodiment, the algorithm may be used for identifying metal composition, purity, strength, and the like. For example, the spectral signature may be used to distinguish between metals. The spectral signature in Fig. 45A is for a pure metal, aluminum, while the spectral signature in Fig. 45B is for an alloy of metals, PbMnTe. The spectral signature may also be used to distinguish between similar substances with different compositions. For example, the spectral signatures in Fig. 45B and Fig. 45C are both for the PbMnTe alloy but the alloy of Fig, 45B is of a different composition as compared to the one in Fig. 45A.

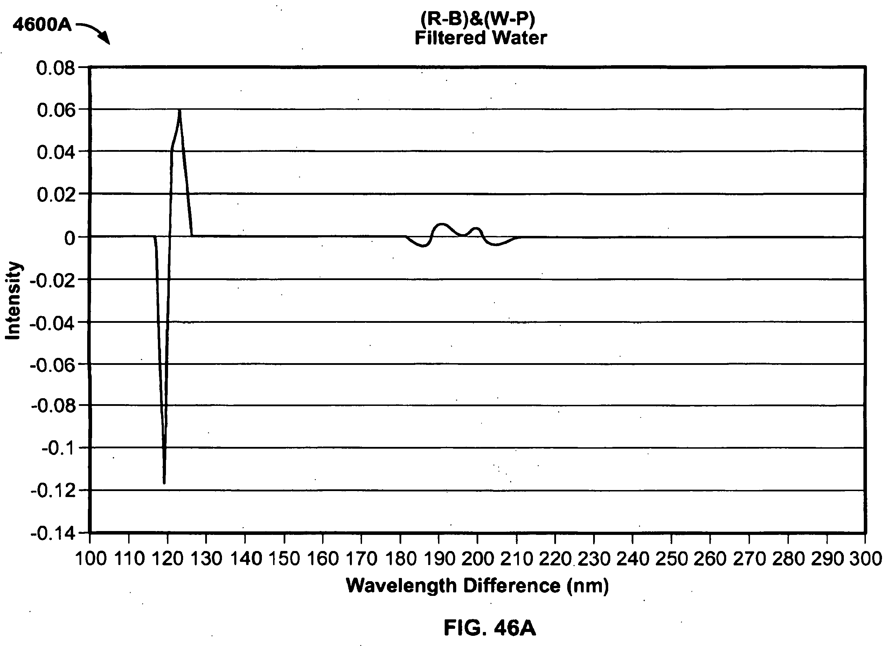

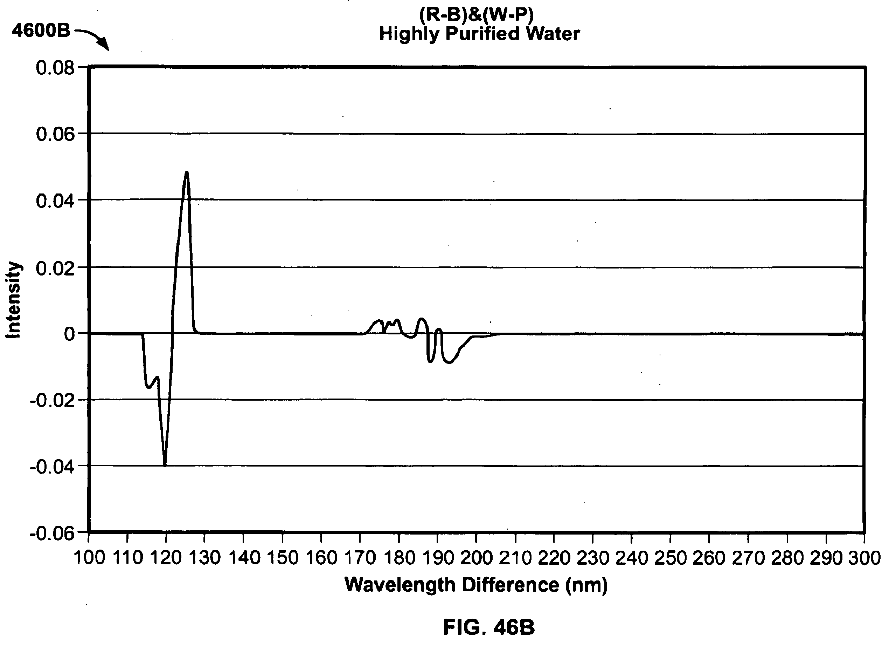

In an embodiment, the algorithm 150 may be used to analyze water quality, composition, purity, and the like. For example, the spectral signature for filtered water is shown in Fig. 46A in comparison with the spectral signature for highly purified water, shown in Fig. 46B.

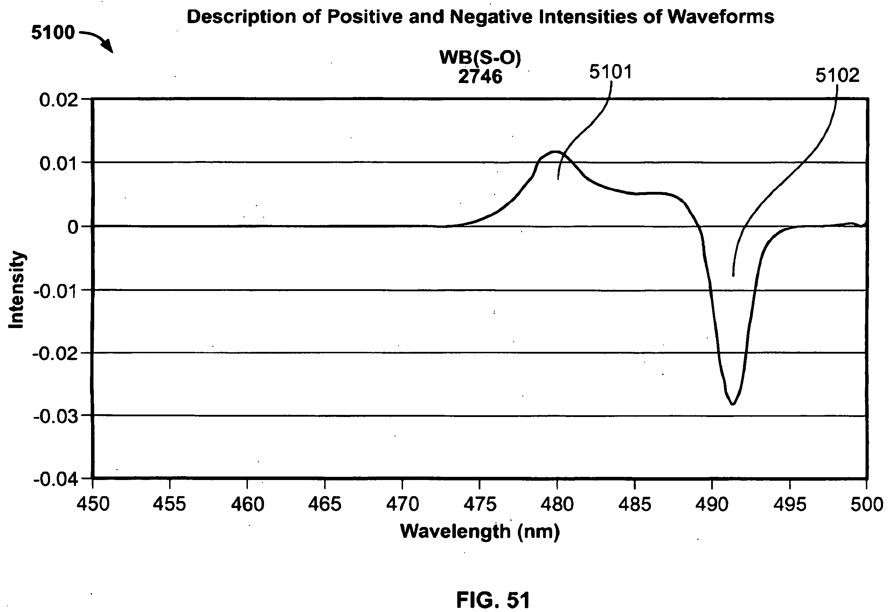

The spectral signature may further be enhanced by subtracting the spectral contribution attributable to the source light from the reflected light spectrum in order to normalize the spectral signature to specific skin conditions. For example the spectral signatures in Figures 51 through 54 may be normalized by subtracting the source spectral signature from the reflected light spectral signature. By subtracting the source spectral signature, the resulting spectral waveform is normalized to only the changes in the skin from the interaction with incident light. In this way, specific type of incident light may be used which may be more amenable to detecting certain structures, compositions, or conditions. In some embodiments, a spectral signature for the subtraction of RGB histograms for angled light from non-angled light may be calculated and used to subtract from the final spectral signature for the material.

Other convolutions may be possible, such as for a yellow color channel or some other color channel. Additionally, pre-determined convolutions may also be possible.

Referring now to Figure 51, positive intensities 5101 represent a net reflection or emission at specific wavelengths based on material characteristics while negative intensities 5102 represent a net absorption from the source light's spectral signature. Negative intensity 5102 indicates no absorption of source light at specific wavelengths based on material characteristics. The source may be selected for use in examining specific biophysical or material criteria in order to produce a specific waveform for analysis.

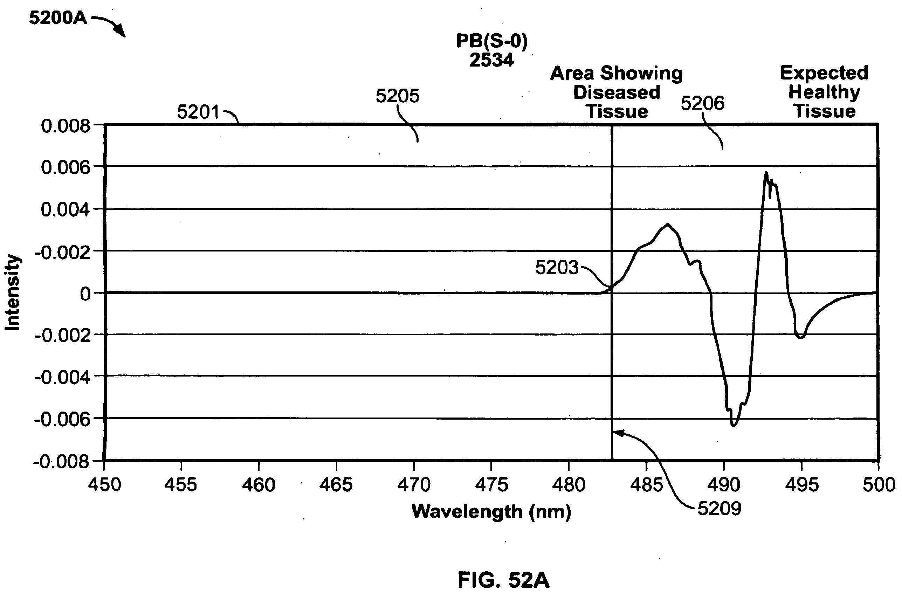

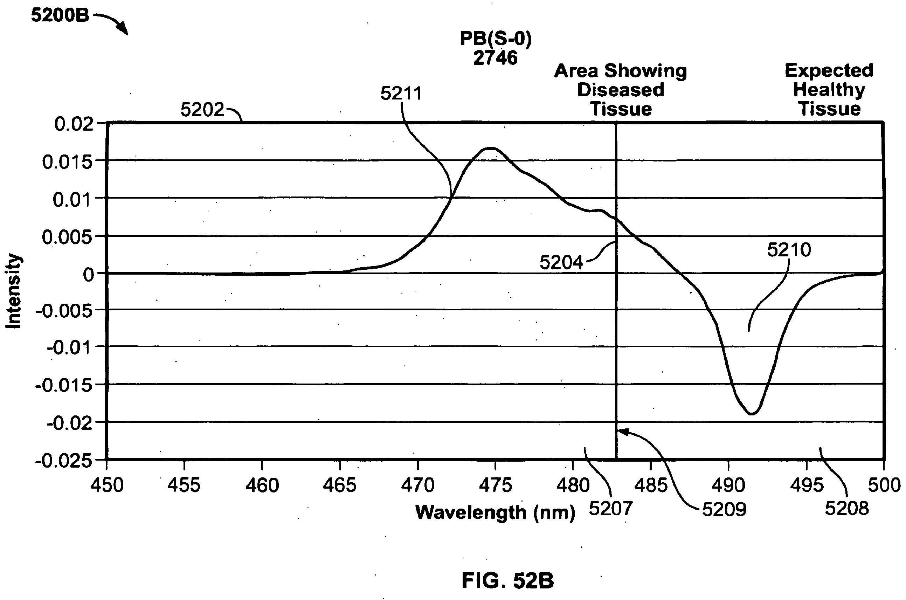

Referring now to Figure 52, it is possible to determine changes in skin state 158 using spectral characteristics of specifically selected light sources based on specific biophysical criteria. Figure 52 shows a comparison of PB(S-O) signatures showing an example for differences between benign/healthy expected tissues and diseased tissue. Changes, such as in the 462nm-485nm range in Fig. 52, such as absorption or emission within the spectral diagram may correspond to additional changes in tissue processes, tissue activity, or presence of other molecules that indicate a changed state of skin. By measuring these changes, it is possible to determine healthy and diseased or disturbed states of the skin. The characterization of healthy tissue based on emission and or absorption may be determined at a specific reference wavelength 5209 that is based on the source light selection. For example, the spectral signature of healthy skin 5201 using a specific source light shows little or no absorption or emission in the spectral range 5205. The spectral diagram shows normal spectral characteristics 5206 right of the reference wavelength at line 5203. Additionally, characteristics in the area 5207 to the left of the reference wavelength at the line 5204 indicate diseased characteristics due to re-emission or emission 5211, while the area 5208 to the right of the line 5204 indicates absorption 5210. The area 5207 corresponding to wavelengths 462nm-485nm shows additional activity due to additional changes in tissue processes, activity, or presence of other molecules that indicated a changed state of skin. The size and shape of peaks, troughs, curves, frequency, spacing, specific sections of wavelength differences, and the like may also correspond to concentrations of molecules, stages of disease progression, skin characteristics, and the like.

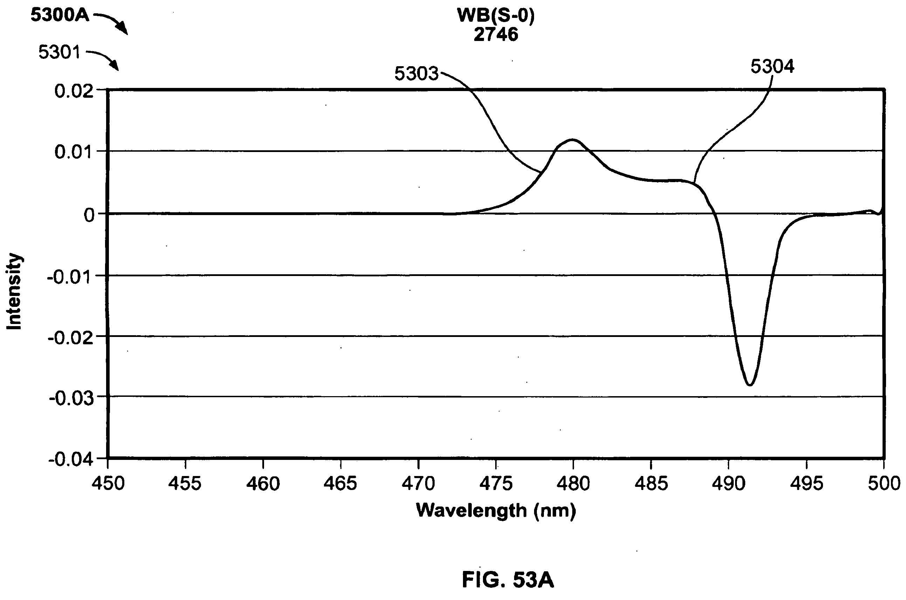

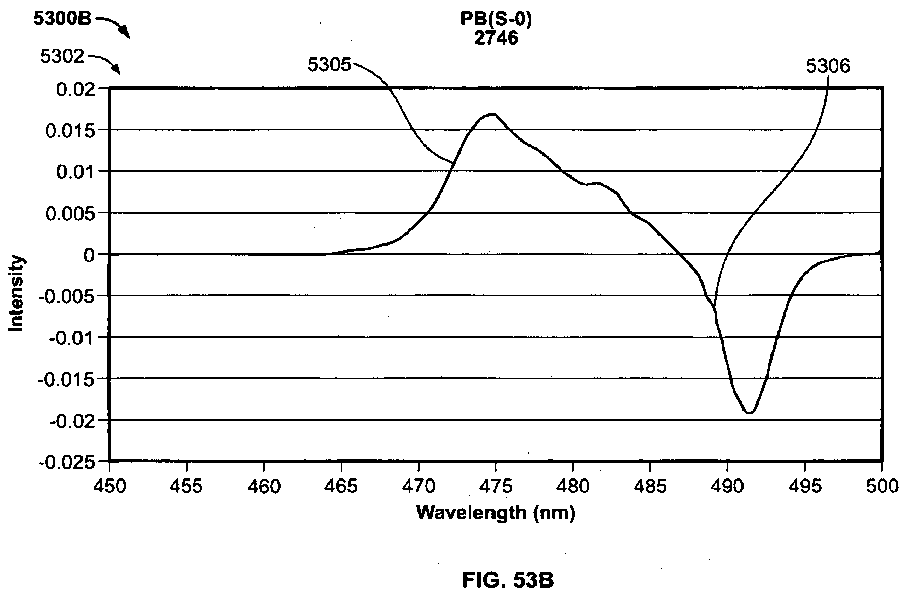

In an embodiment, the algorithm 150 may only use reflected polarized light due to increased selectivity for specific biophysical or material characteristics. For example and referring to Fig 53, the reflected polarized and/or emitted polarized light spectral signature 5302 may be much more sensitive to certain biophysical characteristics than simple white light convolution 5301. Figure 53 depicts the spectral signatures for malignant melanocytic lesions. The spectral diagram showing emission 5305 in the polarized 5302 spectral signature is much taller than the spectral diagram showing emission 5303 in the nonpolarized 5301 spectral signature. Similarly, the spectral diagram showing absorption 5306 in the polarized 5302 spectral signature is much deeper than the spectral diagram showing emission 5304 in the nonpolarized 5301 spectral signature.

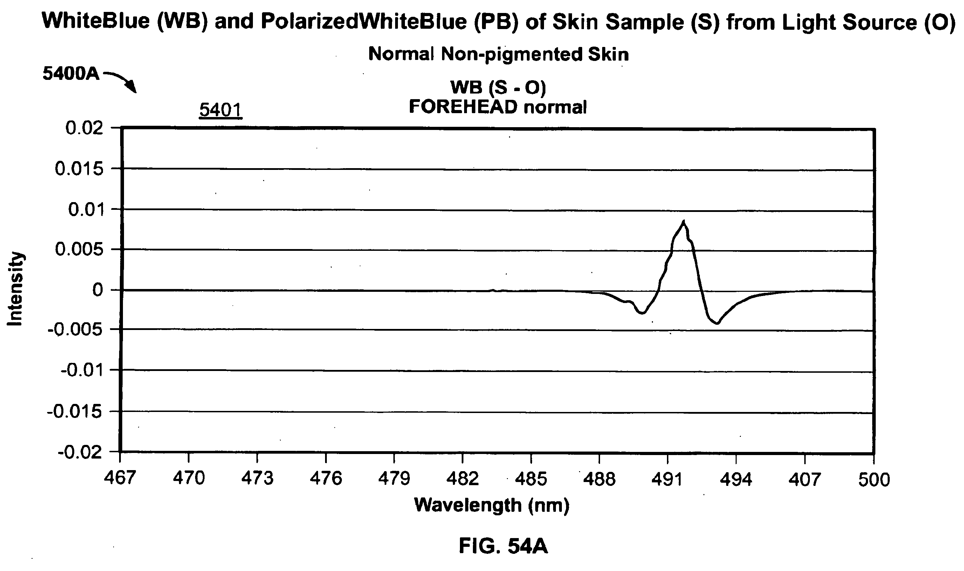

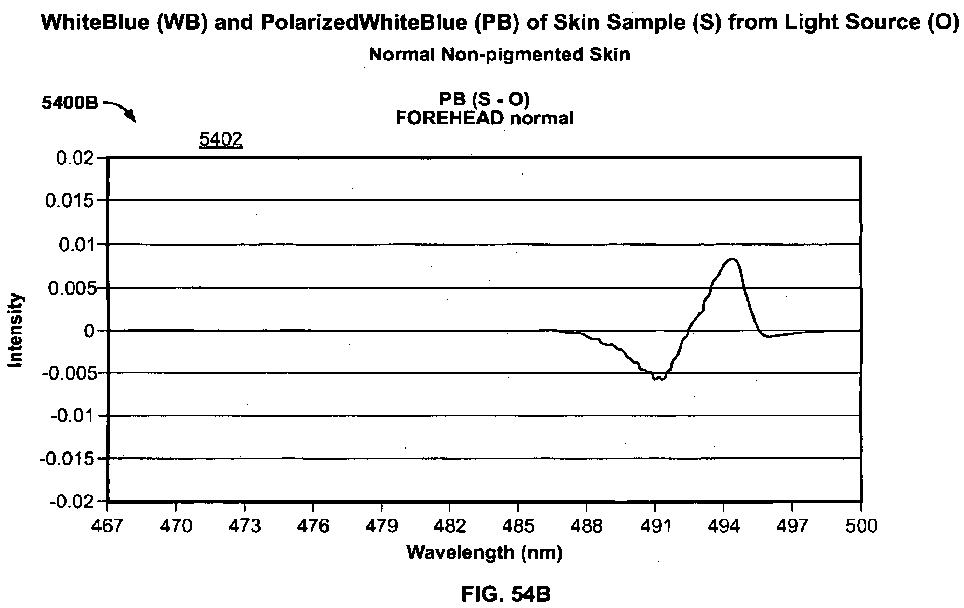

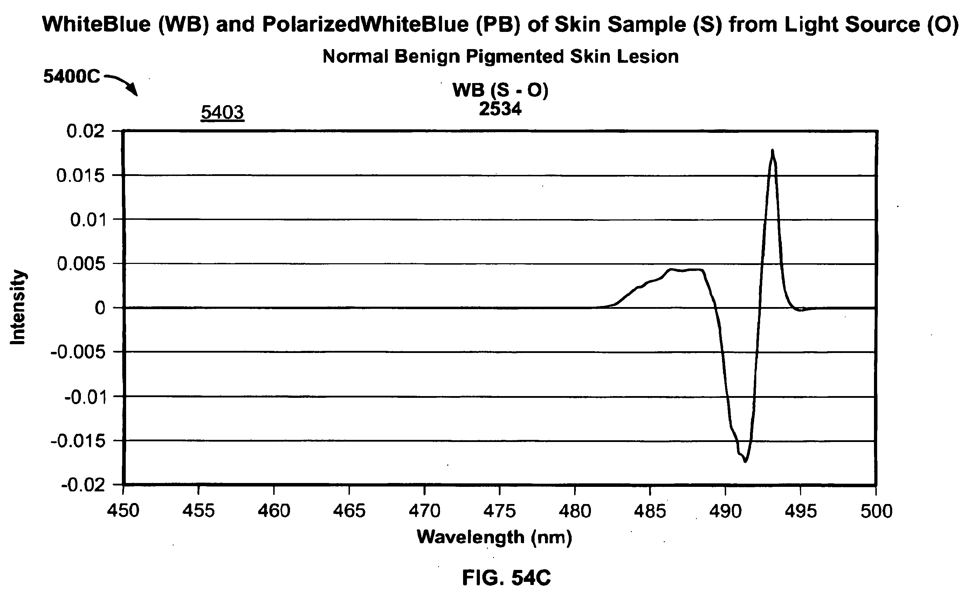

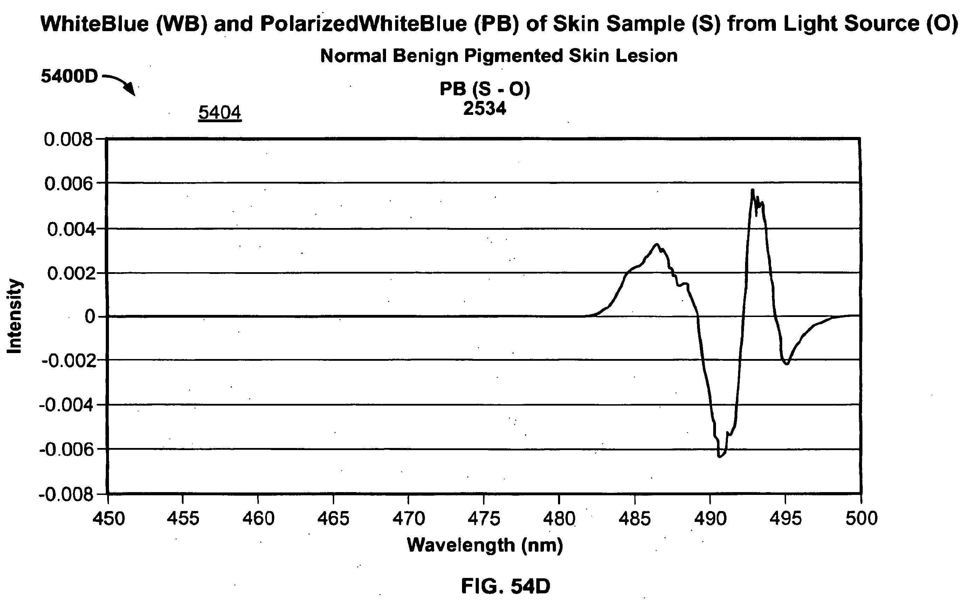

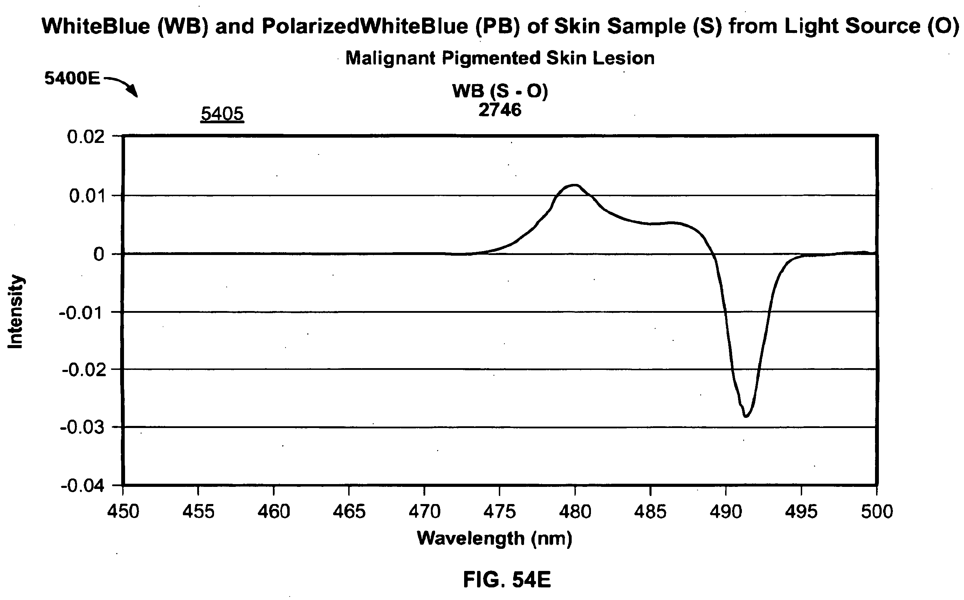

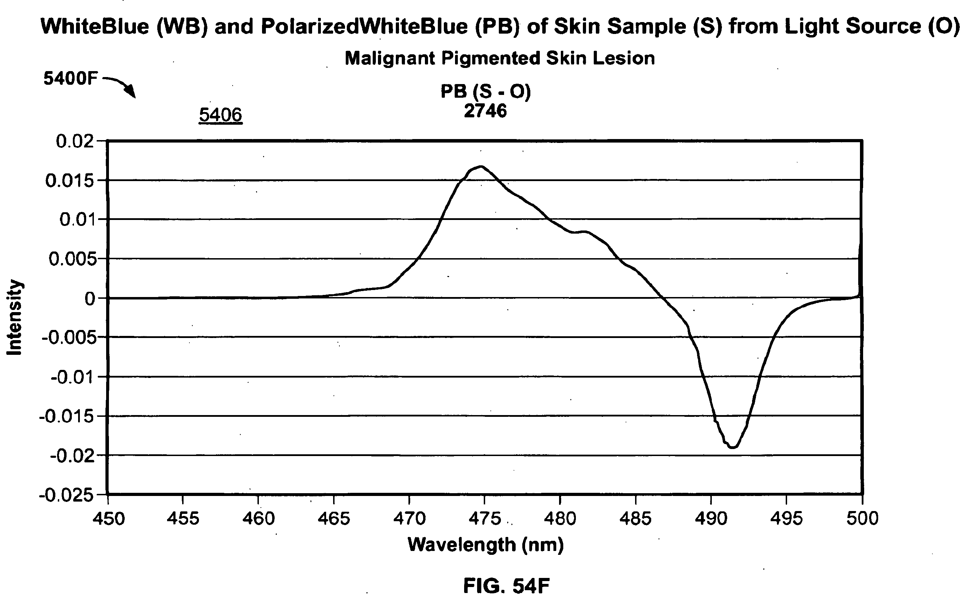

In an embodiment, the algorithm 150 may be used to analyze healthy and non-healthy or malignant skin. For example, the spectral signatures for healthy, non-pigmented skin 5401 and 5402, healthy pigmented skin 5403 and 5404, and malignant pigmented skin 5405 and 5406 are shown in Fig. 54. Both polarized (bottom) and white light (top) spectral signature convolutions are shown for purposes of comparison. The spectral signature of normal, healthy skin 5401 and 5402 shows very little absorption or emission relative to the source light spectrum around referent wavelength 485nm. Similarly, the healthy, benign pigmented skin lesion 5403 and 5404 shows very little absorption or emission to the left or right of the reference wavelength 485nm. The malignant tissue, however, clearly shows absorption and emission effects around the referent wavelengths with higher amplitudes and shifting of the spectral diagram peaks and valleys.

In embodiments, these spectroscopic techniques may be useful for a variety of analytical tests where the test substrate comprises a light-sensitive component.

In an embodiment, elements of the waveform may be tagged and tracked over time in order to track changes in the characteristics of the material or specimen, such as peaks, troughs, curves, frequency, spacing, specific sections of wavelength differences, and the like.

In an embodiment, the algorithm 150 may be incorporated for automated measurement as part of an integrated device that conducts surface analysis, such as a skin imaging device or metal testing device. In an embodiment, the algorithm 150 may be part of a remote analysis system whereby a surface imaging device may capture images and send them to a processing center where the algorithmic computations may be made.

In an embodiment, the algorithm 150 may be used for the analysis of hair in order to determine the health of hair follicles, composition, and the like.

In an embodiment, the algorithm 150 may be used for the counterfeit analysis of money. For example, a unique signature may be created for each series of appointment and/or issue.

In an embodiment, the algorithm 150 may be useful for the analysis of anti-perspirant effectiveness. In certain cases, axillary odor may be an indication of sickness or some other medical condition, such as lymphoma, apocrine gland sweating, hyperhidrosis, hydradenitis suppurativa, or other sweat related medical problems. The algorithm 150 may be useful in determining a scale of deodorant effectiveness based on an individual's specific sweat gland activity and type. The algorithm 150 may enable measuring the activity of sweat glands located in the axilla, feet, palms, and the like. The algorithmic analysis may enable the classification of sweat glands and may enable the suggestion of appropriate products/ingredients for treatment. The algorithm 150 may be able to determine the effectiveness of an antiperspirant based on the impact on sweat gland activity.

In an embodiment, the algorithm 150 may be useful for determining a veterinary condition, such as Mad Cow disease. For example, imaging the tongue of a cow or any mucosal or dermal area where the disease may manifest may allow for the detection of a disease state using the algorithm 150. White light imaging, as described herein, in combination with UV imaging may facilitate detection of a Mad Cow disease state.

In an embodiment, the algorithm 150 may be useful for monitoring post-operative cosmetic concerns, such as stretch mark progression and diminishment, and the like.

In an embodiment, the algorithm 150 may be useful for predicting and monitoring secretion from the mammary glands of lactating women. If milk production is predicted to be low based on the algorithmic analysis, suggestions may be made to increase milk production.

In an embodiment, an algorithm 150 for determining a skin state 158 may facilitate measuring, tracking, and monitoring a skin state 158 as well as the effectiveness of a regimen 118, topical and/or systemic therapies, avoidance routines, diet, and the like. For example, the skin state 158 may be measured at intervals and current measurements may be compared to previous measurements to determine skin health changes. As will be further described herein, the results from the algorithm 150 may feed into a recommendation engine to provide feedback and modifications to aspects of the regimen 118.

In an embodiment, an algorithm 150 for determining a skin state 158 may enable a diagnosis. The diagnosis may be an early diagnosis by distinguishing between critical and non-critical indications. For example, the algorithm 150 may be able to distinguish between a minor sunburn and a third degree sunburn requiring medical attention. Use of the device 108 to capture images enables a user to readily transmit the images to any practitioner for remote assessment, to track progression of a skin condition, rapidly compare images to previous images, other user images or third party images, such as images in a dermascopic database 115, and the like, and to make an immediate assessment with no need for historical knowledge, and the like. Historical data and the results of modeling tools 132 may be compared to the images to assist in analysis, either by an algorithm 150, a practitioner, or a practitioner employing an algorithm. Also, in addition to images, user input in the form of audio, video, or text anecdotes describing the issue, such as a level of pain, a sensation of heat, an itchiness, and the like, may be useful in analyzing the images to determine a diagnosis. The algorithm 150 may enable non-linear regression, such as principal component analysis (PCA), which may be a biomedical analysis used in conjunction with spectrometric analysis for analyzing medical and health conditions. The algorithm 150 may enable a simple pattern analysis for diagnosis. The algorithm 150 may be able to determine the thermo- and electroconductivity conditions of skin lesions. In an embodiment, the algorithm 150 may be able to diagnose a melanocytic lesion by examining the images for the relationship of changes in collagen and porphyrin, as a change in collagen but not porphyrin may indicate a change from a normal lesion to a dysplastic lesion. The skin state 158 may be compared with a table of indicators for various types of lesions. In an embodiment, the algorithm 150 may be able to diagnose UV damage. UV damage may be difficult to assess from a conventional superficial view as UV damage may be present even in wrinkle-free skin. However, UV damage may be assessed by examining skin structures for an increase in melanin production; global distribution, damage and count of superficial blood vessels; change in hemoglobin count: changes in the thickness of the epidermis; changes in the quantity and global distribution of collagen, and the like. In an embodiment, diagnosis may not require processing the border of the lesion, as it may not be a key factor in final analysis of the skin lesion. In an embodiment, the algorithm 150 may be able to diagnose oral cancer.

In an embodiment, an algorithm 150 for determining a skin state 158 may enable cosmetics manufacturing validation or cutaneous clinical trials. For example, a skin state 158 may be determined prior to medical, non-medical, skin care product or cosmetics application and a time lapse series of images may be acquired to track the medical, non-medical, skin care product, and cosmetics effectiveness.

In an embodiment, there may be methods for storing, handling, integrating, and analyzing a skin state 158. The skin state 158 may be stored in the device 108 itself, on a PC, in a central server, a salon record, an e-medicine record, a medical repository, a cosmetic clinical studies database 115, a mobile device, and the like. The device 108 may communicate with a user interface 102, an online platform 120, a mobile platform 124, and the like to upload, deliver, share, and/or port images, analysis 154, skin states 158, data, track history, user profiles, and the like, as will be further described herein. For example, a user may use a device 108 embodied in a mobile device to capture an image of the skin and upload it to a mobile platform 124 for analysis 154 to determine a skin state 158. In response, the user may receive a personalized regimen 118 for sun protection given the user's skin state 158. Other factors that may be used to determine the regimen 118 may be the current UV Index, time of day, location, kind of sun protection product the user prefers, and the like. In the same example, the user may have already obtained a skin state 158 determination and they need not upload a new image but simply request a regimen 118 recommendation from the mobile platform 124 given the already determined and stored skin state 158. Once a skin state 158 is determined, it may be accessible by and/ or integrated with any element of the user interface 102, online platform 120, mobile platform 124 and the like. A user may choose to share the skin state 158 as part of a practitioner record 180.

In an embodiment, an algorithm 150 for determining a skin state 158 may enable an analysis of differences and similarities among peers. The algorithm 150 may determine peers of a user who may be most like them in terms of skin state 158 or other criteria such as gender, age, ethnicity, behaviors such as smoking, working outdoors, and the like, diet, regimen 118, and any other identifying factors. The algorithm 150 may be able to interface with an online platform 120, third party database 115, or third party service provider 111 to access skin states 158 and demographic information for comparison. For example, a user may wish to know what other women in their mid-30's of the same skin color are using for foundation. By employing the algorithm 150, a user may be able to determine their own skin color, identify peers according to the search criteria, and view details on their peers' regimen 118 or the results of the specific search query 103. The algorithm 150 may enable grading of the skin relative to a peer group. Using the algorithm 150, a user's skin state 158 may be compared to a previously defined skin state 158 in order to monitor the skin state 158 over time. A user's skin state 158 may also be compared to the skin state 158 of other individuals or groups of individuals to identify peers whose skin state 158 is closest to the user. Once a peer, such as a similar individual or group, is identified, the system may display the skin care products and/or skin care regimen that is effective for the peer. Similarly, any comparison among users may be made by the system, such as a comparison of at least one of age, gender, location, climate, skin color, ethnicity, and the like, to identify a peer. In an embodiment, as the device 108 captures data from users and determines skin states 158, the information may be fed back into the algorithm 150 to further enhance the peer identification and product recommendation process.

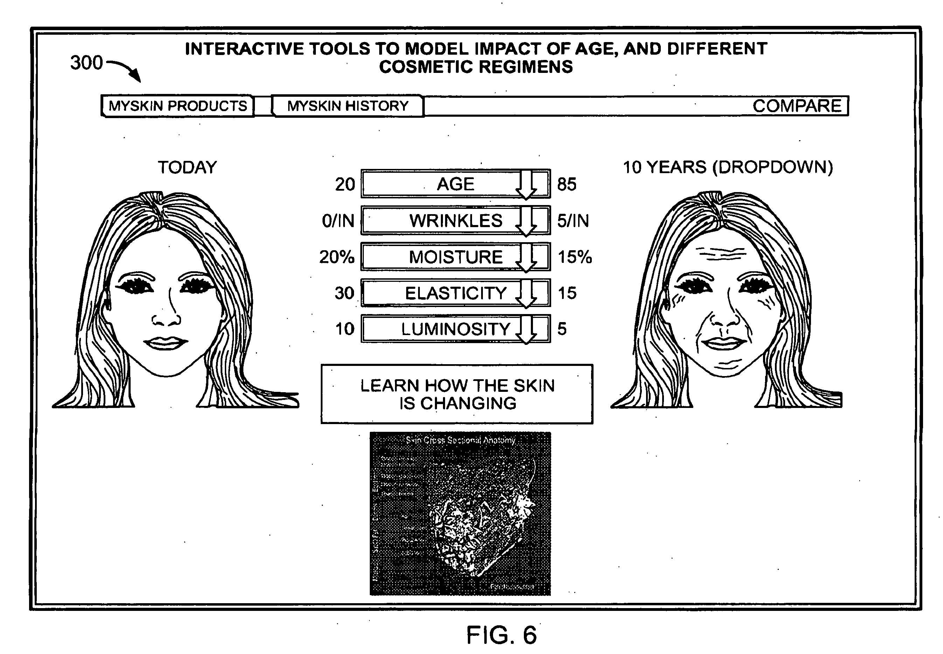

In an embodiment, an algorithm 150 for determining a skin state 158 may enable prediction/simulation tools 132. Having determined a skin state 158, an algorithm 150 may be able to simulate progression of aging, simulate skin care treatment effects and skin care and cosmetic regimens 118, simulate progression of a skin condition, and the like. Referring to Fig. 6, a user may use a user interface 102 to access the simulation tools 132. In the example, the image of an entire face may be used but it should be understood that simulation tools 132 may be used to generate simulations for any size area of concern. After selecting or capturing a starting image, a user may indicate the kind of simulation they would like to perform. For example, the user may like to perform a simulation of aging only, or a simulation of aging and treatment effects. The simulation tool 132 may return data on overall appearance, wrinkle count, elasticity, luminosity, moisture, product usage simulation, and the like. For example, the output may also include a split image with the original face on one half and a new simulated output on the other half.

In an embodiment, an algorithm 150 for determining a skin state 158 may enable skin cycle monitoring 140. By monitoring skin at determined intervals, skin conditions with a cyclical nature may be monitored, predicted, pre-empted and the like. For example, skin conditions associated with a season, weather, pollen count, hormone level, environmental condition and the like may be identified and monitored by a skin cycle monitor 140.

In an embodiment, an algorithm 150 may be used to generate searchable and/or indexable tags to associate with images and may take advantage of image tagging. Images may be tagged with information relating to the content of the image, such as information relating to a skin state, a skin condition, a gender, an ethnicity, an age, a regimen, a treatment, and the like. The information may be gathered by algorithmic analysis, user input, visual inspection of the image, and the like. An algorithm 150 may be used to perform a search 103 using the information associated with the image as a search term. In embodiments, the information may be stored separately from the image, such as an entry in a user profile, or may be stored in association with an image. In an embodiment, a search 103 may be performed against information or images from other users' or a third party database 115 to identify similarities or differences in images or information. For example, a user may use information to search for peers with a similar skin condition in order to determine what to expect as the condition progresses. In another embodiment, the search 103 or query for advice or recommendation from experts may be performed against product information 190, wellness information 192, skin care regimens 118, third party experts 105, and the like. For example, a user may use information to search for product information 190 indicating an effectiveness of a product for the user's skin condition. In an embodiment, the search 103 may be performed to determine an availability of a product, an inventory of a product, a price of a product, and the like. For example, a user may use the information to search a store catalog for a specific product that may be effective for the user. In the example, the user may be pale skinned and be interested in identifying an inventory of a self-tanning product formulated specifically for pale skin. In an embodiment, the image itself may be used as a search query 103. For example, the image itself may be used to search a database 115 of skin images. In an embodiment, images and information entered into the system 104 may be leveraged to develop new algorithms 150 for enhanced diagnosis. For example, algorithms 150 may be developed for non-skin specific diseases with dermal manifestations, such as rheumatoid arthritis.

In an embodiment, an algorithm 150 may be useful for analyzing product characteristics. For example, an algorithm 150 may be able to take product ingredients and match the product up with a projected effectiveness on a particular skin state 158.

In an embodiment, an algorithm 150 may use RGB color analysis. The algorithm may employ standard RGB analysis and correlation with skin structures in determining skin phototype. The calculation of parameters for determining skin phototype is fast and the skin phototype can be found in a very short period of time using a simple skin and cosmetic parameters classification routine.

Exemplary embodiments of the present invention are directed to a method and system for determining skin characteristics and cosmetic features. The method and system provide a minimal error and speed efficient skin analysis. The present technique describes a method and a system for determining a skin phototype of acquired digital image in a Red Green Blue (RGB) color system.

In an exemplary embodiment of the present invention, a method for determining skin characteristics and cosmetic features using color analysis includes a step of analyzing the color of skin images in a pixel by pixel manner in a Red Green Blue (RGB) color system for an acquired digital image. The colors obtained in a device dependent RGB color system are then converted into device independent standard RGB color system (sRGB) which will be used in subsequent color analysis. The step of analyzing the color of skin images in a pixel by pixel manner in a sRGB color system for an acquired digital image comprises analyzing a picture of a part of a person's skin by generating a table of most frequent colors appearing in the picture.

In this embodiment of the invention, the sRGB color system has been used for image analysis. Determination of other skin characteristics (e.g. elasticity, melanin, oil concentration etc.), melanoma, skin related tumors and skin related disorders may require image analysis based on various color systems such as YIQ, YCbCr, L*a*b*, L*u*v* and HSL/HSV. The enhancement of the current algorithm 150 may include at least one of these color systems and its/their correlation with presented sRGB analysis. This will most likely lead to in-depth refinement and overall accuracy of the current results as well as further embodiments of the present invention. Apart from the human skin related issues, this method of image analysis is also applicable to any content whether it be animals, products, plants or any other material whose surface needs to be analyzed by a digital image.

A method for determining skin characteristics and cosmetic features using color analysis includes a step of generating a sample of most frequent sRGB colors responsive to analyzing the color of skin images in a pixel by pixel manner in the RGB color system for the acquired digital image after converting colors obtained in a device-dependent RGB color system into a device-independent standard RGB color system (sRGB). The step of generating a sample of most frequent sRGB colors responsive to analyzing the color of skin images in the sRGB color system for the acquired digital image comprises preserving a plurality of sRGB color values.

A method for determining skin characteristics and cosmetic features using color analysis includes a step of modeling the standard R, G and B component color distribution with Gaussian probabilistic distribution with estimated parameters (expected value and standard deviation) of the generated sRGB color sample for the acquired digital image further including approximating colors of the generated sRGB color samples by a Gaussian normal distribution. In accordance with an exemplary embodiment of the present invention the step of approximating colors of the generated sRGB color samples by a Gaussian normal distribution comprises approximating colors of the generated sRGB color samples by a superposition of a plurality of Gaussian normal distributions.

A method for determining skin characteristics and cosmetic features using color analysis includes a step of generating a phototype of the skin through a decision tree unit responsive to the estimated distribution model parameters colors. The phototype of the skin is generated according to a corrected Fitzpatrick classification, or any other applicable color classifier. In accordance with an exemplary embodiment of the present invention, the step of generating a phototype of the skin according to corrected Fitzpatrick classification includes generating a phototype of the skin according to a skin type scale which ranges from very fair skin to very dark skin.

According to an exemplary embodiment of the present invention, the system for skin phototype determination using photograph analysis includes a subsystem for determination of cosmetic features for a human element and a veterinary element. The cosmetic features further include features pertaining to hair, nail and skin.

According to an exemplary embodiment of the present invention, the image of the skin sample of a person's body can be captured by any digital camera. The acquired digital image sample of the person's skin may be analyzed in a pixel by pixel manner in the RGB color system. After the conversion of colors from a device-dependent RGB color system into a device-independent standard RGB color system (sRGB), a table of most frequent sRGB colors which appear in the image may be generated. According to an example, the generated table may consist of 256 most frequent colors which appear in the image of the person's skin. The color samples obtained from the image may be approximated by a Gaussian normal distribution (or a (scaled) superposition of few Gaussian normal distributions). Therefore the estimates of expected value (using weighted mean) and standard deviation (using unbiased (n-1) method as the precise expected value is unknown / estimated) for each of the acquired digital images may be evaluated. The phototype of the skin may be determined through a decision tree with the estimated expected value and standard deviation. Fitzpatrick classification may be used for categorizing a skin phototype in accordance with a skin type scale which ranges from very fair skin to very dark skin.

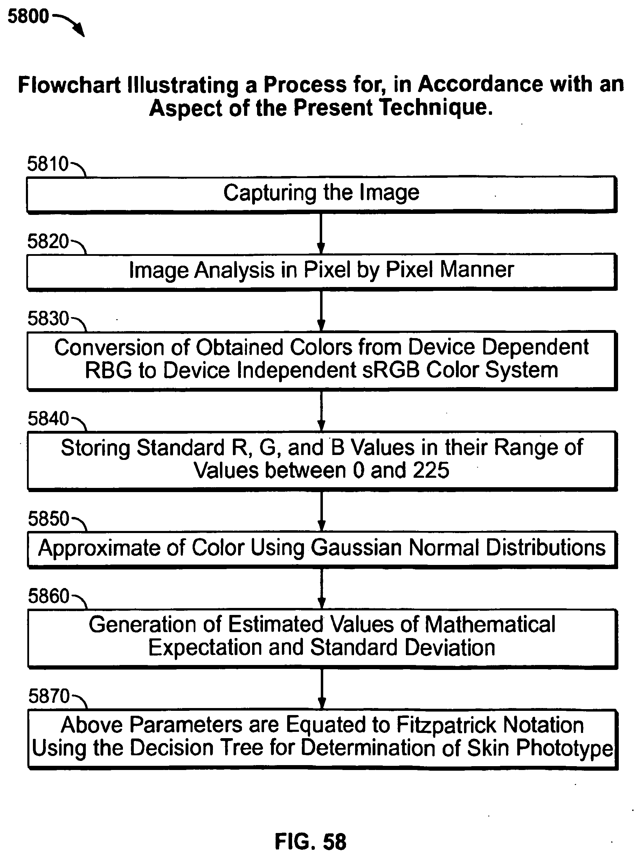

Referring to Fig. 58, a flowchart 5800 illustrating a process for determining a skin phototype of an acquired digital image of a part of a person's skin is shown. The process starts at block 5810 wherein an image of a part of a person's skin is captured. The image capturing device may be a digital camera or the like. Processing flow continues to logical block 5820 wherein analysis of the acquired digital image is done in a pixel by pixel manner in a RGB color system. After converting all colors from the device-dependent RGB color system into a device-independent standard RGB color system (sRGB), a table of most frequent colors which appear in the acquired digital image may be generated using a quantization technique at block 5830. In accordance with an example of the invention, at block 5840 a plurality of sRGB color values/samples generated between a range of values 0 and 255 may be preserved for further analysis. This range of values has been proven to be more convenient for skin type determination than the one between 0 and 1. The transformation from one to another can be done simply by dividing the values with 255 and vice versa. In the next stage 5850 and 5860 approximations of colors on the samples are done by Gaussian normal distribution, at block 5860 the estimates expected value and standard deviation are evaluated. Finally at block 5870, the photoype of skin of the acquired digital image is determined according to the corrected Fitzpatrick classification using a decision tree.

According to an exemplary embodiment of the present invention, the decision tree may be an algorithm wherein the estimated expected value and standard deviation are equated to the values of Fitzpatrick classification/notation values in determining the phototype of the skin. The effectiveness of this approach may be seen in research regarding parametric skin distribution modeling for skin segmentation / detection.

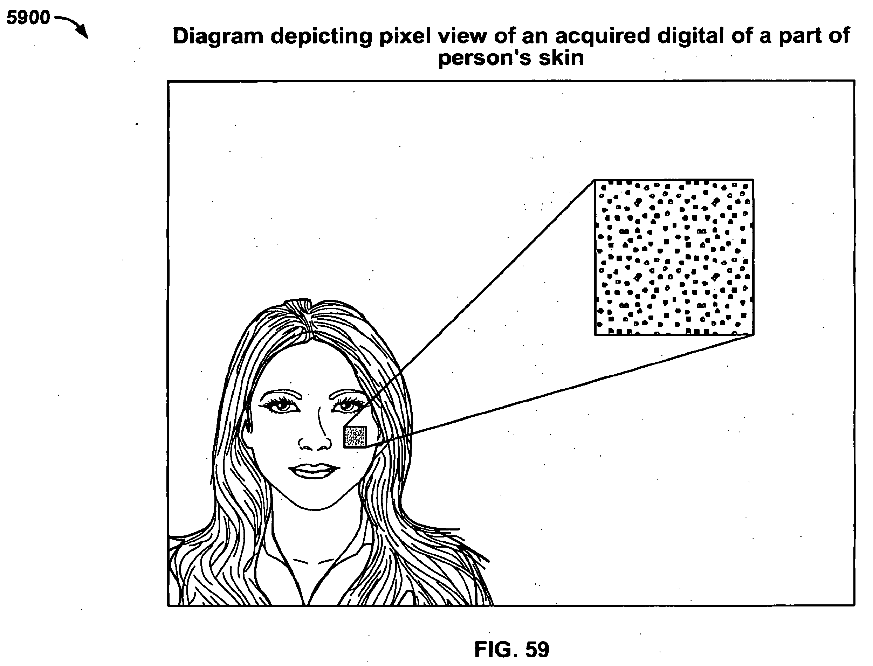

Referring to Fig. 59, a diagram depicting a pixel view of an acquired digital image of a sample of person's skin is shown. The image of a sample of a person's skin is captured under white emitting light. The image may be captured by any digital camera and the like under white emitting light. An analyzer coupled to the image capturing device may analyze the acquired digital image in a pixel by pixel manner in the RGB color system. The analysis of the acquired digital image in a pixel by pixel manner in the sRGB (after RGB to sRGB color system conversion) is not only limited for determining skin phototype but also may be useful for other purposes like classification of other skin characteristics (e.g. elasticity, melanin, oil concentration etc.), melanomas and other skin tumors/disorders and the like.

Digital images captured from a sample of person's skin are usually given in the RGB color system. The present technique employing an algorithm 150 for determining skin phototype in one aspect is dependent on this color system, although device independent due to conversion to the sRGB color system. The calibration of the image capturing device, such as a digital camera or the like, should be taken into consideration carefully, so that the eventual color offset could be corrected. The color offset correction in the present technique can be implemented from any known techniques in the previous art and color offset correction can also be implemented in software used in the present technique in determining skin phototype.

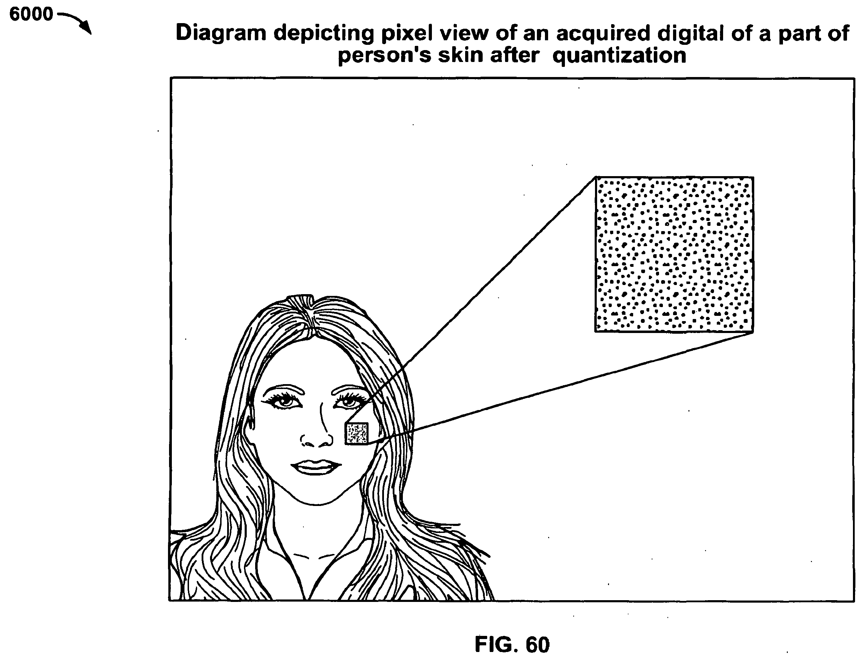

Referring to Fig. 60, a diagram depicting a pixel view of the acquired digital image of a part of person's skin after quantization is shown. The image of the sample of the person's skin is captured under the white emitting light. The image may be captured by any digital camera and the like under white emitting light. The analyzer coupled to the image capturing device analyzes the acquired digital image in a pixel by pixel manner in the RGB color system. The analysis of acquired digital image in a pixel by pixel manner in the sRGB (after RGB to sRGB color system conversion) is not only limited for determining skin phototype but also may be useful for other purposes like classification of other skin characteristics (e.g. elasticity, melanin, oil concentration etc.), melanomas and other skin tumors/disorders and the like. Color quantization or color image quantization is a process that reduces the number of distinct colors used in an image, usually with the intention that the new image should be as visually similar as possible to the original image. Color quantization is critical for displaying images with many colors on devices that can only display a limited number of colors, usually due to memory limitations, and enables efficient compression of certain types of images.

An image quantization technique may be applied to the captured image. A table of 256 most frequent colors which appear on the acquired digital image of the part of person's skin may be generated using a sampling device coupled to the analyzer. The acquired color samples from a digital image may be preserved in the sRGB color system. In accordance with an example of the present invention, the generated color samples may be preserved in their range of values between 0 and 255 in the sRGB color system. This range of values has been proven to be more convenient for skin type determination than the interval ranging between 0 and 1.

Accordingly colors of the samples may be approximated by a Gaussian normal distribution (or a (scaled) superposition of few Gaussian normal distributions) through an approximating device coupled to the sampling device. Further the estimates of expected value (using weighted mean) and standard deviation (using unbiased (n-1) method as the precise expected value is unknown / estimated) for each of the acquired digital image may be calculated with the approximating device coupled to the sampling device.

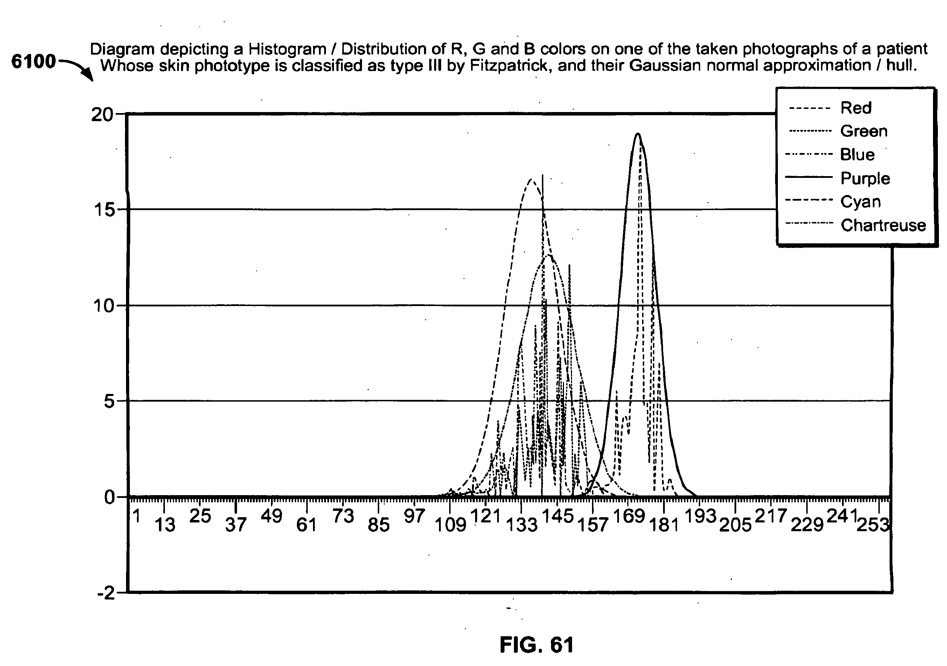

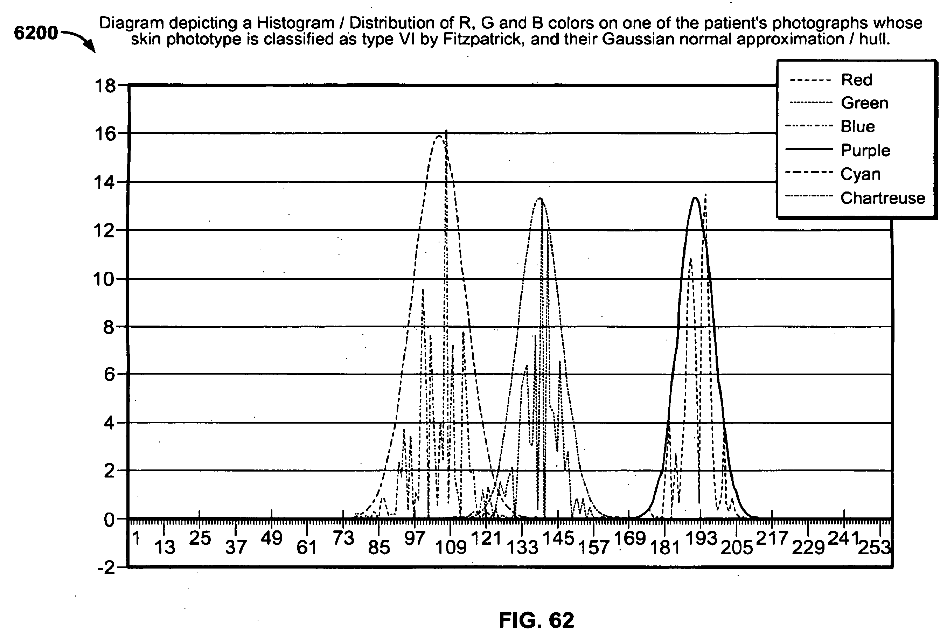

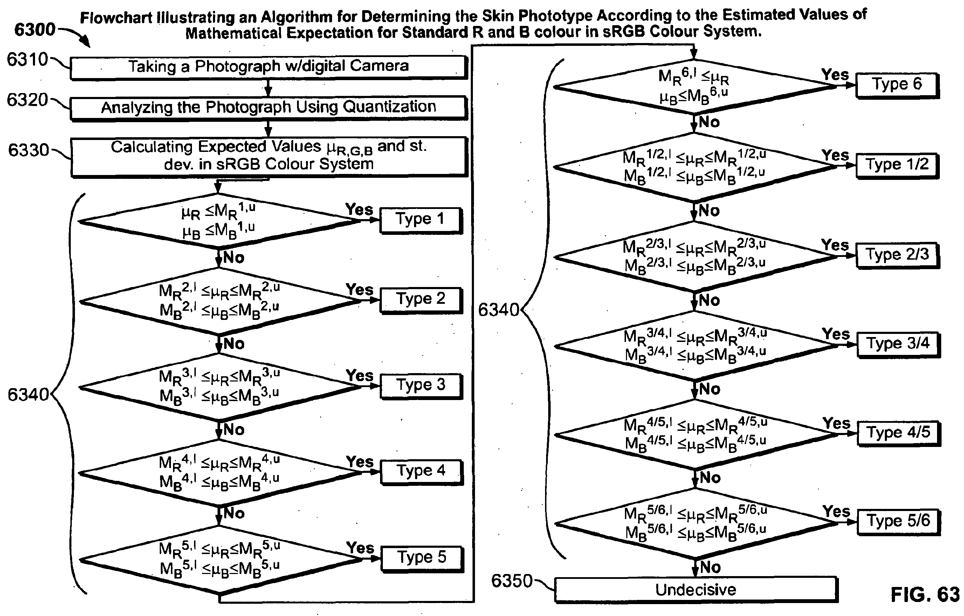

Usage of an algorithm 150 of the present technique is depicted in Fig. 61 and Fig. 62 and the algorithm 150 for RGB color analysis is depicted in Fig. 63.

Referring to Fig. 61, a diagram depicting a Histogram / Distribution of standard R, G and B colors of one of the taken photographs of a patient whose skin phototype is classified as type III by Fitzpatrick, and their Gaussian normal approximation / hull is shown. The relevant estimates are mR (expected value of red) =171.1304 and mB (expected value of blue) =135.3047 , for example. The estimates are compared with the decision tree described below for determining skin phototype. The phototype of skin is determined according to corrected Fitzpatrick classification. The Fitzpatrick Skin Typing Test questionnaire (skin type scale) which ranges from very fair (skin type I) to very dark (skin type VI) is often used to determine skin phototype.

Dermatologists use the Fitzpatrick Classification Scale to classify a person's complexion and tolerance to sunlight. In accordance with an exemplary embodiment of the present invention, the Fitzpatrick scale classifies skin types from I to VI.

> Type I - Very white or freckled skin, always burns with sun exposure (very fair; often in people with red or blond hair and blue eyes)
> Type II - White skin, usually burns with sun exposure (fair; often in people with red or blond hair and blue, green, or hazel eyes)
> Type III - White or olive skin tone, sometime burns with sun exposure (fair; seen in people with any hair or eye color)
> Type IV - Brown skin, rarely burns with sun exposure (common in people of Mediterranean descent)
> Type V - Dark brown skin, very rarely burns with sun exposure (common in people of Middle-Eastern descent)
> Type VI - Black skin, never burns with sun exposure

The images of skin are captured under white emitting light with an image capturing device, such as a digital camera, video camera or the like. An analyzer analyzes the captured image pixel by pixel of a part/sample of a person's skin. A sampling device coupled to the analyzer generates a table of 256 most frequently occurring colors in the captured image. The acquired color samples from the digital image are preserved in the sRGB color system. The generated color samples are preserved in their range of values between 0 and 255 in the sRGB color system. An approximating device coupled to the sampling device may calculate the estimates of expected value (using weighted mean) and standard deviation (using unbiased (n-1) method as the precise expected value is unknown / estimated) for each of the acquired digital images. A decision tree coupled to the approximating device determines the skin phototype. From this imaging, it turns out that expected values of R and B may be sufficient for determining skin phototype according to the following decision tree. An exemplary embodiment of the present invention is illustrated below.

$$\text{Phototype} = \begin{cases} 1, & \left(\mu_R \leq M_R^{1,u}\right) \wedge \left(\mu_B \leq M_B^{1,u}\right) \\ 2, & \left(M_R^{2,l} \leq \mu_R \leq M_R^{2,u}\right) \wedge \left(M_B^{2,l} \leq \mu_B \leq M_B^{2,u}\right) \\ 3, & \left(M_R^{3,l} \leq \mu_R \leq M_R^{3,u}\right) \wedge \left(M_B^{3,l} \leq \mu_B \leq M_B^{3,u}\right) \\ 4, & \left(M_R^{4,l} \leq \mu_R \leq M_R^{4,u}\right) \wedge \left(M_B^{4,l} \leq \mu_B \leq M_B^{4,u}\right) \\ 5, & \left(M_R^{5,l} \leq \mu_R \leq M_R^{5,u}\right) \wedge \left(M_B^{5,l} \leq \mu_B \leq M_B^{5,u}\right) \\ 6, & \left(M_R^{6,l} \leq \mu_R\right) \wedge \left(\mu_B \leq M_B^{6,u}\right) \\ 1/2, & \left(M_R^{1/2,l} \leq \mu_R \leq M_R^{1/2,u}\right) \wedge \left(M_B^{1/2,l} \leq \mu_B \leq M_B^{1/2,u}\right) \\ 2/3, & \left(M_R^{2/3,l} \leq \mu_R \leq M_R^{2/3,u}\right) \wedge \left(M_B^{2/3,l} \leq \mu_B \leq M_B^{2/3,u}\right) \\ 3/4, & \left(M_R^{3/4,l} \leq \mu_R \leq M_R^{3/4,u}\right) \wedge \left(M_B^{3/4,l} \leq \mu_B \leq M_B^{3/4,u}\right) \\ 4/5, & \left(M_R^{4/5,l} \leq \mu_R \leq M_R^{4/5,u}\right) \wedge \left(M_B^{4/5,l} \leq \mu_B \leq M_B^{4/5,u}\right) \\ 5/6, & \left(M_R^{5/6,l} \leq \mu_R \leq M_R^{5/6,u}\right) \wedge \left(M_B^{5/6,l} \leq \mu_B \leq M_B^{5/6,u}\right) \\ \text{Further examination,} & \text{all other cases} \end{cases}$$

The values $M_{R,B}^{n,u\ or\ l}$, n=1,2,3,4,5,6,1/2,2/3,3/4,4/5,5/6 have been determined from the images analyzed by using the programmed neural network.

Fig. 62 is a diagram depicting a Histogram / Distribution of R, G and B colors of one of the patient's photographs whose skin phototype is classified as type VI by Fitzpatrick, and their Gaussian normal approximation / hull. Here the relevant estimates are mR (expected value of red) =189.7173 and mB (expected value of blue) =103.537, in accordance with an example of the present invention. The estimates are compared with the decision tree mentioned above for determining the phototype of the skin.

Referring to Fig. 63, a flowchart 6300 illustrating an algorithm 150 for determining the skin phototype according to the estimated values of mathematical expectation for standard R and B color in sRGB color system is shown. The flow chart describes the algorithm 150 developed in accordance with the present technique wherein the photograph of a part of person's skin is captured with a digital camera or the like under white emitting light at logical block 6310. At logical block 6320 the captured digital image is analyzed in a pixel by pixel manner in the RGB color system. A quantization technique is employed for analyzing the captured image in a pixel by pixel manner in the sRGB color system at logical block 6330. The color samples obtained from the image can be approximated by a Gaussian normal distribution (or a (scaled) superposition of few Gaussian normal distributions). Therefore the estimates of expected value (using weighted mean) and standard deviation unbiased (n-1) method (as the precise expected value is unknown / estimated) for each of the acquired digital images may be evaluated. Now at logical block 6330 the phototype of the skin is determined according to the decision tree.

As will be appreciated by a person skilled in the art, the various implementations of the present technique provide a variety of advantages. Firstly, the present technique determines skin phototype using regular low-cost digital photography equipment under standard environmental conditions. Secondly, the analysis performed on the captured digital image may be useful in recommendation of cosmetic product and medical or surgical purposes. Thirdly, the picture quantization algorithm and calculation of estimates expected value and standard deviation are fast, this makes it easier to determine skin phototype in a short span of time using a simple routine. Fourthly, the analysis performed may be useful for classification of other skin characteristics (e.g. elasticity, melanin, oil concentration etc.), melanomas, skin tumors or disorders and the like.

In an embodiment, new algorithm 150 development by practitioners, users, service providers 111, and the like may be enabled by a software development kit that anyone could use to develop new algorithms 150 and APIs 154 for the device 108.

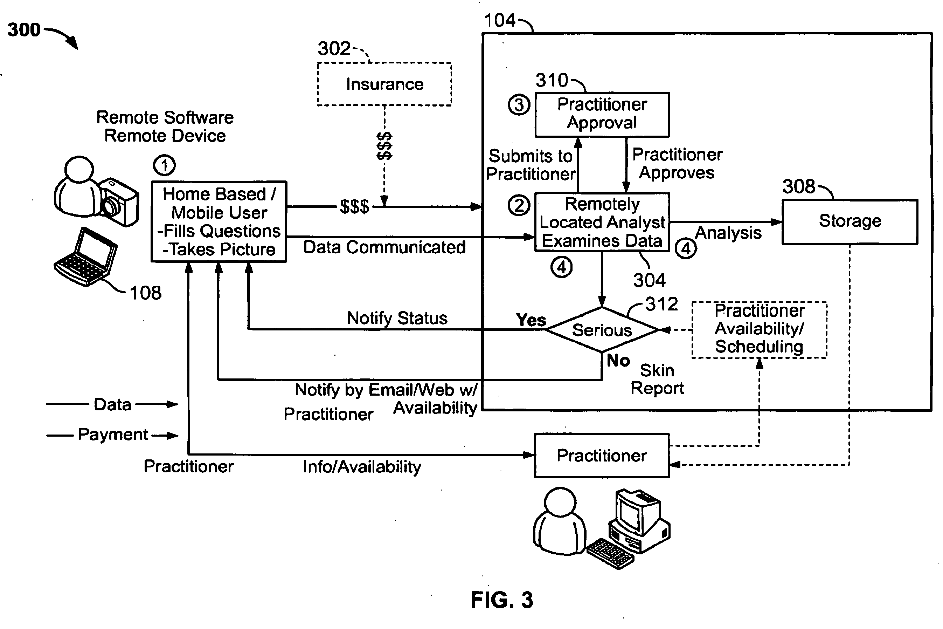

Referring now to Fig. 3, in an embodiment, a process for collecting images, performing skin analysis, communicating findings and scheduling follow up, if required may commence with image capture by a user using a device 108. The user may also answer questions or provide additional details regarding a user-entered imaging, cosmetic regimen, area of concern, or the like. Using the user interface 102, the data may be communicated to an analyst 304 or a computer for analysis 154 by any communication method, such as over a network, the Internet, wirelessly, and the like. In certain embodiments, as the data are collected or communicated, a payment system 302 may be accessed by the user. In the example shown, an insurance company may access the data, however, payment may be effected or requested by any interested entity such as a one-time payment by the user, a subscription by the user, a third party service provider 111, a platform 120,124, a practitioner, and the like. The entered data may be analyzed by the analyst, by software in real-time, by analysts assisted by software assistance, and the like. An initial analysis may be to determine data integrity. In instances where the data do not pass the integrity test, it may be communicated back to the user. The analyst's assessment may be assisted by software that uses an algorithm to determine type of condition and/or recommended care/treatment. Historical analysis and data, and modeling tools may be used to assist the analyst's assessment. Relevant parties (company personnel, payment providers, physicians, medical personnel, users, amongst others) may receive the analysis and/or user specific details for follow up or other actions that may be required. The analysis 154 may be stored 308 by the system and/or submitted to a practitioner for approval 310. In embodiments, storage 308 may require practitioner approval 310. A test of the severity 312 may determine the selection of an appropriate method of communication with the user. If the result of the test 312 is positive, the user may be notified immediately by a preferred communication method, such as telephone, instant message, and the like. If the result of the test 312 is negative, the user may similarly be notified, however, the notification may take a less urgent route, such as by email or postal mail. In any event, the software tool may recommend an appropriate communication method and media, based on the assessment and may populate preset templates with the information/message to be communicated. In addition, notification by any means may also include a notification of practitioner availability. The analysis 154 may trigger a practitioner availability / scheduling tool. For example, prior to transmitting the results on severity 312 to the user, a practitioner availability may be assessed and transmitted simultaneously. The user may access availability and scheduling tools in order to obtain and confirm an appointment time.

In an embodiment, a user interface 102 for a skin analysis system 104 may be used to interface with the device 108, store images, deploy algorithms 150, track a skin state 158 by keeping track of images from any number of areas of concern, the interval between image capture, a projected next image capture date, communicate findings to a practitioner, interact with simulation tools 132, skin type determination tools 130, a skin cycle monitor 140, practitioner availability / scheduling tools, and the like.

In embodiments, the user interface 102 may be operable as an application running on a device 108, a computer, server, kiosk, or the like, on an online platform 120, on a mobile platform 124, and the like. Any and all aspects of the user interface 102 described herein may be applicable to the user interface 102 running in any environment.

In an embodiment, the user interface 102 for the device, as will be further described herein, may be integral with the device 108, such as embodied in the keypad of a communications device or a series of buttons, switches, keys and the like disposed on the device 108, or may be external to the device 108, such as software running on a computer, on the Internet, on an intranet, on a mobile communications device, on an online platform 102, on a mobile platform 124, and the like. The user interface 102 may be used to modify a setting of the device 108, such as the magnification, light source, light intensity, wavelength of light, angle of light, electrical and magnetic properties of the light, positioning of sensor, duration of image capture, image size, data storage, data transmittal, and the like.

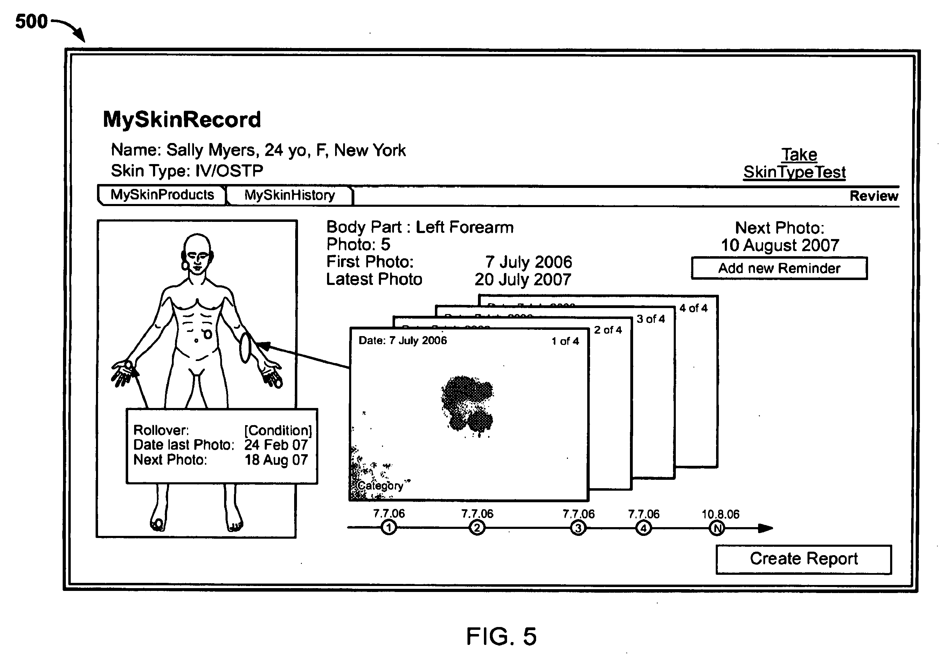

Referring now to Fig. 5, the user interface 102 may organize and index images captured by date, area of concern, skin state, and the like. For example and without limitation, as seen in the Fig. 5, four images captured from the same area of concern are indexed by their number within the series. In an embodiment, the user interface 102 may show in real time the field of view on the skin being imaged as well as populate the user interface 102 with the images once taken or once submitted by the user. The user interface 102 may keep track of the first image, latest image, next image, and the like. The user interface 102 may allow users to shuffle through image s and use the images as a basis for simulation 132, as described herein. The user interface 102 may be used to set a reminder for next image capture. The user interface 102 may be used to create a report of the images and skin state 158. The user interface 102 may be used to transmit the report to a practitioner. In an embodiment, the user interface 102 may be used to launch a skin type test. In an embodiment, the user interface 102 may depict a form of a body. As a user interacts with the depiction of the body, such as with an indicating device, the portions of the body that have been imaged may be linked with the images such that the images may pop-up or be otherwise accessed. The user interface 102 may be adapted to collect data from the user in response to prompts. The user interface 102 may employ an algorithm 150 to check the integrity of the captured images. The user interface 102 may guide the user in capturing images and providing user input in association with the images.

In an embodiment, the user interface 102 may interface with host hardware 108 or third party hardware 109. Hardware 108, 109 may comprise an imaging device that may connect with a computer, online platform 120, mobile platform 124, and the like via the user interface 102 and enable users to capture an image that enables measure various skin health, condition and type parameters. The hardware device 108,109 may be a standalone device or connect via or be embodied in a computing device of either medical or non-medical use. The user interface 102 may guide the connection process for the hardware device 108, 109. The device 108, 109 may store images, reports and recommendations generated and maintain a repository of the image, all as part of a skin health record 121. It may enable a systematic storing of the skin health record 121. Third party hardware 109 may comprise devices such as moisture sensors, cosmetic analysis machines, dermascopes, cameras, x-ray machines, MRIs, medical record providers and software, web cameras, communication devices, and the like. Third party hardware 109 may connect to the system 104 seamlessly to enable the user to gain a better analysis, and share such sets of data with other experts or users.

In an embodiment, the user interface 102 may enable type determination 130. Characteristics may be captured to determine the skin characteristics and the skin state 158 of the users' skin. Broad genetic parameters, such as ethnicity, skin color, location factors, environmental factors (such as pollen count, weather, etc.), and lifestyle factors may be collected in addition to image and skin health data to determine the users' skin state 158. This skin state 158 may be correlated with product experience ranking and ratings 138 to enable providing a recommendation for most effective products.

The user interface 102 may display a regimen 118. The regimen 118 may be a feature that enables users to learn what products and product usage pattern would work best for their skin based on a hardware- or community-led personalized skin care assessment 160 and / or type determination 130 and product experience sharing via ranking and rating 138 and / or comments regarding product effectiveness and experience (such as smell, taste, feel, texture, color, and the like). The regimen 118 may be a dynamic recommendation based on users' collective inputs as well as experts' inputs on products that would best suit the user's individual needs.

In an embodiment, the user interface 102 may enable simulation tools 132. Users may be able to upload an image and model various skin parameters (such as moisture level in skin, collagen level, age, and the like.) and observe changes in the image. Additionally, users may be able to model the impact of various products and regimens 118 (skin care, cosmetic, medical, nail care, hair care, and the like) on the image. Simulation tools 132 may enable users to view changes on the entire image or split half of the image to show a comparison of modeled change with current image. The user's images could also be automatically or manually optimized for the best look and the products or regimen 118 to obtain that look may be provided. Simulation tools 132 may also enable consumers to model the skin characteristics or state 158 of other selected users or non-users, such as celebrities, luminaries, average users, and the like.

In an embodiment, the user interface 102 may enable a daily report 134. The daily report 134 may be a report that provides the user information largely customized and most relevant to the user based on their skin state 158. The daily report 134 may list skin care regimen 118 to be followed based on the environmental and lifestyle factors relevant to the user, may indicate new product information 190, show the current skin care shelf 114 and rankings 138 or change in rankings 138, feedback from users or experts 105 on products most relevant to the user, and the like. The daily report 134 may include information about clinical trials and upcoming results, new product releases and status, events, various factors affecting the skin such as the day's weather forecast, UV index, temperature, pollen count, and the like, and other data to provide value to the user. The daily report 134 may report on whether a product is nearing its shelf life or may require replenishment based on a recommended usage protocol. The daily report 134 may be provided to the user by the user interface 102, paper, email, SMS, RSS, video or any other communication media.

In an embodiment, the user interface 102 may enable a wishlist 134. The wishlist 134 may be a function that a user could select and add products to a part of the skin care shelf 114 using drag and drop functionality or other selection mechanism as they surf the web or otherwise access product information 190. They could share this function with other users, friends and/ or family so that other people could see the wish list 134. Other users could then select the products off the wish list 134 and purchase and send the product to the user.

In an embodiment, the user interface 102 may enable ranking and rating 138. Ranking and rating 138 may be performed for various product characteristics as well as on the various raters and rankers. Product experience may be collected from users in simple ranking and rating 138 format as well as textual comment data to be stored in a database. This ranking and rating 138 may be real time, and may be synthesized to show what is most relevant to the user based on like users or peers, such as users with any of the following characteristics: same age, same sex, same skin type, same ethnicity, geography, moisture levels, and the like. These ranking and ratings 138 may be dynamic ranking and ratings 138. The users may be shown either the total number of rankers / raters and/or the weighted percent score ranking or rating 138. The ranking and rating 138 may comprise any of the following characteristics: perceived effectiveness, smell, touch, feel, texture, ability to absorb product, stains left by product, ease of use, and the like. Users may also be able to upload their images and obtain effectiveness/look ranking and rating 138 for different product recommendations from other users or experts 105. For example and without limitation, a user may upload data and/or images and request rating and feedback on better products from an herbal expert in India, aging expert in Japan, and the like. Users providing ranking and rating 138 for various products may themselves be rated by other users. This may enable selection of the most effective and unbiased users and help identify potential experts 105. A small select group of highly ranked users may be offered exclusive writing / publishing and ranking / rating privileges.

In an embodiment, the user interface 102 may enable a skin cycle monitor 140. The skin cycle monitor 140 may indicate when the last image was collected and countdown to the next scan based on a time interval, such as the time required to replenish the skin or any other interval. Currently, it is believed that the skin replenishes itself every 28 days. The skin cycle monitor 140 may take into consideration age, environmental changes, and other factors to indicate the upcoming scan schedule.

In an embodiment, the user interface 102 may enable wellness/ health 142. The user interface 102 may collect lifestyle data and also provide lifestyle (such as sleep, rest, exercise, and the like) and health (such as vitamins, food, products usage, and the like) recommendations based on the users particular skin state 158 and characteristics. The wellness and health module 142 may enable the user to obtain a personalized best fit health and wellness schedule and regimen 118.

In an embodiment, the user interface 102 may enable games 148. Users may be able to play games 148 that may enable users to model various products, try different hairstyles, model different hairstyles and clothes, and the like. Users may interact with other users or the computer to make the product selection a fun process. This process could also be used to collect information on user preferences and looks.

In an embodiment, the user interface 102 may enable a gift guide 144. Based on the user's skin state 158, personalized gift advice may be provided to others in the user's network.

In an embodiment, the user interface 102 may be embodied in touch screen user navigation. A touch screen system may be employed to enable the user to obtain a visual look and navigate to various parts of the user interface 102, such as navigate to the simulation tools 132, change picture orientation, drag and drop, and the like. Touch screen navigation may be particularly helpful as the hardware device 108 is connected to a computing platform. The user interface 102 may also enable collecting and coordinating information from other devices 109 and/or assessments, such as a dermascope, blood report, biopsy report, and the like to provide additional information for the skin record 121.

In an embodiment, the user interface 102 may enable a purchase/ sample portal. The user interface 102 may include a purchase/sample portal that may enable the user to select products and complete a purchase or request a sample to be delivered to a pre-entered address. The portal may be available in various social networking platforms 188 as well as over various computing platforms, such as an online platform 120, mobile platform 124, computer, laptop, mobile phones, and other mobile devices, medical-use devices, and the like.

In an embodiment, the user interface 102 may enable scheduling and data sharing functionality. A user may be able to schedule online a meeting with a particular expert or practitioner and, if willing, then share a skin state 158 or specific parts of the skin record 121 and history in part or its entirety with the expert or practitioner. Ranked experts and practitioners, availability, and other criteria to aid the selection and scheduling process may be indicated to the user. Experts may also be able to share particular sets of data amongst themselves, such as among practitioners, physician to another physician, physician to spa, spa to spa, and the like.

Other inputs 112, such as devices, features and data, may be used to augment the data submitted by the user or as the primary data to obtain a personalized assessment regarding the users' beauty, cosmetic, or medical concerns related to skin, hair, nails, and the like. For example, certain devices may be available commercially off the shelf, purchased, proprietary, and the like.

In an embodiment, a wearable monitor 182 may be an input 112 to the system 104 and user interface 102. Wearable skin health monitors 182 may enable real time tracking of changes in the environment and the skins health. These devices could be worn directly on the body, or integrated into clothing, apparel and / or accessories carried by the user. An example would be a user having a device that monitors the UV level, and provides a warning if the sun protection level accorded by a product used by the user falls below a set target level. These wearable monitors 182 may have independent user interfaces 102 or can be programmed for personalized parameters using other input devices. Wearable monitors 182 may also capture various physical parameters like heart rate, blood pressure, exercise rate, water consumption, fat counter, calorie meter, and the like. The monitors 182 may be able to assess hydration levels.

In an embodiment, a social network 188 may be an input 112 to the system 104 and user interface 102. The beauty social network 188 may be a collection of users interested in knowing and sharing information on beauty or medical concerns in a personal, private, and social interactive setting. The intent may be to create a beauty social network 188 where users invite and link to other users to discuss such concerns; obtain information 190, 192; perform ranking, rating, and review of products, regimens, experts, practitioners, other rankers/raters, and the like; complete purchases; access a wishlist 119; access a gift guide 144; play a game 148; review their daily report 134; and the like, all the while sharing experiences with other users in their network.

In an embodiment, product information 190 may be an input 112 to the system 104 and user interface 102. A database of product information 190 may comprise product, name, claims, manufacturer information, ranking and ratings 138, packaging information, images, usage parameters, product development history or forecast, special handling, upcoming changes, safety information, effectiveness information, smell, taste, color, texture, price, geography of manufacturing, brand information, consumer feedback and experiences, and other such parameters that may be obtained and/ or maintained to assist in the selection of the best product suited to the users' individual preferences or conditions to obtain the best beauty or medical outcome for their skin, hair, nails, and the like. Additionally, similar information on service oriented products such as massages, facials, hair toning, and the like may also be captured as well as information on procedures such as liposuction, Botox treatments, laser hair removal and other beauty, cosmetic and/ or medical procedures related to helping the user look good, improve or maintain a skin state 158, and the like. Manufacturers may register product information 190, contribute information on procedures, products in the pipeline, products in clinical trials, and the like. Users may rank and rate 138 products. A database update utility may update the database with new product information 190, store inventory, and the like.

In an embodiment, wellness information 192 may be an input 112 to the system 104 and user interface 102. Health and wellness information 192 may be captured, such as the impact of various products, primarily but not limited to non-prescription medications, supplements and other consumables that assist and maintain health and wellness (such as vitamins, protein shakes, supplements, and the like). Additionally, information on lifestyle recommendations (such as sleep, rest, diet and exercise recommendations for particular age groups/ ethnicities, etc.) may be collected and correlated with user preferences and characteristics to enable and provide a holistic health, wellness, and beauty/ cosmetic optimal personalized solution and service.

In an embodiment, a plug-in web capture 194 may be an input 112 to the system 104 and user interface 102. A software component-plug in for internet web browsers and basket or repository may recognize graphic objects on any browsed web page and allow the user to select, and drag-and-drop the graphic object onto a basket or repository onto a page of the web browser, such as a page comprising the skin care shelf 114. The graphic objects would be recognized through a standard reference table that would be accessed remotely or reside on the user's PC as part of the plug-in module 194, or as part of a resident software program on the computing platform. Graphic objects may include images for commercial products, such as skin care products or creams, or other objects that are part of any web e-commerce site. Once recognized, the plug-in 194 may highlight the picture, notifying the user that is it recognized, or provide additional information or reference. The plug-in 194 may also recognize brand names, trade names, generic pharmaceutical names, trademarks, and the like.

In an embodiment, barcode scan 198 may be an input 112 to the system 104 and user interface 102. Bar code information on various products may be captured to assist tracking, identification, price determination and correlation with other product information 190 for identifying similar substitute products, or other allied product information, usage recommendation, other user experience, pricing and delivery information, amongst other relevant sets of data. The bar code scanner 198 could be part of the hand held user device 108, a standalone system, a manual entry mechanism, and the like.

In an embodiment, conventional information/ questionnaires 101 may be an input 112 to the system 104 and user interface 102. Information 101 on the users and products may be captured via dynamic and static questions. Information such as age, sex, location, personal lifestyle traits, smoking habits, sleep patterns, skin dryness / oiliness and moisture levels, product likes and dislikes, experiences with other products along parameters such as smell, taste, absorption, staining propensity, and the like may be captured in a fun manner using questions and answers, games and other interactive tools interspersed at various points of the users' interaction with the service product, system 104, or user interface 102. Information 101 may be captured directly form the user or via an intermediary, and augmented automatically via computer data population, as an output of an algorithm 150 or by experts based on their assessment. Information 101 may be obtained by quizzes, badge- and widget-based forms, on-the-fly, through adaptive, investigative questioning, and the like. Information 101 may be obtained through questionnaires, such as How often do you go shopping?, When do you shop for cosmetics?, Where do you typically go? Why that spot?, Who do you shop with? Why?, What do you ask your friends when asking for advice?, Where do you go for new products/ information about cosmetics?, When do you have to go to a dept store, vs buying online?, When would you want to know something immediately from your friends?, What do you ask from your friends?, How do you choose a mobile phone?, What do you care about menus on a cell phone?, When do you get a new cell phone?, and the like.

In an embodiment, third party experts 105 may be an input 112 to the system 104 and user interface 102. The system 104 may connect various experts such as practitioners, physicians, medical experts, aestheticians, schedulers, product ingredient experts, cosmetologists, herbal, ayurvedic and homeopathic experts, health and wellness experts, media experts, photograph enhancement experts, and the like with users and one another. Users may be able to direct questions to such experts 105 who may be located at different places geographically over the system to obtain personalized advice. The experts 105 may be provided with users' data and characteristics collected and a record of the experts assessment may be retained in the record 121. The recommendation provided by the expert may be offered to the user for purchase / sample request, and the like. Experts may also be able to flag certain cases or sets of data for discussion or referrals within the expert community or with users.

In an embodiment, third party hardware 109 may be an input 112 to the system 104 and user interface 102. The system may connect with various third party hardware 109, such as existing imaging solutions, camera devices, computers, lighting systems, sports devices such as pedometers, and the like.

In an embodiment, third party service providers 111 may be an input 112 to the system 104 and user interface 102. Third party service providers 111 may be integrated into the system 104 to enable users to make the best personalized product or service selection for their hair, skin, nails, and the like for medical or cosmetic / beauty needs, and the like.

Third party service providers 111 may include hospitals, physicians, spas, salons, aestheticians, beauticians, cosmetic counters, drug stores, cosmetics sales representatives and websites, ranking and rating services, product information databases, testing laboratories, magazines and information providers, insurance companies, social networking sites, health and wellness services, photograph enhancement services, and the like. For example, based on a skin concern, the scheduling system for a physician may be integrated and scheduling options offered online to users, while also connecting with insurance providers to confirm coverage with the user. In addition, pre-assessments on the condition, availability of historical medical and/or cosmetic products prescribed either over the counter or by medical prescription, and / or recommended services may be captured to make the selection process for the user convenient and easy.

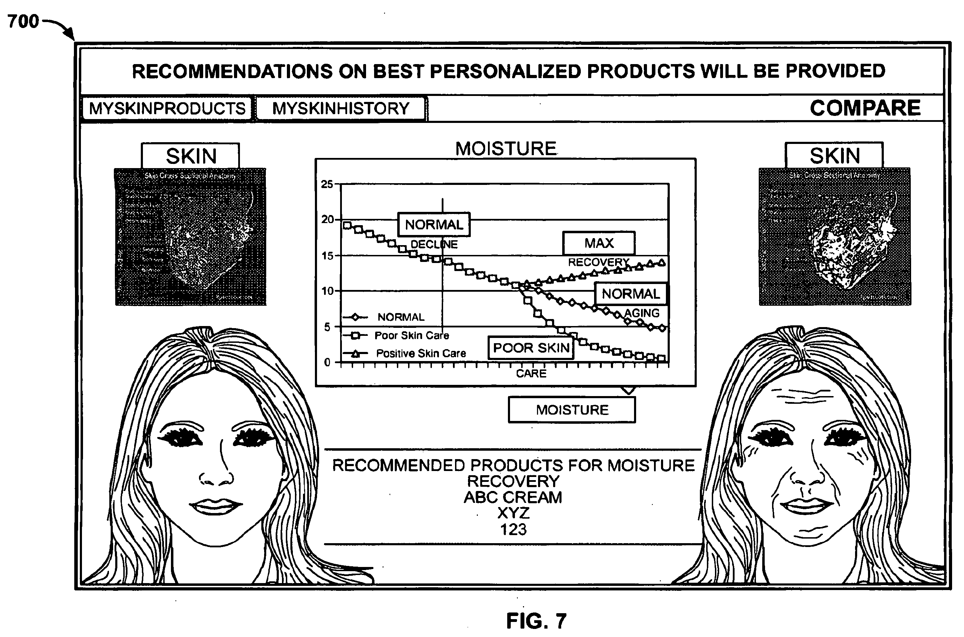

Referring to Fig. 7, a system for providing recommendations for skin care based on a skin state 158, a skin care goal, and environmental factors affecting the skin may comprise obtaining a skin state 158 of an individual, categorizing the individual by skin state 158, and recommending products and regimens that may be effective in achieving a skin care goal. The system may be computer-based, Internet based, network based, and the like. The system may be a community-led provision of skin services. In an embodiment, the recommendation may be made on the basis of identifying other users with similar skin states and identifying a product or regimen that is effective for them. In an embodiment, the recommendation may be made on the basis of product information 190, wellness information 192, a third party database 115, an expert 105, a service provider 111, and the like. As seen in Fig.7, a user may acquire an initial image and perform an analysis for a specific endpoint, such as moisture in this case. The system may automatically recommend certain products based on the moisture level that may be effective given the moisture level, a skin state 158, and the like. Additionally, the system may perform a projection of skin state 158 based on various skin care regimens 118, such as maximum care, normal care, or poor care. In an embodiment, the images may be captured using the device 108 or third party hardware 109. Images may be captured using any image capture device or technique, employing any kind of incident light, such as unpolarized light, polarized light, monochromatic light, diffuse light, white light, multiple single wavelength light, and the like. Any captured image may be used to obtain a skin state 158.

An embodiment of a skin care recommendation page of a skin care system may include a report of products the user is currently using, user input to obtain a skin state 158, a recommendation request, and the like. The report on the products the user is currently using may include ranking or ratings 138. For example, when a user accesses the user interface 102, they may access an adaptive questionnaire to determine their experience with their current regimen 118, current products or therapies used, or any products or regimens 118 used in the past. For example, the user may be asked to respond to questions such as How effective is it?, How is its fragrance?, How does it absorb?, Does it cause breakouts?, How does it feel?, Do you think this product is of good value?, and the like. Of course, rankings and ratings need not be prompted by questions but may simply be anecdotal, deployed in a non-question format, deployed in a drop down menu, and the like. To obtain a skin state 158, the user may enter data relating to aspects such as gender, age, ethnicity, location, skin color, environmental factors, and the like. In embodiments, analysis 154 of images obtained from the device 108 or third party hardware 109 may also be used to determine a skin state 158. Based on the skin state 158, either derived from user input, analysis of images, or a combination thereof, users may be able to determine products and regimens 118 that may work best for their skin state 158 by connecting to a database containing wellness 192, regimen 118, expert 105, service provider 111, and product information 190, wherein the information may comprise product ingredients, product claims, product indications, product pairing, product usage protocol, product ratings and rankings 138, and the like. By including rankings and ratings 138, community-lead recommendations may be made for skin related products adjusted for age, skin color, location, ethnicity, environmental factors, and the like. In an embodiment, the user may perform a recommendation request which may involve selecting a skin goal, such as moisturize, protect, cleanse, tone, beautify, anti-aging, wrinkle protection, skin tightening, deep cleanse, pore diminishing, treat rosacea, exfoliate, lighten skin, tan, sun protect, self-tan, treat acne, avoid pimples, improve luminosity, skin rejuvenation, treat spots, treat Crow's feet, hair removal, scar treatment, and the like. In embodiments, a skin goal may be automatically selected by the system 104. Automatic selection may be based on an aspect of the skin state 158. For example, if analysis 154 reveals that the skin is severely dry, the system may recommend moisturizing products for severely dry skin, or the system may recommend ingredients to look for in a product. The user may be able to purchase products directly from the recommendations page, such as by placing the product in an electronic shopping cart 113, or may be directed to another site for purchase. In an embodiment, the user may add the product to a wishlist 119 for future purchasing. In an embodiment, the user may add the product to a skin care shelf 114, which may be an interface to or depiction of a regimen 118 that enables users to organize their products and regimen 118 in a logical fashion based on the user's specific skin characteristics 130, by usage scenario (e.g. Morning, afternoon, night, etc.), intent (e.g. work, fun, etc.), and the like. The beauty shelf 114 may have multiple screens for recommendations by various bodies (e.g. Physicians, dermatologists, aestheticians, spa specialists, overall users, experts, people most like you, etc.). The beauty shelf 114 may be a personalized arrangement of products. Users may drag and drop products (or select to add) as they are surfing the web and discover new products as well as having auto-populated recommendations. The functionality may include a program that will highlight products of interest while surfing the web. The beauty shelf 114 may be an application that can also sit independently on social networking sites and other personal pages and or toolbars. The beauty shelf 114 may also indicate purchase date and purchase history, product expiration alerts and other usage updates. A purchase made off the website may automatically add to the user's beauty shelf 114, while manual entries for offline purchases may also be possible.

In an embodiment, the user may be able to obtain samples of recommended or non-recommended products directly from the recommendations page. The shopping cart 113 may be a functionality that integrates with the skin care shelf 114. Users may be able to use the personalized recommendations and select products either for purchase, or for sample delivery. The user may be prompted for personal information such as address, shipping method, credit card number and the like, and that information may be retained by the shopping cart 113. The shopping cart 113 may be an independent program, in similar fashion to the skin care shelf 114, that may reside in a toolbar, as part of a user interface 102 or as a program on a webpage, so that products could be highlighted and dragged into the shopping cart 113 for later purchase. Dragging the product into the cart 113 may also initiate queries across the database and across various websites for best price, location and availability of product, consumer experience, rankings and ratings and the like.

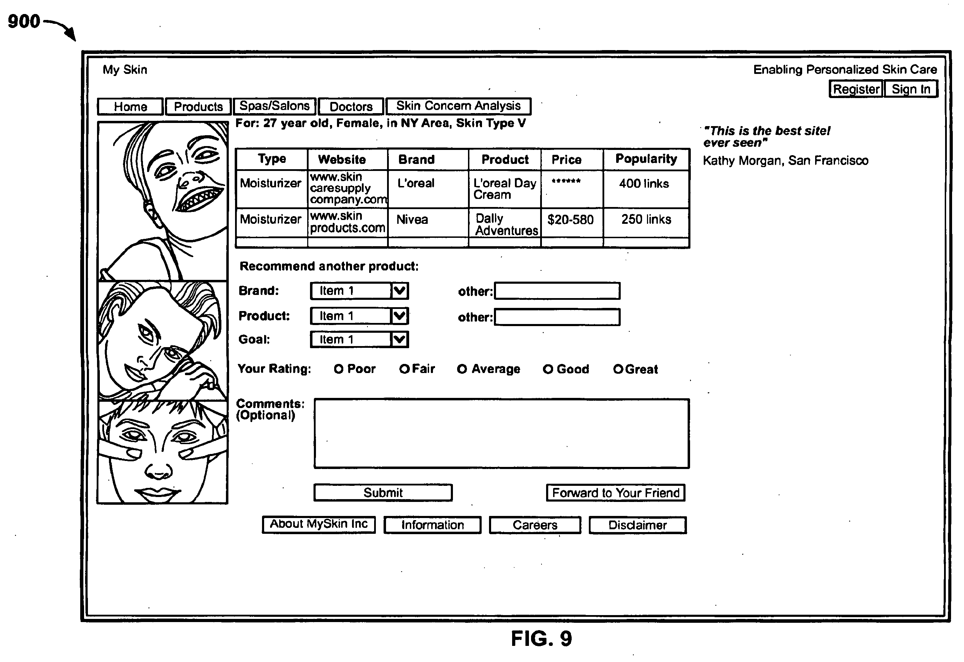

Referring to Fig. 9, a product rating page of a skin care system is depicted. To obtain recommendations, users may be asked to respond to their medical, non-medical, cosmetic and skin care product experiences, thereby scaling data collection inexpensively. For example, a user may identify a product and provide an effectiveness assessment, rankings and ratings 138 for the product, anecdotal information, usage information, and the like. This information may be stored in a wellness 192, regimen 118, and product information 190 database in order to refine future recommendations. In an embodiment, user responses to product experiences may be shared with friends and/or other users automatically or upon request.

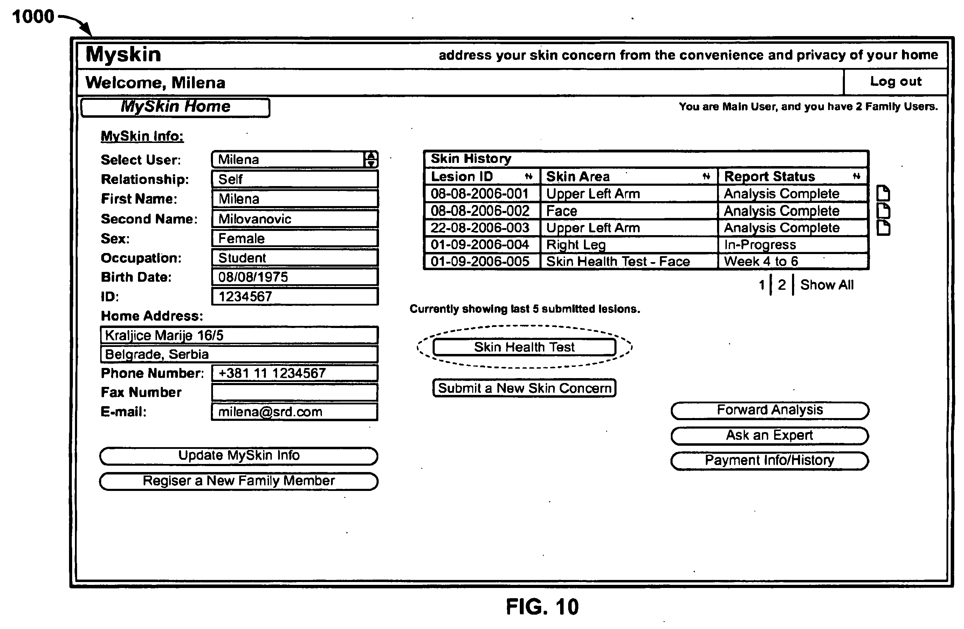

Referring to Fig. 10, a user interface 102 home page 1000 of a skin care system 104 is depicted. The user may be prompted to input demographic information such as name, gender, age, occupation, ID, address, telephone number, email address, payment information, new related users, and the like, which may be stored in a user profile or as part of a skin record 121. The home page may show a skin record 121, or a listing of areas imaged, date imaged, and status of analysis. Once a task is complete in the skin history/record 121, an icon may be displayed near the Status. The user may be able to launch a new Skin Health Test from the home page 1000 or submit a new skin concern. The user may be able to forward the analysis 154 to an interested party; Ask an Expert a question regarding an aspect of the skin, skin history/record 121, image analysis, and the like; view payment information and history; and the like.

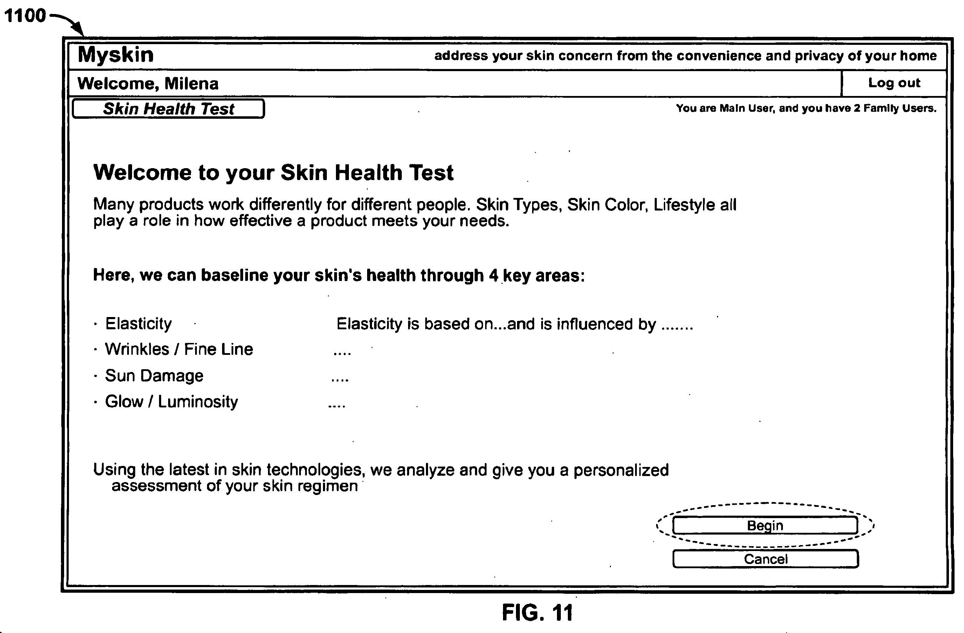

Referring to Fig. 11, a welcome page 1100 of a skin health test is depicted. The welcome page may provide information on the skin health test, what endpoints will be tested for, such as elasticity, wrinkles/ fine lines, sun damage, glow / luminosity, and the like. Using the analysis of the skin health test, the system may provide a personalized assessment of the user's skin regimen 118. The user may initiate the skin health test from the welcome page 1100.

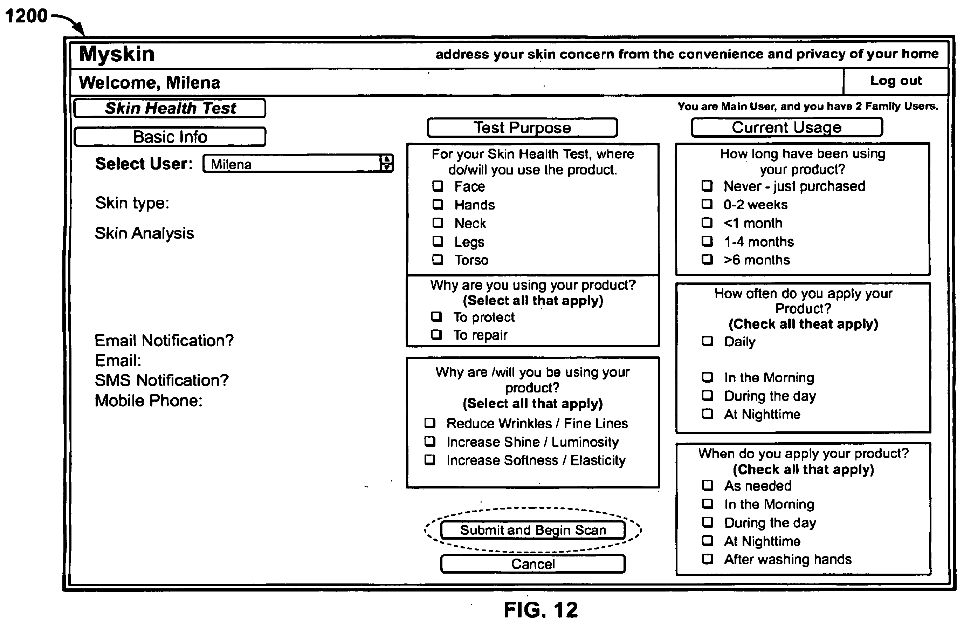

Referring to Fig. 12, a questionnaire page 1200 of a skin care system is depicted. The questionnaire may capture relevant skin history that may be useful for subsequent image analysis. The questions may be asked in multiple choice fashion or as open-ended questions. For example, a question may be 'Where do you use your product?' with responses including face, hands, neck, legs, torso, and the like. Another question may be 'Why are you using your product?' with responses including to protect, repair, moisturize, and any other skin care goal. Another question may be, 'Why are/will you be using your product?' with responses including reduce wrinkles / fine lines, increase shine / luminosity, increase softness / elasticity, and any other skin care goal. Other questions may include, 'How long have you been using your product?', 'How often do you apply your product?', 'When do you apply your product?', and the like, with responses including stated intervals of time. Other information gathered may be how the user prefers notification, where products were purchased, if the user employs a seasonal usage of products, and the like. From the questionnaire page 1200, the user may launch the skin health test.

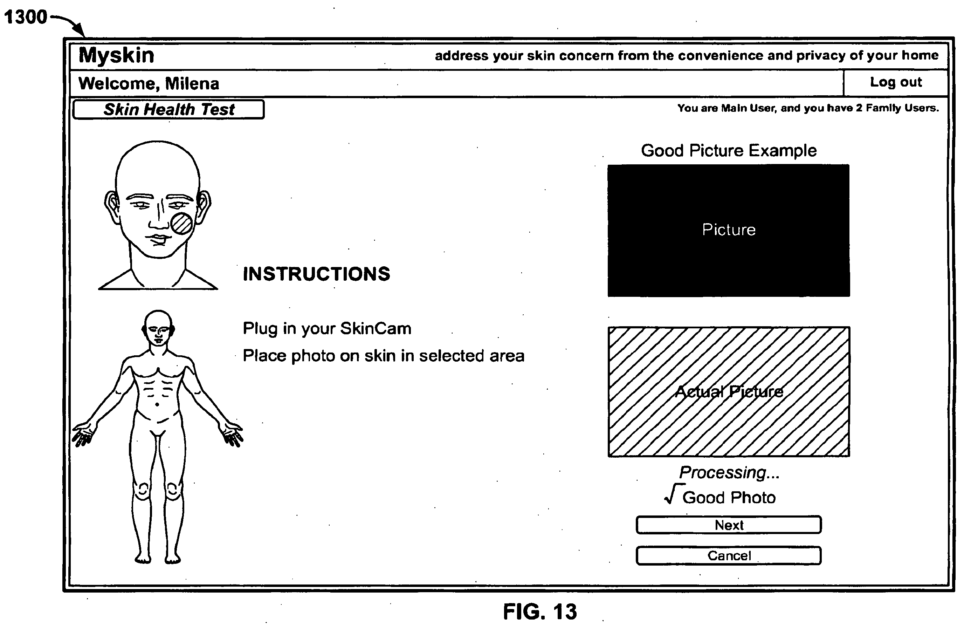

Referring to Fig. 13, a skin image capture page 1300 of a skin care system is depicted. In the example, the user interface 102 may access a device 108 in order to capture images, however, it should be understood that other devices 109 may be conveniently used in the system. The page 1300 may show a real time view of the area being imaged. The user may be able to employ positioning tools to be able to take an exact image of an area previously imaged. Once an image has been captured and submitted, an algorithm 150 may verify the integrity of the image. Once an image suitable for analysis has been captured, the user may proceed to an analysis page 1400.

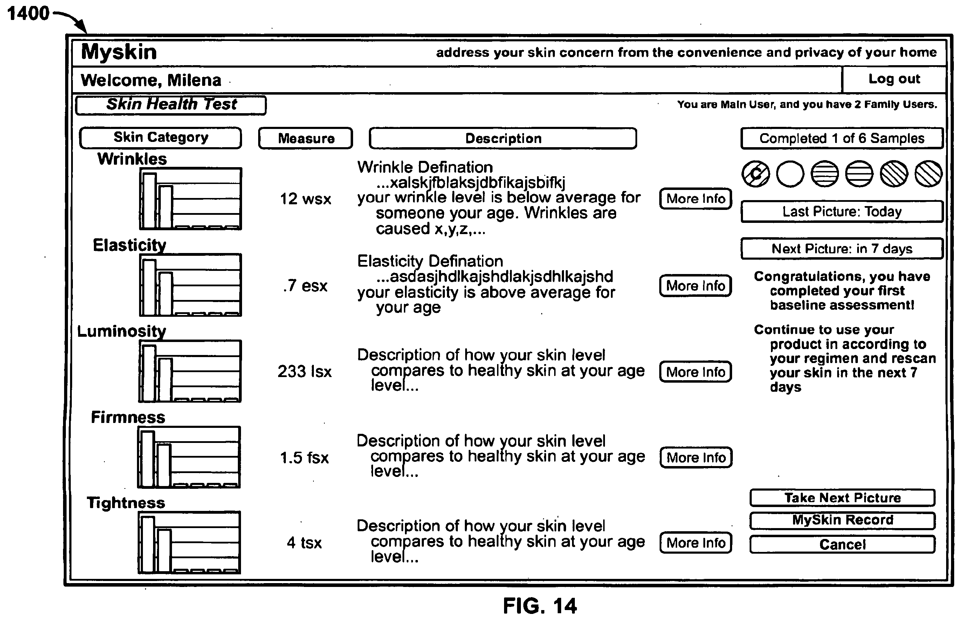

Referring to Fig. 14, a results page of a skin care system with bar graphs is depicted. Algorithms 150 may be used to analyze the image and provide measurements of wrinkles, elasticity, luminosity, firmness, tightness, and the like, as described previously herein. In an embodiment, the measurements may be quantitative measurements. The first analysis may be considered a baseline for purposes of tracking. For each measure, the user may be compared against the baseline for their age, skin state, gender, ethnicity, or any other category. For example, the graph depicts the reading for the user in the first bar on each graph and the average baseline for people of the same age in the second bar. It is apparent from visual inspection that the user is better than average, in this case. These results may be color-coded for ease of interpretation. The results page 1400 may include a description of each measure. The user may be able to request More Information for each of the measures, such as why a certain condition is caused and hints and tips on how to improve a skin condition. The user may be given instructions on when to re-scan the area, which products to use, which regimen 118 to employ, and the like. Desired improvements may be correlated to ingredients and most effective products for the user's skin may be recommended. The user may access and/or edit a skin record 121, which may contain information about the user, images, a chronology of images, information derived from the images, recommendations, products, regimen 118, and the like. The user may access a report facility to obtain a report.

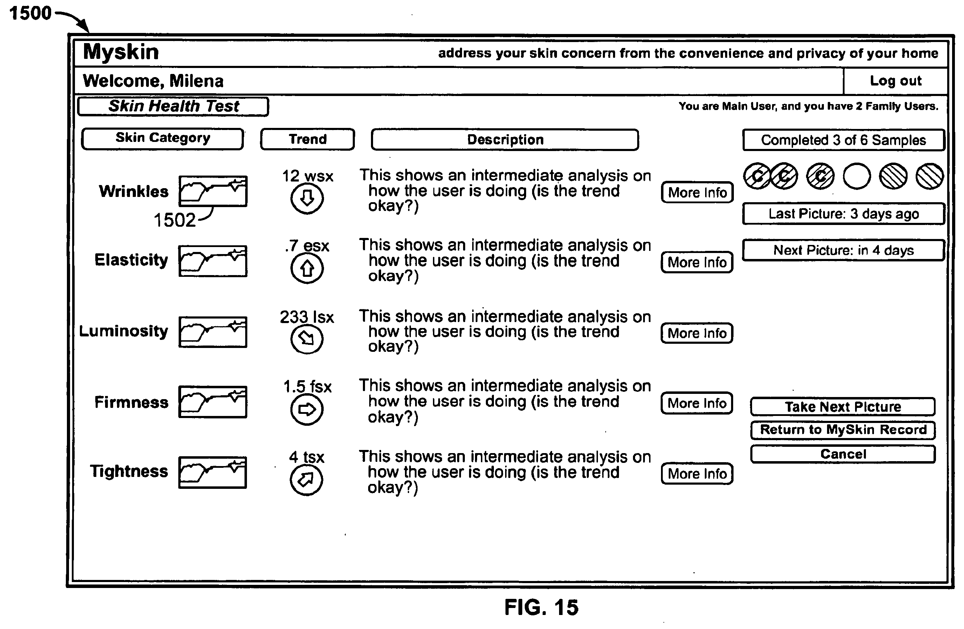

Referring to Fig. 15, a results page of a skin care system with trend analysis is depicted. A method for tracking the effectiveness of a skin care product or regimen may comprise obtaining a baseline skin health assessment; recommending a monitoring interval based on at least one of the skin care goal, product, and regimen; obtaining a second skin health assessment; comparing the second assessment to the baseline assessment to determine progress towards a skin care goal; and, optionally, optimizing the regimen 118 or product in order to improve a skin health assessment. When a subsequent image is acquired and submitted to the system 104, a trend analysis may be performed. Subsequent images may be used to track effectiveness of products and/or regimens 118 and, ultimately, advise the user on and optimize their skin regimen 118, product and/or condition. The trend analysis 1502 may be useful for determining an intermediate skin state 158 during a regimen 118. The trend analysis 1502 may show a baseline reading, an average reading for healthy skin for someone of the user's age, and individual measurements for each type of skin condition. Progress may be shown over time. A time series of images, such as over a twenty-eight day skin cycle, over a treatment timeframe, seasonally, periodically over a year and the like may be captured in order to track progress of a skin state 158. The data may be presented in a pictorial view with data on the picture, graphical view, trend view, numerical view, text view, and the like. Progress may be sorted by the concerns / skin care goals that the user may have indicated at the beginning of the test. The user may be told when to take the next image, how much longer to continue with a regimen 118, how to modify the regimen 118, be reassured about the effectiveness of a product or regimen 118, receive useful tips, and the like. The user may view and/or edit a skin record 121. The user may be able to view past images and perform a simulation 132 of future progress. The user may access a report facility to obtain a report.

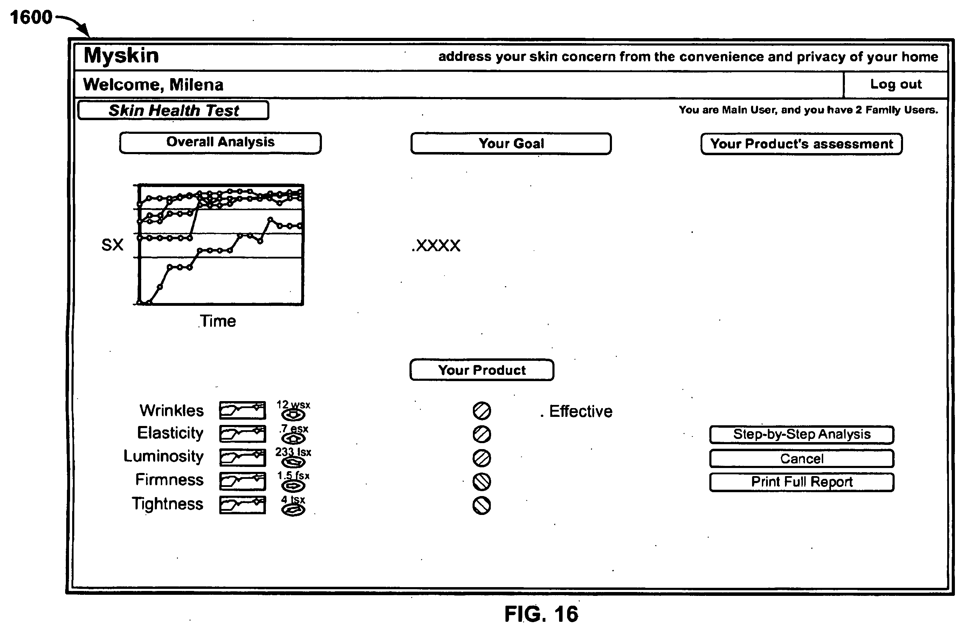

Referring to Fig. 16, a summary screen of a skin care system is depicted. An overall analysis for a time interval may be shown, current measurements, progress towards reaching a skin care goal, a product assessment, a regimen 118 assessment, advice on continuing, modifying, or terminating a regimen 118 or product usage, and the like. The user may view a step-by-step analysis or obtain a full report. At an interval, such as at the end of a suggested regimen 118, a report may include information on how the user's skin state 158 changed over time, if the user's skin is healthier than when they started the regimen 118, if the product or regimen 118 met their initial goals, feedback on regimen 118/ product effectiveness, and the like. Given the current skin state 158, a new product or regimen 118 may be recommended. For example, the system may recommend specific ingredients to look for in order to increase a user's luminosity given a current skin state 158. Reports may be on-screen, printed, custom, and the like. Reports may be shared with a practitioner for ongoing treatment and consultation.

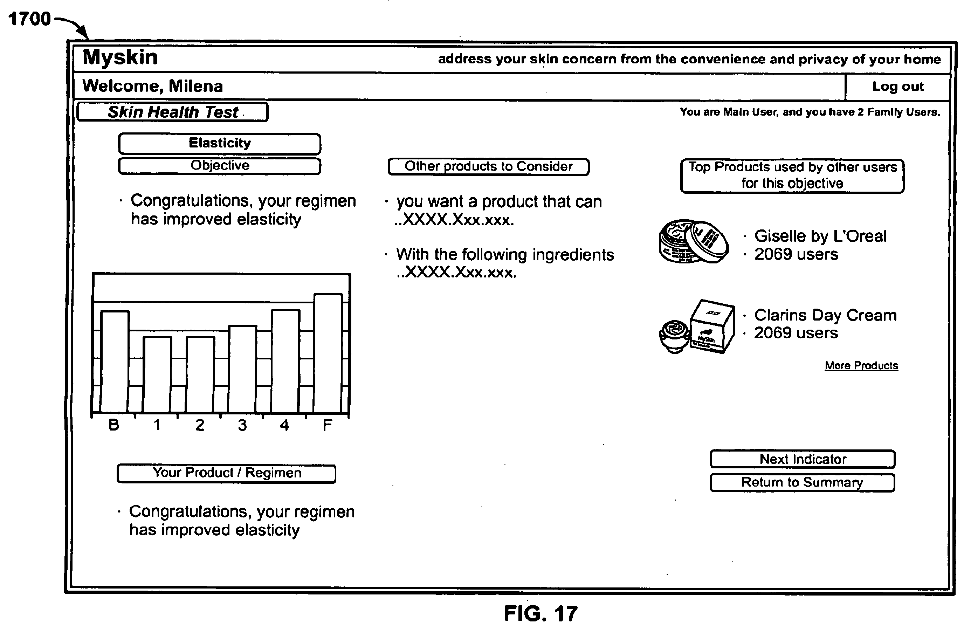

Referring to Fig. 17, an elasticity summary page 1700 of a skin care system is depicted. A step-by-step analysis of each indicator may be performed. For example, a step-by-step analysis of the elasticity measurement is shown in Fig. 17. The summary page 1700 may depict all of the data captured over an interval, such as in a bar graph, for each indicator on separate summary pages 1700. It should be understood that while Fig. 17 depicts an elasticity summary page, the summary page may summarize data related to any and all concerns. Progress towards meeting a skin care goal may be indicated by the data and its analysis or from user input. An assessment of a user's product or regimen 118 in meeting the skin care goal may be made. Products or regimens 118 that may enable meeting future needs may be indicated. The system may also indicate products used or regimens 118 employed by other users in meeting the stated skin care goal.

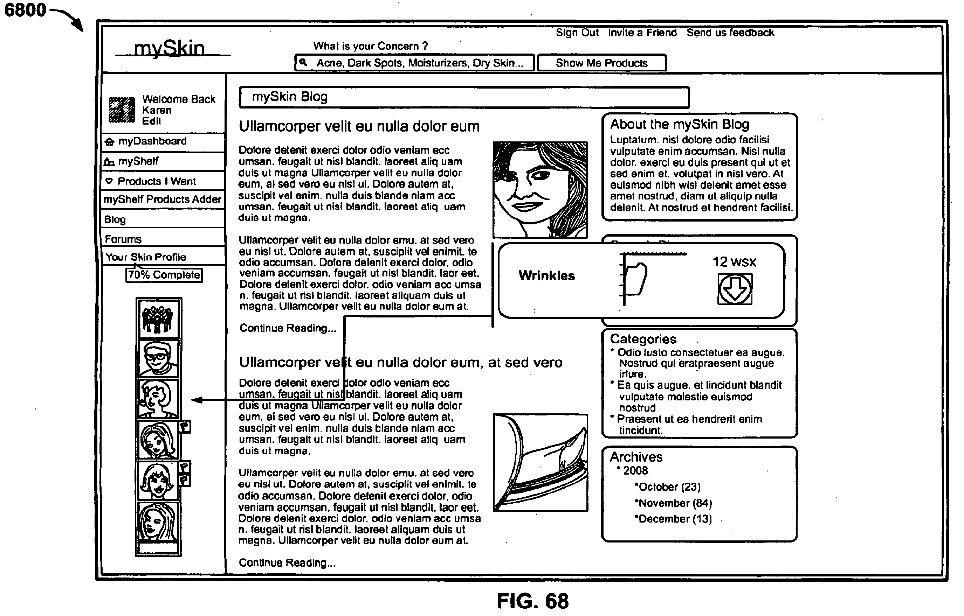

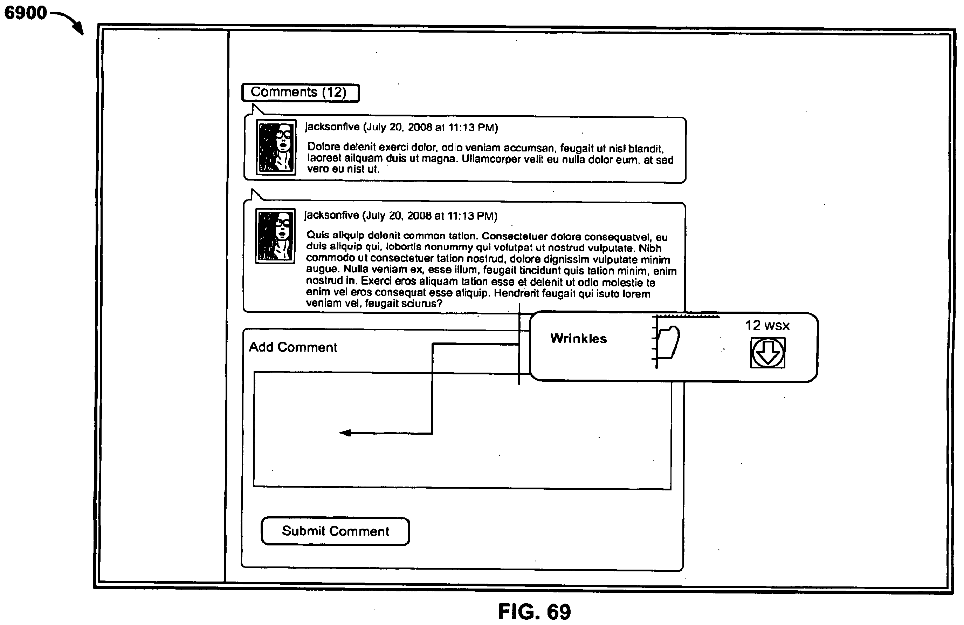

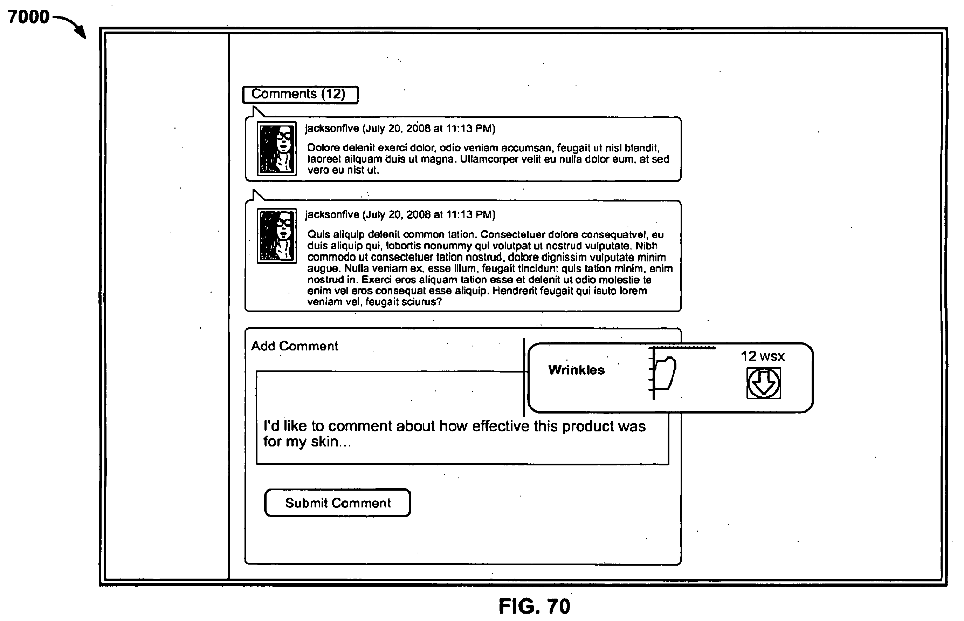

In an embodiment, the data acquired at a single timepoint or over a time interval may be shared with other users of the skin care system, practitioners, and the like. In an embodiment, the data may be shared as a data object with users of an online platform 120 or mobile platform 124 of the skin care system, posted to blogs, e-mailed to third parties, and the like. In some embodiments, the data may be a drag-and-droppable data object. For example, the wrinkle trend analysis 1502 shown in Fig. 15 may be shared with friends as in Fig. 68, posted on a blog or forum where users may discuss the data as in Fig. 69, become part of the content that a user may wish to discuss as in Fig. 70, and the like.

In embodiments, a system for providing recommendations for skin care based on a skin state 158, a skin care goal, and environmental factors affecting the skin may comprise interaction with tools and algorithms 150 on an online platform 120, a mobile platform 124, a social networking interface, and the like to receive product and regimen recommendations and track product and regimen 118 effectiveness. The system may be a communication platform, online 120 or mobile 124, that connects geographically separate consumers, manufacturers, product information, experts, service providers and others related to or allied to the beauty and medical field to provide personalized assessment regarding the consumers skin, hair, or nails queries and concerns. The user interface 102 may reside on an online platform 120, mobile platform 124, or social networking interface. In some embodiments, a skin care assessment may be provided by algorithms 150 operating on an online platform 120 without the use of images or data from a device 108, that is, a user need not have data from a device 108 to participate in the online platform 120. The online platform 120 may be a standalone skin health assessment and skin care recommendation tool. However, in embodiments, image data may also be used by the online platform 120 to provide skin health assessments and skin care recommendations. A user interface 102 may interface with the online platform 120. For example, a user may access an online platform 120 of the system for skin health analysis, monitoring, and recommendation to: monitor skin health, download, process, analyze, track, and store data from an imaging device 108 or other device 109 or monitor 182, receive product and /or regimen recommendations from an analysis/ API 154 or from peers, compare skin state 158 and regimen 118 with peers, receive product information 190, purchase products; add recommendations to a skin care shelf 114; organize a skin care shelf 114 by regimen 118, rankings, expiration date, cost, skin care goal, time of day, frequency, friends, and the like; view community ratings, rankings and comments on products/ regimen in a skin care shelf 114; rank/rate products; leave comments on products, regimens, peers products and/or regimens; and the like, receive new product alerts or product recalls, receive a daily report 134, interact with a social network 188, and the like. The user interface 102 may enable users to conveniently take and submit images, enter data, track history, obtain recommendations and analysis and perform a purchase regarding their skin, hair, and/or nail's beauty/cosmetic or medical concern. The user interface 102 may reside on an online platform 120 and guide the user while also serving as a data repository to maintain a skin record 121 and history tracking tool, and may help the user organize information relevant to their condition in a logical fashion.

In an embodiment, the user interface may comprise a skin care shelf 114. The skin care shelf 114 may be a structure that enables users to organize their products and regimen 118 in a logical fashion based on users' specific skin characteristics 130 / skin state 158 by usage scenario (such as morning, afternoon, night, and the like), intent (such as work, fun, etc.), skin care goal (such as moisture, glow, protect, and the like), and the like. The skin care shelf 114 may have multiple "pages" for recommendations by various entities (such as practitioner, physicians, dermatologists, aestheticians, spa specialists, overall users, experts, people most like you, and the like). The skin care shelf 114 may be a personalized arrangement of products, regimen 118, and/or information 190, 192. Users may drag and drop products (or select to add) as they are surfing the web and discover new products as well as having auto populated recommendations. The functionality may include a facility that may highlight products of interest while surfing the web. For example, a plug-in 194 may be used to allow a user to capture information from any location on the Internet. For example, a user may access a web page for a makeover article in a beauty magazine and wish to include the products from the makeover in their skin care shelf 114 and/or shopping cart.113. The user may click on the product name and drag it over to at least one of the skin care shelf 114 and shopping cart 113 to obtain additional product information 190, include in their regimen 118, purchase, request samples, and the like. The skin care shelf 114 may an application that may also sit independently on social networking sites 188 and other personal pages and or toolbars. The skin care shelf 114 may also indicate purchase date and purchase history, product expiration alerts and other usage updates. In an embodiment, a purchase made off a website may automatically add to the users' shelf 114, while manual entries for offline purchases may also be possible.

In an embodiment, the user interface 102 may interface with a mobile platform 124. The user interface 102 may support plug and play with various mobile devices 184 such as mobile phones, laptops, digital cameras, medical-use devices, and the like. For example, the mobile phone may have an attachment or an integrated feature that may enable a user to take an image of the skin and input/ capture data and have it connect via the web, wirelessly or via cable, to the user interface 102 and enable seamless connectivity and data transfer. The mobile device could be used to take images and data at various locations for obtaining various information from the community (such as at the beach to measure effectiveness of sun screen, an image of a specific location, a product image or a bar code image to get product feedback, best price, nearest physical selling location, coupons, and the like). Users may also be able to share data / ask questions regarding products instantaneously to other users. The mobile device could have an internal lens system that may be internally charges or an independently attached lens system that would enable using the battery power and light source of the device to take an image and use the in-built communication method for submitting the image.

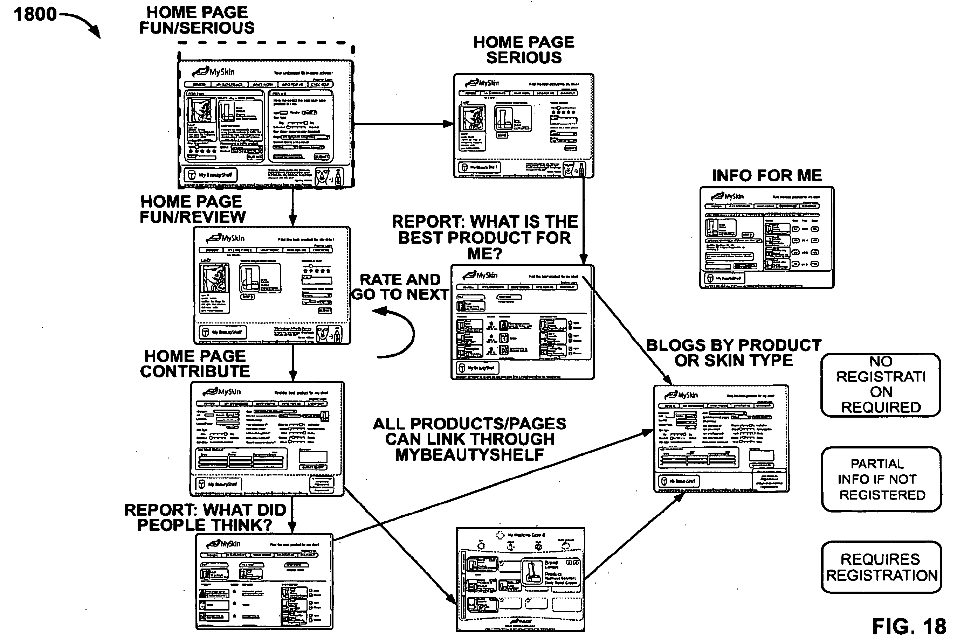

Referring to Fig. 18, the user interface for the online platform 120 may be depicted as a map. The home page may have a different theme or feel depending on the user profile, the user preference, or any other criteria. For example, it may be fun, serious, clinical, and the like. From the user interface, a user may review products, contribute anecdotes, report, review reports, review blogs by product, skin type, and the like, visit their beauty shelf 114, and the like. Information may be accessed freely, with registration, or only partially freely and partly with registration. All products and pages may link through the beauty shelf 114.

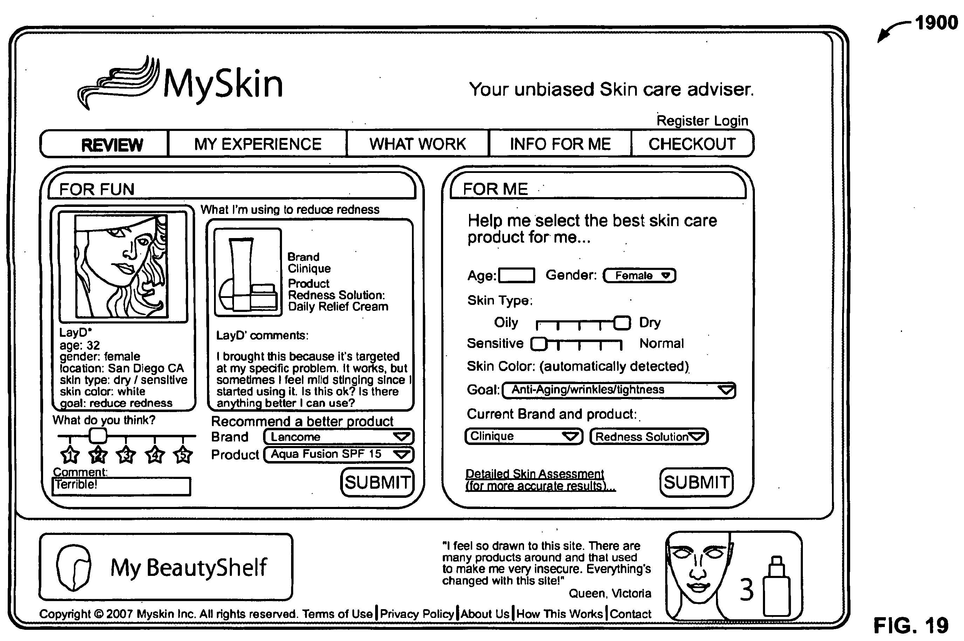

For example, Fig. 19 depicts a review page of the user interface of a skin care system. The menu across the top of the user interface may enable a user to access Reviews, Experience, Recommendation, Info For Me, Checkout, and the like. The user interface may depict a portion of the user profile, such as the age, gender, location, skin type, skin color, skin goal, picture, and the like for the user. The user interface may also depict what products or regimen 118 the user may be using and any associated review, rating, or comments of the product. Other users accessing a user profile may make comments on the regimen 118 or products in use, give the products or regimen 118 a rating, recommend a different product or regimen 118, and the like. The user interface may present tools to aid a user in selecting a product or regimen 118. For example, the tools may be in the form a questionnaire or wizard guising the user to describe their skin. The user may provide age, gender, skin type (oiliness, sensitivity), skin color, goal, current brand or product, current regimen 118 and the like. In some embodiments, the skin type and/ or color may be detected automatically if the user interface is interfaced with an imaging device 108. The user may also access their beauty shelf 114 from the user interface.

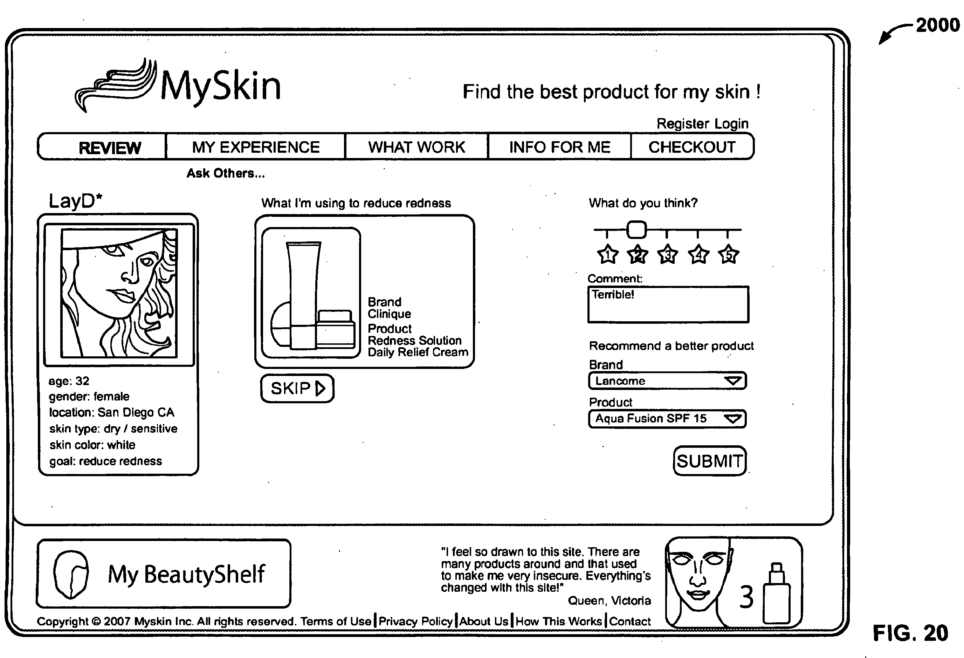

Referring to Fig. 20, a review page of a user interface of a skin care system is depicted. The review page is shown in a different layout than the compact view depicted in Fig. 19.

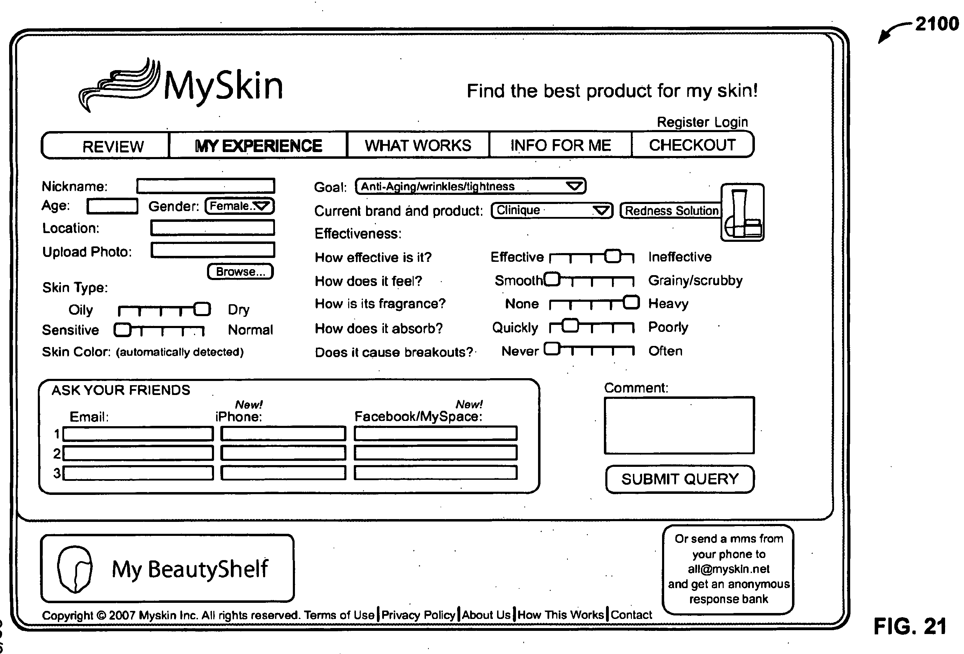

Referring to Fig. 21, an experience page of a user interface of a skin care system is depicted. The experience page allows users to provide a detailed report of experience with a product or regimen 118. For example, the user may note the effectiveness of a product or regimen 118, such as by answering questions. For example, the questions may be "How effective is it?", "How does it feel?", "How is its fragrance?", "How does it absorb?", "Does it cause breakouts?", and the like. The experience page may also allow a user to update a user profile with age, gender, nickname, location, a photo, skin type, skin color, goal, and the like. The user may be able to query other users for their experience or make a general inquiry by submitting a request to an email, MMS, SMS, phone number, mobile device, social network, and the like.

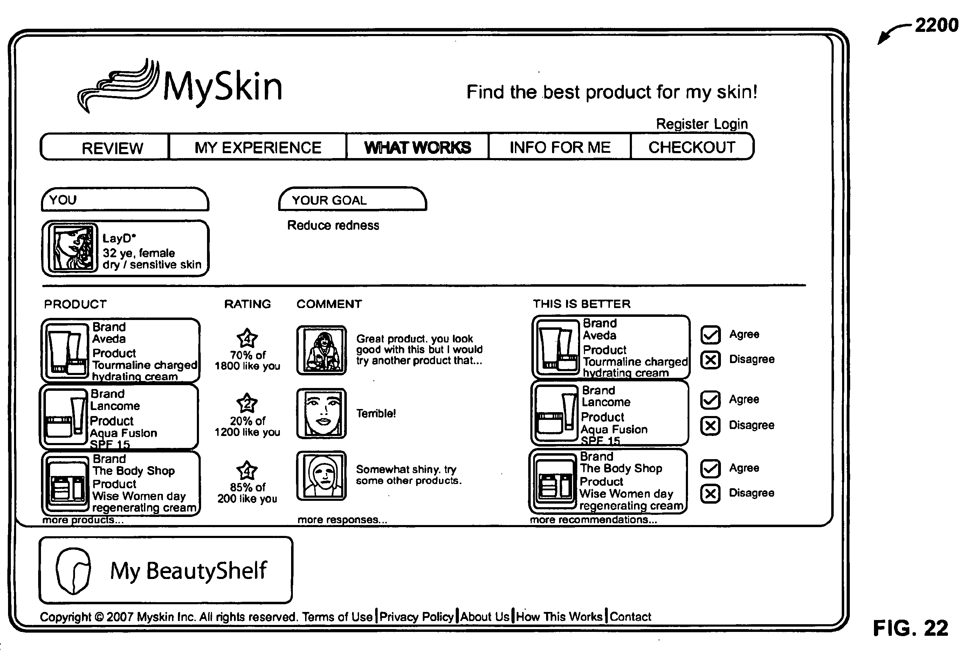

Referring to Fig. 22, a recommendation page of a user interface of a skin care system is depicted. Given the goal, various products or regimens 118 that may be effective in meeting the goal may be shown on the recommendation page. The brand and product or regimen 118 may be shown along with a rating from the community of users, comments from users, the ability to indicate of the user believes the product may better than the current product or regimen 118 in use, and the like. If the user believes the product or regimen 118 may be better than what they are currently using, the product or regimen 118 may be stored for future consideration on the beauty shelf 114.

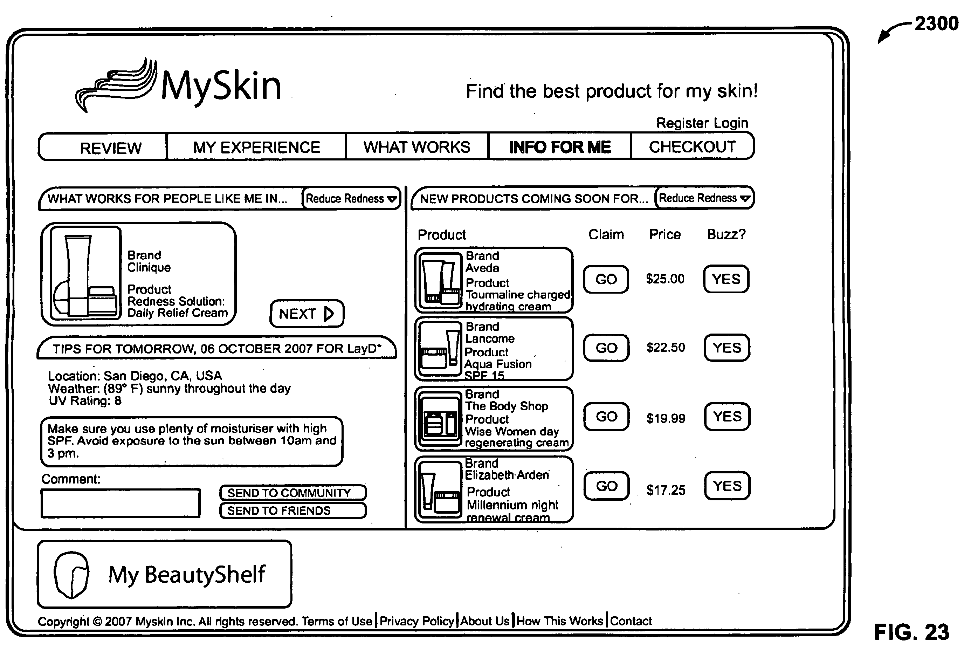

Referring to Fig. 23, an Info For Me page of a user interface of a skin care system is shown. A People Like Me algorithm 150 may be used to sort the community of users of the skin care system. Given the aspects of the user profile, the algoirthm 150 may determine which other users are most similar along all criteria, along custom-selected criteria, along a combination of skin color and skin type, and the like. Once the algorithm 150 has determined a subset of the community of users who are most like the user, the user can view data for the community. For example, the user can find out which products work best for the subset generally, for a specific issue, for a specific time of day, for a specific season, and the like. The Info for Me page may also depict the weather for the location given in the user profile and a UV rating and any specific tips given the location / weather/ environment. The Info for Me page may also alert the users of new products being launched. The user may sort the products according to effectiveness.

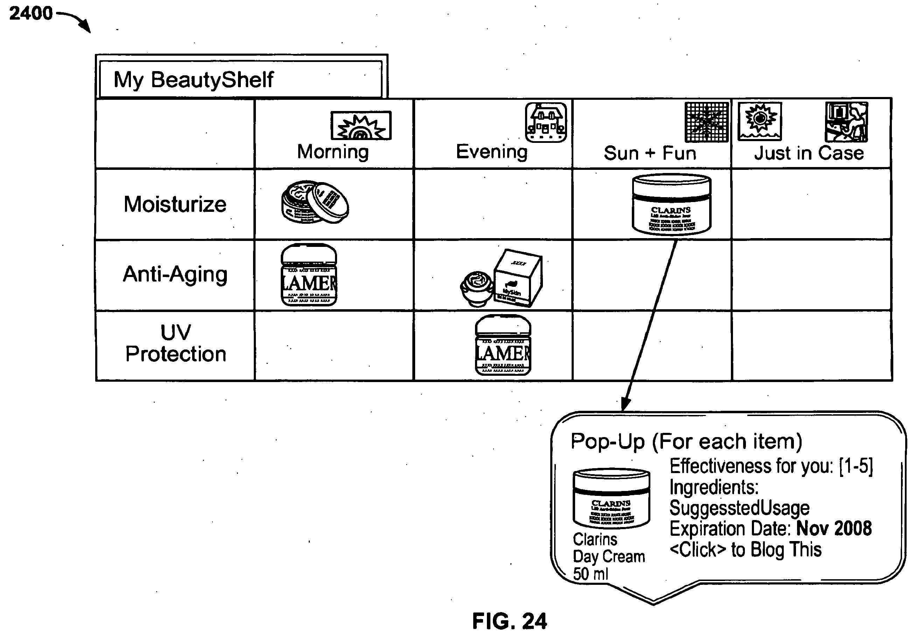

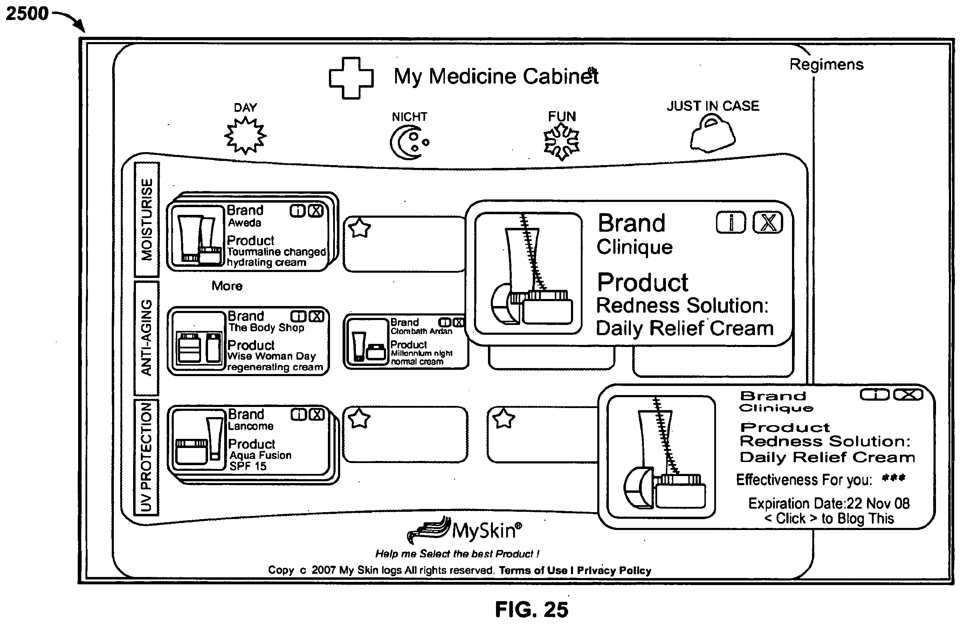

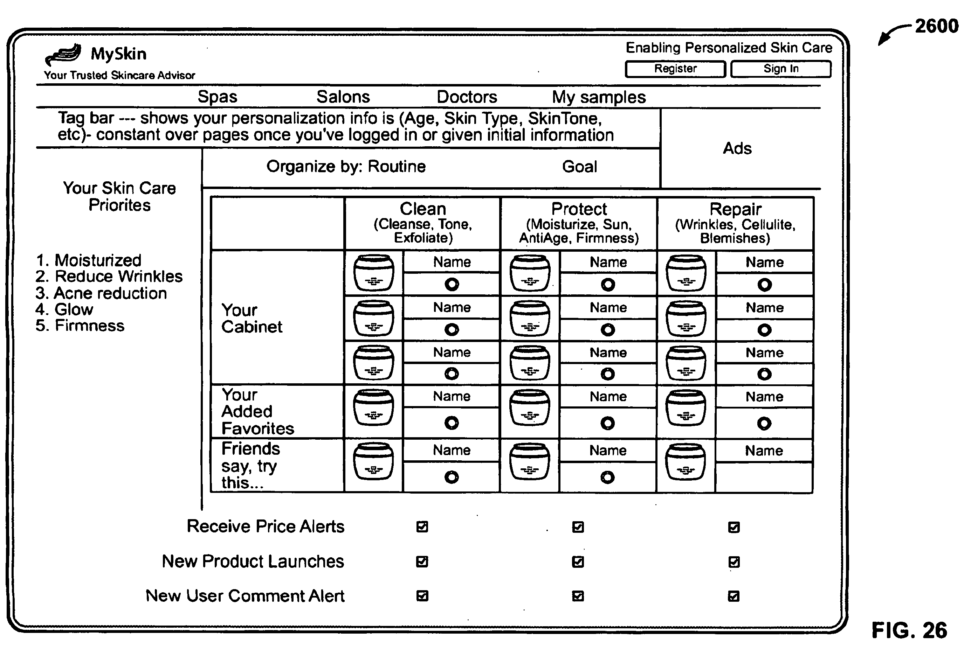

Referring to Fig. 24, an example of a beauty shelf 114 portion of a user interface of a skin care system is shown. Products or regimens 118 used by the user may be categorized by time of day use, specific effectiveness, cost, expiration, and the like. Each item may be clicked on to pop-up additional details about the product or regimen 118, such as effectiveness, ingredients, suggested use, expiration date, a link to purchase more, a link to blog about the product or regimen 118, a link to write a review or read reviews, a link to the manufacturer's site, a link to an in-store coupon, and the like. Fig. 25 depicts another example of a beauty shelf 114 portion of a user interface of a skin care system. Fig. 26 depicts an alternate view of the beauty shelf 114 of the user interface of a skin care system. In this example, friends have the ability to comment on the products or regimen 118 and suggest an alternative product or regimen 118. The user also has the option to receive price alerts, new product launch alerts, new user comment alerts, and the like.

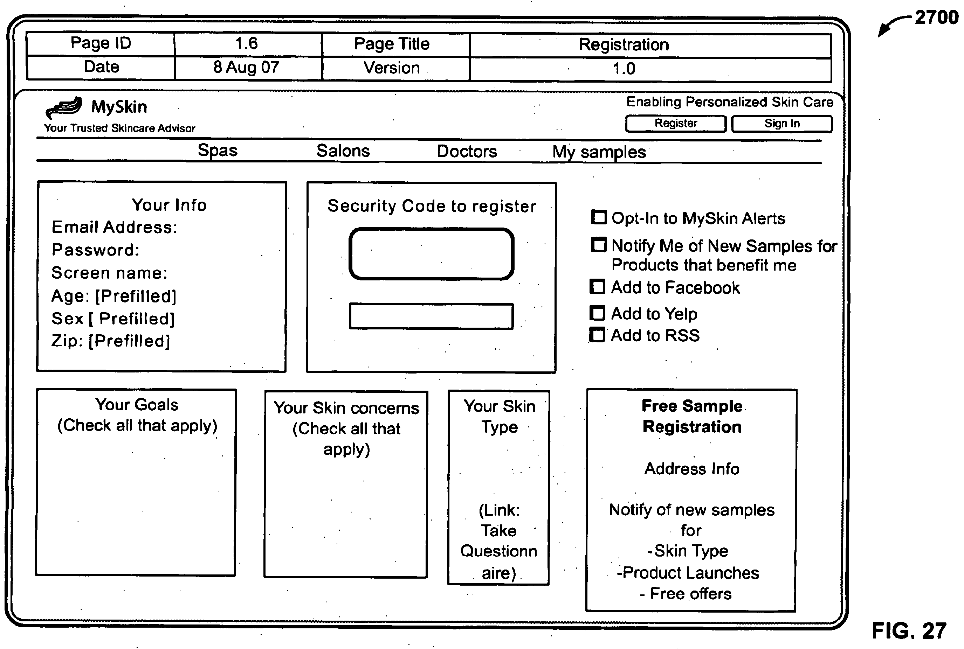

Referring to Fig. 27, a registration page of a user interface of a skin care system is depicted. Information may be entered by the user, goals may be indicated, a security code may be entered, skin concerns, color, and/or type may be entered, samples may be registered for, and the like. Additionally, the user may indicate that the want to add a feed from the skin care system to their RSS feed, and application from the skin care system to a social networking site, and the like. The user may have the option to opt-in to alerts, to be notified of samples and products, and the like.

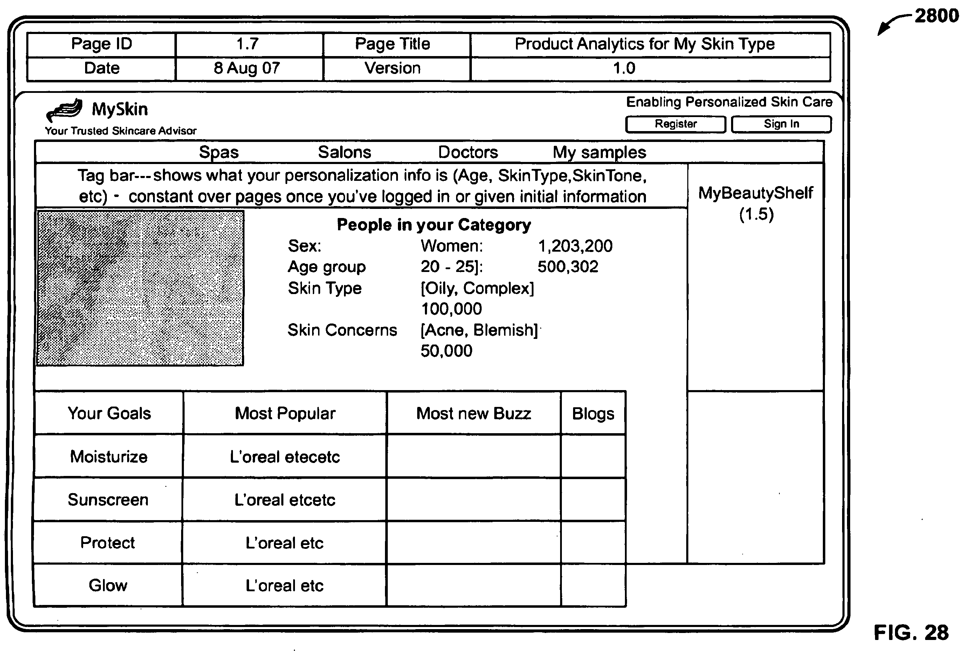

Referring to Fig. 28, another embodiment of a recommendation page of a user interface of a skin care system is shown. This page may show people in the user's category, such as number of people of the same gender, same age group, sith similar skin type, with similar concerns, and the like. For each stated goal, a product may be recommended that is most popular, has the most buzz, has been reviewed, has been rated, has been blogged about, and the like.

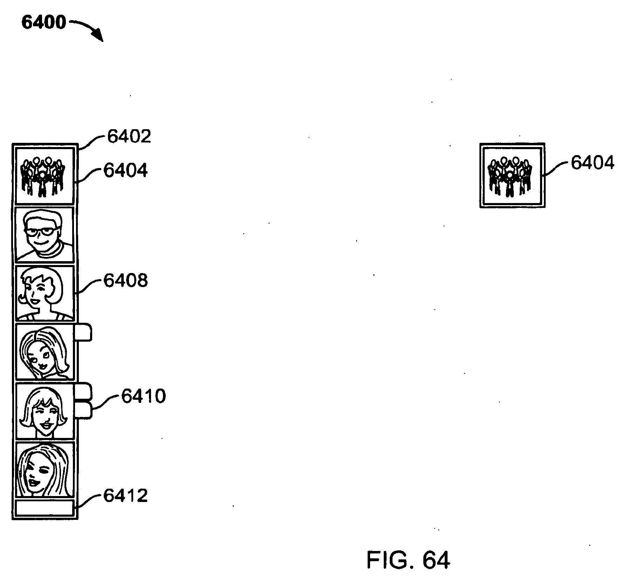

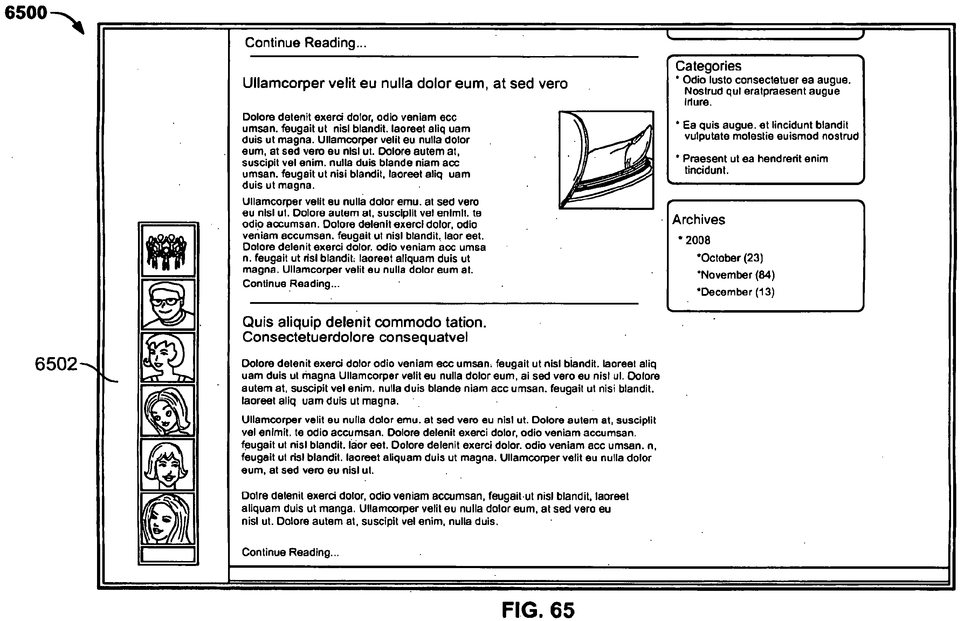

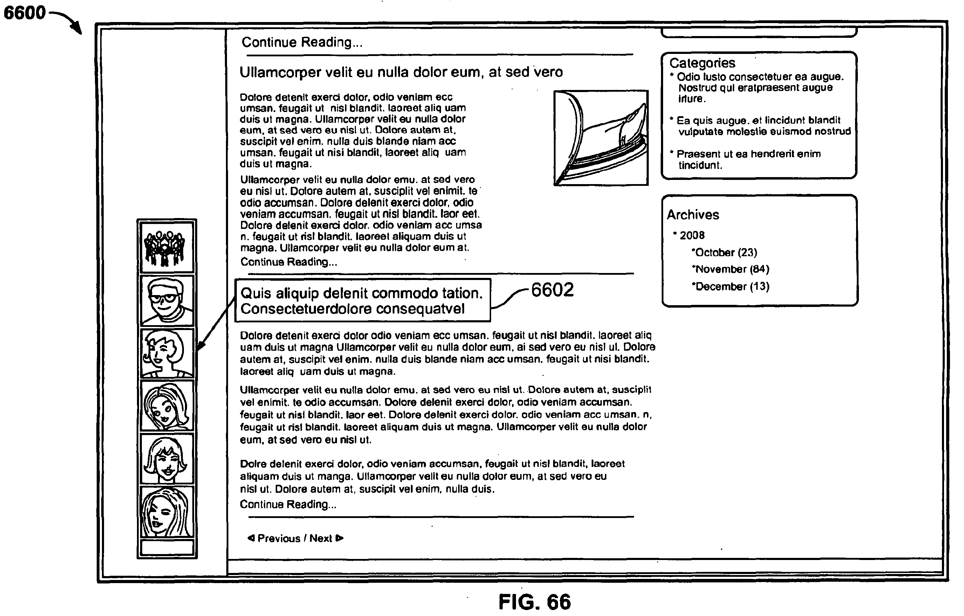

Referring to Fig. 64, the user interface may include a friend toolbar. The friend toolbar may float over a current website, or any website, such as by using a plug-in. Friends may upload images and the images 6408 may be displayed on the friend toolbar 6402. A home key 6404 may be part of the toolbar 6402, where the whole toolbar can be reduced to just the home key 6404. When an alert is associated with a friend, such as a new product being added to their beauty shelf 114 or a new review being written, a flag alert 6410 may pop-up next to their image on the toolbar 6402. A bottom bar 6412 may be used for shuffling friends or accessing other options related to the toolbar 6402. Referring to Fig. 65, the toolbar 6402 may auto-scroll 6502 as the user scrolls the webpage they are viewing. Referring to Fig. 66, objects may be shared with friends in the friends' toolbar 6402 using a drag-and-drop functionality 6602. For example, a blog posting may be shared as in Fig. 66 by dragging and dropping the blog title onto a friend's image. Similarly, products may be recommended to a friend by dragging and dropping 6702 the product into the friends' image, as in Fig, 67. Rolling over a friends' image may result in a pop-up, dialog box or other manifestation of additional information about the friend, such as a view of their user profile, beauty shelf 114, reviews, blogs, and the like.

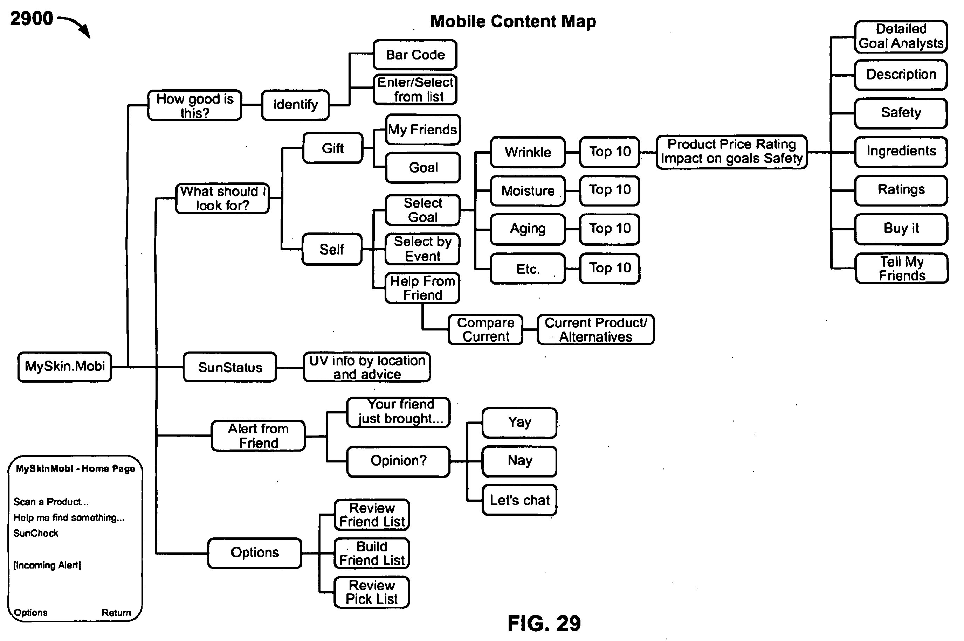

Referring to Fig. 29, a mobile content map for a mobile user interface of a skin care system on a mobile platform 124 is depicted. The content map depicted shows an example of content that can be accessed from a mobile platform 124 home page. For example, starting from the home page, a product may be scanned or identified from a list and searched for using the internet on the mobile device. For example, a bar code may be scanned for a product and prices, reviews, ratings and the like for the product may be returned. The user may be helped to find something, such as an item for themselves, a gift for a friend, and the like. The product may be searched for based on a goal, an issue, a skin type, a skin color, and the like. The mobile skin care system may return a list of products, such as the top 10 products, and information about the products such as rating, impact on goals, safety, reviews, and the like. The user may access a Suncheck application to be given UV information by location and advice, as well as based on an image captured by an imaging device 108 embodied in a mobile device, as described previously herein.

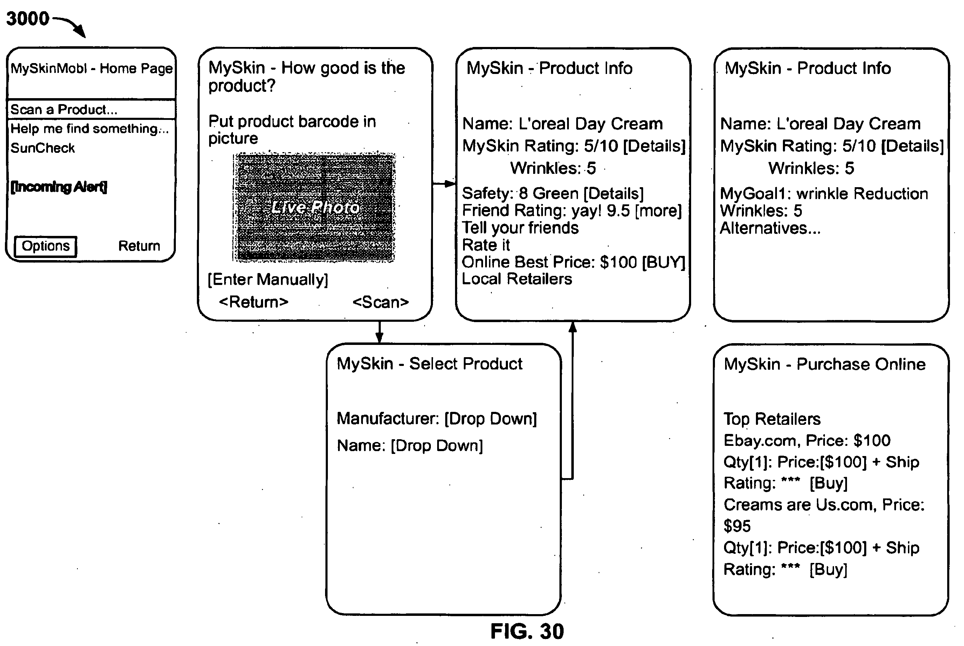

Referring to Fig. 30, a How Good Is This Product message flow is depicted. In the example, a bar code may be scanned to obtain product info, the bar code numbers may be manually entered, or the product may be chosen from a list. The system may return product information such as the product name, rating, ingredients, a general rating, a rating for a specific concern, a friend's rating, a price, where the product can be found, and the like. If the mobile device is enabled, a purchase may be initiated on the mobile platform 124.

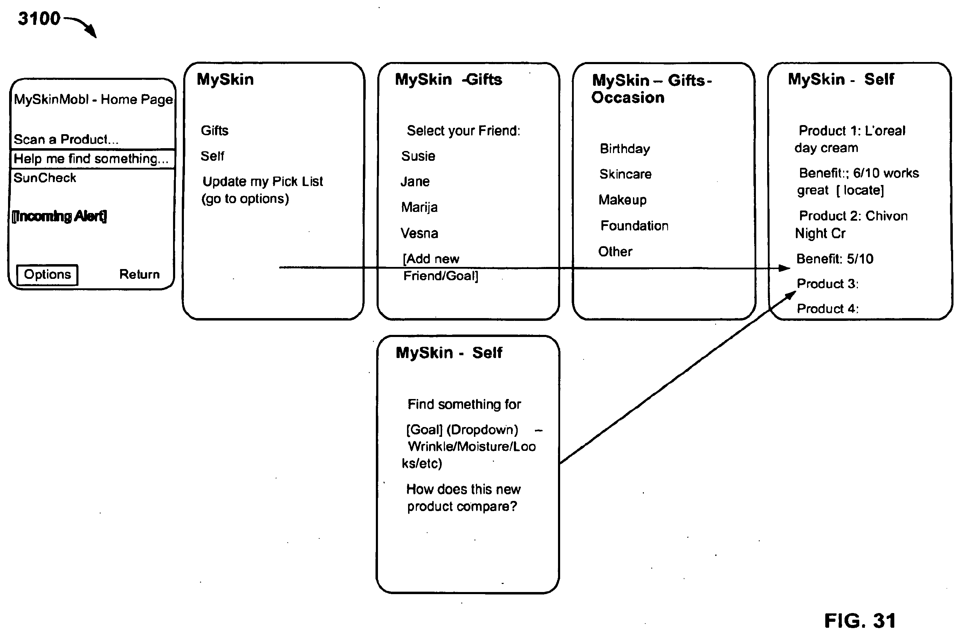

Referring to Fig. 31, a What Should I Look For? message flow is depicted. The message flow may begin by giving the user the option to indicate if the item searched for is a gift, for the user, to update a pick list, and the like. For gifts, a recipient may be selected from a pre-populated list or a new recipient may be indicated. An occasion may be indicated. Based on the recipient and occasion and any other criteria entered, products may be recommended along with any information associated with the product, a price, a location, and an option to purchase on the mobile platform 124. In looking for something for the user, the user may indicate a goal, such as from a drop down menu, and receive a list of recommended products. Once a product is selected, the user may request to locate the product at a store or initiate a purchase on the mobile platform 124, or the like.

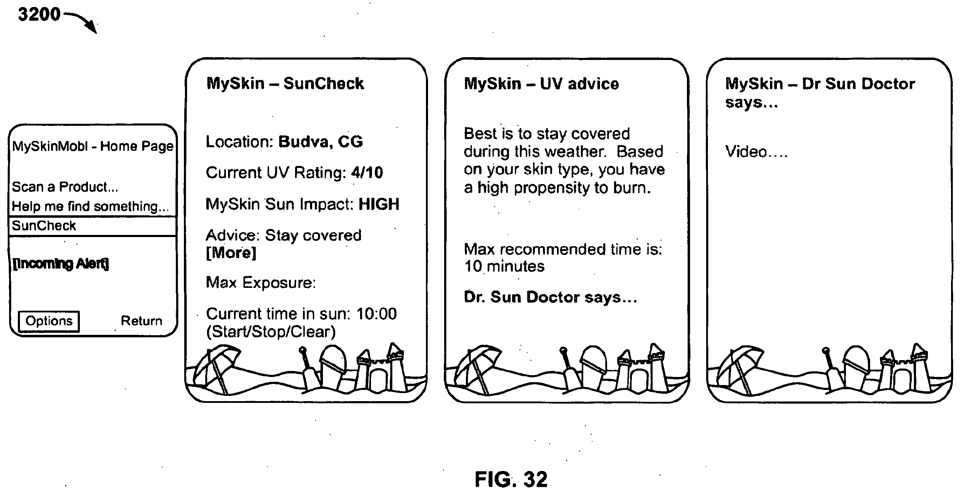

Referring to Fig. 32, a Suncheck message flow is depicted. The initial message may contain information about the user's location, the weather, a UV index, a sun impact rating, an indication of the maximum exposure time, and a timer for measuring the current time in the sun. Advice may be generated based on the information, such as what level of sun protection factor to apply, a maximum recommended time of exposure, and the like.

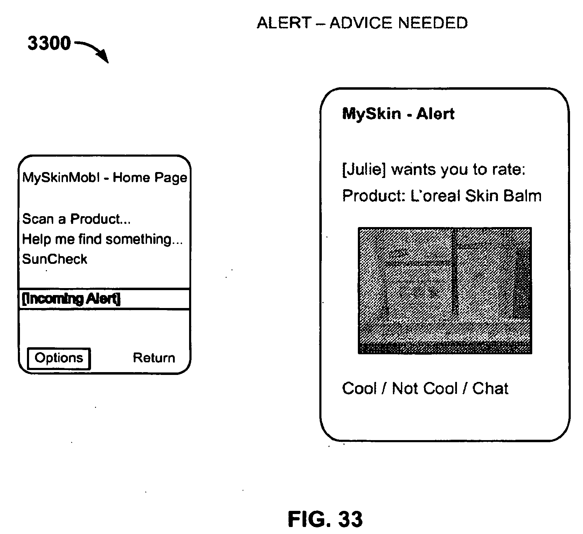

Referring to Fig. 33, an Alert message flow is depicted. The user may be linked to other users on the mobile platform 124 so that when another user requests a review or rating of a product, an alert may be sent to the user. The user may respond with a review, a rating, a chat message, an SMS, an MMS, a phone call, a voicemail, and the like.

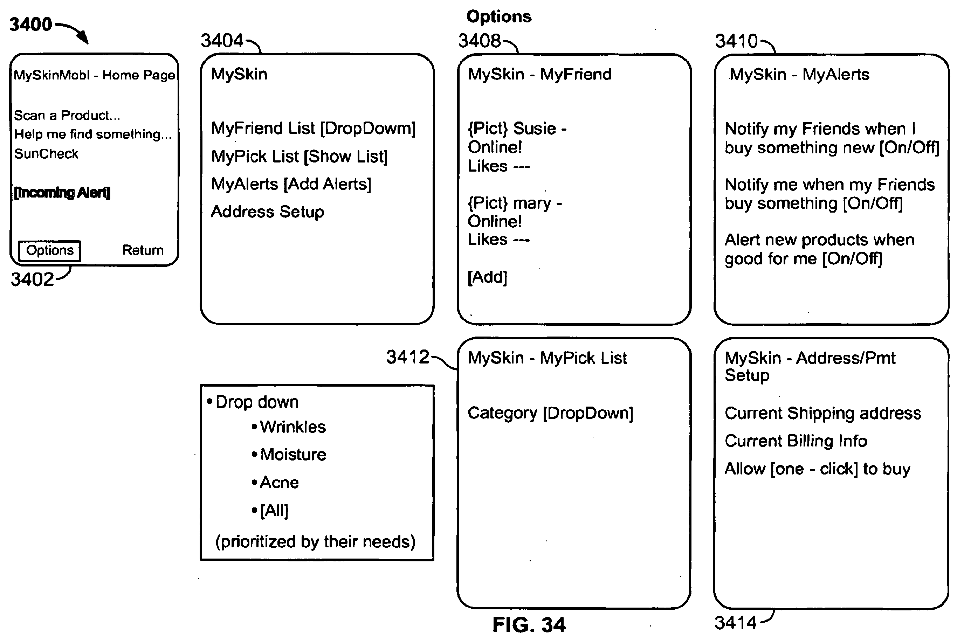

Referring to Fig. 34, an Options message flow is depicted. From the mobile platform 124 home page 3402, Options may be selected. Options 3404 may be a friend list, a pick list, alerts, address/location, and the like. For example, a friend list 3408 may be accessed to pick and choose friends to follow, receive alerts from and the like. The friends list may indicate if the friend is online. Alerts 3410 may also be set on the mobile platform 124, for example to notify the user when their friends buy something new, notify the user when a new product that is good for them is available, and the like. Address / location / payment setup may allow the user to initiate purchases from the mobile platform 124.

In certain arrangements, systems and methods for analysis of skin diseases (or disorders) by image processing detection (or image processing-based detection) of dermoscopic structures (or skin lesions) are disclosed. More particularly, there is disclosed the design and implementation of a system for automated diagnosis of seborrheic keratosis by image processing detection of multiple milia-like cysts or comedo-like openings and methods thereof. Still more specifically, there is a disclosed an improved system with enhanced qualitative and quantitative parameters, such as non-invasive, automatic, reliable, accurate and easily operable, for automated diagnosis of seborrheic keratosis by image processing detection of multiple milia-like cysts or comedo-like openings and methods thereof and a method for the design and implementation of such a system.

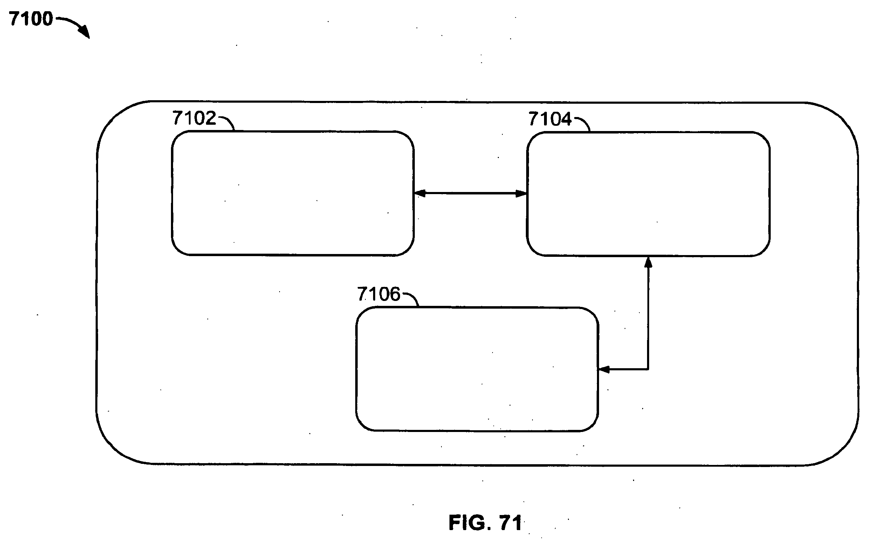

FIG. 71 is a schematic view of a system for automated diagnosis of skin disorders by image processing detection of skin lesions or dermoscopic structures, designed and implemented in accordance with at least some embodiments of the invention.

The system 7100 is in essence an Automatic Seborrheic Keratosis Diagnosis System (or ASKDS).

The ASKDS 100 consists of an illumination subsystem 7102, a sensor subsystem 7104 and a host computing subsystem 7106.

The ASKDS 100, by virtue of its design and implementation, facilitates automatic diagnosis of seborrheic keratosis based on detection of multiple milia-like cysts or comedo-like openings through image processing.

In certain embodiments, the ASKDS 7100 for automated diagnosis of skin disorders and processes thereof has been disclosed. Specifically, in such embodiments, the ASKDS 7100 comprises one or more illumination sources. The illumination sources comprise incident light sources to direct light upon skin. In consequence, the incident light sources may be unpolarized or polarized light sources. For example, and by no way of limitation, the unpolarized light may be white light, multiple selected wavelengths, or a single wavelength. Further, the illumination source may be positioned to direct light at a selected angle alpha. By way of example, and in no way limiting the scope of the invention, the ASKDS 7100 implements the processes for non-invasive processing including, but not limited to, imaging, analysis, and the like, as disclosed in United States Provisional Patent Applications "METHOD AND ALGORITHM FOR ANALYSIS OF LIGHT-MATTER INTERACTION BASED ON SPECTRAL CONVOLUTION" and "IMAGING DEVICE UTILIZING WHITE LIGHT FOR COMPSOITION ANALYSIS" and United States Non-Provisional Patent Applications "SYSTEM, DEVICE, AND METHOD FOR DERMAL IMAGING" to MYSKIN, INC. Thus, all remaining ins-and-outs in connection with the process of non-invasive processing of materials, both organic and inorganic, will not be further detailed herein.

As shown in the FIG. 71, in certain embodiments, the illumination subsystem 7102 may be coupled to the sensor subsystem 7104.

As shown in the FIG. 71, the sensor subsystem 7104 may in essence be a device that converts optical images (or optical signals) to electric signals. In certain embodiments, the sensor subsystem 7104 captures continuous digital images of skin. Specifically, in such embodiments, the sensor subsystem 7104 captures continuous digital images of the metallic surface illuminated with white light both, non-angled and angled. By way of, and by no way of limitation, the sensor subsystem 7104 may be anyone selected from a group consisting of a Complementary Metal-Oxide-Semiconductor (CMOS) image sensor, Charged Coupled Device (CCD) image sensor, and the like.

Again, as shown in FIG. 71, the sensor subsystem 7104 may be coupled to the host computing subsystem 7106 and the illumination subsystem 7102, respectively.

The term "digital image" refers to a representation of a two-dimensional image using ones and zeros (or binary digits or bits). The digital image may be of vector or raster type depending on whether or not the image resolution is fixed. However, without qualifications the term "digital image" usually refers to raster images.

Likewise, the term "digital imaging or digital image acquisition" refers to creation of digital images, typically from a physical object. The term is often assumed to imply or include the processing, compression, storage, printing and display of such images.

Digital image processing is the use of computer algorithms to perform image processing on digital images. As a subfield of digital signal processing, digital image processing has many advantages over analog image processing; it allows a much wider range of algorithms to be applied to the input data, and can avoid problems such as the build-up of noise and signal distortion during processing.

For example, and in no way limiting the scope of the invention, in certain embodiments the sensor subsystem 7104 may be selected on the basis of the following specifications: color is color or monochrome; optical format; horizontal pixels X vertical pixels; pixel size; one or more performance parameters, such as maximum frame rate, data rate, maximum power dissipation, quantum efficiency, dynamic range and supply voltage; output; one or more features, such as integrated Analog-to-Digital Converter (ADC) and microlenses; and environment, such as operating temperature.

In certain embodiments, the host computing subsystem 7106 may comprise a skin disorder management module designed and implemented, in accordance with the principles of the invention.

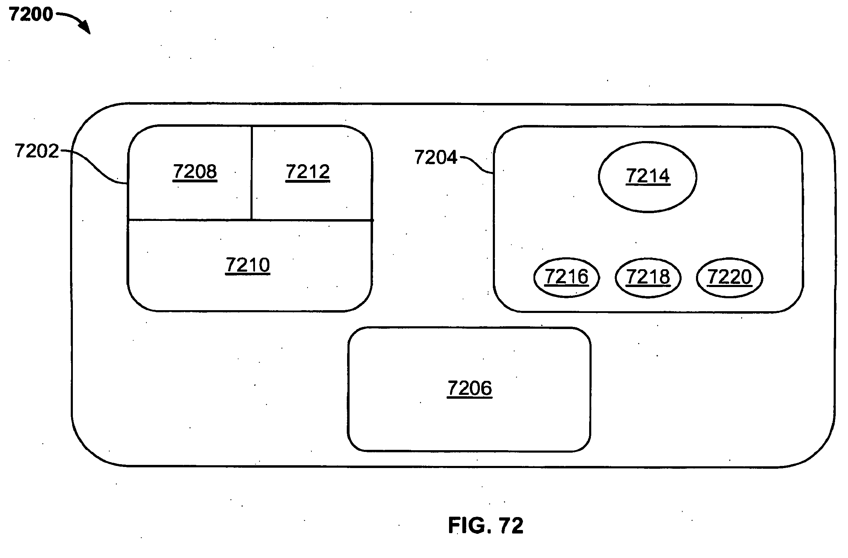

FIG. 72 is an exploded diagrammatic representation of the host computing subsystem, of the Fig. 71, comprising the skin disorder management module designed and implemented in accordance with at least some embodiments.

The host computing subsystem 7200 may comprise a processing unit 7202, a memory unit 7204 and an Input / Output (or I/O) unit 7206 respectively.

The host computing subsystem 7200, by virtue of its design and implementation, performs overall management of one or more disorders of skin.

The processing unit 7202 may comprise an Arithmetic Logic Unit (or ALU) 7208, a Control Unit (or CU) 7210 and a Register Unit (or RU) 7212.

The memory unit 7204 comprises a skin disorder management module 7214.

In certain embodiments, the skin disorder management module for real- or point-time analysis of the continuously captured digital skin information and methods thereof is disclosed, in accordance with the principles of the invention. Specifically, in such embodiments, the skin disorder management module captures the skin information using at least one of Diffused Reflectance Spectroscopy, Red (R)-Green (G)-Blue (B) analysis of re-emitted white light and any combination thereof.

The terms "Diffused (or Diffuse) Reflectance Spectroscopy (or DRS)" and "Diffuse Reflectance Infrared Fourier Transform Spectroscopy (DRIFTS)" refer to a technique that collects and analyzes scattered Infrared (or IR) energy. It is used for measurement of fine particles, powders as well as rough surface. Specifically, it assesses the interaction of a surfactant with the inner particle or the adsorption of molecules on the particle surface. In DRS or DRIFTS, sampling is fast and easy because little or no sample preparation is required.

In certain other embodiments, the skin disorder management module may comprise one or more processes for determination of an assortment of qualitative and quantitative parameters thereby facilitating overall management of disorders of skin. In such embodiments, at least a first process of the one or more processes determines moisture levels of skin. Specifically, this process may comprise one or more phases comprising emission of incident electromagnetic signals to skin, detection of degree of polarization of the electromagnetic signals reflected or re-emitted from skin and determination of the moisture levels based on the amount of polarized and reflected or re-emitted electromagnetic signals. Yet, in such embodiments, the first process may comprise one or more phases comprising combination of the determined moisture levels with skin color measurements thereby resulting in determination of skin luminosity.

Still, in certain such embodiments, at least a second process of the processes determines elasticity of skin. Specifically, this process may comprise one or more phases comprising the emission of the incident electromagnetic signals to skin, detection of a first aspect of polarization of the electromagnetic signals reflected by skin, correlation of the aspect of polarization with a concentration of elastin and determination of elasticity level based on the concentration of elastin.

Still further, in certain such embodiments, at least a third process of the processes determines firmness of skin. Specifically, this process may comprise or more phases comprising the of the incident electromagnetic signals to skin, the detection of a second aspect of polarization of the electromagnetic signals reflected by skin, the correlation of the aspect of polarization with the concentration of at least one of the elastin, a collagen, an activity of a sebaceous gland and any combination thereof and determination of the firmness based on the concentration of at least one of the elastin, collagen and sebaceous gland activity. In such embodiments, the sebaceous gland activity may be indicated by at least one of a number of glands, percent of glands open / closed and level of clog / fill.

Yet, in certain such embodiments, at least a fourth process of the processes obtains biophysical properties and may comprise performing a spectral analysis of image data acquired from the degree of polarization of reflections and absorption and re-emission of incident light from skin. Specifically, the biophysical properties is at least one of a structure, form, concentration, number, size, state, and stage of at least one of a: melanocyte, melanin, hemoglobin, porphyrin, keratin, carotene, collagen, elastin, sebum, sebaceous gland activity, pore (sweat and sebaceous), moisture level, elasticity, luminosity, firmness, fine line, wrinkle count and stage, pore size, percent of open pores, skin elasticity, skin tension line, spot, skin color, psoriasis, allergy, red area, general skin disorder or infection, tumor, sunburn, rash, scratch, pimple, acne, insect bite, itch, bleeding, injury, inflammation, photodamage, pigmentation, tone, tattoo, percent burn/ burn classification, mole (naevi, nevus), aspect of a skin lesion (structure, color, dimensions/asymmetry), melanoma, dermally observed disorder, cutaneous lesion, cellulite, boil, blistering disease, congenital dermal syndrome, (sub)-cutaneous mycoses, melasma, vascular condition, rosacea, spider vein, texture, skin ulcer, wound healing, post-operative tracking, melanocytic lesion, non-melanocytic lesion, basal cell carcinoma, seborrhoic keratosis, sebum (oiliness), nail- and/or hair-related concern, and the like.

Alternatively, in certain embodiments, there is disclosed a system for obtaining dermal biophysical properties, designed and implemented in accordance with the principles of the invention. In certain such embodiments, the skin disorder management module facilitates acquisition of dermal biophysical properties.

As shown in the FIG. 72, the skin disorder management module 7214 comprises a Fourier transform sub-module 7216, a spectral analyzer sub-module 7218 and a diagnostics sub-module 7220.

In certain embodiments, the Fourier transform sub-module 7216 is in essence a Discrete-Time Fourier Transform (or DTFT).

The term "DTFT", as used herein, refers to one of the specific forms of Fourier analysis. As such, it transforms one function into another, which is called the frequency domain representation, or simply the "DTFT", of the original function, which is often a function in the time-domain. But, the DTFT requires an input function that is discrete. Such inputs are often created by sampling a continuous function, like a person's voice. The DTFT frequency-domain representation is always a periodic function. Since one period of the function contains all of the unique information, it is sometimes convenient to say that the DTFT is a transform to a "finite" frequency-domain (the length of one period), rather than to the entire real line.

The DTFT 7216 converts time-domain digital signals into corresponding frequency-domain digital signals.

The DTFT 7216 is coupled to the spectrum analyzer sub-module 7218.

As used herein, the term "spectrum analyzer" refers to a device used to examine the spectral composition of some electrical, acoustic, or optical waveform. It may also measure the power spectrum. In general, there are three types of spectrum analyzers, such as analog, digital and real-time spectrum analyzers. Firstly, an analog spectrum analyzer uses either a variable band-pass filter whose mid-frequency is automatically tuned (i.e. shifted, swept) through the range of frequencies of the spectrum to be measured or a superheterodyne receiver, wherein the local oscillator is swept through a range of frequencies. Secondly, a digital spectrum analyzer computes the Discrete Fourier transform (or DFT), a mathematical process that transforms a waveform into the components of its frequency spectrum. Eventually, some spectrum analyzers, such as "real-time spectrum analyzers", use a hybrid technique where the incoming signal is first down-converted to a lower frequency using superheterodyne techniques and then analyzed using fast Fourier transformation (FFT) techniques.

In operation, the illumination subsystem 7102 illuminates the skin. It may be noted here that all ins-and-outs in connection with the illumination subsystem 7102 has been disclosed earlier and thus will not be detailed herein. The sensor subsystem 104 captures the electromagnetic signals reflected, absorbed and re-emitted from the skin. As mentioned earlier, the ADC integrated in the sensor subsystem 7104 converts the analog electromagnetic signals into corresponding digital signals. The skin disorder management module 7214 of the host computing subsystem 7106 facilitates automated diagnosis of seborrheic keratosis based on detection of multiple milia-like cysts or comedo-like openings through image processing. Specifically, the DTFT 7216, of the skin disorder management module 7214, converts time-domain digital signals into corresponding frequency-domain digital signals. The spectrum analyzer sub-module 7218, of the skin disorder management module 7214, performs a spectral analysis of the corresponding frequency-domain digital signals. The diagnostics sub-module 7220, of the skin disorder management module 7214, detects the presence of one or more skin lesions or dermascopic structures, such as milia-like cysts or comedo-like openings through implementation of suitable image processing algorithms.

In certain other embodiments, the host computing subsystem configuration, discussed in conjunction with FIG. 72, implements one or more processes facilitating acquisition of biophysical properties of organ systems, analysis of characteristics of the organ systems and determination of a state of the organ systems. Specifically, the processes comprise one or more sequences of process stages comprising acquisition of dermal biophysical properties of skin, analysis of the skin characteristics and determination of a skin state and potential permutations and combinations thereof.

Specifically, in certain such embodiments, a customized image processing algorithm (not depicted herein), designed and implemented in accordance with the principles of the invention, may be useful for the analysis of skin characteristics, obtaining the biophysical properties of the skin and determining a skin state. The skin state may capture a combination of underlying skin structure with time-based variance. Some variation may be predictable but some may be based on a transient condition like infection, sunburn, hormonal imbalance, and the like. The algorithm may be able to measure aspects such as the structure, form, concentration, number, size, state, stage, and the like of melanocytes / melanin, hemoglobin, porphyrin, keratin, carotene, collagen, elastin, sebum, sebaceous gland activity, pores (sweat and sebaceous), wrinkles, moisture, elasticity, luminosity, all forms of the aforementioned, such as derivatives, salts, complexes, and the like. The algorithm may be used to make a quantitative assessment of clinical, medical, non-medical, and cosmetic indications, such as moisture level, firmness, fine lines, wrinkle count and stage, pore size, percent of open pores, skin elasticity, skin tension lines, spots, skin color, psoriasis, allergies, red areas, general skin disorders and infections, or other skin related concerns for the user such as tumors, sunburns, rashes, scratches, pimples, acne, insect bites, itches, bleeding, injury, inflammation, photodamage, pigmentation, tone, tattoos, percent burn / burn classification, moles (naevi, nevus), aspects of skin lesions (structure, color, dimensions / asymmetry), melanoma, dermally observed disorders and cutaneous lesions, cellulite, boils, blistering diseases, management of congenital dermal syndromes, (sub)-cutaneous mycoses, melasma, vascular conditions, rosacea, spider veins, texture, skin ulcers, wound healing, post-operative tracking, melanocytic lesions, non-melanocytic lesions, basal cell carcinoma, seborrhoic keratosis, sebum (oiliness), nail- and / or hair-related concerns, and the like. The algorithm may also be useful for the analysis of and obtaining the physical properties and composition of hair, nails, biological substances, gaseous substances, food, wine, water, liquid, metal, non-metals, plastics, polymers, and the like. Either manually or as determined by an algorithm, a targeted wavelength or wavelengths may be employed for specific endpoint measurements.

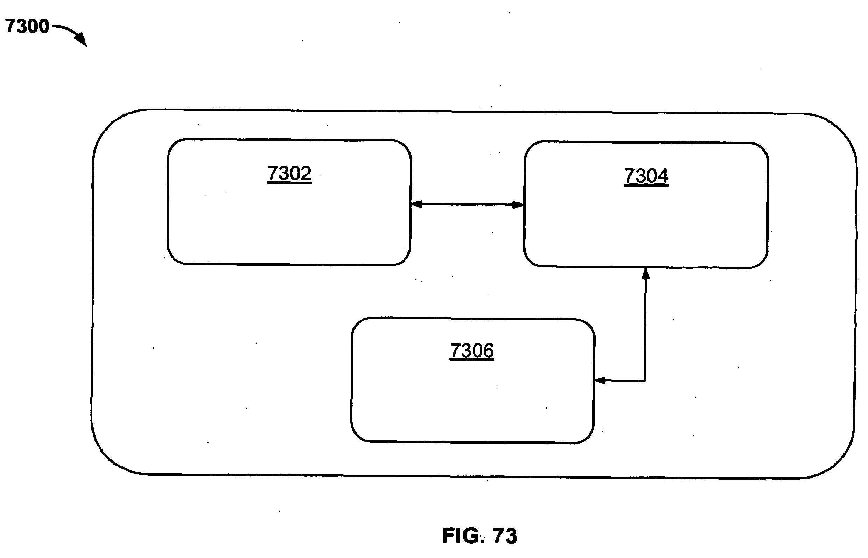

FIG. 73 is a block diagrammatic view of a system facilitating implementation of an Opto-Magnetic process based on light-matter interaction using digital imaging for detection of EPV and CMV viruses in blood plasma samples, designed and implemented in accordance with certain embodiments of the invention;

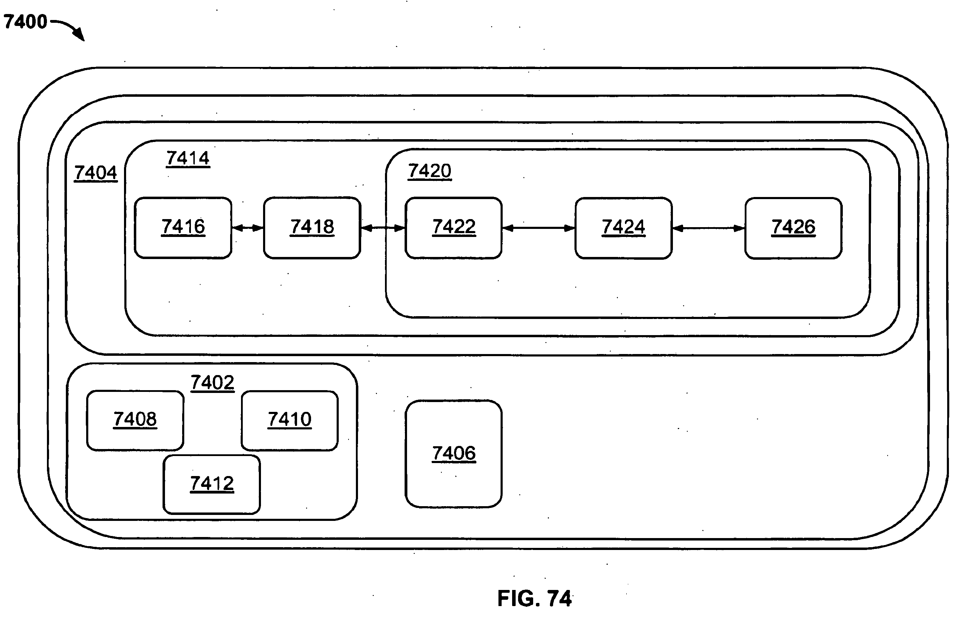

FIG. 74 is an exploded diagrammatic representation of the host computing subsystem, of the Fig. 1, comprising the Opto-Magnetic Fingerprint (or OMF) Generator module designed and implemented in accordance with at least some embodiments;

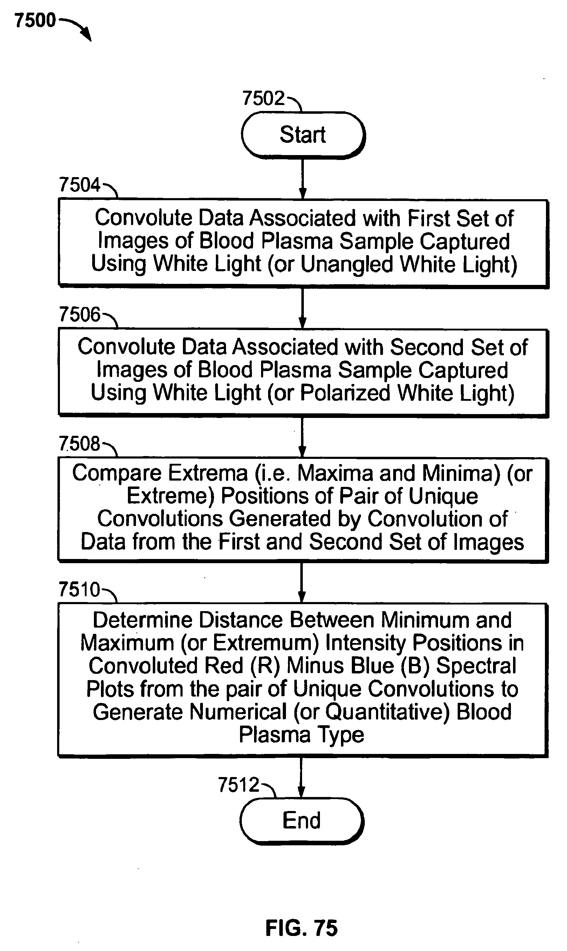

FIG. 75 depicts a flow diagram delineating at least one process implemented by the system configuration of FIGS. 1 and 2 thereby facilitating estimation of blood plasma type and properties (or characteristics) thereof and creation of a unique spectral signature;

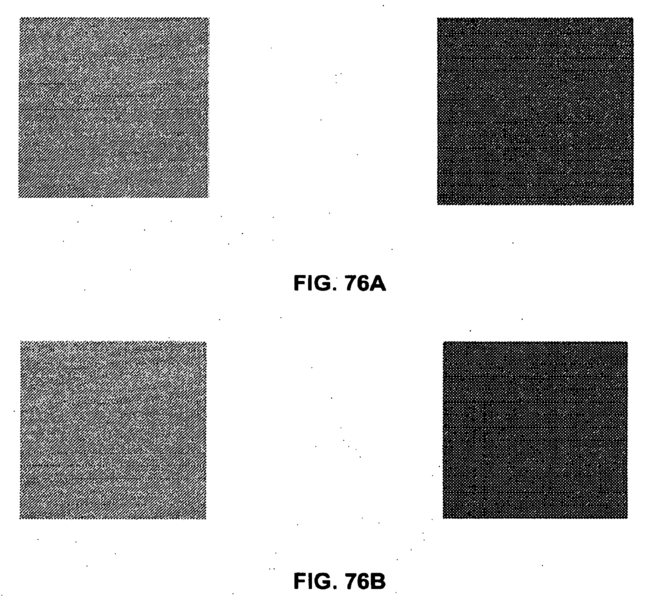

FIGS. 76A and 76B depict a dual pair of typical digital images of samples, tested positive and negative for EBV and CMV, captured with diffuse white light (W) and reflected polarized light (P), in that order;

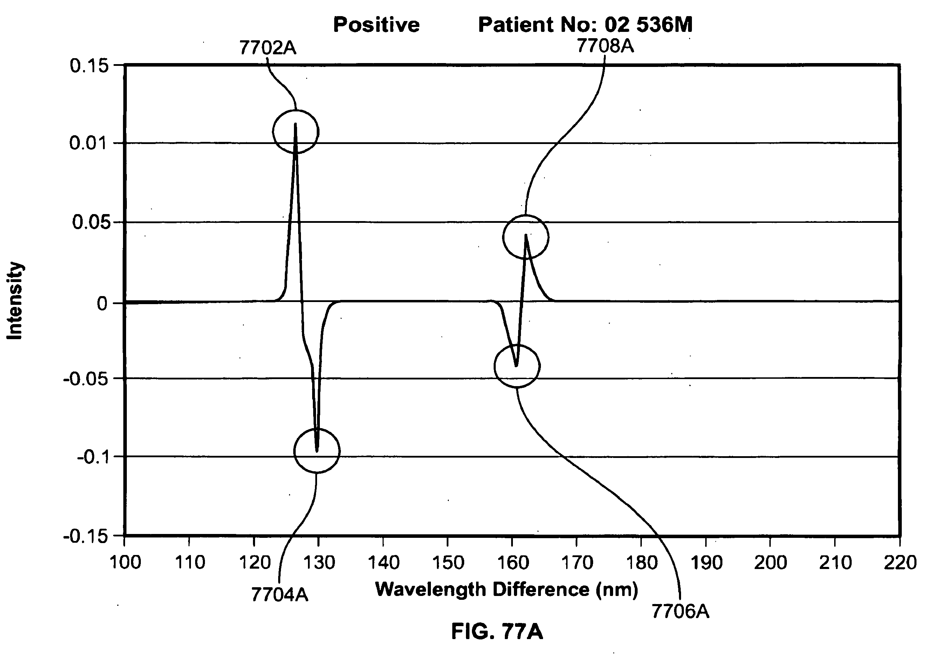

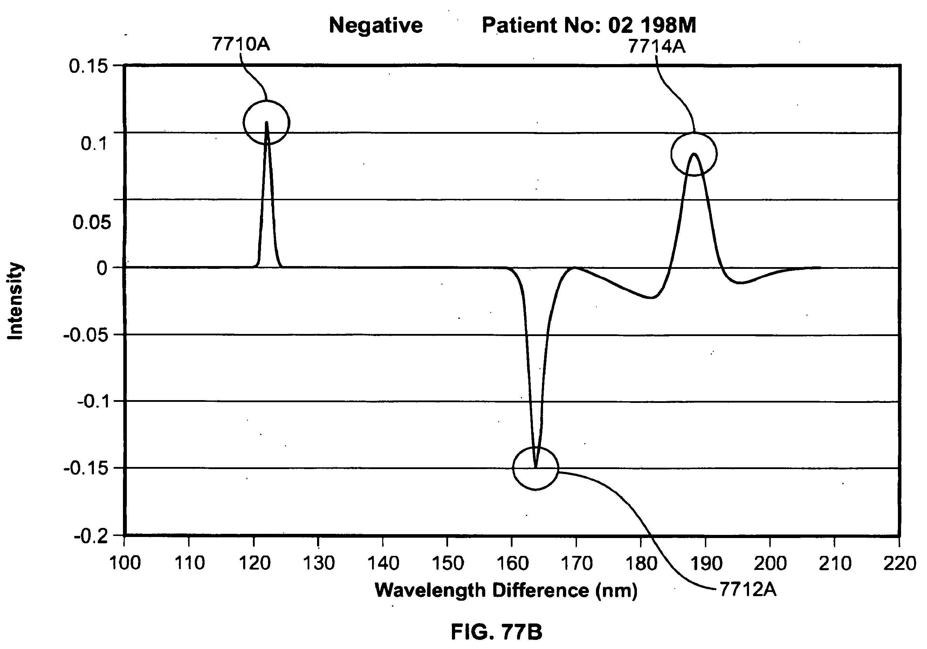

FIGS. 77A and 77B depict a first pair of plots of typical spectral data obtained on implementation of the OMF method for processing digital images of unique samples from a first set of two patients subjected to a first test case for confirmation of EBV, namely "Case I: EBV-IgM", designed and implemented in accordance with certain embodiments of the invention;

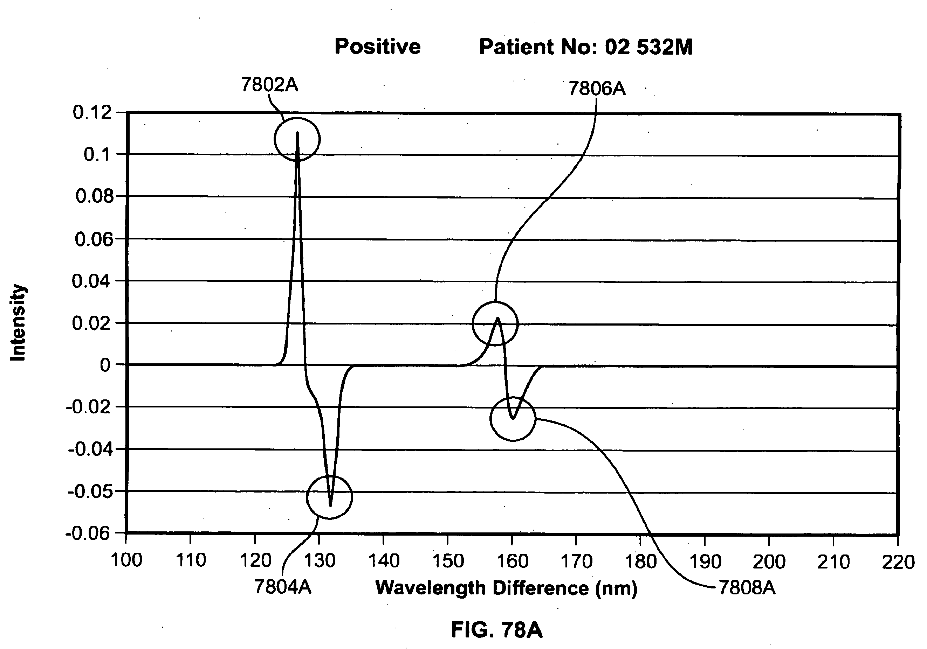

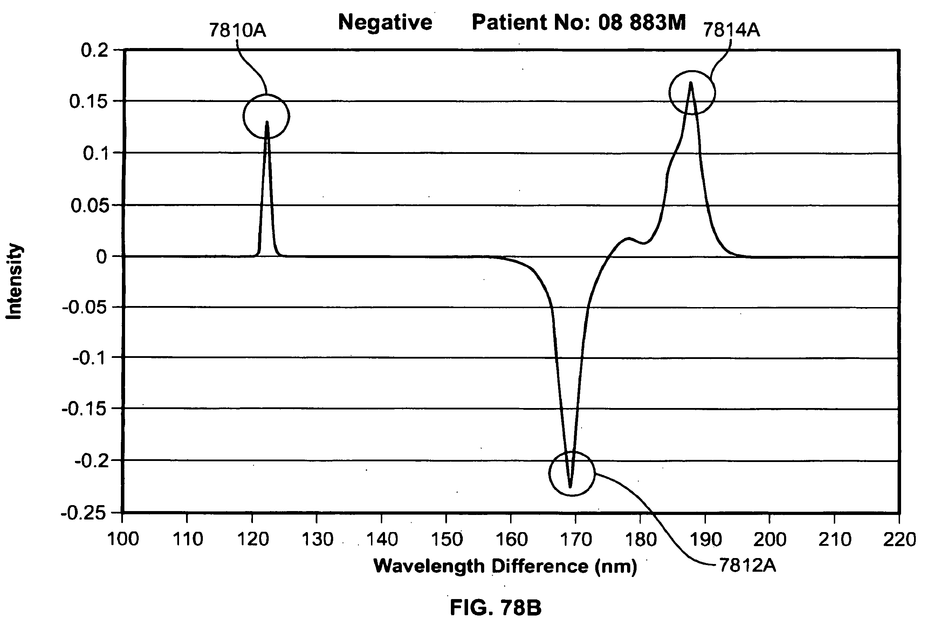

FIGS. 78A and 78B depict a second pair of plots of typical spectral data obtained on implementation of the OMF method for processing digital images of unique samples from a second set of two different patients subjected to a second test case for confirmation of EBV, namely "Case II: EBV-IgM", designed and implemented in accordance with certain embodiments of the invention;

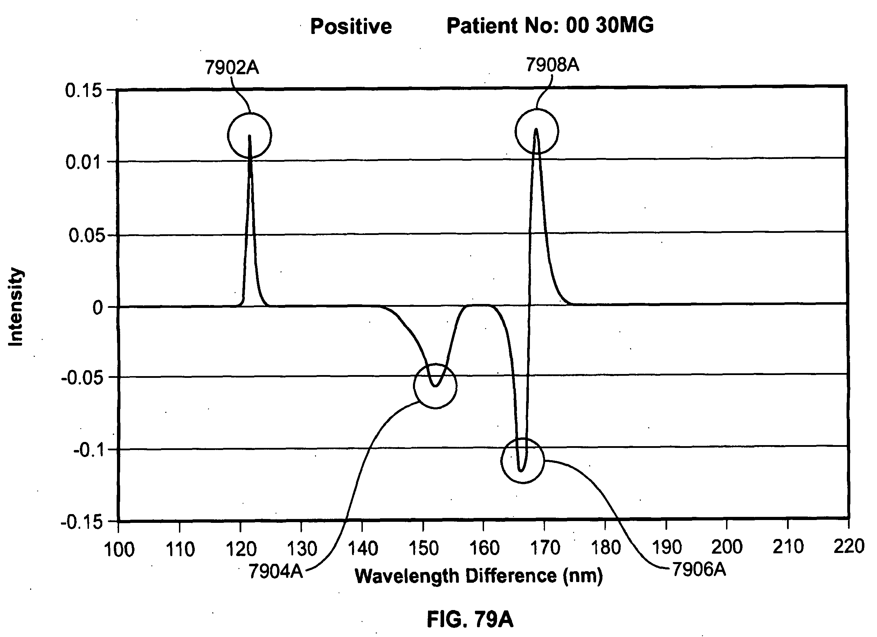

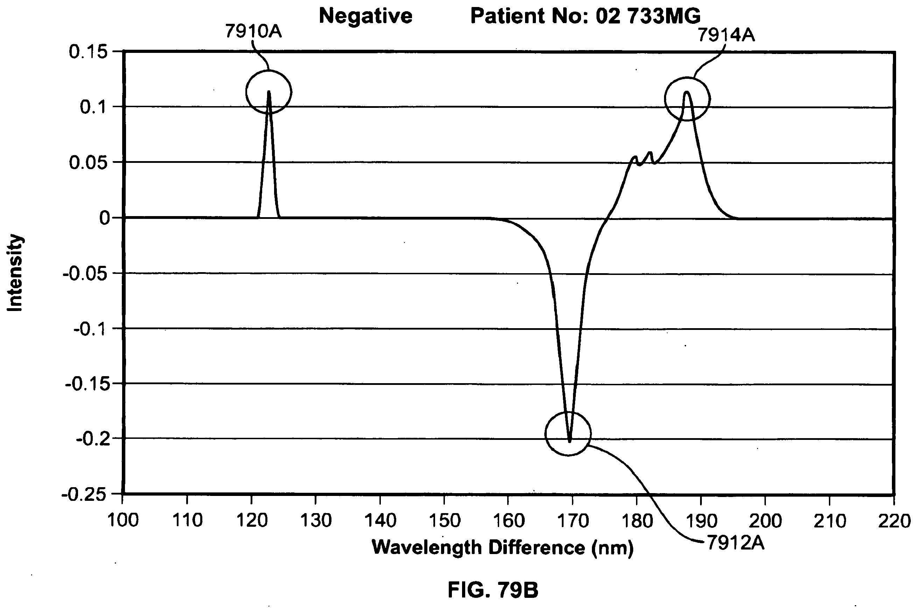

FIGS. 79A and 79B depict a third pair of plots of typical spectral data obtained on implementation of the OMF method for processing digital images of unique samples from a third set of two different patients subjected to a third test case for confirmation of EBV, namely "Case III: EBV-IgG", designed and implemented in accordance with certain embodiments of the invention; and

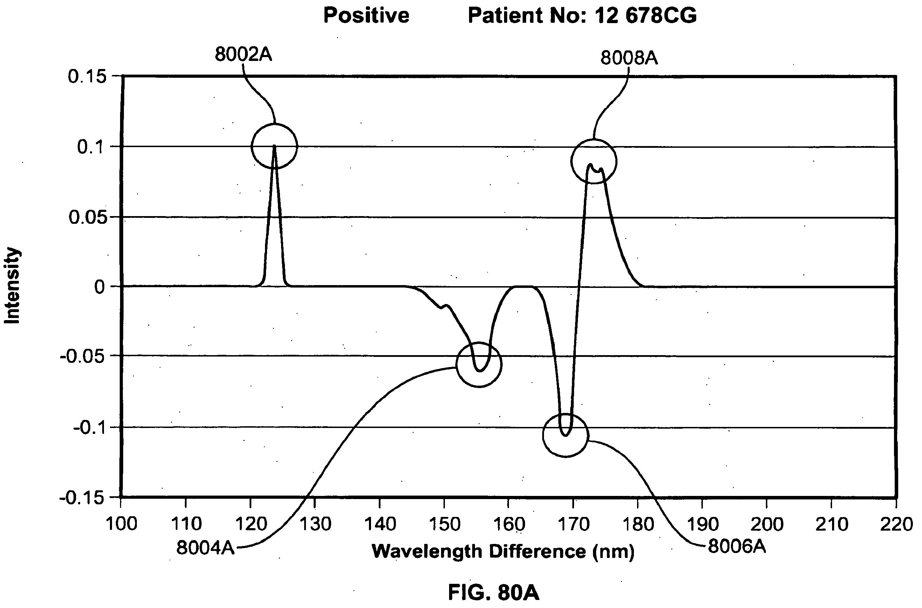

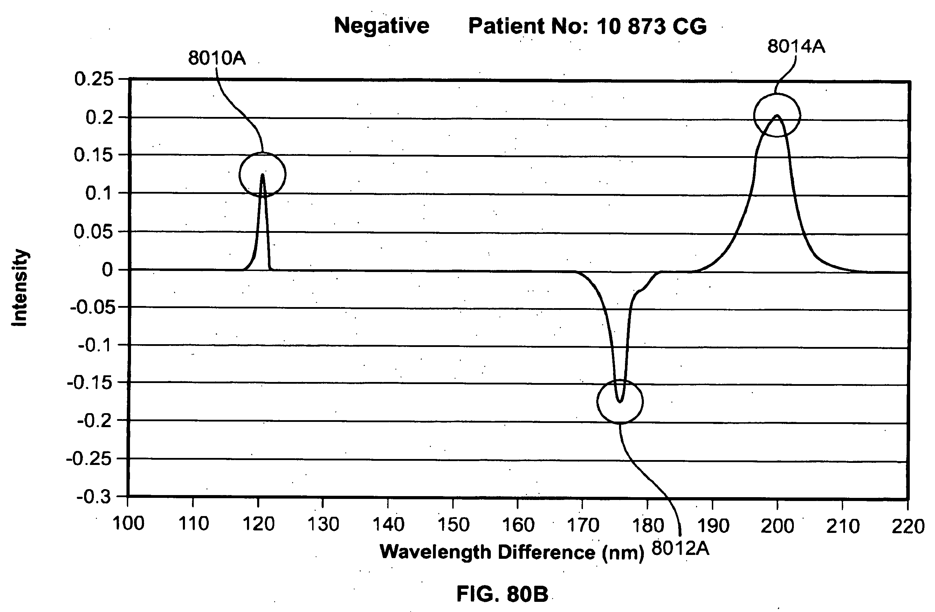

FIGS. 80A and 80B depict a fourth pair of plots of typical spectral data obtained on implementation of the OMF method for processing digital images of unique samples from a fourth set of two different patients subjected to a fourth test case for confirmation of EBV, namely "Case IV: EBV-IgG", designed and implemented in accordance with certain embodiments of the invention.

In certain embodiments, methods for detection of DNA viruses based on the interaction between matter and electromagnetic radiation and systems and apparatuses facilitating implementation of such methods are disclosed. Stated differently, in certain such embodiments, systems and apparatuses for practicing the principles of the invention are disclosed. More specifically, the systems and apparatuses facilitate implementation of an Opto-Magnetic method with enhanced qualitative and quantitative parameters for detection of Herpesviridae in blood plasma samples based on Opto-Magnetic properties of light-matter interaction. Still more specifically, the systems and apparatuses facilitate implementation of an Opto-Magnetic method with enhanced qualitative and quantitative parameters, such as novel, easily operable, rapid, economical, precise, timely and minute variation sensitive, for detection of EPV and CMV in blood plasma samples based on Opto-Magnetic properties of light-matter interaction.

In certain other situations, the sample set is subjected to diagnosis using OMF method. Specifically, the preparation of digital pictures for OMF is made by usage of non-invasive imaging device that has previously been successfully used in biophysical skin characterization, such as skin photo type, moisture, conductivity, etc. By way of example and in no way limiting the scope of the invention, systems, devices and methods for non-invasive dermal imaging has been disclosed in US Pat. App. No. PCT/US2008/050438, Publication No: WO/2008/086311, Publication Date: 2008-07-17 "SYSTEM, DEVICE AND METHOD FOR DERMAL IMAGING" to J. Bandic, Dj. Koruga, R. Mehendale and S. Marinkovich of MYSKIN, INC. Thus, all remaining ins-and-outs in connection with the process of generating the spectral signature will not be further detailed herein.

In certain specific embodiments, the design and implementation of an Opto-Magnetic Fingerprint (OMF) process for detection of EPV and CMV in blood plasma samples has been disclosed. Specifically, the OMF process is based on electron properties of matter and its interaction with light. By way of example, and in no way limiting the scope of the invention, the concept of light-matter interaction and Opto-magnetic thereof has been disclosed in United States Provisional Patent Application "METHOD AND ALGORITHM FOR ANALYSIS OF LIGHT-MATTER INTERACTION BASED ON SPECTRAL CONVOLUTION" to MYSKIN, INC. Thus, all remaining ins-and-outs in connection with the process of generating the spectral signature will not be further detailed herein.

Typically, valence electrons build a major link network of matter. The orbital velocity of the valence electrons in atoms is on the order of $10^6$ m/s. This gives the ratio between magnetic force ($F_M$) and electrical force ($F_E$) of matter of approximately $10^{-4}$ (or $F_M / F_E \approx 10^{-4}$.) Since, force ($F$) is directly related to quantum action (or Planck action) through the following equation: $h = F \times d \times t = 6.626 \times 10^{-34}$ Js, where $F$ is force, $d$ is displacement and $t$ is time of action. This means that the action of magnetic forces is four orders of magnitude closer to quantum action than the electrical ones. Further, since the quantum state of matter is primarily responsible for conformational changes on the molecular level, this means that detecting differences between tissue states is by far more likely to give greater sensitivity on the level of magnetic forces than it would be on the level of measurement of electrical forces.

The term "conformational change" refers to a transition in shape of a macromolecule. Typically, a macromolecule is flexible or dynamic. Thus, it can change its shape in response to changes in its environment or other factors. Each possible shape is called a conformation. A macromolecular conformational change may be induced by many factors, such as a change in temperature, pH, voltage, ion concentration, or the binding of a ligand.

In certain other embodiments, a comparative analysis of pictures of materials captured by classical optical microscopy and OMF has been discussed. Specifically, pictures captured by classical optical microscopy are based on electromagnetic property of light. On the contrary, in OMF pictures captured are based on difference between diffuse white light and reflected polarized light. Noticeable, here is the fact that reflected polarized light is produced when source of diffuse light irradiates the surface of matter under certain angle, such as Brewster's angle. Each type of matter has special different angle value of light polarization.

In here, the fact that the angle of reflected polarized light of blood plasma is about $52 \pm 0.8$ degree is disclosed. Since, reflected polarized light contains electrical component of light-matter interaction. Thus, taking the difference between white light (i.e. electromagnetic) and reflected polarized light (i.e. electrical) yields magnetic properties of matter based on light-matter interaction.

FIG. 73 is a block diagrammatic view of a system facilitating implementation of an Opto-Magnetic process based on light-matter interaction using digital imaging for detection of EPV and CMV viruses in blood plasma samples, designed and implemented in accordance with certain embodiments of the invention.

System 7300 is in essence a Virus Detection System (or VDS). The VDS 100 includes an illumination subsystem 7302, an imaging (or sensor) subsystem 7304 and a host computing subsystem 7306.

VDS 7300, by virtue of its design and implementation, facilitates execution of an Opto-Magnetic method based on interaction between electromagnetic radiation and matter, for instance light-matter interaction, using digital imaging for detection of EPV and CMV viruses in blood plasma samples. Specifically, the Opto-Magnetic process employs apparatuses for generation of unique spectral signatures from digitally captured images of blood plasma samples thereby facilitating detection of EPV and CMV viruses in blood plasma samples based on Opto-Magnetic properties of light-blood plasma interaction.

Illumination subsystem 7302 may be one or more electromagnetic radiation sources. In certain specific embodiments, the Illumination subsystem 7302 may be a set of Light Emitting Diodes (LEDs).

Illumination subsystem 7302 may be adapted to emit polarized and unpolarized electromagnetic signals. The polarized electromagnetic signal is angled white light and unpolarized electromagnetic signal is non-angled white light.

As shown in the FIG. 73, in certain embodiments, the illumination subsystem 7302 may be coupled to the sensor subsystem 7304.

As shown in the FIG. 73, the sensor subsystem 7304 may in essence be a device that converts optical images (or optical signals) to electric signals. In certain embodiments, the sensor subsystem 7304 captures continuous digital images of blood plasma samples. Specifically, in such embodiments, the sensor subsystem 7304 captures continuous digital images of the blood plasma samples illuminated with white light both, non-angled and angled. By way of, and by no way of limitation, the sensor subsystem 7304 may be any one selected from a group consisting of a Complementary Metal-Oxide-Semiconductor (CMOS) image sensor, Charged Coupled Device (CCD) image sensor, and the like.

Again, as shown in FIG. 73, the sensor subsystem 7304 may be coupled to the host computing subsystem 7306.

FIG. 74 is an exploded diagrammatic representation of the host computing subsystem, of the Fig. 73, comprising the Opto-Magnetic Fingerprint (or OMF) Generator module designed and implemented in accordance with at least some embodiments.

The host computing subsystem 7400 may comprise a processing unit 7402, a memory unit 204 and an Input / Output (or I/O) unit 206 respectively.

The host computing subsystem 7400, by virtue of its design and implementation, performs overall management of blood plasma samples.

The processing unit 7402 may comprise an Arithmetic Logic Unit (or ALU) 7408, a Control Unit (or CU) 7410 and a Register Unit (or RU) 7412.

As shown in FIG. 74, the memory unit 7404 comprises a blood plasma virus detection module 7414.

In certain embodiments, the blood plasma virus detection module for detection of EPV and CMV via generation of unique spectral signatures from the digitally captured images of blood plasma samples and methods thereof are disclosed, in accordance with the principles of the invention. Specifically, in such embodiments, the blood plasma virus detection module utilizes the continuously captured digital images of the blood plasma samples illuminated with white light both, non-angled and angled. More specifically, the blood plasma virus detection module takes into consideration the digital images in Red (R), Green (G) and Blue (B) (or RGB) system for purposes of analysis.

Further, as shown in FIG. 74, the blood plasma virus detection module 7414 includes a Fourier transform sub-module 7416, a spectral analyzer sub-module 7418 and an Opto-Magnetic Fingerprint Generator (or OMFG) sub-module 7420, respectively.

In certain embodiments, the Fourier transform sub-module 7416 is in essence a Discrete-Time Fourier Transform (or DTFT).

The term "DTFT", as used herein, refers to one of the specific forms of Fourier analysis. As such, it transforms one function into another, which is called the frequency domain representation, or simply the "DTFT", of the original function, which is often a function in the time-domain. But, the DTFT requires an input function that is discrete. Such inputs are often created by sampling a continuous function, like a person's voice. The DTFT frequency-domain representation is always a periodic function. Since one period of the function contains all of the unique information, it is sometimes convenient to say that the DTFT is a transform to a "finite" frequency-domain (the length of one period), rather than to the entire real line.

DTFT 7416 converts time-domain digital signals into corresponding frequency-domain digital signals.

DTFT 7416 is coupled to the spectrum analyzer sub-module 7418.

As used herein, the term "spectrum analyzer" refers to a device used to examine the spectral composition of some electrical, acoustic, or optical waveform. It may also measure the power spectrum. In general, there are three types of spectrum analyzers, such as analog, digital and real-time spectrum analyzers. Firstly, an analog spectrum analyzer uses either a variable band-pass filter whose mid-frequency is automatically tuned (i.e. shifted, swept) through the range of frequencies of the spectrum to be measured or a superheterodyne receiver, wherein the local oscillator is swept through a range of frequencies. Secondly, a digital spectrum analyzer computes the Discrete Fourier transform (or DFT), a mathematical process that transforms a waveform into the components of its frequency spectrum. Eventually, some spectrum analyzers, such as "real-time spectrum analyzers", use a hybrid technique where the incoming signal is first down-converted to a lower frequency using superheterodyne techniques and then analyzed using fast Fourier transformation (FFT) techniques.

In certain embodiments, the spectrum (or spectral) analyzer sub-module for analysis of digitally captured images of blood plasma samples thereby facilitating detection of EBV and CMV is disclosed. Specifically, the spectrum (or spectral) analyzer sub-module in order to analyze the blood plasma samples takes into consideration digital images of blood plasma in Red (R), Green (G) and Blue (B) (or RGB) system. In certain such embodiments, basic pixel data in Red (R) and Blue (B) channels for both white diffuse light (or W) and reflected polarized light (or P) is selected. In here, the algorithm for data analysis is based on chromaticity diagram called "Maxwell's triangle" and spectral convolution.

In certain specific embodiments, the digital images in Red (R), Green (G) and Blue (B) (or RGB) system are taken into consideration for purposes of spectral analysis. Specifically, basic pixel data in Red (R) and Blue (B) channels for white diffuse light (or W) and reflected polarized white light (or P) is selected. More specifically, the algorithm for data analysis is based on a chromaticity diagram called "Maxwell's triangle" and spectral convolution operation, in accordance with a ratio of (R - B) & (W - P). Noticeably, the abbreviated designation implies that Red (R) minus Blue (B) wavelength of White light (W) and reflected Polarized light (P) are used in a spectral convolution algorithm to calculate data for an Opto-Magnetic Fingerprint (OMF) of matter both, organic and inorganic. Consequently, the method and algorithm for creating unique spectral fingerprints are based on the convolution of RGB color channel spectral plots generated from digital images that capture single and multi-wavelength light-matter interaction for different paramagnetic materials, such as Al, Mn and Ti, diamagnetic materials, such as Cu, C and Zn, alloys, such asPb1-xMnxTe, Biomolecules and biological tissues as paramagnetic / diamagnetic materials, such as skin, biological water, amniotic fluid, blood plasma and the like.

Further, incident white light can give different information about properties of thin layers of matter, such as a blood plasma sample surface, depending on the angle of light incidence. In use, when the incident white light is diffuse, the reflected white light is then composed of electrical and magnetic components, whereas diffuse incident light that is inclined under certain angle will produce reflected light which contains only electrical component of light.

As shown in FIG. 74, the spectrum analyzer sub-module 7418 may be coupled to the OMFG sub-module 7420.

OMFG sub-module 7420 includes a color histogram generator unit 7422, a spectral plot generator unit 7424 and a convolution unit 7426.

OMFG sub-module 7414, by virtue of its design and implementation, facilitates generation of unique spectral signatures from digitally captured images of blood plasma samples, Specifically, the generated spectral signatures of blood plasma samples facilitate detection of EPV and CMV based on Opto-Magnetic properties of light-blood plasma interaction.

Color histogram generator unit 7422, by virtue of its design, generates a normalized Red (R) and Blue (B) color channel histogram for each of the one or more images of the blood plasma samples.

The term "color histogram", as used in computer graphics and photography, refers to is a representation of the distribution of colors in an image, derived by counting the number of pixels of each of given set of color ranges in a typically two-dimensional (2D) or three-dimensional (3D) color space. A histogram is a standard statistical description of a distribution in terms of occurrence frequencies of different event classes; for color, the event classes are regions in color space. An image histogram of scalar pixel values is more commonly used in image processing than is a color histogram. The term "image histogram" refers to a type of histogram which acts as a graphical representation of the tonal distribution in a digital image. It plots the number of pixels for each tonal value. By looking at the histogram for a specific image a viewer is able to judge the entire tonal distribution at a glance.

Typically, color histograms are flexible constructs that can be built from images in various color spaces, whether RGB, rg chromaticity or any other color space of any dimension. A histogram of an image is produced first by discretization of the colors in the image into a number of bins, and counting the number of image pixels in each bin. For example, a Red-Blue chromaticity histogram can be formed by first normalizing color pixel values by dividing RGB values by R+G+B, then quantizing the normalized R and B coordinates into N bins each, where N = 4, which might yield a 2D histogram that is similar to Table 2:

Table 2 exhibits a tabular representation in connection with a 2D Red-Blue chromaticity histogram generated by first normalizing color pixel values by dividing RGB values by R+G+B, then quantizing the normalized R and B coordinates into N bins each, where N = 4.

| | | R | | | |
|---|---|---|---|---|---|
| | | 0-63 | 64-127 | 128-191 | 192-255 |
| B | 0-63 | 43 | 78 | 18 | 0 |
| | 64-127 | 45 | 67 | 33 | 2 |
| | 128-191 | 127 | 58 | 25 | 8 |
| | 192-255 | 140 | 47 | 47 | 13 |

As shown in FIG. 74, the color histogram generator unit 7422 may be coupled to the spectral plot generator unit 7424.

Spectral plot generator unit 7424 generates Red (R) and Blue (B) color channel spectral plots by correlating the normalized Red (R) and Blue (B) color channel histograms to a wavelength scale. In certain embodiments, a unit scale on the spectral signature is a difference of wavelength.

In general, color digital images are made of pixels and, in turn, pixels are made of combinations of primary colors. As used in the current context, the term "channel" refers to the grayscale image of the same size as a color image, made of just one of these primary colors. For instance, an image from a standard digital camera will have a red, green and blue channel. A grayscale image has just one channel. Further, an RGB image has three channels, namely Red (R), Green (G) and Blue (B). For example, if the RGB image is 24-bit then each channel has 8 bits, for R, G and B. Stated differently, the image is composed of three grayscale images, where each grayscale image can store discrete pixels with conventional brightness intensities between 0 and 255. Whereas, if the RGB image is 48-bit (i.e. very high resolution), each channel is made of 16-bit grayscale images.

The periodogram is an estimate of the spectral density of a signal. The term "spectral plot" refers to a smoothed version of the periodogram. Smoothing is performed to reduce the effect of measurement noise.

Convolution unit 7426 convolutes the Red (R) and Blue (B) color channel spectral plots by subtracting the spectral plot for the polarized optical electromagnetic signal from the non-polarized optical electromagnetic signal for each color to generate Red (R) and Blue (B) normalized, composite color channel spectral plots and subtracting the normalized, composite Blue (B) channel spectral plot from the normalized, composite Red (R) channel spectral plot thereby resulting in generation of a spectral signature for the blood plasma samples.

In certain embodiments, the spectral signature is analyzed for at least one of number of crests and troughs, amplitude, shape of peaks, intermediate structures and patterns. In certain such embodiments, the spectral signature is analysed for material composition, identification, purity and the like.

In certain other embodiments, the system configuration, discussed in conjunction with FIGS. 73 and 74, implement one or more processes facilitating estimation of blood plasma type and properties (or characteristics) thereof to create a unique spectral signature.

FIG. 75 depicts a flow diagram delineating at least one process implemented by the system configuration of FIGS. 73 and 74 thereby facilitating estimation of blood plasma type and properties (or characteristics) thereof and creation of a unique spectral signature.

The process 7500 starts at stage 7502 and proceeds to stage 7504, wherein the process 7500 comprises the phase of convolution of data associated with a first set of images of a blood plasma sample captured by illuminating the sample with a white light (or unangled white light.) Noticeable here is the fact that the data associated with the first set of images of the blood plasma sample illuminated with the white light (or unangled white light) may comprise one or more combinations of reflected and re-emitted angled and unangled white light.

At stage 7506, the process 7500 comprises the phase of convolution of data associated with a second set of images of the blood plasma sample captured by illuminating the sample with an angled white light. It must be noted here that the data associated with the second set of images of the blood plasma sample illuminated with the angled white light may comprise one or more combinations of reflected and re-emitted angled white light.

At stage 7508, the process 7500 comprises the phase of comparison of extrema (i.e. maxima and minima) (or extreme) positions of at least a pair of unique convolutions generated by convolution of data from the first set of images and second set of images.

At stage 7510, the process 7500 comprises the phase of determination of a distance between minimum and maximum (or extremum) intensity positions in convoluted Red (R) minus Blue (B) spectral plots from the pair of unique convolutions generated by convolution of data from the first set of images and second set of images to generate a numerical (or quantitative) blood plasma type. The process 7500 ends at stage 7512.

In certain embodiments, the phase of comparison of extrema (i.e. maxima and minima) (or extreme) positions of at least a pair of unique convolutions comprises implementation of one or more sub-phases. Specifically, the one or more sub-phases include comparison of a first component Red (R) minus Blue (B) of unangled white light (or W) minus angled white light (or polarized white light or P) (i.e. (R - B) (W - P)) versus a second component Red (R) minus Blue (B) of unangled white light (or W) (i.e. (R - B) W). The two unique convolutions in unangled white light and angled (or polarized) white light further include a White Red component (WR), a White Blue component (WB), a reflected and / or re-emitted Polarized Blue component (PB) and a reflected and / or re-emitted Polarized Red component (PR). The two unique convolutions are based on a numerical value difference correlating to medical standards.

In certain alternative embodiments, the step of comparing extreme positions of at least two unique convolutions includes comparing a component (R - B) (W - P) for the reflected and / or re-emitted polarized light, and a component (R - B) W for the white light. Yet, in certain embodiments, the step of comparing extreme positions of at least two unique convolutions includes a spectral convolution scheme, wherein multiple combinations of subtraction of Blue (B) spectrum from Red (R), in white light and polarized white light are determined, wherein the spectral interval is expressed in a wavelength scale interval of 100 nanometers to 300 nanometers.

In certain circumstances, the investigation of viral infection performed over a sample set taken from 40 pregnant women is disclosed. In such circumstances, the sample set is classified by blood test in two groups, namely EBV group (32 cases, M, GM) and CMV group (8 cases M, GM). Further, each group is separated into two categories, namely positive (virus present, 16 EBV and 4 CMV) and negative (virus absent, 16 EBV and 4 CMV) respectively.

Still further, in certain situations the sample set is subjected to diagnosis using standard Enzyme Immunoassay Method (or ELISA).

FIGS. 76A and 76B depict a dual pair of typical digital images of samples, tested positive and negative for EBV and CMV, captured with diffuse white light (W) and reflected polarized light (P), in that order.

As shown in FIG. 76A, a first pair of the dual pair of digital photography images of blood plasma samples of pregnant women captured with diffuse white light and reflected polarized tested positive for presence of EBV. For purposes of expediency and clarity, both the positively tested blood plasma samples have been referred to as "POSITIVE 00 30MG".

In contrast, a second pair of the dual pair of digital photography images of blood plasma samples of pregnant women captured with diffuse white light and reflected polarized tested negative for presence of EBV are shown in FIG. 76B. For purposes of expediency and clarity, both the negatively tested blood plasma samples have been referred to as "NEGATIVE 02 733MG".

Observation of images in FIGS. 76A and 76B by naked eye would probably testify that there are no differences between them. However, using Computer Assisted Analysis (CAA) based on pixel by pixel count and Spectral Convolution Algorithm (SCA), significant differences are found, the final result of which is illustrated in conjunction with FIGS. 77A-B, 78A-B, 79A-B and 80A-B, respectively.

In certain embodiments, a limited number of typical cases of EBV are selected and presented for purposes of illustration. Specifically, four typical cases of EBV, namely two IgM and two IgG, to illustrate the difference between positive and negative of same cases (i.e. IgM or IgG) and similarity of spectral data.

The term "IgG or Immunoglobulin G" refers to a monomeric immunoglobulin built of two heavy chains y and two light chains. Each IgG has two antigen binding sites. It is the most abundant immunoglobulin and is approximately equally distributed in blood and in tissue liquids, constituting 75% of serum immunoglobulins in humans. IgG molecules are synthesized and secreted by plasma B cells.

The term "Immunoglobulin M or IgM" refers to a basic antibody that is present on B cells. It is the primary antibody against A and B antigens on red blood cells. IgM is by far the physically largest antibody in the human circulatory system. It is the first antibody to appear in response to initial exposure to antigen.

In certain specific embodiments, CAA based on pixel by pixel count and SCA is implemented taking into consideration only four typical cases of EBV, namely two IgM and two IgG, thereby facilitating illustration of difference between positive and negative of same cases (i.e. IgM or IgG) and similarity of spectral data. In such specific embodiments, for purposes of illustration of the spectral data obtained on implementation of the CAA and SCA, a two (or 2 D)-dimensional coordinate system including a horizontal X-axis and a vertical Y-axis is selected. Specifically, the horizontal X-axis represents the wavelength difference in nanometers whereas the vertical Y-axis represents the intensity in suitable units. More specifically, the 2D coordinate system exhibits the comparative analysis of wavelength difference versus intensity for given samples collected from given patients and subjected to tests for presence or absence of EBV, wherein the wavelength difference is the independent variable and the intensity is the dependent variable.

FIGS. 77A and 77B depict a first pair of plots of typical spectral data obtained on implementation of the OMF method for processing digital images of unique samples from a first set of two patients subjected to a first test case for confirmation of EBV, namely "Case I: EBV-IgM", designed and implemented in accordance with certain embodiments of the invention.

As shown in FIGS. 77A-B, the 2D coordinate system is in essence a Difference Versus Intensity plot (or DI plot) obtained on plotting a plurality of DI ordered pairs. Each of the plurality of ordered pairs includes a Wavelength Difference value and a corresponding Intensity value. It must be noted here that the plurality of ordered pairs are obtained on processing the digital images of blood plasma samples, captured using diffuse white light and reflected polarized light, using the OMF method. Specifically, the OMF method implements the SCA and CAA to analyze the processed digital images of the blood plasma samples. Further, the blood plasma samples are collected from two different patients subjected to test for presence or absence of EBV-IgM.

As depicted in FIG. 77A, a first DI plot of the first pair of DI plots possess the following specifications and associated test information thereof: ordered (or DI) pair is (Wavelength Difference Value, Intensity Value); horizontal X-axis includes a closed interval of Wavelength Difference Values ranging from a minimum of equal to 100 nanometers (nm) to a maximum of equal to 220 nanometers (nm) (or [100, 220]); vertical X-axis includes a closed interval of Intensity Values ranging from a minimum of equal to -0.15 to a maximum of equal to +0.15; test is analysis for confirmation of presence or absence of EBV in blood plasma sample; patient information is a first patient of the first set is a pregnant woman bearing optional or exemplary patient number is patient no. 02 536M; test input sample is blood plasma of the patient; test case is EBV-IgM; test output is positive; operation is OMF method; number of intensity peaks (or extrema or maxima and minima) is 4; identifiers for the 4 intensity peaks are first 7702A, second 7704A, third 7706A and fourth 7708A respectively; values for Wavelength Difference / Intensity associated with the first 7702A, second 7704A, third 7706A and fourth 7708A intensity peaks are 126.6 nm / 0.113, 129.7 nm / -0.095, 160.8 nm / -0.041, 162.1 nm / 0.041 in that order.

As depicted in FIG. 77B, a second DI plot of the first pair of DI plots possess the following specifications and associated test information thereof: ordered (or DI) pair is (Wavelength Difference Value, Intensity Value); horizontal X-axis includes a closed interval of Wavelength Difference Values ranging from a minimum of equal to 100 nanometers (nm) to a maximum of equal to 220 nanometers (nm) (or [100, 220]); vertical X-axis includes a closed interval of Intensity Values ranging from a minimum of equal to -0.2 to a maximum of equal to +0.15; test is analysis for confirmation of presence or absence of EBV in blood plasma sample; patient information is a second patient of the first set is a pregnant woman bearing optional or exemplary patient number is patient no. 09 198M; test input sample is blood plasma of the patient; test case is EBV-IgM; test output is negative; number of intensity peaks (or extrema or maxima and minima) is 3; identifiers for the 3 intensity peaks are fifth 7710A, sixth 7712A and seventh 7714 A respectively; values for Wavelength Difference / Intensity associated with the fifth, sixth and seventh intensity peaks are 122.0 nm/0.107, 163.4 nm / -0.151, 187.8 mn / 0.084 in that order.

FIGS. 78A and 78B depict a second pair of plots of typical spectral data obtained on implementation of the OMF method for processing digital images of unique samples from a second set of two different patients subjected to a second test case for confirmation of EBV, namely "Case II: EBV-IgM", designed and implemented in accordance with certain embodiments of the invention.

As depicted in FIG. 78A, a third DI plot of the second pair of DI plots possess the following specifications and associated test information thereof: ordered (or DI) pair is (Wavelength Difference Value, Intensity Value); horizontal X-axis includes a closed interval of Wavelength Difference Values ranging from a minimum of equal to 100 nanometers (nm) to a maximum of equal to 220 nanometers (nm) (or [100, 220]); vertical X-axis includes a closed interval of Intensity Values ranging from a minimum of equal to -0.06 to a maximum of equal to +0.12; test is analysis for confirmation of presence or absence of EBV in blood plasma sample; patient information is a first patient of the second set is a pregnant woman bearing optional or exemplary patient number is patient no. 02 532M; test input sample is blood plasma of the patient; test case is EBV-IgM; test output is positive; operation is OMF method; number of intensity peaks (or extrema or maxima and minima) is 4; identifiers for the 4 intensity peaks are first 7802A, second 7804A, third 7806A and fourth 7808A respectively; values for Wavelength Difference / Intensity associated with the first 7802A, second 7804A, third 7806A and fourth 7808A intensity peaks are 126.6 nm / 0.110, 132.3 nm / -0.060, 157.8 nm / 0.023, 160.2 nm / - 0.026 in that order.

As depicted in FIG. 78B, a fourth DI plot of the second pair of DI plots possess the following specifications and associated test information thereof: ordered (or DI) pair is (Wavelength Difference Value, Intensity Value); horizontal X-axis includes a closed interval of Wavelength Difference Values ranging from a minimum of equal to 100 nanometers (nm) to a maximum of equal to 220 nanometers (nm) (or [100, 220]); vertical X-axis includes a closed interval of Intensity Values ranging from a minimum of equal to -0.25 to a maximum of equal to +0.2; test is analysis for confirmation of presence or absence of EBV in blood plasma sample; patient information is a second patient of the second set is a pregnant woman bearing optional or exemplary patient number is patient no. 08 883M; test input sample is blood plasma of the patient; test case is EBV-IgM; test output is negative; number of intensity peaks (or extrema or maxima and minima) is 3; identifiers for the 3 intensity peaks are fifth 7810A, sixth 7812A and seventh 7814A respectively; values for Wavelength Difference / Intensity associated with the fifth 7810A, sixth 7812A and seventh 7814A intensity peaks are 122.2 nm / 0.132, 169.3 nm /-0.225, 187.8 nm / 0.169 in that order.

FIGS. 79A and 79B depict a third pair of plots of typical spectral data obtained on implementation of the OMF method for processing digital images of unique samples from a third set of two different patients subjected to a third test case for confirmation of EBV, namely "Case III: EBV-IgG", designed and implemented in accordance with certain embodiments of the invention.

As depicted in FIG. 79A, a fifth DI plot of the third pair of DI plots possess the following specifications and associated test information thereof: ordered (or DI) pair is (Wavelength Difference Value, Intensity Value); horizontal X-axis includes a closed interval of Wavelength Difference Values ranging from a minimum of equal to 100 nanometers (nm) to a maximum of equal to 220 nanometers (nm) (or [100, 220]); vertical X-axis includes a closed interval of Intensity Values ranging from a minimum of equal to -0.15 to a maximum of equal to +0.15; test is analysis for confirmation of presence or absence of EBV in blood plasma sample; patient information is a first patient of the third set is a pregnant woman bearing optional or exemplary patient number is patient no. 00 30MG; test input sample is blood plasma of the patient; test case is EBV-IgG; test output is positive; operation is OMF method; number of intensity peaks (or extrema or maxima and minima) is 4; identifiers for the 4 intensity peaks are first 7902A, second 7904A, third 7906A and fourth 7908A respectively; values for Wavelength Difference / Intensity associated with the first 7902A, second 7904A, third 7906A and fourth 7908A intensity peaks are 121.7 nm / 0.120, 151.3 nm / -0.059, 166.3 nm / -0.117, 168.4 nm / 0.121 in that order.

As depicted in FIG. 79B, a sixth DI plot of the third pair of DI plots possess the following specifications and associated test information thereof: ordered (or DI) pair is (Wavelength Difference Value, Intensity Value); horizontal X-axis includes a closed interval of Wavelength Difference Values ranging from a minimum of equal to 100 nanometers (cm) to a maximum of equal to 220 nanometers (nm) (or [100, 220]); vertical X-axis includes a closed interval of Intensity Values ranging from a minimum of equal to -0.25 to a maximum of equal to +0.15; test is analysis for confirmation of presence or absence of EBV in blood plasma sample; patient information is a second patient of the third set is a pregnant woman bearing optional or exemplary patient number is patient no. 02 733MG; test input sample is blood plasma of the patient; test case is EBV-IgG; test output is negative; number of intensity peaks (or extrema or maxima and minima) is 3; identifiers for the 3 intensity peaks are fifth 7910A, sixth 7912A and seventh 7914A respectively; values for Wavelength Difference / Intensity associated with the fifth 7910A, sixth 7912A and seventh 7914A intensity peaks are 122.0 nm / 0.115, 169.3 nm /-0.203, 187.8 nm / 0.114 in that order.

FIGS. 80A and 80B depict a fourth pair of plots of typical spectral data obtained on implementation of the OMF method for processing digital images of unique samples from a fourth set of two different patients subjected to a fourth test case for confirmation of EBV, namely "Case IV: EBV-IgG", designed and implemented in accordance with certain embodiments of the invention.

As depicted in FIG. 80A, a seventh DI plot of the fourth pair of DI plots possess the following specifications and associated test information thereof: ordered (or DI) pair is (Wavelength Difference Value, Intensity Value); horizontal X-axis includes a closed interval of Wavelength Difference Values ranging from a minimum of equal to 100 nanometers (nm) to a maximum of equal to 220 nanometers (nm) (or [100, 220]); vertical X-axis includes a closed interval of Intensity Values ranging from a minimum of equal to -0.15 to a maximum of equal to +0.15; test is analysis for confirmation of presence or absence of EBV in blood plasma sample; patient information is a first patient of the fourth set is a pregnant woman bearing optional or exemplary patient number is patient no. 12 678 CG; test input sample is blood plasma of the patient; test case is EBV-IgG; test output is positive; operation is OMF method; number of intensity peaks (or extrema or maxima and minima) is 4; identifiers for the 4 intensity peaks are first 8002A, second 8004A, third 8006A and fourth 8008A respectively; values for Wavelength Difference / Intensity associated with the first 8002A, second 8004A, third 8006A and fourth 8008A intensity peaks are 123.6 nm / 0.098, 155.7 nm / -0.061, 168.4 nm / -0.106, 172.2 nm / 0.087 in that order.

As depicted in FIG. 80B, a eighth DI plot of the fourth pair of DI plots possess the following specifications and associated test information thereof: ordered (or DI) pair is (Wavelength Difference Value, Intensity Value); horizontal X-axis includes a closed interval of Wavelength Difference Values ranging from a minimum of equal to 100 nanometers (nm) to a maximum of equal to 220 nanometers (nm) (or [100, 220]); vertical X-axis includes a closed interval of Intensity Values ranging from a minimum of equal to -0.3 to a maximum of equal to +0.25; test is analysis for confirmation of presence or absence of EBV in blood plasma sample; patient information is a second patient of the fourth set is a pregnant woman bearing optional or exemplary patient number is patient no. 10 873 CG; test input sample is blood plasma of the patient; test case is EBV-IgG; test output is negative; number of intensity peaks (or extrema or maxima and minima) is 3; identifiers for the 3 intensity peaks are fifth, sixth and seventh respectively; values for Wavelength Difference / Intensity associated with the fifth, sixth and seventh intensity peaks are 120.5 nm / 0.123, 176.1 nm / -0.175, 200.3 nm / 0.203 in that order.

Noticeable here is the fact that the 40 samples examined for presence of EBV or CMV the following distinctive features are observed in the FIGS. 77A-B, 78A-B, 79A-B and 80A-B: number of peaks, position of peaks, distribution of peaks (up and down), and individual peak intensity. Regarding all the aforementioned features it is seen that it is possible to group the FIGS. 77A-B, 78A-B, 79A-B and 80A-B based on the antibody type (i.e. IgG / IgM) and the test results (i.e. positive/negative). The intensities as well as wavelength differences for IgM antibodies differ from those for IgG antibodies. All positive samples are approximated by four peaks while negative ones are approximated by only three. As a consequence, this is a promising evidence for using this OMF process as a fast, accurate and economically affordable screening tool. Another feature, visible in the group of negative samples (i.e. around 180 nm), does not exhibit an easily observable shape or peak position therefore is excluded from this analysis.

In addition, spectral data of all 40 cases presented in the FIGS. 77A-B, 78A-B, 79A-B and 80A-B display information regarding the difference between normal (i.e. negative) and virus infected (i.e. positive) blood plasma samples. Owing to the fact that the OMF spectral plots (or DI-OMF) for EBV-GM and CMV-GM appear similar, this algorithm still needs to be refined in order to more clearly distinguish which type of virus infection is present. However, OMF method could be used as an adjunct method in virus detection since it yields good results in quick identification of virus infection presence. It can save time and money when used in parallel with expensive biochemical analysis.

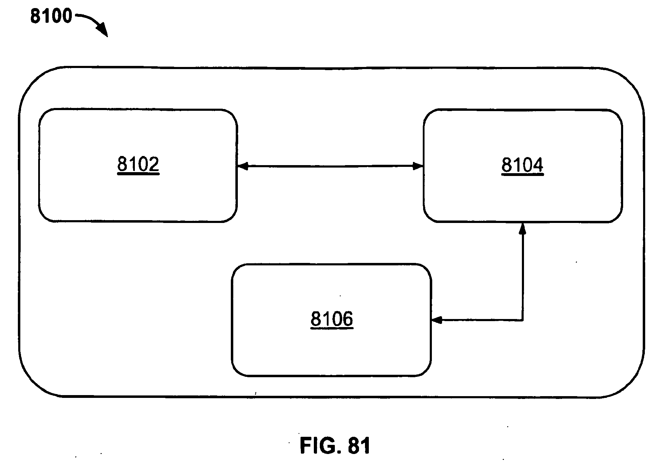

FIG. 81 is a block diagrammatic view of a system facilitating implementation of an Opto-Magnetic process based on light-matter interaction using digital imaging for Papanicolau Test Analysis of samples, designed and implemented in accordance with certain embodiments of the invention.

System 8100 is in essence a Papanicolau Test Analyzer (or PTA). The PTA 8100 includes an illumination subsystem 8102, an imaging (or sensor) subsystem 8104 and a host computing subsystem 8106.

PTA 8100, by virtue of its design and implementation, facilitates execution of an Opto-Magnetic method based on interaction between electromagnetic radiation and matter, for instance light-matter interaction, using digital imaging for analysis of samples subjected to Papanicolau Test. Specifically, the Opto-Magnetic process employs apparatuses for generation of unique spectral signatures from digitally captured images of samples thereby facilitating analysis of the samples subjected to Papanicolau Test based on Opto-Magnetic properties of light-blood plasma interaction.

Illumination subsystem 8102 may be one or more electromagnetic radiation sources. In certain specific embodiments, the Illumination subsystem 8102 may be a set of Light Emitting Diodes (LEDs).

Illumination subsystem 8102 may be adapted to emit polarized and unpolarized electromagnetic signals. The polarized electromagnetic signal is angled white light and unpolarized electromagnetic signal is non-angled white light.

As shown in the FIG. 81, in certain embodiments, the illumination subsystem 8102 may be coupled to the sensor subsystem 8104.

As shown in the FIG. 81, the sensor subsystem 804 may in essence be a device that converts optical images (or optical signals) to electric signals. In certain embodiments, the sensor subsystem 8104 captures continuous digital images of blood plasma samples. Specifically, in such embodiments, the sensor subsystem 8104 captures continuous digital images of the blood plasma samples illuminated with white light both, non-angled and angled. By way of, and by no way of limitation, the sensor subsystem 8104 may be anyone selected from a group consisting of a Complementary Metal-Oxide-Semiconductor (CMOS) image sensor, Charged Coupled Device (CCD) image sensor, and the like.

Again, as shown in FIG. 81, the sensor subsystem 8104 may be coupled to the host computing subsystem 8106.

For example, and in no way limiting the scope of the invention, in certain embodiments the sensor subsystem 8104 may be selected on the basis of the following specifications: color is color or monochrome; optical format; horizontal pixels X vertical pixels; pixel size; one or more performance parameters, such as maximum frame rate, data rate, maximum power dissipation, quantum efficiency, dynamic range and supply voltage; output; one or more features, such as integrated Analog-to-Digital Converter (ADC) and microlenses; and environment, such as operating temperature.

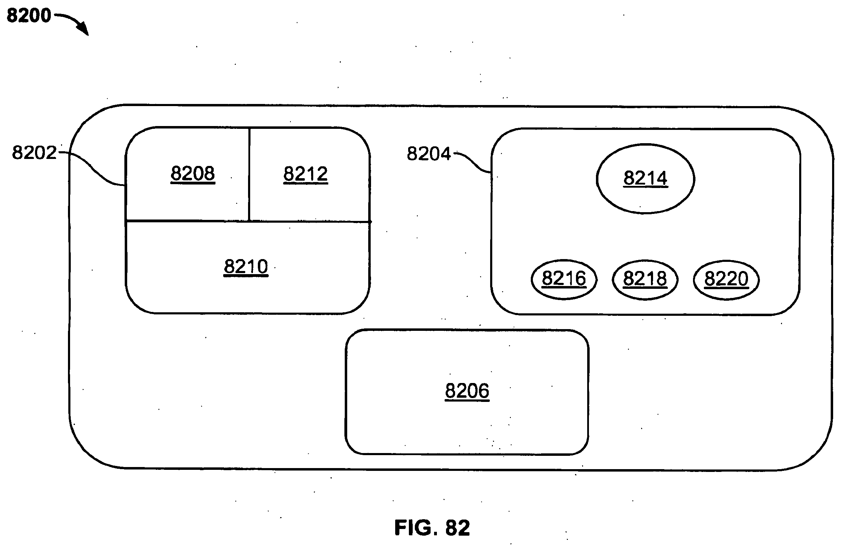

FIG. 82 is an exploded diagrammatic representation of the host computing subsystem, of the Fig. 81, comprising the Opto-Magnetic Fingerprint (or OMF) Generator module designed and implemented in accordance with at least some embodiments.

The host computing subsystem 8200 may comprise a processing unit 8202, a memory unit 8204 and an Input / Output (or I/O) unit 206 respectively.

The host computing subsystem 8200, by virtue of its design and implementation, performs overall management of blood plasma samples.

The processing unit 8202 may comprise an Arithmetic Logic Unit (or ALU) 8208, a Control Unit (or CU) 8210 and a Register Unit (or RU) 8212.

As shown in FIG. 82, the memory unit 8204 comprises a test analysis module 8214.

In certain embodiments, the test analysis module for analysis of samples subjected to Papanicolau Test via generation of unique spectral signatures from the digitally captured images of the samples and methods thereof are disclosed, in accordance with the principles of the invention. Specifically, in such embodiments, the test analysis module utilizes the continuously captured digital images of the samples illuminated with white light both, non-angled and angled. More specifically, the blood plasma virus detection module takes into consideration the digital images in Red (R), Green (G) and Blue (B) (or RGB) system for purposes of analysis.

Further, as shown in FIG. 82, the test analysis module 8214 includes a Fourier transform sub-module 8216, a spectral analyzer sub-module 8218 and an Opto-Magnetic Fingerprint Generator (or OMFG) sub-module 8220, respectively.

In certain embodiments, the Fourier transform sub-module 8216 is in essence a Discrete-Time Fourier Transform (or DTFT).

The term "DTFT", as used herein, refers to one of the specific forms of Fourier analysis. As such, it transforms one function into another, which is called the frequency domain representation, or simply the "DTFT", of the original function, which is often a function in the time-domain. But, the DTFT requires an input function that is discrete. Such inputs are often created by sampling a continuous function, like a person's voice. The DTFT frequency-domain representation is always a periodic function. Since one period of the function contains all of the unique information, it is sometimes convenient to say that the DTFT is a transform to a "finite" frequency-domain (the length of one period), rather than to the entire real line.

DTFT 8216 converts time-domain digital signals into corresponding frequency-domain digital signals.

DTFT 8216 is coupled to the spectrum analyzer sub-module 8218.

As used herein, the term "spectrum analyzer" refers to a device used to examine the spectral composition of some electrical, acoustic, or optical waveform. It may also measure the power spectrum. In general, there are three types of spectrum analyzers, such as analog, digital and real-time spectrum analyzers. Firstly, an analog spectrum analyzer uses either a variable band-pass filter whose mid-frequency is automatically tuned (i.e. shifted, swept) through the range of frequencies of the spectrum to be measured or a superheterodyne receiver, wherein the local oscillator is swept through a range of frequencies. Secondly, a digital spectrum analyzer computes the Discrete Fourier transform (or DFT), a mathematical process that transforms a waveform into the components of its frequency spectrum. Eventually, some spectrum analyzers, such as "real-time spectrum analyzers", use a hybrid technique where the incoming signal is first down-converted to a lower frequency using superheterodyne techniques and then analyzed using fast Fourier transformation (FFT) techniques.

In certain embodiments, the spectrum (or spectral) analyzer sub-module for analysis of digitally captured images of samples thereby facilitating analysis of the samples subjected to Papanicolau Test is disclosed. Specifically, the spectrum (or spectral) analyzer sub-module in order to analyze the samples takes into consideration digital images of the samples in Red (R), Green (G) and Blue (B) (or RGB) system. In certain such embodiments, basic pixel data in Red (R) and Blue (B) channels for both white diffuse light (or W) and reflected polarized light (or P) is selected. In here, the algorithm for data analysis is based on chromaticity diagram called "Maxwell's triangle" and spectral convolution.

In certain specific embodiments, the digital images in Red (R), Green (G) and Blue (B) (or RGB) system are taken into consideration for purposes of spectral analysis. Specifically, basic pixel data in Red (R) and Blue (B) channels for white diffuse light (or W) and reflected polarized white light (or P) is selected. More specifically, the algorithm for data analysis is based on chromaticity diagram called "Maxwell's triangle" and spectral convolution operation, in accordance with a ratio of (R - B) & (W - P). Noticeably, the abbreviated designation implies that Red (R) minus Blue (B) wavelength of White light (W) and reflected Polarized light (P) are used in spectral convolution algorithm to calculate data for Opto-Magnetic Fingerprint (OMF) of matter both, organic and inorganic. Consequently, method and algorithm for creating unique spectral fingerprint are based on the convolution of RGB color channel spectral plots generated from digital images that capture single and multi-wavelength light-matter interaction for different paramagnetic materials, such as Al, Mn and Ti, diamagnetic materials, such as Cu, C and Zn, alloys, such as $Pb_{1-x}Mn_xTe$, Biomolecules and biological tissues as paramagnetic / diamagnetic materials, such as skin, biological water, amniotic fluid, blood plasma and the like.

Further, incident white light can give different information about properties of thin layer of matter, such as blood plasma sample surface, depending on the angle of light incidence. In use, when the incident white light is diffuse, the reflected white light is then composed of electrical and magnetic components, whereas diffuse incident light that is inclined under certain angle will produce reflected light which contains only electrical component of light.

As shown in FIG. 82, the spectrum analyzer sub-module 8218 may be coupled to the OMFG sub-module 8220.

OMFG sub-module 8220 includes a color histogram generator unit 8222, a spectral plot generator unit 8224 and a convolution unit 8226.

OMFG sub-module 8214, by virtue of its design and implementation, facilitates generation of unique spectral signatures from digitally captured images of Pap test samples. Specifically, the generated spectral signatures of Pap test samples facilitate detection of cancer based on Opto-Magnetic properties of light-blood plasma interaction.

Color histogram generator unit 8222, by virtue of its design, generates a normalized Red (R) and Blue (B) color channel histogram for each of the one or more images of the blood plasma samples.

The term "color histogram", as used in computer graphics and photography, refers to is a representation of the distribution of colors in an image, derived by counting the number of pixels of each of given set of color ranges in a typically two-dimensional (2D) or three-dimensional (3D) color space. A histogram is a standard statistical description of a distribution in terms of occurrence frequencies of different event classes; for color, the event classes are regions in color space. An image histogram of scalar pixel values is more commonly used in image processing than is a color histogram. The term "image histogram" refers to a type of histogram which acts as a graphical representation of the tonal distribution in a digital image. It plots the number of pixels for each tonal value. By looking at the histogram for a specific image a viewer is able to judge the entire tonal distribution at a glance.

Typically, color histograms are flexible constructs that can be built from images in various color spaces, whether RGB, rg chromaticity or any other color space of any dimension. A histogram of an image is produced first by discretization of the colors in the image into a number of bins, and counting the number of image pixels in each bin. For example, a Red-Blue chromaticity histogram can be formed by first normalizing color pixel values by dividing RGB values by R+G+B, then quantizing the normalized R and B coordinates into N bins each, where N = 4, which might yield a 2D histogram that looks like this table:

Table 3 exhibits a tabular representation in connection with a 2D Red-Blue chromaticity histogram generated by first normalizing color pixel values by dividing RGB values by R+G+B, then quantizing the normalized R and B coordinates into N bins each, where N = 4.

| | | R | | | |
|---|---|---|---|---|---|
| | | 0-63 | 64-127 | 128-191 | 192-255 |
| B | 0-63 | 43 | 78 | 18 | 0 |
| | 64-127 | 45 | 67 | 33 | 2 |
| | 128-191 | 127 | 58 | 25 | 8 |
| | 192-25 | 140 | 47 | 47 | 13 |

As shown in FIG. 82, the color histogram generator unit 8222 may be coupled to the spectral plot generator unit 8224.

Spectral plot generator unit 224 generates Red (R) and Blue (B) color channel spectral plots by correlating the normalized Red (R) and Blue (B) color channel histograms to a wavelength scale. In certain embodiments, a unit scale on the spectral signature is a difference of wavelength.

In general, color digital images are made of pixels and, in turn, pixels are made of combinations of primary colors. As used in the current context, the term "channel" refers to the grayscale image of the same size as a color image, made of just one of these primary colors. For instance, an image from a standard digital camera will have a red, green and blue channel. A grayscale image has just one channel. Further, an RGB image has three channels, namely Red (R), Green (G) and Blue (B). For example, if the RGB image is 24-bit then each channel has 8 bits, for R, G and B. Stated differently, the image is composed of three grayscale images, where each grayscale image can store discrete pixels with conventional brightness intensities between 0 and 255. Whereas, if the RGB image is 48-bit (i.e. very high resolution), each channel is made of 16-bit grayscale images.

The periodogram is an estimate of the spectral density of a signal. The term "spectral plot" refers to a smoothed version of the periodogram. Smoothing is performed to reduce the effect of measurement noise.

Convolution unit 8226 convolutes the Red (R) and Blue (B) color channel spectral plots by subtracting the spectral plot for the polarized optical electromagnetic signal from the non-polarized optical electromagnetic signal for each color to generate Red (R) and Blue (B) normalized, composite color channel spectral plots and subtracting the normalized, composite Blue (B) channel spectral plot from the normalized, composite Red (R) channel spectral plot thereby resulting in generation of a spectral signature for the Pap test samples.

In certain embodiments, the spectral signature is analyzed for at least one of number of crests and troughs, amplitude, shape of peaks, intermediate structures and patterns. In certain such embodiments, the spectral signature is analysed for material composition, identification, purity and the like.

In certain other embodiments, the system configuration, discussed in conjunction with FIGS. 81 and 82, implement one or more processes facilitating estimation of blood plasma type and properties (or characteristics) thereof to create a unique spectral signature.

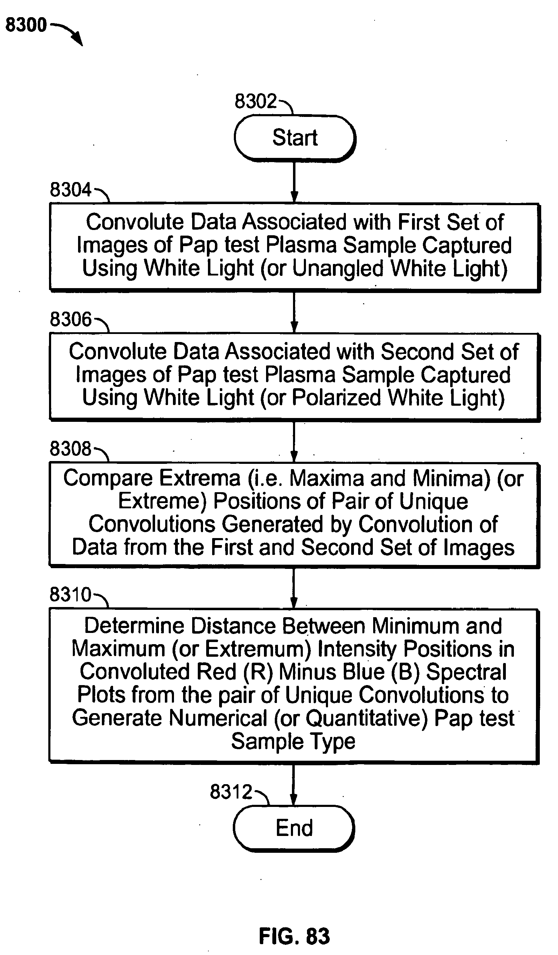

FIG. 83 depicts a flow diagram delineating at least one process implemented by the system configuration of FIGS. 81 and 82 thereby facilitating estimation of Pap test sample type and properties (or characteristics) thereof and creation of a unique spectral signature.

The process 8300 starts at stage 8302 and proceeds to stage 8304, wherein the process 8300 comprises the phase of convolution of data associated with a first set of images of a Pap test sample captured by illuminating the sample with a white light (or unangled white light.) Noticeable here is the fact that the data associated with the first set of images of the Pap test sample illuminated with the white light (or unangled white light) may comprise one or more combinations of reflected and re-emitted angled and unangled white light.

At stage 8306, the process 8300 comprises the phase of convolution of data associated with a second set of images of the Pap test sample captured by illuminating the sample with an angled white light. It must be noted here that the data associated with the second set of images of the Pap test sample illuminated with the angled white light may comprise one or more combinations of reflected and re-emitted angled white light.

At stage 8308, the process 8300 comprises the phase of comparison of extrema (i.e. maxima and minima) (or extreme) positions of at least a pair of unique convolutions generated by convolution of data from the first set of images and second set of images.

At stage 8310, the process 8300 comprises the phase of determination of a distance between minimum and maximum (or extremum) intensity positions in convoluted Red (R) minus Blue (B) spectral plots from the pair of unique convolutions generated by convolution of data from the first set of images and second set of images to generate a numerical (or quantitative) Pap test sample type. The process 8300 ends at stage 8312.

In certain embodiments, the phase of comparison of extrema (i.e. maxima and minima) (or extreme) positions of at least a pair of unique convolutions comprises implementation of one or more sub-phases. Specifically, the one or more sub-phases include comparison of a first component Red (R) minus Blue (B) of unangled white light (or W) minus angled white light (or polarized white light or P) (i.e. (R - B) (W - P)) versus a second component Red (R) minus Blue (B) of unangled white light (or W) (i.e. (R - B) W). The two unique convolutions in unangled white light and angled (or polarized) white light further include a White Red component (WR), a White Blue component (WB), a reflected and / or re-emitted Polarized Blue component (PB) and a reflected and / or re-emitted Polarized Red component (PR). The two unique convolutions are based on a numerical value difference correlating to medical standards.

In certain embodiments, the exploded diagrammatic representation in FIG. 74 of the host computing subsystem, of the Fig. 71, may comprise the Opto-Magnetic Fingerprint (or OMF) Generator sub-module designed and implemented in accordance with at least some embodiments. Thus, all ins-and-outs in connection with the OMFG sub-module 8220 have not been detailed herein.

In certain alternative embodiments, the step of comparing extreme positions of at least two unique convolutions

includes comparing a component (R - B) (W - P) for the reflected and / or re-emitted polarized light, and a component (R - B) W for the white light. Yet, in certain embodiments, the step of comparing extreme positions of at least two unique convolutions includes a spectral convolution scheme, wherein multiple combinations of subtraction of Blue (B) spectrum from Red (R), in white light and polarized white light are determined, wherein the spectral interval is expressed in a wavelength scale interval of 100 nanometers to 300 nanometers.

In certain circumstances, the investigation of Pap test performed, as adjunct to yearly screening, over a sample set taken from 40 women is disclosed. In such circumstances, the 40 samples are prepared for standard Pap test and examined as double-blind experiment using digital imaging software that analyzes the difference between reflected diffuse white light and reflected polarized light (Opto-Magnetic Fingerprint-OMF) in order to detect normal, dysplastic and cancerous cells. Specifically, the samples were prepared according to standard fixation and staining procedures used for Pap smear tests during regular colposcopic examination. More specifically, the Opto-magnetic images of samples are analyzed using a digital camera customized for capturing OMF pictures (or DI-OMF) and light-mater interaction analysis software (DI-OMF), which guides the diagnostic decision to more refined distinction between normal smear and the one containing either dysplastic or cancerous cells.

The term "double-blind experiment or double-blind trials" refers to an especially stringent way of conducting an experiment, usually on human subjects, in an attempts to eliminate subjective bias on the part of both experimental subjects and the experimenters. In most cases, double-blind experiments are held to achieve a higher standard of scientific rigor. In a double-blind experiment, neither the individuals nor the researchers know who belongs to the control group and the experimental group. Only after all the data have been recorded (and in some cases, analyzed) do the researchers learn which individuals are which. Performing an experiment in double-blind fashion is a way to lessen the influence of the prejudices and unintentional physical cues on the results (the placebo effect, observer bias, and experimenter's bias). Random assignment of the subject to the experimental or control group is a critical part of double-blind research design. The key that identifies the subjects and which group they belonged to is kept by a third party and not given to the researchers until the study is over.

Still, in certain situations, the DI-OMF diagrams are separated into five groups. Subsequent to completion of DI-OMF analysis, randomized samples codes were removed and a comparative analysis of results of DI-OMF vis-a-vis Pap test is performed. Analysis of the results of comparison show that 40 slides were categorized by standard Pap test examination into five groups, namely Group I (or normal tissue state) 7 cases, Group II (or non-typical inflammation) 8 cases, Group III (or dysplasia) 17 cases, Group IV (or carcinoma in situ) 5 cases and Group V (or suspicion to carcinoma) 3 cases.

Table 4 exhibits a tabular representation in connection with the comparative analysis of results of Pap test vis-a-vis DI-OMF and matching results thereof.

| CASE | TOTAL CASES | TRUE POSITIVE | FALSE POSITIVE | TRUE NEGATIVE | FALSE NEGATIVE |
|---|---|---|---|---|---|
| GROUP I - NORMAL | 7 | 0 | 1 | 6 | 0 |
| GROUP II - NON-TYPICAL INFLAMMATION | 8 | 7 | 0 | 0 | 1 |
| GROUP III DYSPLASIA | 17 | 16 | 0 | 0 | 1 |
| GROUP IV CARCINO MA IN SITU | 5 | 5 | 0 | 0 | 0 |
| GROUP V - SUSPICION TO CARCINO MA | 3 | 3 | 0 | 0 | 0 |
| TOTAL | 40 | 31 | 1 | 7 | 2 |

According to data from Table 3, for all 40 cases, sensitivity of DI-OMF method compared to Pap test is 93.9% and specificity is 87.5%.

In certain cases, one or more typical digital images of Pap smear slide samples, categorized as Group I, captured using diffuse white light and reflected polarized light are selected for purposes of observation and analysis.

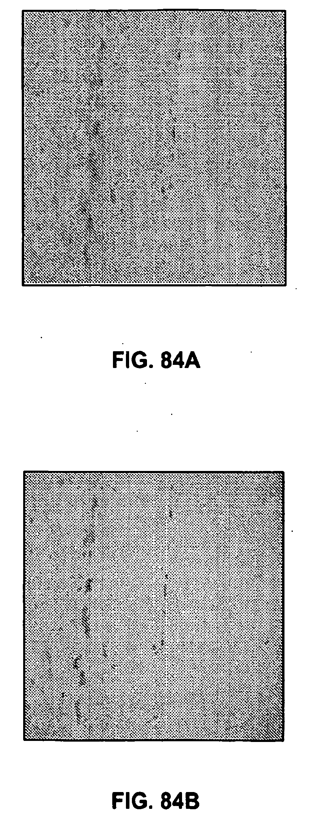

FIGS. 84A-B, 85A-B and 86A-B depict a triple pair of typical digital images of samples (or Pap smear slides), categorized as Group I (or normal tissue state), captured with diffuse white light (W) and reflected polarized light (P), in that order.

As shown in FIGS. 84A-B, a first pair of the triple pair of digital photography images of a given, selected first sample (or Pap smear slide) categorized as Group I (or normal tissue state), is captured with diffuse white light and reflected polarized light. For purposes of expediency and clarity, the sample categorized as Group I (or normal tissue state) is collected from a first patient herein referred to as Group I Patient 1. For purposes of further convenience, the digital photography images of the sample captured using the diffuse white light and reflected polarized light have been labeled as "LEFT" and "RIGHT", in that order.

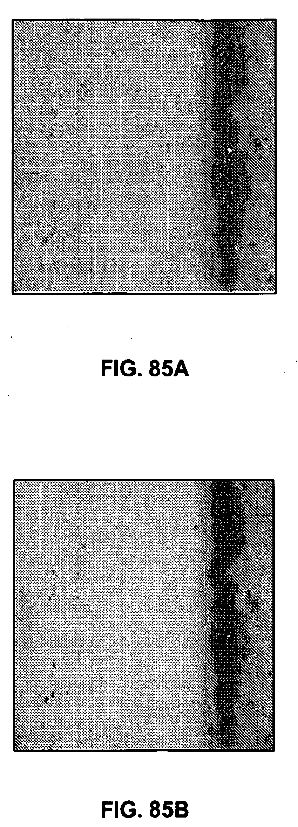

Likewise, as shown in FIGS. 85A-B, a second pair of the triple pair of digital photography images of a given, selected second sample (or Pap smear slide) categorized as group I (or normal tissue state), is captured with diffuse white light and reflected polarized light. For purposes of expediency and clarity, the sample categorized as Group I (or normal tissue state) is collected from a second patient herein referred to as Group I Patient 2. For purposes of further convenience, the digital photography images of the sample captured using the diffuse white light and reflected polarized light have been labeled as "LEFT" and "RIGHT", in that order.

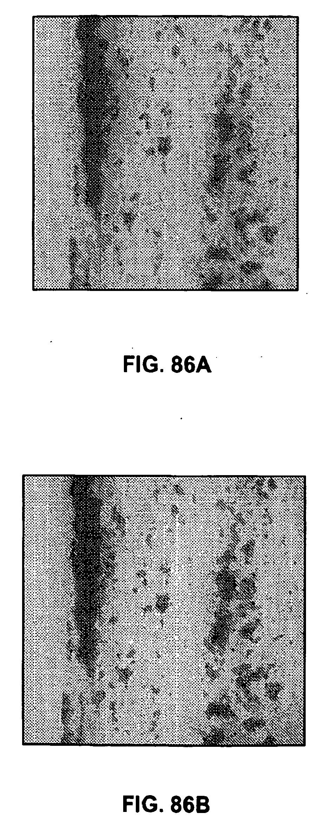

Likewise, as shown in FIGS. 86A-B, a third pair of the triple pair of digital photography images of a given, selected third sample (or Pap smear slide) categorized as group I (or normal tissue state), is captured with diffuse white light and reflected polarized light. For purposes of expediency and clarity, the sample categorized as Group I (or normal tissue state) is collected from a third patient herein referred to as Group I Patient 3. For purposes of further convenience, the digital photography images of the sample captured using the diffuse white light and reflected polarized light have been labeled as "LEFT" and "RIGHT", in that order.

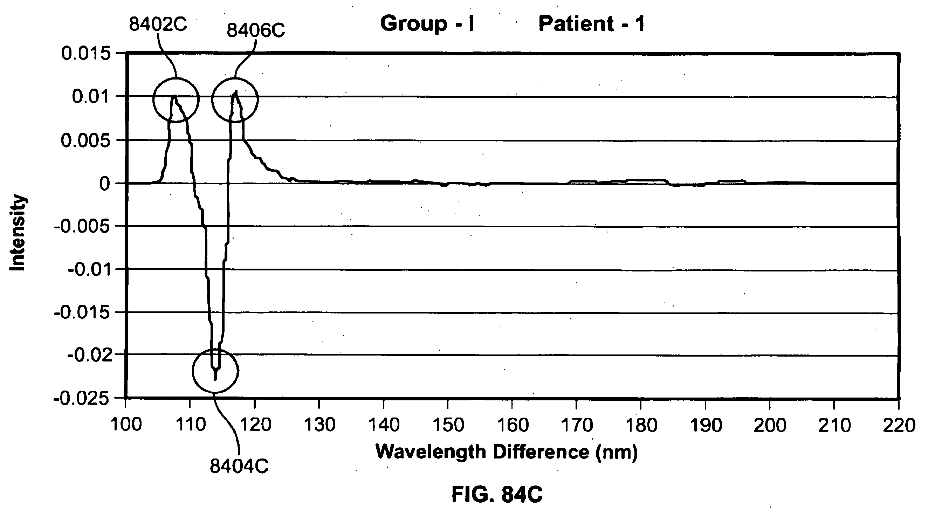

Observation of the triple pair of digital photography images in FIGS. 84A-B, 85A-B and 86A-B by naked eye would probably testify that there are no quantifiable differences between them. However, using Computer Assisted Analysis (CAA) based on pixel by pixel count and Spectral Convolution Algorithm (SCA) significant differences are found the final result of whose is illustrated in conjunction with FIGS. 84C, 85C and 86C respectively.

In certain embodiments, a limited number of typical cases comprising samples (or Pap smear slides) categorized into one or more groups based on states of samples, such as "Group I (or normal tissue state)," "Group II (or non-typical inflammation)," "Group III (or dysplasia)," "Group IV (or carcinoma in situ)," and "Group V (or suspicion to carcinoma)", are selected and presented for purposes of illustration. Specifically, three typical cases of Group I, namely one "Group I Patient 1," one "Group I Patient 2," and one "Group I Patient 3", and one case from each of the Groups II, III, IV and V, namely "Group II Patient 17," "Group III Patient 16," "Group IV Patient 4," and "Group V Patient 7", are selected and presented for purposes of illustration.

In certain specific embodiments, CAA based on pixel by pixel count and SCA is implemented taking into consideration only three typical cases of Group I, namely one "Group I Patient 1," one "Group I Patient 2," and one "Group I Patient 3", and one case from each of the Groups II, III, IV and V, namely "Group II Patient 17," "Group III Patient 16," "Group IV Patient 4," and "Group V Patient 7", thereby facilitating illustration of characteristics of spectral data thereof. In such specific embodiments, for purposes of illustration of the spectral data obtained on implementation of the CAA and SCA, a two (or 2 D)-dimensional coordinate system including a horizontal X-axis and a vertical Y-axis is selected. Specifically, the horizontal X-axis represents the wavelength difference in nanometers whereas the vertical Y-axis represents the intensity in suitable units. More specifically, the 2D coordinate system exhibits the comparative analysis of wavelength difference versus intensity for given samples collected from given patients and subjected to tests for presence or absence of normal, dysplastic and cancerous cells, wherein the wavelength difference is the independent variable and the intensity is the dependent variable.

FIG. 84C depicts a plot of a typical spectral data (or OMF diagram) obtained on implementation of the OMF method on digital images of FIGS. 84A-B of the given, selected first sample (or Pap smear slide) categorized as Group I (or normal tissue state), in accordance with certain embodiments of the invention.

As shown in FIG. 84C, the 2D coordinate system is in essence a Wavelength Difference Versus Intensity plot (or DI plot or OMF diagram) obtained on plotting a plurality of DI ordered pairs. Each of the plurality of ordered pairs includes a Wavelength Difference value and a corresponding Intensity value. It must be noted here that the plurality of ordered pairs are obtained on processing the digital image of the first sample, captured using diffuse white light and reflected polarized light, using the OMF method. Specifically, the OMF method implements the SCA and CAA to analyze the processed digital image of the sample. Further, the sample is the given, selected first sample (or Pap smear slide) categorized as Group I (or normal tissue state) of the given, selected first patient subjected to Pap test.

As depicted in FIG. 84C, a first DI plot possesses the following specifications and associated analytical information thereof: ordered (or DI) pair is (Wavelength Difference Value, Intensity Value); horizontal X-axis includes a closed interval of Wavelength Difference Values ranging from a minimum of equal to 100 nanometers (nm) to a maximum of equal to 220 nanometers (nm) (or [100, 220]); vertical X-axis includes a closed interval of Intensity Values ranging from a minimum of equal to -0.025 to a maximum of equal to +0.015; analytical information is analysis of the first DI plot (or OMF Diagram) of the sample; patient information is a given, selected first patient of the Group I (or normal tissue state) or Group I Patient 1; test input sample is the Pap smear slide categorized as the Group I (or normal tissue state) of the patient referred to as Group I Patient 1; operation is implementation of OMF method on digital images of FIGS. 4A-B of the given, selected first sample (or Pap smear slide) categorized as Group I (or normal tissue state); number of intensity peaks (or extrema or maxima and minima) is 3; number of peaks with positive intensity values is 2; number of peaks with negative intensity value is 1; identifiers for the 3 intensity peaks are first 8402A, second 8404A and third 8408A respectively; values for Wavelength Difference / Intensity associated with the first 8402A, second 8404A and third 8406A intensity peaks are 105.5 nm / 0.095 Intensity (arb units), 113.7 nm / -0.022 arb and 119.2 nm / 0.012 arb in that order.

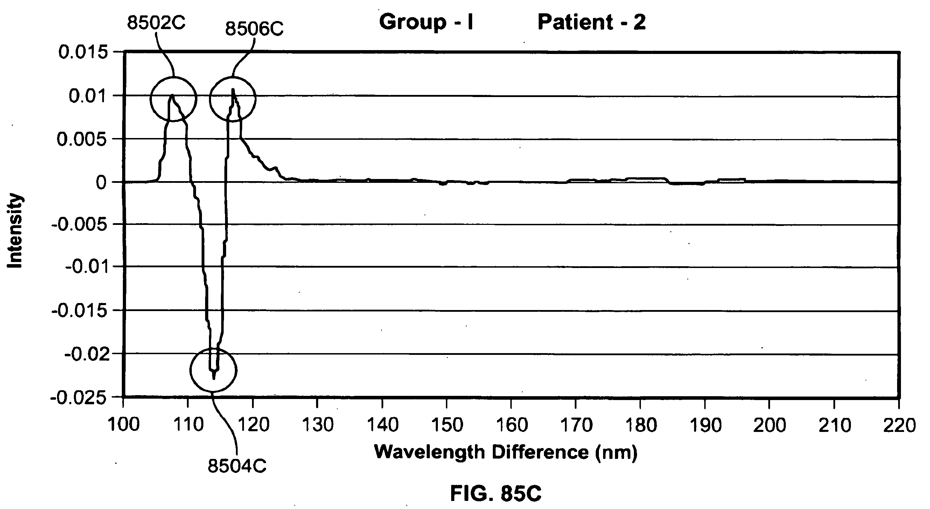

FIG. 85C depicts a plot of a typical spectral data (or OMF diagram) obtained on implementation of the OMF method on digital images of FIGS. 85A-B of the given, selected second sample (or Pap smear slide) categorized as Group I (or normal tissue state), in accordance with certain embodiments of the invention.

As depicted in FIG. 85C, a second DI plot possess the following specifications and associated analytical information thereof: ordered (or DI) pair is (Wavelength Difference Value, Intensity Value); horizontal X-axis includes a closed interval of Wavelength Difference Values ranging from a minimum of equal to 100 nanometers (nm) to a maximum of equal to 220 nanometers (nm) (or [100, 220]); vertical X-axis includes a closed interval of Intensity Values ranging from a minimum of equal to -0.025 to a maximum of equal to +0.015; analytical information is analysis of the second DI plot (or OMF Diagram) of the digital photography image of the sample; patient information is the given, selected second patient of the Group I (or normal tissue state) or Group I Patient 2; test input sample is the Pap smear slide categorized as the Group I (or normal tissue state) of the patient referred to as Group I Patient 2; operation is implementation of OMF method on digital images of FIGS. 85A-B of the given, selected second sample (or Pap smear slide) categorized as Group I (or normal tissue state); number of intensity peaks (or extrema or maxima and minima) is 3; number of intensity peaks (or extrema or maxima and minima) is 3; number of peaks with positive intensity values is 2; number of peaks with negative intensity value is 1; identifiers for the 3 intensity peaks are first 8502A, second 8504A and third 8506A respectively; values for Wavelength Difference / Intensity associated with the first, second and third intensity peaks are 107.5 nm / 0.010 arb, 114.2 nm / -0.023 arb and 118.9 nm / 0.011 arb in that order.

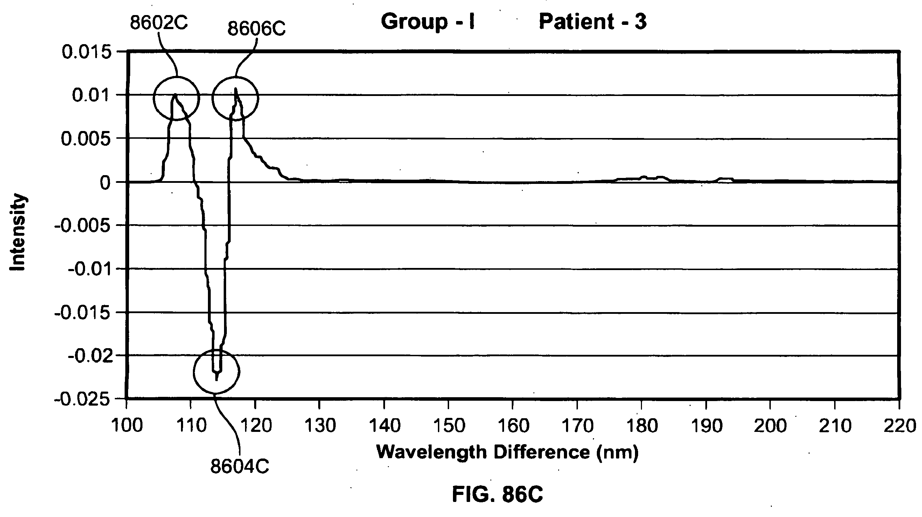

FIG. 86C depicts a plot of a typical spectral data (or OMF diagram) obtained on implementation of the OMF method on digital images of FIGS. 86A-B of the given, selected third sample (or Pap smear slide) categorized as Group I (or normal tissue state), in accordance with certain embodiments of the invention.

As depicted in FIG. 86C, a third DI plot possess the following specifications and associated analytical information thereof: ordered (or DI) pair is (Wavelength Difference Value, Intensity Value); horizontal X-axis includes a closed interval of Wavelength Difference Values ranging from a minimum of equal to 100 nanometers (nm) to a maximum of equal to 220 nanometers (nm) (or [100, 220]); vertical X-axis includes a closed interval of Intensity Values ranging from a minimum of equal to -0.025 to a maximum of equal to +0.015; analytical information is analysis of the third DI plot (or OMF Diagram) of the digital photography image of the sample; patient information is the given, selected third patient of the Group I (or normal tissue state) or Group I Patient 3; test input sample is the Pap smear slide categorized as the Group I (or normal tissue state) of the patient referred to as Group I Patient 3; operation is implementation of OMF method on digital images of FIGS. 86A-B of the given, selected third sample (or Pap smear slide) categorized as Group I (or normal tissue state); number of intensity peaks (or extrema or maxima and minima) is 3; number of intensity peaks (or extrema or maxima and minima) is 3; number of peaks with positive intensity values is 2; number of peaks with negative intensity value is 1; identifiers for the 3 intensity peaks are first 8602A, second 8604A and third 8606A respectively; values for Wavelength Difference / Intensity associated with the first, second and third intensity peaks are 109.0 nm / 0.0098 arb, 114.0 nm / -0.024 arb and 117.9 nm / 0.0102 arb in that order.

Despite the fact that the digital images in FIGS. 84A-B, 85A-B and 86A-B are different, their OMF diagrams appear almost identical. Apparently, in the FIGS. 84C, 85C and 86C three peaks are seen, wherein a pair of the peaks possesses very similar positive intensity values (i.e. 108 nm and 118 nm) and one with a larger negative intensity value (i.e. 113 nm). These values are valid for spectral convolution field. They are symmetrical and indicate normal tissue state. Reason for this is same Pap group, which is in this case normal.

However, the similarity of OMF diagrams for samples categorized as Group II (non-typical inflammation) is not nearly ubiquitous as for Group I (normal), while for Group III (dysplasia) there are significant differences between samples. Reason for this is because there is different intensity of dysplasia (week, middle, strong). All samples belong to the same group but with diversity from case to case, and peaks varying in intensity and in difference of their position.

In certain other embodiments, one or more typical cases comprising samples (or Pap smear slides) categorized as group II (or non-typical inflammation) are selected and presented for purposes of illustration. Specifically, one typical case including a sample categorized as group II (or non-typical inflammation) is taken into consideration and presented for purposes of illustration.

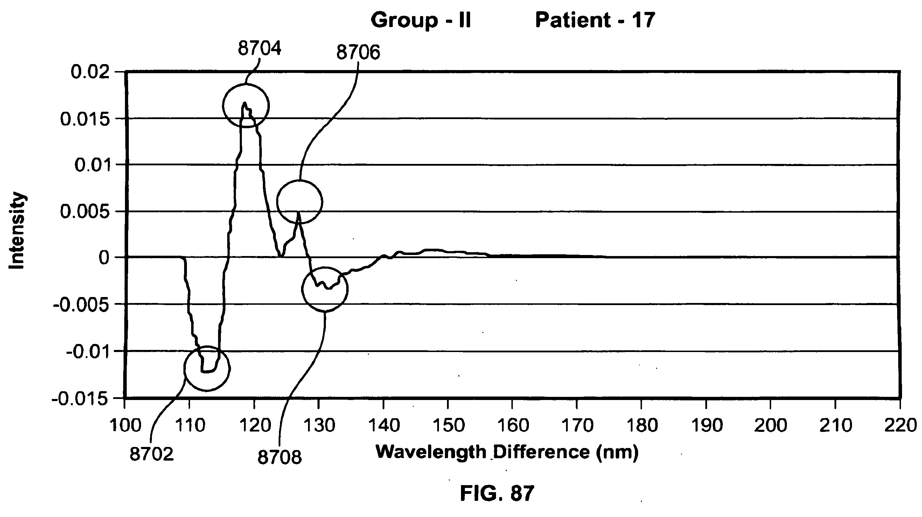

FIG. 87 depicts a plot of a typical spectral data (or OMF diagram) obtained on implementation of the OMF method on digital images of a given, selected sample (or Pap smear slide) categorized as Group II (or non-typical inflammation), in accordance with certain embodiments of the invention.

As depicted in FIG. 87, a fourth DI plot possess the following specifications and associated analytical information thereof: ordered (or DI) pair is (Wavelength Difference Value, Intensity Value); horizontal X-axis includes a closed interval of Wavelength Difference Values ranging from a minimum of equal to 100 nanometers (nm) to a maximum of equal to 220 nanometers (nm) (or [100, 220]); vertical X-axis includes a closed interval of Intensity Values ranging from a minimum of equal to -0.015 to a maximum of equal to +0.02; analytical information is analysis of the fourth DI plot (or OMF Diagram) of the digital photography image of the sample; patient information is the given, selected seventeenth patient of the Group II (or non-typical inflammation) or Group II Patient 17; test input sample is the Pap smear slide categorized as the Group II (or non-typical inflammation) of the patient referred to as Group II Patient 17; operation is implementation of OMF method on digital images of the given, selected seventeenth sample (or Pap smear slide) categorized as the Group II (or non-typical inflammation); number of intensity peaks (or extrema or maxima and minima) is 4; number of peaks with positive intensity values is 2; number of peaks with negative intensity value is 2; identifiers for the 4 intensity peaks are first 8702, second 8704, third 8706 and fourth 8708 respectively; values for Wavelength Difference / Intensity associated with the first, second, third and fourth intensity peaks are 112.5 nm / -0.013 arb, 118.9 nm / 0.016 arb, 126.8 nm / 0.005 arb, 131.4 nm / -0.003 arb in that order.

Investigation of FIG. 87 suggests that the OMF diagram presented therein has a different diagram pattern vis-à-vis the diagrams discussed in conjunction with the FIGS. 84C, 85C and 86C. Noteworthy is the fact that all higher order Pap groups can be described with distinctive diagrams depicting the characteristic intensity to wavelength relationship thereof. Particularly, noteworthy is the fact that these patterns differ in an easily detectable manner. For example, the diagram for Group II shown in FIG. 87 has one peak more than the sample from Group I. More particularly, four peaks belonging to following wavelengths: 112 nm, 120 nm, 128 nm and 132 nm, have intensities and wavelengths whose distribution differs from that of the group I.

The same kind of analysis can be conducted in a straightforward manner for the sample diagram in Group III, shown in FIG. 86. The four peaks for Group III differ from FIG. 85 in intensities and also possess a slight shift in corresponding wavelengths.

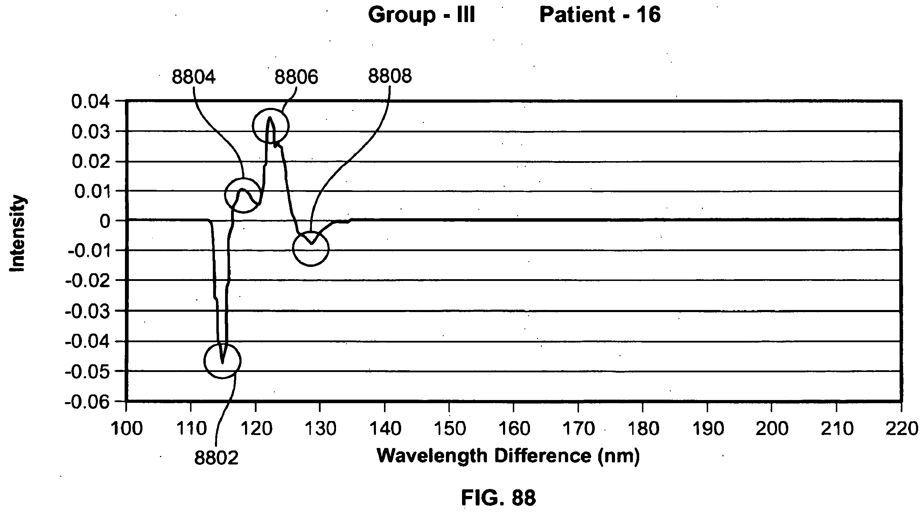

FIG. 88 depicts a plot of a typical spectral data (or OMF diagram) obtained on implementation of the OMF method on digital images of a given, selected sample (or Pap smear slide) categorized as Group III (dysplasia), in accordance with certain embodiments of the invention.

As depicted in FIG. 88, a fifth DI plot possess the following specifications and associated analytical information thereof: ordered (or DI) pair is (Wavelength Difference Value, Intensity Value); horizontal X-axis includes a closed interval of Wavelength Difference Values ranging from a minimum of equal to 100 nanometers (nm) to a maximum of equal to 220 nanometers (nm) (or [100, 220]); vertical X-axis includes a closed interval of Intensity Values ranging from a minimum of equal to -0.06 to a maximum of equal to +0.04; analytical information is analysis of the fifth DI plot (or OMF Diagram) of the sample; patient information is a given, selected seventeenth patient of the Group III (or non-typical inflammation); test input sample is the Pap smear slide categorized as Group III of a patient referred to as Group III Patient 16; operation is implementation of OMF method on digital images of the given, selected seventeenth sample (or Pap smear slide) categorized as the group II (or non-typical inflammation); number of intensity peaks (or extrema or maxima and minima) is 4; number of peaks with positive intensity values is 2; number of peaks with negative intensity value is 2; identifiers for the 4 intensity peaks are first 8802, second 8804, third 8806 and fourth 8808 respectively; values for Wavelength Difference / Intensity associated with the first, second, third and fourth intensity peaks are 112.5 nm / -0.013 arb, 118.9 nm / 0.016 arb, 126.8 nm / 0.005 arb, 131.4 nm / -0.003 arb in that order.

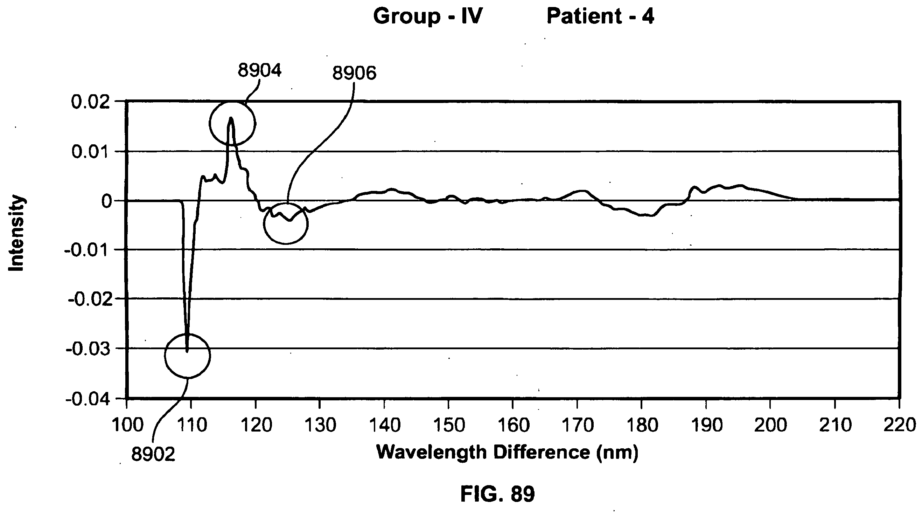

FIG. 89 depicts a plot of a typical spectral data (or OMF diagram) obtained on implementation of the OMF method on digital images of a given, selected sample (or Pap smear slide) categorized as Group IV (carcinoma in situ), in accordance with certain embodiments of the invention.

As depicted in FIG. 89, a sixth DI plot possess the following specifications and associated analytical information thereof: ordered (or DI) pair is (Wavelength Difference Value, Intensity Value); horizontal X-axis includes a closed interval of Wavelength Difference Values ranging from a minimum of equal to 100 nanometers (nm) to a maximum of equal to 220 nanometers (nm) (or [100, 220]); vertical X-axis includes a closed interval of Intensity Values ranging from a minimum of equal to -0.04 to a maximum of equal to +0.02; analytical information is analysis of the sixth DI plot (or OMF Diagram) of the sample; patient information is a given, selected fourth patient of the Group IV (or carcinoma in situ) or Group IV Patient 4; test input sample is the Pap smear slide categorized as the Group IV (or carcinoma in situ) of the patient referred to as Group IV Patient 4; operation is implementation of OMF method on digital images of the sample; number of intensity peaks (or extrema or maxima and minima) is 3; number of peaks with positive intensity values is 1; number of peaks with negative intensity value is 2; identifiers for the 3 intensity peaks are first 8902, second 8904 and third 8906 respectively; values for Wavelength Difference / Intensity associated with the first, second and third intensity peaks are 109.4 nm / -0.031 arb, 115.9 nm / 0.016 arb and 125.0 nm / -0.004 arb in that order.

Table 5 exhibits a tabular representation in connection with parameter values of OMF study for 5 cases (carcinoma in situ) as True Positive.

| PEAK | VALUE OF GROUP IV | |
|---|---|---|
| | WAVELENGTH DIFFERENCE | INTENSITY (ARB) |
| FIRST | 110 ± 3.0 NM | - 0.03 ± 0.008 |
| SECOND | 116 ± 3.0 NM | 0.01 ± 0.008 |

(continued)

| PEAK | VALUE OF GROUP IV | |
|---|---|---|
| | WAVELENGTH DIFFERENCE | INTENSITY (ARB) |
| THIRD | 126 ± 5.0 NM | - 0.005 ± 0.003 |
| A FEW | 140 -220 NM | WEEK CORRUGATION |

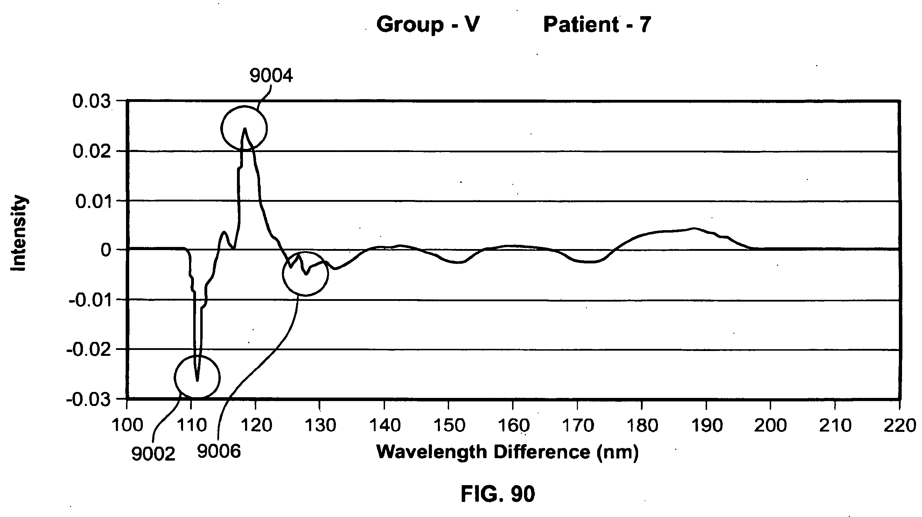

FIG. 90 depicts a plot of a typical spectral data (or OMF diagram) obtained on implementation of the OMF method on digital images of a given, selected sample (or Pap smear slide) categorized as Group V (suspicion to carcinoma), in accordance with certain embodiments of the invention.

As depicted in FIG. 90, a seventh DI plot possess the following specifications and associated analytical information thereof: ordered (or DI) pair is (Wavelength Difference Value, Intensity Value); horizontal X-axis includes a closed interval of Wavelength Difference Values ranging from a minimum of equal to 100 nanometers (nm) to a maximum of equal to 220 nanometers (nm) (or [100, 220]); vertical X-axis includes a closed interval of Intensity Values ranging from a minimum of equal to -0.03 to a maximum of equal to +0.03; analytical information is analysis of the seventh DI plot (or OMF Diagram) of the sample; patient information is a given, selected seventh patient of the Group V (suspicion to carcinoma) or Group V Patient 7; test input sample is the Pap smear slide categorized as the Group V (suspicion to carcinoma) of the patient referred to as Group V Patient 7; operation is implementation of OMF method on digital images of the sample; number of intensity peaks (or extrema or maxima and minima) is 3; number of peaks with positive intensity values is 1; number of peaks with negative intensity value is 2; identifiers for the 3 intensity peaks are first 9002A, second 9004A and third 9006A respectively; values for Wavelength Difference / Intensity associated with the first, second and third intensity peaks are 110.9 nm / -0.027 arb, 118.2 nm / 0.025 arb and 128.1 nm / -0.005 arb in that order.

OMF diagrams for samples categorized as Group IV (carcinoma in situ) and Group V (suspicion to carcinoma) share some qualitative similarity but differ markedly from Groups I, II, and III. The difference is obvious not only in distribution of peaks within lower wavelength difference range (<140 nm) but also throughout the higher spectral range of wavelength differences that is captured by this method (100-220 nm). The patterns in higher wavelength differences are unseen in lower grade groups and are likely to be produced by malignant cells.

In certain embodiments, systems for generating enhanced heterogeneous signals for use in non-invasive processing of materials using an Opto-Magnetic Antenna (or OMA), and methods thereof are disclosed.

In the description, the terms "system" and "Opto-Magnetic Amplifier (or OMA)" are used interchangeably, unless otherwise prescribed. For example, in some embodiments, the terms "system" and "Opto-Magnetic Amplifier (or OMA)" are used interchangeably to refer to a system which has been designed and implemented herein for generating enhanced heterogeneous (or mixed) signals for use in non-invasive processing of materials. Whereas, in some other embodiments, the terms "first signal processing subsystem" and "Opto-Magnetic Signal Processor (or OMSP)" are used interchangeably to refer to a subsystem which has been designed and implemented herein for generating spectral signatures for materials. In yet other some embodiments, the terms "second signal processing subsystem" and "Direct EM Signal Processor (or DEMSP)" are used interchangeably to refer to a subsystem which has been designed and implemented to process EM signals.

In certain embodiments, systems and / or methods for non-invasive surface and / or bulk processing of materials have been disclosed. Specifically, such systems and / or methods for non-invasive detection, analysis, characterization, indication, identification, and determination of materials are based on valence electrons. Such systems and / or methods measure the magnetic change in the valence orbitals. This implies that such methods measure Electro-Magnetic (EM) changes in underlying structures, such as skin, collagen, elastin or a metal. Thus, such systems and / or methods can provide information about the composition of the materials. For example, theoretically such systems and / or methods can be used down to a level approximately 1 millimeter by 1 millimeter to measure material properties.

In addition, the aforementioned systems and / or methods may be implemented as an antenna amplifier. These systems and / or methods can measure the variance in the magnetic receptance of the antenna and get highly enhanced antenna reception. In certain situations involving antennae supplied with an input signal, such systems and / or methods can give a result based on the antennae properties of the input signal. In such situations, the output signal can be enhanced based on the antenna properties.

As used in the current context, the term "magnetic reception" refers to sensitivity to magnetic stimuli. For example, the very weak magnetic stimuli occurring naturally in the environment.

In certain dermatological applications, on illuminating the skin with polarized light only the electrical properties of skin will be apparent. But, on illuminating the skin with unpolarized incident light may reveal both the electrical and magnetic properties of skin. Further, usage of the polarized light may generate improved induction of optical activity. However, the data sets generated on illumination of skin with polarized light may be of less value as compared to the data sets captured using incident unpolarized light. For example, by measuring the effects between 10-34 and 10-30 Js measurements can be made at the border area of quantum and classical physics effects on skin and as a difference of action of electrical and magnetic forces of valence electrons of skin's biomolecules.

In general, unpolarized light includes any permutations and / or combinations of diffused light, white light, monochromatic light, light of multiple single wavelengths and the like. Specifically, the white light is a light consisting of photons of all wavelengths. Thus, when a material is illuminated by the white light, photons can make the valence electrons of an atom transition to a higher electronic energy level.

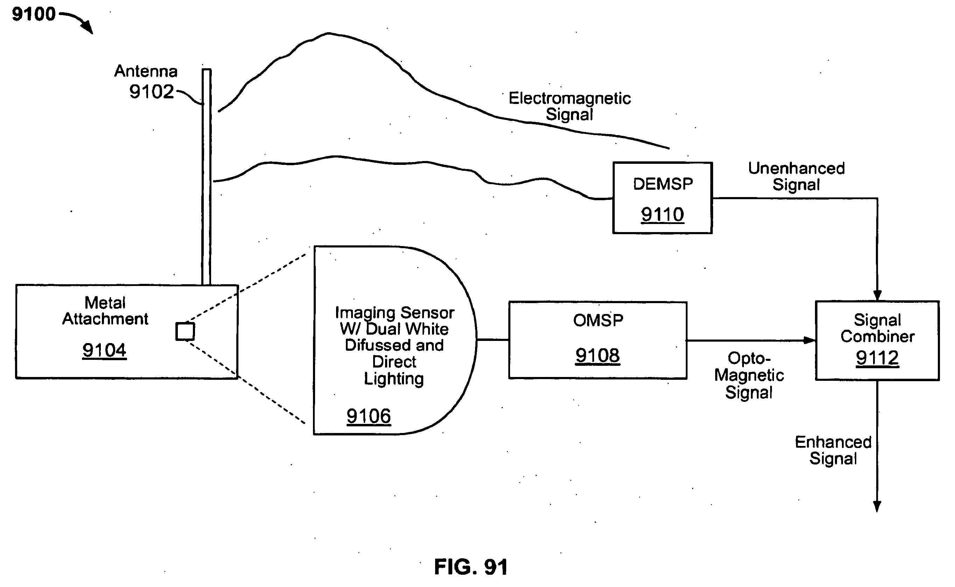

FIG. 91 depicts a system for generating enhanced heterogeneous signals for use in non-invasive processing of materials utilizing an Opto-Magnetic Antenna (or OMA), designed and implemented in accordance with certain embodiments of the invention.

The system 9100 is in essence an Opto-Magnetic Amplifier (or OMAMP.)

The OMAMP 9100 consists of the OMA 9102, a metal attachment 9104, an imaging sensor 9106, an Opto-Magnetic Signal Processor (or OMSP) 9108, a Direct Electro-Magnetic Signal Processor (or DEMSP) 9110 and a signal combiner (or mixer) 9112.

The OMAMP 9100, by virtue of its design and implementation, processes Electro-Magnetic (or EM) and photomagnetic (or photo-magnetic Optomagnetic or Opto-Magnetic) signals thereby facilitating detection, analysis, characterization, indication, identification, assessment and determination of the materials.

The OMAMP 9100 can be coupled to a metallic surface (not shown), for example as a regular antenna.

In certain embodiments, the OMA 9102 may be a transmitting antenna.

The OMA 9102 transmits EM signals. The OMA 9102 receives the EM signals and generates a response based on the received EM signals. It must be noted here that the output signal of the OMA 9102 can be boosted based on the response of the OMA 102.

The OMA 9102 is coupled to the metal attachment 9104 and the DEMSP 9110. This is shown in FIG. 91. Specifically, the OMA 9102 feeds the EM signals to an input of the DEMSP 9110.

The term "transmitting antenna or transmitter" refers to an electronic device which, usually with the aid of an antenna, propagates an EM signal, such as in radio, television, or other telecommunications applications. In other applications signals can also be transmitted using an analog 0/4 - 20 mA current loop signal.

The metal attachment 9104 is in essence a receiving antenna. The metal attachment 9104 receives EM signals.

The term "metal attachment or attachment", as used in the current context refers to a special hardware specific to an antenna model for attachment to an antenna mounting pipe or concealment structure. The antenna attachment is located at the base end of the antenna element. The antenna attachment has a capacitive reactance. In addition, the antenna attachment can cancel the inductive reactance of the antenna thereby causing the impedance of the antenna to approach a prescribed value.

As depicted in FIG. 91, the metal attachment 9104 is coupled to the OMA 9102.

The imaging sensor 9106 is in essence a device that converts an optical image to an electric signal. In certain embodiments, the imaging sensor 9106 captures continuous digital images of the metallic surface. Noticeable here is the fact that the OMAMP 9100 is attached to the metallic surface. Specifically, in such embodiments, the imaging sensor 9106 captures continuous digital images of the metallic surface illuminated with white light both, non-angled and angled. By way of, and by no way of limitation, the imaging sensor 106 may be anyone selected from a group consisting of a Complementary Metal-Oxide-Semiconductor (CMOS) image sensor, Charged Coupled Device (CCD) image sensor, and the like.

The imaging sensor 9106 is coupled to the metal attachment 9104, as depicted in FIG. 91. In addition, the imaging sensor 9106 is coupled to the OMSP 9108. Specifically, an output of the imaging sensor 9106 is coupled to an input of the OMSP 9108.

The term "digital image" refers to a representation of a two-dimensional image using ones and zeros (or binary digits or bits). The digital image may be of vector or raster type depending on whether or not the image resolution is fixed. However, without qualifications the term "digital image" usually refers to raster images.

For example, and in no way limiting the scope of the invention, in certain embodiments the imaging sensor 9106 may be selected on the basis of the following specifications: color is color or monochrome; optical format; horizontal pixels X vertical pixels; pixel size; one or more performance parameters, such as maximum frame rate, data rate, maximum power dissipation, quantum efficiency, dynamic range and supply voltage; output; one or more features, such as integrated Analog-to-Digital Converter (ADC) and microlenses; and environment, such as operating temperature.

The OMSP 9108 may be a customized digital signal processor.

As seen in FIG. 91, the OMSP 9108 has a single input and a single output.

The OMSP 9108 processes the continuously captured non-angled and angled white light digital images of the metallic surface.

In certain embodiments, the process of generating a spectral signature for materials and the system thereof (for implementing or facilitating implementation of) the process is disclosed, in accordance with the principles of the invention. In certain specific embodiments, the OMSP 9108 implements the process of generating the spectral signature for materials.

Specifically, the process comprises the stages of capturing an image of a material illuminated with incident non-angled and angled white light, generating a normalized red and blue color channel histogram for each image, correlating the normalized red and blue color channel histograms to a wavelength scale to obtain red and blue color channel spectral plots, and convoluting the spectral plots by subtracting the spectral plot for angled light from the spectral plot for non-angled light for each color channel to generate red and blue normalized, composite color channel spectral plots, and subtracting the normalized, composite blue channel spectral plot from the normalized, composite red channel spectral plot to generate a spectral signature for the material. By way of example, and in no way limiting the scope of the invention, the OMSP 108 implements a process for generating the spectral signature for materials as disclosed in United States Provisional Patent Application "METHOD AND ALGORITHM FOR ANALYSIS OF LIGHT-MATTER INTERACTION BASED ON SPECTRAL CONVOLUTION" to MYSKIN, INC. Thus, all remaining ins-and-outs in connection with the process of generating the spectral signature will not be further detailed herein.

As seen in FIG. 91, the input of the OMSP 9108 is coupled to the output of the imaging sensor 9106. Thus, the input of the OMSP 9108 is fed with the continuously captured non-angled and angled white light digital images of the material.

Further, the output of the OMSP 9108 generates Opto-Magnetic signals.

The output of the OMSP 9108 is coupled to the signal combiner 9112.

The term "digital image processing", as used herein, refers to the use of computer algorithms to perform image processing on digital images. As a subfield of digital signal processing, digital image processing has many advantages over analog image processing. For example, digital image processing allows a much wider range of algorithms to be applied to the input data and can avoid problems, such as the build-up of noise and signal distortion during processing.

The term "spectral signatures" as used herein refers to specific combination of reflected and absorbed electromagnetic radiation at varying wavelengths that can uniquely identify an object. The spectral signature of an object is a function of incidental Electro-Magnetic (EM) wavelength and material interaction with that section of the electromagnetic spectrum. The measurements can be made with various instruments, including but not limited to, a task specific spectrometer. For instance, the most common method is separation of the Red (R), Green (G), Blue (B) and Near Infrared (NIR) portion of the EM spectrum as acquired by digital cameras. In certain airborne or satellite imagery applications, calibrations of spectral signatures under specific illumination are collected in order to apply an empirical correction to airborne or satellite imagery digital images.

In general, all of the antenna parameters are expressed in terms of a transmission antenna, but are identically applicable to a receiving antenna, due to reciprocity. However, impedance is not applied in an obvious way. The impedance at the load, where the power is consumed, is most critical. For a transmitting antenna, this is the antenna. On the other hand, for a receiving antenna this is at the radio receiver rather than at the antenna. Tuning is done by adjusting the length of an electrically long linear antenna to alter the electrical resonance of the antenna.

Antenna tuning is done by adjusting an inductance or capacitance combined with the active antenna (but distinct and separate from the active antenna). The inductance or capacitance provides the reactance which combines with the inherent reactance of the active antenna to establish a resonance in a circuit including the active antenna. The established resonance being at a frequency other than the natural electrical resonant frequency of the active antenna. Adjustment of the inductance or capacitance changes this resonance.

Antennas used for transmission have a maximum power rating, beyond which heating, arcing or sparking may occur in the components, which may cause them to be damaged or destroyed. Raising this maximum power rating usually requires larger and heavier components, which may require larger and heavier supporting structures. This is a concern only for transmitting antennas, as the power received by an antenna rarely exceeds the microwatt range.

Antennas designed specifically for reception might be optimized for noise rejection capabilities. An antenna shield is a conductive or low reluctance structure (such as a wire, plate or grid) which is adapted to be placed in the vicinity of an antenna to reduce, as by dissipation through a resistance or by conduction to ground, undesired electromagnetic radiation, or electric or magnetic fields, which are directed toward the active antenna from an external source or which emanate from the active antenna. Other methods to optimize for noise rejection can be done by selecting a narrow bandwidth so that noise from other frequencies is rejected, or selecting a specific radiation pattern to reject noise from a specific direction, or by selecting a polarization different from the noise polarization, or by selecting an antenna that favors either the electric or magnetic field.

For instance, an antenna to be used for reception of low frequencies (below about ten megahertz) will be subject to both man-made noise from motors and other machinery, and from natural sources such as lightning. Successfully rejecting these forms of noise is an important antenna feature. A small coil of wire with many turns is more able to reject such noise than a vertical antenna. However, the vertical will radiate much more effectively on transmit, where extraneous signals are not a concern.

The term "tuning" refers to adjusting a device to a desired frequency.

In general, there are two basic types of mixer, namely additive mixers and multiplying mixers. Additive mixers add two or more input (or source) signals thereby outputting a composite signal that contains the frequency components of each of the input signals. For example, the simplest additive mixers are simple resistor networks, and thus purely passive, whereas more complex mixers employ active components such as, buffer amplifiers for impedance matching and better isolation.

On the other hand, the multiplying mixers (or product) multiply two or more input (or source) signals together thereby producing an output containing both the input signals and new signals that comprise the sum and difference of the frequency of the input signals. For example, ideal product mixers act as signal multipliers thereby producing an output signal equal to the product of the input signals. In certain communications-based applications, the product mixers are often used in conjugation with an oscillator to modulate signal frequencies. For instance, the product mixers can either up-convert or down-convert an input signal frequency, but it is more common to down-convert to a lower frequency to allow for easier filter design. In many typical circuits, the single output signal actually contains multiple waveforms, namely those at the sum and difference of the two input frequencies and harmonic waveforms. The ideal signal may be obtained by removing the other signal components with a filter.

As shown in FIG. 91, the DEMSP 9110 has a single input and a single output. For example, and by no way of limitation, in certain embodiments the DEMSP 9110 may be a customized Analog Signal Processor (ASP). Thus, in such embodiments, the DEMSP 9110 may employ analog signal processing to process the EM signals:

The term "analog signal processing" refers to any signal processing conducted on analog signals by analog means. For example, analog signal processing include crossover filters in loudspeakers, "bass", "treble" and "volume" controls on stereos, and "tint" controls on TVs. Common analog processing elements include capacitors, resistors, inductors and transistors.

The input of the DEMSP 9110 is fed with the EM signals. The input of the DEMSP 9110 is coupled to the OMA 9102.

The output of the DEMSP 9110 outputs unenhanced signals. The output of the DEMSP 9110 is coupled to the signal combiner 9112.

In general, the signal combiner 9112 combines (or mixes) two or more signals into one composite output signal.

As shown in FIG. 91, the signal combiner 9112 consists of a pair of inputs and a single output.

The first input of the pair of inputs of the signal combiner 9112 is coupled to the DEMSP 9110. The first input of the pair of inputs of the signal combiner 9112 is fed with the unenhanced signal.

The second input of the pair of inputs of the signal combiner 9112 is coupled to the OMSP 9108. The second input of the pair of inputs of the signal combiner 9112 is fed with the Opto-magnetic signal.

In operation, the signal combiner 9112 combines (or mixes) the unenhanced signal from the DEMSP 9110 and the Opto-magnetic signal from the OMSP 9108 thereby producing the enhanced signal.

In operation, the OMAMP 9100 is coupled to a test material surface. The imaging sensors 9106 capture continuous digital images of the material illuminated with non-angled and angled white light. The output of the imaging sensors 9106 is fed as input to the OMSP 9108. The OMSP 9108 processes the continuously captured digital images of the material to generate a spectral signature of the material, in accordance with the principles of the invention disclosed earlier. The antenna 9102 transmits EM signals to the DEMSP 9110. The DEMSP 9110 processes the EM signals and outputs an unenhanced EM signal. The output of the OMSP 9108 (i.e. the Opto-Magnetic signal) and the output of DEMSP 9110 (i.e. the unenhanced EM signal) are fed as inputs to the signal combiner 9112. The signal combiner 9112 combines (or mixes) the Opto-Magnetic signal and unenhanced EM signal to generate an enhanced mixed signal.

In certain embodiments, the wavelengths and algorithm varies by the frequency of the target antenna. Multiple detectors may be placed on the same metal surface in order to take images in parallel in order to increase processing speed based on wavelength, etc. Tuning to different frequencies is done by analyzing the resulting spectrum as well as adjusting the speed of the images taken.

In certain embodiments, design and implementation of one or more workable configurations for the system of FIG. 91 for facilitating high frequency imaging and processes thereof have been disclosed. Specifically, such configurations can use multiple sensors that allow rapid lighting sequences for rapid imaging thereby resulting in high frequency imaging of materials.

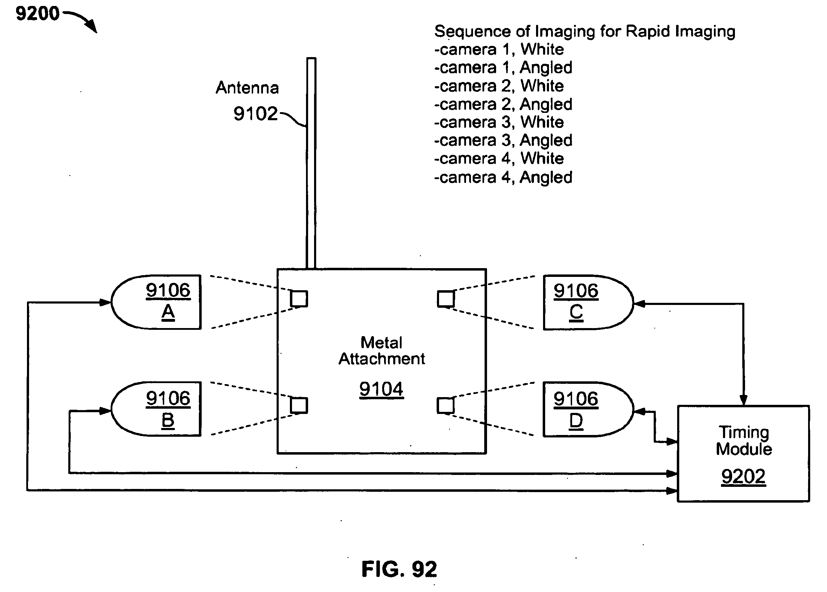

FIG. 92 is block diagrammatic view of at least one workable configuration for use in tandem with the system of FIG. 91.

The configuration 9200 comprises the OMA 9102, metal attachment 9104, at least two pairs of the imaging sensors 9106 and a timing module 9202.

The configuration 9200 may be coupled to surface of materials. For example, and by no way of limitation, materials may be anyone selected from a group of both inorganic and organic materials consisting of skin, collagen, elastin, metal and the like.

The two pairs of imaging sensors 9106 consists of a first imaging sensor 9106A, second imaging sensor 9106B, third imaging sensor 9106C and fourth imaging sensor 9106D.

Reiterating again, each individual sensor 9106 of the two pairs of imaging sensors 9106 captures continuous digital images of materials illuminated with the unangled and angled white light.

Timing module (or Timer) 9202 is a specialized type of clock. The timer 9202 can be used to control the sequence of an event or process.

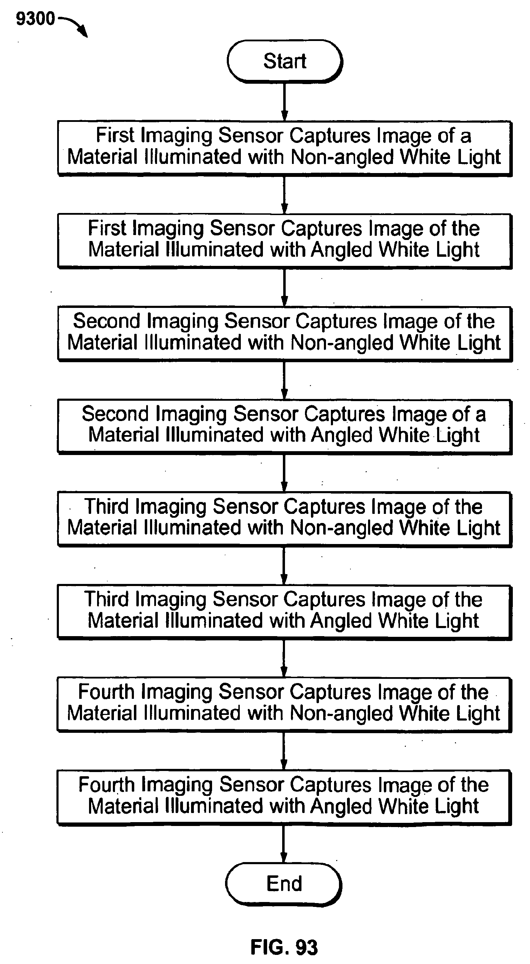

In operation, the configuration 9200 implements a process facilitating high frequency imaging of materials by employment of multiple sensors. Specifically, the process implements a sequence of process stages of imaging for rapid imaging using the multiple sensors. It must be noted here that the use of the multiple sensors allow rapid lighting sequences thereby resulting in high frequency imaging of materials. This sequence has been explained in conjunction with the process of FIG. 93 and TABLE 1.

As seen in FIG. 91, the timing module 9202 is separately coupled to each individual sensor 9106 of the two pairs of the imaging sensors 9106.

In certain other embodiments, the system configuration, discussed in conjunction with FIG. 92, implement one or more processes facilitating high frequency imaging by employment of multiple sensors. Specifically, the processes comprise one or more sequences of process stages of imaging for rapid imaging using the multiple sensors. It must be noted here that the use of the multiple sensors allow rapid lighting sequences thereby resulting in high frequency imaging of materials.

FIG. 93 depicts a flow diagram delineating at least one process implemented by the system configuration of FIG. 92 thereby facilitating multi sensor high frequency imaging.

The process 9300 starts at stage 9301 and proceeds to stage 9302, where the process 9300 comprises the phase of capturing images of a material illuminated with a white light (or unangled white light.) Noticeable here is the fact that the process 9300 initiates the first imaging sensor for capturing images of the material illuminated with the white light.

At stage 9304, the process 9300 comprises the phase of capturing images of the material illuminated with an angled white light. In here, it is worth notable that the process 9300 initiates the first imaging sensor for capturing images of the material illuminated with the angled white light.

At stage 9306, the process 9300 comprises the phase of capturing images of the material illuminated with the white light. It must be noted here that the process 9300 initiates the second imaging sensor for capturing images of the material illuminated with the white light.

At stage 9308, the process 9300 comprises the phase of capturing images of the material illuminated with the angled white light using the second imaging sensor.

At stage 9310, the process 9300 comprises the phase of capturing images of the material illuminated with the white light using the third imaging sensor.

At stage 9312, the process 9300 comprises the phase of capturing images of the material illuminated with the angled white light using the third imaging sensor.

At stage 9314, the process 9300 comprises the phase of capturing images of the material illuminated with the white light using the fourth imaging sensor.

At stage 9316, the process 9300 comprises the phase of capturing images of the material illuminated with the angled white light using the fourth imaging sensor.

The process 9300 ends at the stage 9318. It is worth notable that the timer 9202 can be used to control the sequence of the process 9300.

Table 6 below provides at least one sequence of imaging for rapid imaging.

| SEQUENCE EVENT # | IMAGING SENSOR OR CAMERA # | TYPE OF WHITE LIGHT (POLARIZED / NON-POLARIZED) |
|---|---|---|
| 1. | FIRST IMAGING SENSOR (OR CAMERA 1) 9106 A | WHITE (NON-ANGLED WHITE) |
| 2. | FIRST IMAGING SENSOR (CAMERA 1) 9106A | ANGLED (OR ANGLED WHITE) |
| 3. | SECOND IMAGING SENSOR (OR CAMERA 2) 9106B | WHITE (NON-ANGLED WHITE) |
| 4. | SECOND IMAGING SENSOR (OR CAMERA 2) 9106B | ANGLED (OR ANGLED WHITE) |
| 5. | THIRD IMAGING SENSOR (OR CAMERA 3) 9106C | WHITE (NON-ANGLED WHITE) |

(continued)

| SEQUENCE EVENT # | IMAGING SENSOR OR CAMERA # | TYPE OF WHITE LIGHT (POLARIZED / NON-POLARIZED) |
|---|---|---|
| 6. | THIRD IMAGING SENSOR (OR CAMERA 3) 9106C | ANGLED (OR ANGLED WHITE) |
| 7. | FOURTH IMAGING SENSOR (OR CAMERA 4) 9106D | WHITE (NON-ANGLED WHITE) |
| 8. | FOURTH IMAGING SENSOR (OR CAMERA 4) 9106D | ANGLED (OR ANGLED WHITE) |

Advantageously, in certain embodiments, the invention may find application in highly accurate Digital Video Disc (or DVD) readings. Still advantageously, the invention may find application in material optical characterization. For example, the invention may be used in material identification, lot-based assessment of materials, and the like.

In certain embodiments, a system for managing physiological state, based on one or more physiological parameters, with improved qualitative and quantitative parameters and methods thereof are disclosed.

In the description of this invention, the terms "system," "device" and "Wearable Hydration Monitor (or WHM)" are used interchangeably, unless otherwise prescribed. For example, in some embodiments, the terms "system," "device" and "Wearable Hydration Monitor (or WHM)" are used interchangeably to refer to a wearable computing system, which has been designed and implemented herein for managing (i.e. monitoring) hydration level of skin. Whereas, in some other embodiments, the terms "sensor subsystem" and "sensor" are used interchangeably to refer to a device for capturing the polarized and unpolarized electromagnetic signals reflected from the physiological organs. In yet other some embodiments, the terms "physiological parameter management module," "skin hydration management module" and "hydration management module" are used interchangeably to refer to a software module which has been designed and implemented for overall management of hydration level of skin.

Typically, there are many factors that can impact on the hydration status of sports people, such as social activities, diet, climate and activity level. It is very important for sports people to be well hydrated. As far as health is concerned, dehydrated athletes competing in a hot climate are at greater risk of heat injury. In addition, as far as performance is concerned, research has shown that a dehydration percentage of 2% of body weight or greater can have a significant effect on performance.

Conventionally, there are many methods for determining hydration status including, but not limited to, monitoring body mass changes, measuring sweat, various blood markers and analysis of urine. For example, USG measurement using refractometers, urine color, sweat analysis, sweat rate, and the like.

In certain embodiments, the skin care devices and systems may be adapted for managing physiological state based on one or more physiological parameters. Specifically, such skin care devices and systems can be worn by a user in one or more forms, such as necklace, earrings, bracelets, a patch, or as a sensor attached to a strap, and the like. For example, and by no way of limitation, such wearable devices and systems can be persistent, personalized skin care monitors.

In certain specific embodiments, the wearable skin care devices and systems may be a Wearable Hydration Monitor (or WHM). Similar to the skin care device, the WHM may comprise an electromagnetic radiation source, a radiation detector, and a skin condition analysis module. In such embodiments of the wearable skincare device and systems, the electromagnetic radiation source may be one or more LEDs. Each of the LEDs may have unique predetermined frequencies. In other such embodiments, the one or more LEDs may be arranged in a line to form a light strip.

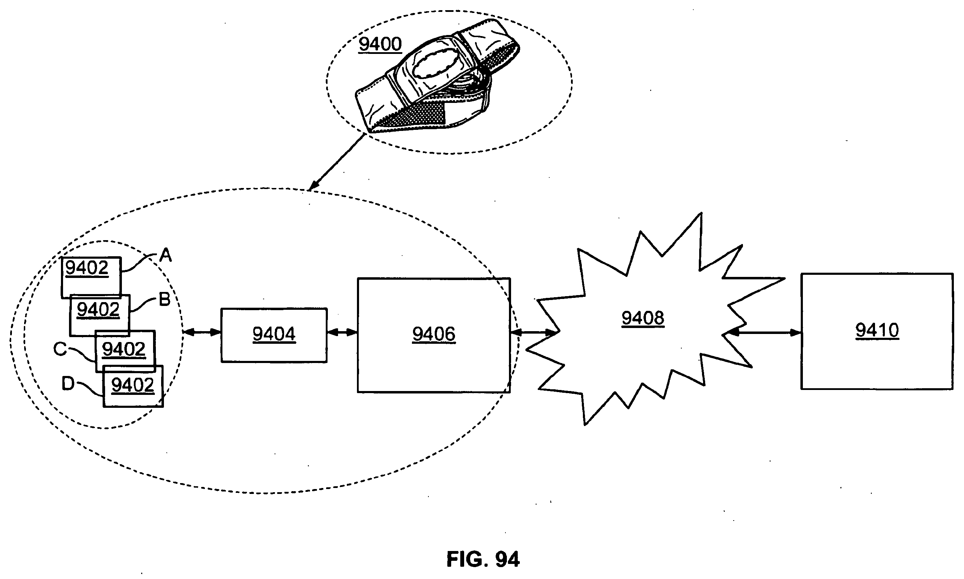

FIG. 94 is a schematic view of a wearable computing system for monitoring of one or more physiological parameters designed and implemented in accordance with at least some embodiments of the invention.

The system 9400 may in essence be a Wearable Hydration Monitor (or WHM.) The WHM 9400 may consist of one or more Light Emitting Diodes (LEDs) 9402, a sensor subsystem 9404, a host computing subsystem 9406, an optional network 9408 and a remote computing subsystem 9410. By way of example and by no way of limitation the WHM 9400 may be a polar arm or chest band. This is shown in FIG. 94.

As depicted in a partially disassembled view of FIG. 94, in certain specific embodiments, the one or more Light Emitting Diodes (LEDs) 9402 consists of a first LED 9402A, a second LED 9402B, a third LED 9402C, a fourth LED 9402D respectively.

In some embodiments, the WHM 9400 may be powered via a USB coupled to an external power source or through built-in batteries, motion power, solar power, or other similar power source. All these have not been shown explicitly in FIG. 94.

In certain embodiments, the WHM 9400 for managing one or more physiological parameters and processes thereof has been disclosed, in accordance with the principles of the invention. Specifically, in such embodiments, the WHM 9400 comprises one or more illumination sources. The illumination sources comprise incident light sources to direct light upon skin. In consequence, the incident light sources may be unpolarized or polarized light sources. For example, and by no way of limitation, the unpolarized light may be white light, multiple selected wavelengths, or a single wavelength. Further, the illumination source may be positioned to direct light at a selected angle alpha. By way of example, and in no way limiting the scope of the invention, the WHM 9400 implements the processes for non-invasive processing including, but not limited to, imaging, analysis, of materials, as disclosed in United States Provisional Patent Applications "METHOD AND ALGORITHM FOR ANALYSIS OF LIGHT-MATTER INTERACTION BASED ON SPECTRAL CONVOLUTION" and "IMAGING DEVICE UTILIZING WHITE LIGHT FOR COMPSOITION ANALYSIS" and United States Non-Provisional Patent Applications "SYSTEM, DEVICE, AND METHOD FOR DERMAL IMAGING" to MYSKIN, INC.. Thus, all remaining ins-and-outs in connection with the process of non-invasive processing of materials will not be further detailed herein.

Embodiments of the WHM 9400 may also have one or more sensors for measuring various body and environmental parameters. Examples of body parameters that could be measured by the wearable skincare device are hydration level, skin turgor, body temperature, hemoglobin antioxidant level, etc. Examples of environmental parameters that could be measured by the WHM 9400 are air cleanliness, humidity, temperature, UV index, external air quality, smoke index, etc.

As shown in FIG. 94, the sensor subsystem 9404 may in essence be a device that converts optical images (or optical signals) to electric signals. In certain embodiments, the sensor subsystem 9404 captures continuous digital images of skin. Specifically, in such embodiments, the sensor subsystem 9404 captures continuous digital images of the metallic surface illuminated with white light both, non-angled and angled. By way of, and by no way of limitation, the sensor subsystem 9404 may be anyone selected from a group consisting of a Complementary Metal-Oxide-Semiconductor (CMOS) image sensor, Charged Coupled Device (CCD) image sensor, and the like.

Again, as shown in FIG. 94, the sensor subsystem 9404 may be coupled to the host computing subsystem 9406 and the first, second, third and fourth LEDs 9402A, 9402B, 9402C and 9402D, respectively.

The term "digital image" refers to a representation of a two-dimensional image using ones and zeros (or binary digits or bits). The digital image may be of vector or raster type depending on whether or not the image resolution is fixed. However, without qualifications the term "digital image" usually refers to raster images.

Likewise, the term "digital imaging or digital image acquisition" refers to creation of digital images, typically from a physical object. The term is often assumed to imply or include the processing, compression, storage, printing and display of such images.

Digital image processing is the use of computer algorithms to perform image processing on digital images. As a subfield of digital signal processing, digital image processing has many advantages over analog image processing; it allows a much wider range of algorithms to be applied to the input data, and can avoid problems such as the build-up of noise and signal distortion during processing.

For example, and in no way limiting the scope of the invention, in certain embodiments the sensor subsystem 9404 may be selected on the basis of the following specifications: color is color or monochrome; optical format; horizontal pixels X vertical pixels; pixel size; one or more performance parameters, such as maximum frame rate, data rate, maximum power dissipation, quantum efficiency, dynamic range and supply voltage; output; one or more features, such as integrated Analog-to-Digital Converter (ADC) and microlenses; and environment, such as operating temperature.

In certain embodiments, the host computing subsystem 9406 may comprise a skin hydration management module designed and implemented, in accordance with the principles of the invention.

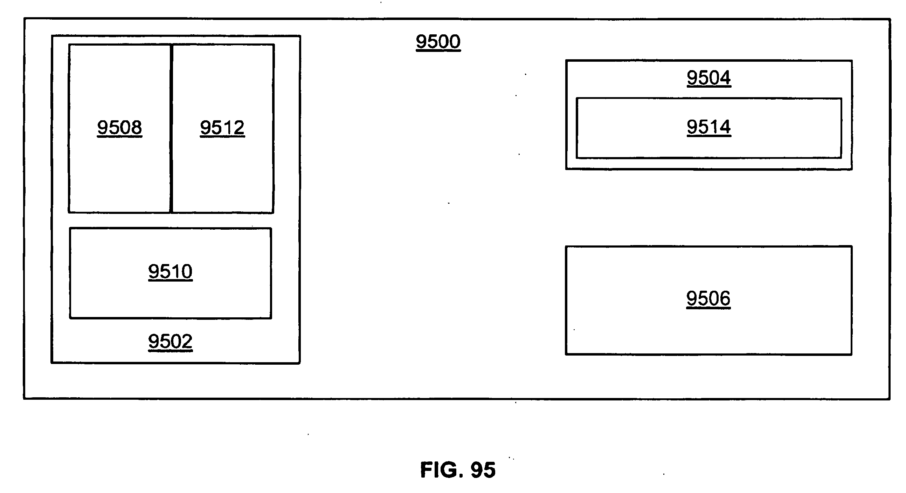

FIG. 95 is an exploded diagrammatic representation of the host computing subsystem, of Fig. 1, comprising the skin hydration management module designed and implemented in accordance with at least some embodiments.

The host computing subsystem 9500 may comprise a processing unit 9502, a memory unit 9504 and an Input / Output (or I/O) unit 9506 respectively.

The host computing subsystem 9500, by virtue of its design and implementation, performs overall management of the hydration level of skin.

The processing unit 9502 may comprise an Arithmetic Logic Unit (or ALU) 9508, a Control Unit (or CU) 9510 and a Register Unit (or RU) 9512.

The memory unit 9504 comprises a skin hydration management module 9514.

In certain embodiments, the skin hydration management module for real- or point-time analysis of the continuously captured digital skin information and methods thereof is disclosed, in accordance with the principles of the invention. Specifically, in such embodiments, the skin hydration management module captures the skin information using at least one of Diffused Reflectance Spectroscopy, Red (R)-Green (G)-Blue (B) analysis of re-emitted white light and any combination thereof.

The terms "Diffused (or Diffuse) Reflectance Spectroscopy (or DRS)" and "Diffuse Reflectance Infrared Fourier Transform Spectroscopy (DRIFTS)" refer to a technique that collects and analyzes scattered Infrared (or IR) energy. It is used for measurement of fine particles, powders as well as rough surface. Specifically, it assesses the interaction of a surfactant with the inner particle or the adsorption of molecules on the particle surface. In DRS or DRIFTS, sampling is fast and easy because little or no sample preparation is required.

In certain other embodiments, the skin hydration management module may comprise one or more processes for determination of an assortment of qualitative and quantitative parameters thereby facilitating overall management of hydration level of skin. In such embodiments, at least a first process of the one or more processes determines moisture levels of skin. Specifically, this process may comprise one or more phases comprising emission of incident electromagnetic signals to skin, detection of degree of polarization of the electromagnetic signals reflected or re-emitted from skin and determination of the moisture levels based on the amount of polarized and reflected or re-emitted electromagnetic signals. Yet, in such embodiments, the first process may comprise one or more phases comprising combination of the determined moisture levels with skin color measurements thereby resulting in determination of skin luminosity.

Still, in certain such embodiments, at least a second process of the processes determines elasticity of skin. Specifically, this process may comprise one or more phases comprising the emission of the incident electromagnetic signals to skin, detection of a first aspect of polarization of the electromagnetic signals reflected by skin, correlation of the aspect of polarization with a concentration of elastin and determination of elasticity level based on the concentration of elastin.

Still further, in certain such embodiments, at least a third process of the processes determines firmness of skin. Specifically, this process may comprise or more phases comprising the of the incident electromagnetic signals to skin, the detection of a second aspect of polarization of the electromagnetic signals reflected by skin, the correlation of the aspect of polarization with the concentration of at least one of the elastin, a collagen, an activity of a sebaceous gland and any combination thereof and determination of the firmness based on the concentration of at least one of the elastin, collagen and sebaceous gland activity. In such embodiments, the sebaceous gland activity may be indicated by at least one of a number of glands, percent of glands open / closed and level of clog / fill.

Yet, in certain such embodiments, at least a fourth process of the processes obtains biophysical properties may comprise performing a spectral analysis of image data acquired from the degree of polarization of reflections and absorption and re-emission of incident light from skin. Specifically, the biophysical properties is at least one of a structure, form, concentration, number, size, state, and stage of at least one of a: melanocyte, melanin, hemoglobin, porphyrin, keratin, carotene, collagen, elastin, sebum, sebaceous gland activity, pore (sweat and sebaceous), moisture level, elasticity, luminosity, firmness, fine line, wrinkle count and stage, pore size, percent of open pores, skin elasticity, skin tension line, spot, skin color, psoriasis, allergy, red area, general skin disorder or infection, tumor, sunburn, rash, scratch, pimple, acne, insect bite, itch, bleeding, injury, inflammation, photodamage, pigmentation, tone, tattoo, percent burn/burn classification, mole (naevi, nevus), aspect of a skin lesion (structure, color, dimensions/asymmetry), melanoma, dermally observed disorder, cutaneous lesion, cellulite, boil, blistering disease, congenital dermal syndrome, (sub)-cutaneous mycoses, melasma, vascular condition, rosacea, spider vein, texture, skin ulcer, wound healing, post-operative tracking, melanocytic lesion, non-melanocytic lesion, basal cell carcinoma, seborrhoic keratosis, sebum (oiliness), nail- and/or hair-related concern, and the like.

In certain embodiments, the WHM 9400 may include the one or more LEDs 9402 capable of directing incident electromagnetic radiation to a location on the skin of a user, the sensor subsystem 9404 for measuring various parameters of radiation re-emitted from the location, and the skin hydration management module 9514, as disclosed in FIG. 95, capable of managing skin hydration level in real- or point-time, based partly on at least one of RGB analysis and diffused reflectance analysis of the radiation parameters. It must be noted here that the aforementioned embodiments have been explained in conjunction with FIGS. 94 and 95.

Typically, imaging spectroscopy (or spectral imaging or chemical imaging) is similar to color photography. But, unlike color photography, in imaging spectroscopy each pixel acquires many bands of light intensity data from the spectrum, instead of just the three bands of the RGB color model. More precisely, it is the simultaneous acquisition of spatially coregistered images in many spectrally contiguous bands.

Further, hyperspectral data is often used to determine materials present in images. For example, materials of interest could include roadways, vegetation, and specific targets (i.e. pollutants, hazardous materials, etc.) Trivially, each pixel of a hyperpsectral image could be compared to a material database to determine the type of material making up the pixel. However, many hyperspectral imaging platforms have low resolution (i.e. > 5 m per pixel) thereby causing each pixel to be a mixture of several materials. The process of unmixing one of these 'mixed' pixels is called hyperspectral image unmixing or simply hyperspectral unmixing.

In general, there are many algorithms to unmix hyperspecectral data each with their own strengths and weaknesses. Many such algorithms assume that pure pixels (i.e. pixels that contain only one material) are present in images. For example, some algorithms to perform unmixing are Pixel Purity Index (or PPI), N-Finder Algorithm (or NFINDR), Gift Wrapping Algorithm, Independent Component Analysis Endmember Extraction Algorithm (or ICA-EEA), Vertex Component Analysis (or VCA), Principal component analysis (or PCA), Multi Endmembers Spatial Mixture Analysis (or MESMA), Support Vector Machines (or SVM) or Analytical Neural Network (or ANN), and the like.

In certain embodiments, the WHM 9400 employs white light (or other specific wavelengths) for measuring the concentration of specific ions in the blood stream and the skin layers. By way of example, and in no way limiting the scope of the invention, the specific ions may be at least one of sodium ($[Na+]$), potassium ($[K+]$), and chloride ($[Cl-]$). It must be noted here that the presence of these salts / ions and levels thereof tracked in due course indicates normal level of user vis-à-vis specific metabolism and body of the user.

The term "skin turgor" as used herein refers to an abnormality in the skin's ability to change shape and return to normal (i.e. elasticity.) Skin turgor is a sign commonly used by health care workers to assess the degree of fluid loss or dehydration. Fluid loss can occur from common conditions, such as diarrhea or vomiting. In certain situations, infants and young children with vomiting, diarrhea and decreased or no fluid.intake can rapidly lose a significant amount of fluid. Fever speeds up this process. To determine skin turgor, the health care provider grasps the skin on the back of the hand, lower arm, or abdomen between two fingers so that it is tented up. The skin is held for a few seconds then released. Skin with normal turgor snaps rapidly back to its normal position. Skin with decreased turgor remains elevated and returns slowly to its normal position.

In certain such embodiments, the WHM 9400 measures skin turgor as a secondary measurement tool to create a combined hydration impact score. By way of example, and in no way limiting the scope of the invention, the WHM 100 may implement methods and systems for management of skin hydration as disclosed in an article "SENSITIVITY AND SPECIFICITY OF CLINICAL SIGNS FOR ASSESSMENT OF DEHYDRATION IN ENDURANCE ATHLETES" to James McGarvey et al. and published online in Br J Sports Med. on 3rd November 2008. Thus, all other ins-and-outs in connection with the aforementioned embodiment have not been further disclosed herein.

In certain embodiments, the WHM 9400 of FIG. 94 may be capable of transmitting to and / or receiving from the remote computing subsystem 9410 pluralities of information including the skin hydration assessment information through the network 9408. Specifically, the skin hydration management module, residing in the memory of the host computing subsystem, generates the skin hydration assessment information that is transmitted to the remote computing subsystem 9410 through the network 9408.

In certain specific embodiments, the remote computing subsystem 9410 may in essence be similar to the host computing subsystem 9406. Specifically, the remote computing subsystem 9410 may comprise a processing unit, a memory unit and an Input / Output (or I/O) unit (all not shown explicitly) respectively. By way of example, and in no way limiting the scope of the invention, the remote computing subsystem 9410 may be a wristwatch or a Bluetooth™-enabled or -capable device.

The remote computing subsystem 9410 may be coupled to the WHM 9400. Specifically, the remote computing subsystem 9410 may be coupled to the I / O unit of the host computing subsystem of the WHM 9400, through the network 9408.

The remote computing subsystem 9410, by virtue of its design and implementation, may perform at least one of the following operations: processing the received (or unprocessed) skin hydration assessment information, displaying the processed and / or received skin hydration assessment information and performing any combination thereof.

The processing unit may comprise an Arithmetic Logic Unit (or ALU), a Control Unit (or CU) and a Register Unit (or RU).

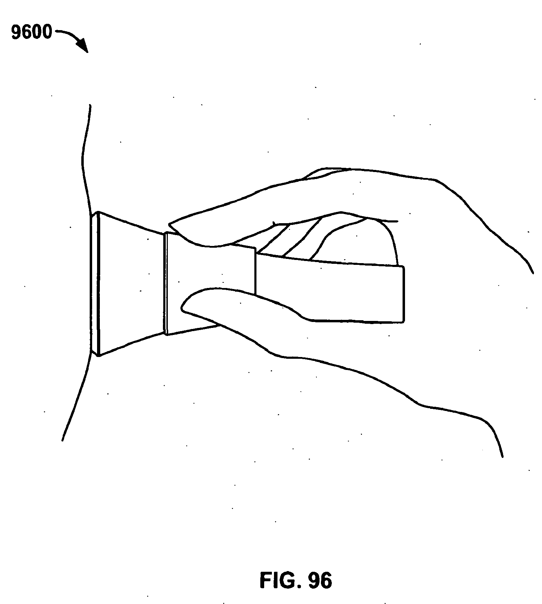

FIG. 96 is a perspective view of the WHM of FIG. 94 designed and implemented as a handheld hydration monitor, in accordance with some other embodiments of the invention

As shown in FIG. 96, the WHM 9400 may be a simple handheld device that checks for hydration status. In such specific embodiments, the WHM 9400 could be used in places, such as saunas, spas, desert environments, and the like.

Electrical Impedance Tomography (or EIT) is a medical imaging technique in which an image of the conductivity or permittivity of part of the body is inferred from surface electrical measurements. Typically, conducting electrodes are attached to the skin of the subject and small alternating currents are applied to some or all of the electrodes. The resulting electrical potentials are measured, and the process may be repeated for numerous different configurations of applied current.

In general, the electrical conductivity and permittivity in biological tissues varies between tissue types and depending on temperature and physiological factors. For example, lungs become less conductive as the alveoli become filled with air. In EIT, adhesive electrodes are applied to the skin and an electric current, typically a few milli-Amperes (or mA) of Alternating Current (or AC) at a frequency of 10-100 kHz, is applied across two or more electrodes. Other electrodes are used to measure the resulting voltage. This is repeated for numerous "stimulation patterns", such as successive pairs of adjacent electrodes.

Operationally, the currents used are relatively small and certainly below the threshold at which they would cause stimulation of nerves. The frequency of the AC is sufficiently high not to give rise electrolytic effects in the body. In addition, the Ohmic power dissipated is sufficiently small and diffused over the body to be easily handled by the body's thermoregulatory system. Specifically, the current is applied using current sources, either a single current source switched between electrodes using a multiplexor or a system of Voltage-to-Current converters, one for each electrode, each controlled by a Digital-to-Analog Converter (or DAC). The measurements again may be taken either by a single voltage measurement circuit multiplexed over the electrodes or a separate circuit for each electrode. Earlier systems typically used an analog demodulation circuit to convert the alternating voltage to a direct current level then an analog to digital converter. Many recent systems convert the alternating signal directly, the demodulation then being performed digitally. Many EIT systems are capable of working at several frequencies and can measure both the magnitude and phase of the voltage.

The voltages measured are then passed to a computer to perform the reconstruction and display of the image. If images are required in real time a typical approach is the application of some form of regularized inverse of a linearization of the forward problem. In most practical systems used in a medical setting a 'difference image' is formed. That is, the differences in voltage between two time points are left-multiplied by the regularized inverse to produce an approximate difference between the permittivity and conductivity images. Another approach is to construct a finite element model of the body and adjust the conductivities (for example using a variant of Levenburg-Marquart method) to fit the measured data. This is more challenging as it requires an accurate body shape and the exact position of the electrodes.

In certain specific embodiments, the WHM 9400 may employ electrical impedance techniques for imaging skin, in accordance with the principles of the invention.

In certain embodiments, the WHM 9400 may operate in one or more distinct modes thereby performing at least one of State-Independent and State-Dependent Hydration Management of organ systems.

In certain such embodiments, the WHM 9400 may be implemented as an Organ System State-Independent WHM. By way of example and in now way limiting the scope of the invention, in a first mode of operation the WHM 9400 may be applied to the epidermal layer. In such embodiments, the WHM 9400 may measure the amount of intracellular water / hydration level in the skin.

In yet certain other embodiments, the WHM 9400 may be implemented as an Organ System State-Dependent WHM. By way of example and in now way limiting the scope of the invention, in a second mode of operation, the WHM 9400 may be implemented as a dynamic hydration level indicator. In the second mode of operation, the WHM 9400 may measure the sweat from sweat pores and ions thereof, such as Potassium (or K), Sodium (or Na), and the like, to measure the current activity level and hydration, where user is in a state of motion (or inertia of motion).

Likewise, in a third mode of operation, the WHM 9400 may be implemented as a static hydration level indicator. In the third mode of operation, the WHM 9400 may measure the hydration level in the epidermal and dermal layers and the blood stream when user is in a state of rest (or inertia of rest).

In general, hydrogen bonds have dual properties, namely classical, i.e. electrostatic interaction based on Coulomb's law, and quantum, i.e. wave function based on Schrödinger equation. In certain embodiments, there are disclosed methods, apparatuses and systems for analysis of water using OMF. In certain such embodiments, owing to the fact that Planck's constant is one of the main criteria for decisions in connection with processes and quantum properties thereof use is made of electrical and magnetic forces of valence electrons as a point of departure to develop the method for Opto-Magnetic Fingerprinting of matter. It must be noted here that during the study of different types of matter, observation of a phenomena is obtained from spectral convolution data of digital images. These digital images characterize matter from both covalent and non-covalent bonding. By way of example, and in no way limiting the scope of the invention, water is matter that is most abundant with hydrogen bonds. In certain such situations, the results of 18.2 M$\Omega$ water investigations at different temperatures and under the influence of constant and variable magnetic fields by OMM are disclosed.

In certain specific embodiments, based on the data obtained neutron diffraction experiments it is observable that the product of distance between center of hydrogen and oxygen atoms in a covalent bond, i.e. d (O-H), of different structures is between 95 pm and 120 pm, while distance of center of hydrogen and oxygen atoms in non-covalent bond d (O$\cdots$H) is between 120 pm and 200 pm. However, for each type of matter product value d (O-H) $\times$ d (O$\cdots$H) is about 162 pm. Still further, systematic investigation and quantitative analysis of bond lengths of O-H$\cdots$O showed that bond-valence parameters of hydrogen bonds follow Golden ratio rule, whose value is around 1.62.

As a general rule, taking into consideration the fact that water is matter that is most abundant with hydrogen bonds, which may be organized in molecular networks thereby providing an indication that water via hydrogen bonds (i.e. with classical and quantum properties), may play a role in molecular and biomolecular recognition. From this viewpoint, there two primary goals in modern day pharmacy are: (1) understanding mechanism of molecular recognition in water solution and (2) water structure for drug design. Further, some pharmacologists are aware of importance of water structure for drug design owing to the fact that modeling ligand-receptor interaction has to include specific geometry, which relates to water structure. Still further, it is well known that hydrogen bonds are a link between two nucleotide chains in DNA and support existence of secondary, ternary and quaternary structure of proteins. Since, hydrogen bonds play important role in water, biomolecular structures, hydrated crystals and nanostructures research to characterize water and its hydrogen bonds by Opto-Magnetic Method. By this method, based on light-water interaction, it is possible to collect data of both classical and quantum actions of water molecules and interactions between them.

Operationally, this method is based on light-matter interaction and ratio of electrical and magnetic forces of covalent bonds and intermolecular bonds of matter. DNA research indicates that both classical and quantum mechanical approach give same phenomenological results for structures thereof. This is owing to one simple reason that is for stationary quantum state Hamiltonian is a sum of kinetic (T) and potential (V) energy, while Lagrangian is a difference between them when system is in equilibrium with external forces. Two similar pictures, one classical and another quantum, of same object with very close similar results from energy point of view exist. The goal is to find out how hydrogen bonds participate in water to be more or less classical or quantum entity. Therefore, use is made of Planck's constant (h) as the first criteria to estimate whether an object is classical or quantum. Since Planck's constant by nature is action than product of force (F), distance (d) and time (t) of action have to has value h (6.626 × 10-34 Js), or close to if system is quantum one. However, what will be value for coupling quantum-classical system, and when classical one becomes dominant, it is unknown.

Reiterating again, Planck's constant is link between energy (E) and electromagnetic wave oscillation (v), as E = hv. In certain situations, an analysis of the electrical vis-à-vis magnetic interaction between two electron charges in neighboring atoms in relative motion in matter may provide a solution. Further, it is known that is exigent to calculate the magnetic interaction between two charged particles in motion relative to an observer O in a form similar to the electric interaction given by Coulomb's law. In operation, a comparative study of the order of magnitude of the magnetic interaction with the electrical interaction. For example, and in no way of limiting the inventions, on taking into consideration two charges q and q' of neighboring atoms moving with velocities v and v' relative to observer may simplify the formulas, because only order of magnitude is important. Thus, the electrical force produced by q' on q as measured by O is qE.

Further, the magnetic field produced by q', on using equation B = 1/c2 (v×E), is of order of magnitude of v'E/c2 and the magnetic force on q is of the order of qvB = (w'/c2) qE. Since, qE is the electrical force on q than magnetic force/electrical force (FM/FE) ≈ vv'/c2. Still further, if the velocities of the charges are small compared with the velocity of light c, the magnetic force is negligible compared to the electrical force and in many cases can be ignored. The orbital velocity of valence electrons in atoms is about 106 m/s, FM/FE ≈10-4. This implies that existence of semi-classical/quantum could be 6,626 × 10-34 < h* < 6,626 × 10-30. In this action area, from energy point of view, simultaneously exists both classical and quantum phenomena. Because, this value of action coupling classical and quantum phenomena, means that this action area is perfect one for hydrogen bond investigation. Therefore, if action is h*>6,626 × 10-30 Js than phenomena are classical, while if it is 6,626 × 10-34 Js, it is quantum. Electrical force is closer to classical interaction (Coulomb's law), while magnetic force is closer for order four to quantum interaction than electrical one.

Specifically, in order to calculate action we should know values of force, distance and time of hydrogen bonds activity. In certain specific embodiments, the hydrogen bonds may posses the following specifications: Average values for force 2.5×10-10 N, distance 1.6 × 10-10 m and time 50 × 10-15 s. Based on the quantitative parameters and the values thereof the values give action of $h^* = F \times d \times t$ = (2.5×10-10) × (1.6 × 10-10) × (50 × 10-15) = 0.5 × 10-33 Js, what is semi-quantum action. Hydrogen bond in water is for three orders closer to quantum (6,626 × 10-34 Js) than to classical (6,626 × 10-30 Js) action. According to ratio FM/FE ≈10-4 it means that magnetic and electrical fingerprint of hydrogen bond of water will be different, because action of magnetic force is separated it two pats (quantum and classical), while electrical force is only classical, because domain of its action is 10-29Js (0.5 × 10-33 × 104 ≈ 10-29 Js).

In certain other embodiments, experimental measurements of quantum and classical contribution of hydrogen bonds action in water are disclosed. Specifically, there is disclosed experimental measurements of quantum and classical contribution of hydrogen bonds action in water using OMF device. Further, there is also disclosed separate electrical and magnetic action in light-water interaction. In operation, pictures of surfaces that are captured by classical optical microscope is based on electromagnetic property of light, while OMF is based on difference between diffuse white light and reflected polarized light. In here, reflected polarized light is produced when source of diffuse light irradiates the surface of matter under certain angle (Brewster's angle). Each type of matter has special different angle value of light polarization.

Further, it is found that angle of reflected polarized light of water is about 53 degree. Since reflected polarized light contains electrical component of light-matter interaction, taking the difference between white light (electromagnetic) and reflected polarized light (electrical) fields gives magnetic properties of matter (Opto-Magnetic Fingerprint).

Still further, digital images in RGB (R-red, G-green, B-blue) system are used in analysis, therefore basic pixel data in red and blue channels for white diffuse light (W) and reflected polarized white light (P). Algorithm for data analysis is based on chromaticity diagram called "Maxwell's triangle" and spectral convolution operation according to ratio of (R-B)&(W-P). The abbreviated designation means that Red minus Blue wavelength of White light and reflected Polarized light are used in spectral convolution algorithm to calculate data for Opto-Magnetic Fingerprint of matter. Therefore, method and algorithm for creating unique spectral fingerprint are based on the convolution of RGB color channel spectral plots generated from digital images that capture single and multi-wavelength light-matter interaction.

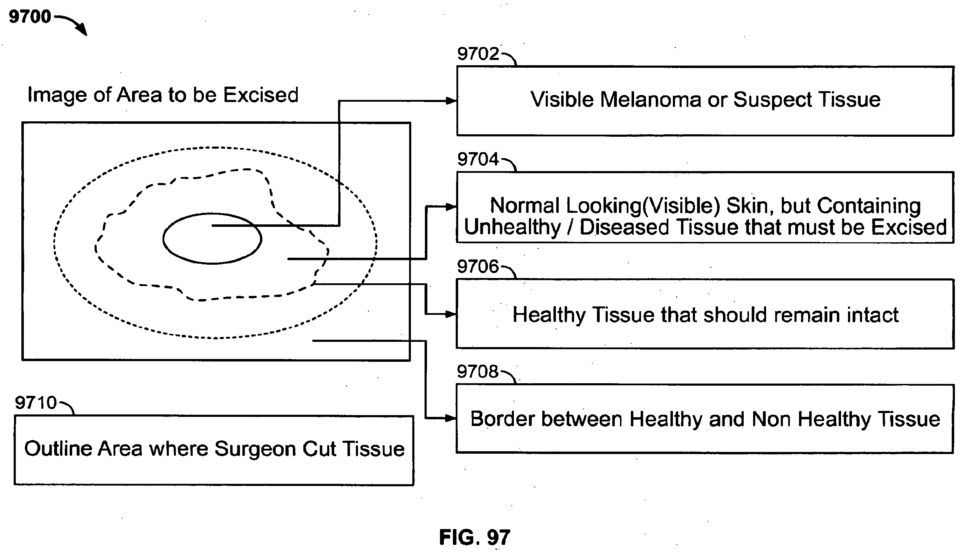

Accordingly, the foregoing description of the present technique should be considered as merely illustrative of the principles of the present technique and not in limitation thereof. Referring to FIG. 97 is a diagram 9700 depicting an image of area to be exercised. The image of the skin is captured for distinguishing between a healthy biological skin tissue and an unhealthy biological skin tissue for enabling an excision proximate to the healthy biological skin tissue. The biological skin tissue may be of the human skin tissue, the veterinary skin tissue, the agricultural product skin tissue including a finite and natural life cycle, and the like. In accordance with an example of the present invention, 9702 depicts the visible melanoma or suspect tissue in the captured, 9704 depicts the normal looking (visible) skin (this comprises unhealthy/ diseased tissue that must be excised), 9706 depicts the healthy skin tissue that should remain intact, 9708 depicts the border between healthy and non healthy tissue and 9710 depicts the outlined area for where the surgeon should cut the tissue. The image capturing device captures the image of the skin site for identifying the healthy biological skin tissue, the diseased biological skin tissue and tracking growth of the unhealthy biological skin tissue. The biological skin tissue comprises a finite and natural life cycle. The captured image of the particular site of skin is analyzed in pixel by pixel manner by analyzer of skin images for generating a sample of most frequent of a standard R G B (sRGB) color component.

According to an exemplary embodiment of the present invention, an algorithmic method based on optical analysis of skin biophysical characteristics of captured image under white light and standard RGB analysis of image in pixel by pixel manner may be employed for precisely determining the presence of a healthy tissue and suspect tissue. This helps the surgeon for leaving a larger amount of healthy tissue around a site, decrease recurrance and micrometastasis in surrounding skin while allowing minimal surgical morbidity. The method may be used to image a particular site, and determine border area, suspect tissue, either before surgery, in pre-surgery, or during surgery. The method would also show post surgical analysis of affected skin tissue.

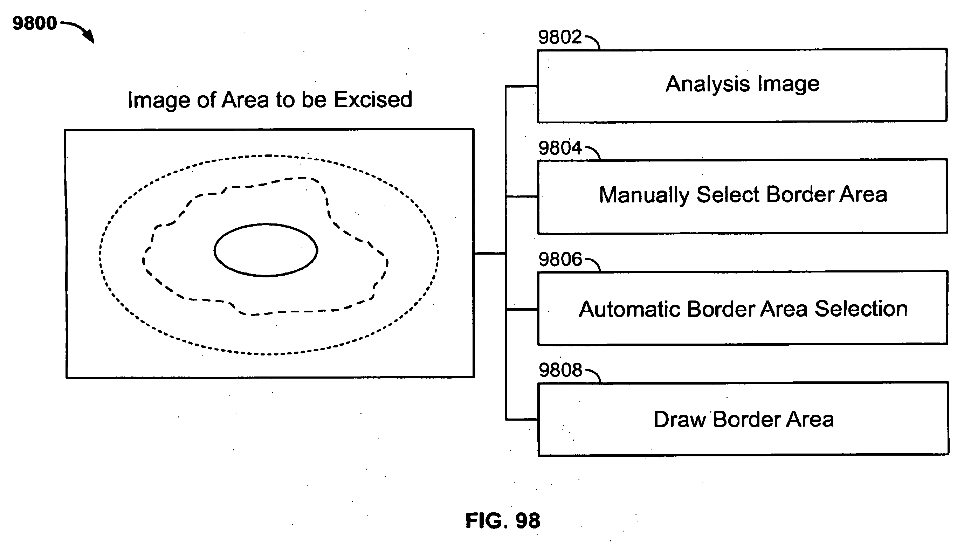

Referring to FIG. 98 is a diagram 9800 depicting the process employed for automatically determining the area to be exercised. According to an example, analysis of image 9802 is done using an optical analysis device coupled to the image capturing device and the surgical intervention unit. The analysis would include controls for type of diseased tissue. The border area is selected manually 9804 for distinguishing between healthy biological skin tissue and suspect skin tissue. Border area is selected manually based on the implied healthy non healthy tissue. In accordance with an example of the present invention, automatically the border area is selected 9806 by the system so that the surgeon could leave a larger amount of healthy tissue around a site, decrease recurrance and micrometastasis in surrounding skin while allowing minimal surgical morbidity. The algorithmic method to best determine the border area based on user-definable parameters such as minimally width, desired shape (circular, square, for example).Finally a border area is drawn 9808 for determining the exact area to be excised for treatment. A hypo-allergenic ink or other marking substance may be used to draw on the surface of the skin automatically using an attached device.

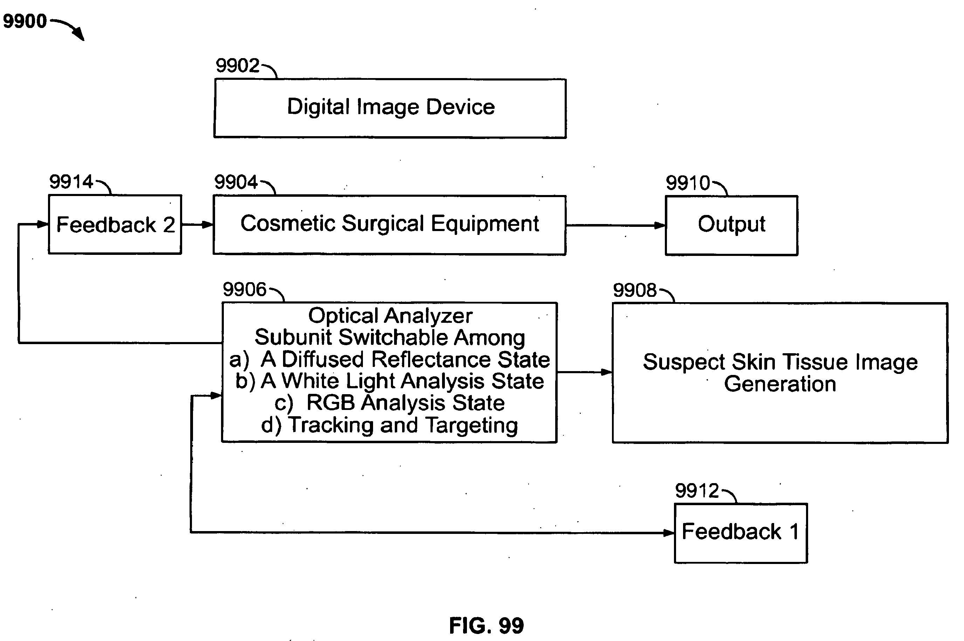

**[0667]** [0041] Referring to FIG. 99 is a diagram 9900 depicting a system for distinguishing between the healthy skin biological skin tissue and an unhealthy biological skin tissue for enabling an excision proximate to the healthy biological tissue. The image of skin site may be captured by the digital imaging device 9902. The digital imaging device may be used for identifying a healthy biological skin tissue; a diseased biological skin tissue; and tracking growth of the unhealthy biological skin tissue. The digital imaging device may comprise a real time digital camera device. The captured image may be submitted to cosmetic surgical equipment 9904 for further analysis of the image for distinguishing between the healthy biological skin tissue and the suspect biological skin tissue. The optical analyzer 9906 is coupled to the feedback unit 9912 and cosmetic surgical unit. The optical analyzer further comprises sub unit switchable among a diffused reflectance state, a white light analysis state, RGB analysis state and tracking and targeting state. The optical analysis device coupled to the image capturing device comprises the Red Green Blue (RGB) unit further comprising, the sampler coupled to a pixel by pixel by analyzer of skin images for generating a sample of most frequent of a standard R G B (sRGB) color component, the Gaussian probabilistic distributer for modeling the sRGB component color distribution with estimated parameters on the generated sRGB color sample for the captured image and the photo type generator coupled to the Gaussian probabilistic distributer for generating the phototype of the biological skin tissue through a decision tree unit.

According to an exemplary embodiment of the present invention, the white light unit further comprises the comparison unit for comparing extreme positions of at least two unique convolutions in white light and in polarized light responsive to convoluting data of the first skin image and a second skin image and an output unit for determining a distance between minimum and maximum intensity positions in convoluted red minus blue wavelength scale in the at least two unique convolutions for generating a numerical skin type output. According to an example, the optical analyzer further comprises the skin biophysical analysis unit further including at least one of the following parameters: a skin fairness parameter, a skin darkness parameter, systemic hydration, skin hydration, skin firmness, skin wrinkles, pore size on skin, spots on skin, glow on skin, melanocyte, melanin, hemoglobin, porphyrin, keratin, carotene, collagen, elastin, sebum, sebaceous gland activity, sweat pore, sebaceous pore, moisture level, elasticity, luminosity, firmness, fine line, wrinkle count, pore size, percent of open pores, skin elasticity, skin tension line, spots, viscosity, epidermal, dermal sebum levels, skin color, psoriasis, allergy, red area, general skin disorder, infection, tumor, sunburn, rash, scratch, pimple, acne, insect bite, itch, bleeding, injury, inflammation, photodamage, pigmentation, tone, tattoo, percent burn, burn classification, mole, aspect of a skin lesion, melanoma, dermally observed disorder, cutaneous lesion, cellulite, boil, blistering disease, congenital dermal syndrome, cutaneous mycoses, melasma, vascular condition, rosacea, spider vein, texture, skin ulcer, wound healing, post-operative tracking, melanocytic lesion, nonmelanocytic lesion, basal cell carcinoma and seborrhoic keratosis.

According to an example, the optical analysis device further comprising a diffused reflectance unit for generating the predetermined set of wavelengths for reflection intensity measurement of the spectral data, utilizing the plurality of reflection intensity values and the plurality of reflection intensity ratio values of the spectral data for classification of the skin type responsive to generating a predetermined set of wavelengths, normalizing the reflection intensity values of spectral data with respect to spectral source and spectral classification of the skin type and generating a skin photo type output by applying nonparametric regression analysis on measured spectral data responsive to normalizing the reflection intensity values of spectral data.

In accordance with an example of the present invention, the output of optical analyzer is fed to the suspect skin tissue image generation unit 9908. The suspect skin tissue image generator coupled to the optical analysis device for imaging a site on the biological skin area, determining the border area on the site and determining the suspect skin tissue. The suspect tissue image generator comprises the image of an area to be excised which includes the visible suspect skin tissue, the normal visible skin tissue surrounding the visible suspect tissue for excision, the border between the visible suspect tissue and the normal visible skin tissue, the healthy skin tissue surrounding both the visible suspect skin tissue and the normal visible skin tissue, outlined area for the surgeon to cut a predetermined skin tissue portion including the visible suspect skin tissue, the normal visible skin tissue, the border and the healthy skin tissue.

The output of suspect skin tissue image generation unit 9908 is fed to the feed back unit 9912. The feed back obtained is fed to the optical analyzer 9906 wherein the analysis is further done based on the obtained feedback. The analysis data is further fed to the cosmetic surgical equipment 9904 through another additional feed back unit 9914 coupled between the optical analyzer 9906 and cosmetic surgical equipment 9904. Finally an accurate area to be excised is given as output 9910.

As will be appreciated by a person skilled in the art, the various implementations of the present technique provide a variety of advantages. Firstly, the process employed for distinguishing between a healthy biological skin tissue and an unhealthy biological skin tissue for enabling an excision proximate to the healthy biological skin tissue Allows more precise determination of the border area instead of relying on subjective experience or fixed tables. Secondly, the algorithmic method may be used to image a particular site, and automatically determine border area, suspect tissue, either before surgery, in pre-surgery, or during surgery. The algorithmic method would also show post surgical analysis of affected skin tissue. Thirdly, the advantage of this system is better isolated suspect tissue and retaining a greater degree of healthier tissue. Fourthly, the system allows a surgeon or other specialist to precisely determine the border area around a surgical intervention for primary cutaneous melanoma, skin cancers, and other skin diseases that require excision around the skin.

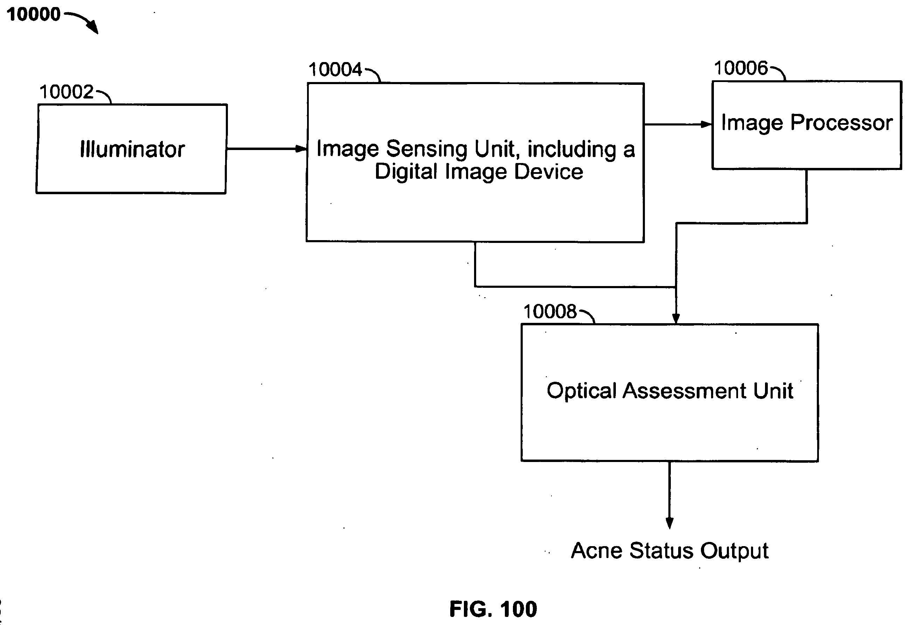

Referring to FIG.100 is a schematic diagram 10000 depicting a system for determining a predisposition of sebaceous pores and skin structures. The system may include an illuminator 10002, an image sensing unit including a digital imaging device 10004 coupled to the illuminator and image processor 10006 for imaging the portion of the surface on the skin and an optical assessment unit 10008 is coupled to the image sensing unit including the digital imaging device 10004 and the image processor 10006. According to an example of the present invention, the optical assessment unit 10008 may include a spectroscopic analysis unit, which may further include a diffused reflectance color analysis unit.

In accordance with an exemplary embodiment of the present invention, the illuminator 10002 for illuminating a portion of a surface on the skin may include the white light source, the blue light source, and an ultraviolet light source and the like. The images of skin are captured with the imaging sensing unit including the digital imaging device 10004 coupled to the illuminator 10002. The images may be captured under white light or blue light or ultra violet light source and the like. According to an example, the propensity to get acne and acne status output can be ascertained based on anatomical-physiological factors. The characteristics of the skin may be measured on at least one of discrete scale and a continuous scale. The continuous scale comprises a plurality of acne improvement and worsening conditions further including a predetermined number of acne status outcomes. The continuous scale and discrete scale may include at least one of the following acne conditions of an acne condition unit closed, partially open and open for sebaceous pore opening; full, partially full and empty for sebaceous pore contents; blocked, partially blocked and clear for gland and hair connection; full, partially full and empty for sebaceous gland contents; active, partially active and inactive for sebaceous gland activity; and high, medium, low and none for inflammation. The acne condition unit may comprise a questionnaire unit for generating an acne status questionnaire.

According to an exemplary embodiment of the present invention, the image processor may include a plurality of characteristic acne elements elimination unit for isolating sebaceous pore openings, sebaceous pore channel, sebaceous pore intersection, sebaceous gland intersection, blockage of sebaceous pore openings, contents of the sebaceous pore, unhealthiness arising out of age of the sebaceous gland, inflammation around the gland, inflammation around the sebaceous pores, inflammation around the sebaceous gland, inflammation around hair follicles and level of p-acne bacteria. The plurality of characteristic acne elements elimination unit may also include determining age of sebum, whether the sebaceous gland is actively producing sebum and a level of p-acne bacteria.

In accordance with an exemplary embodiment of the present invention, the output of the image processor 10006 is fed to the optical assessment unit 10008. The optical assessment unit 10008 may include Red Green Blue (RGB) analysis device further including a standard RGB (sRGB) color unit for analysis of the captured digital image. The white light polarization device coupled to the RGB analysis device compares extreme positions of at least two unique convolutions in white light and in polarized light in response to the convoluting data of the first captured image and the second captured image. According to an example, the white light polarization device may further include an output generator for determining the distance between minimum and maximum intensity positions in the convoluted red minus blue wavelength scale in the at least two unique convolutions to generate a numerical skin type output. The correlation level may include at least one of a fuzzy logic, a non-linear regression, a genetic algorithm and a neural network The digital color analysis device coupled to both the white light polarization device and the RGB analysis device for generating a combination of color systems for determining the health status of the imaged portion of the surface on the skin. The combination of color systems may include at least one of the YIQ, YCbCr, L*a*b* (CIELAB color space); L*u*v* (CIELUV color space); HSL (Hue, Saturation, Lightness) and HSV (Hue, Saturation, Value) color systems for image analysis in accordance with an example of the present invention, which is not limited to the listed color systems. According to an example of the present invention the system may further include a marking unit for outlining and marking areas on the surface on the skin to thereby enable surgical excision of the skin structure. Finally the optical assessment unit 10008 outputs the acne status.

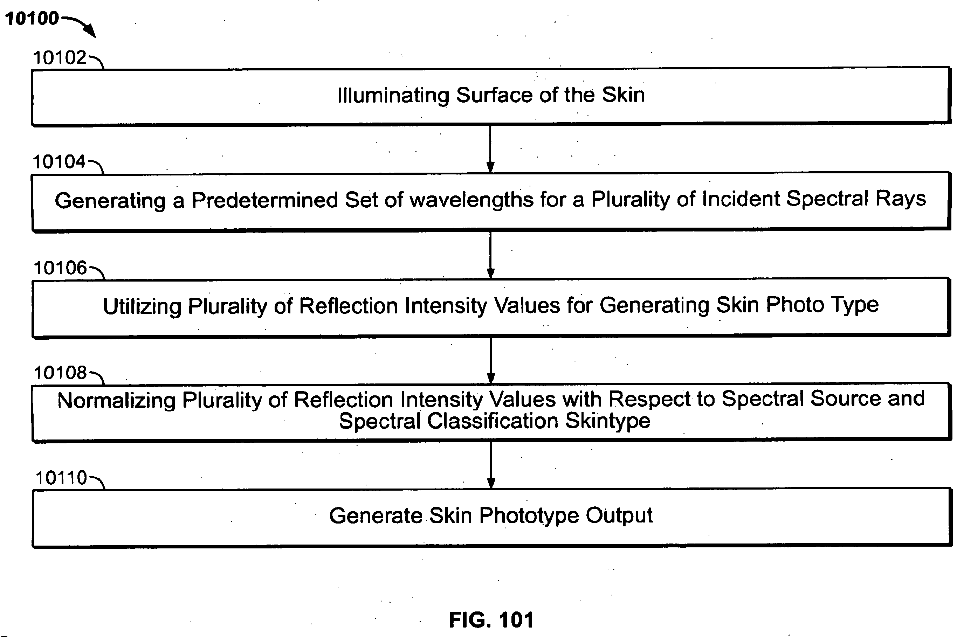

Referring to FIG. 101 is a flowchart 10100 illustrating a process for, in accordance with an aspect of the present technique. The process starts at block 10100 wherein the surface of the skin is illuminated by a light source. Spectral rays are reflected back once the light is illuminated on the surface of the skin. Now at block 10102, a predetermined set of wave lengths may be generated for reflection intensity measurements of the spectral data. The set of wave lengths may be generated for a plurality of incident spectral rays. In accordance with an example of the invention, at block 10103 a plurality of reflection intensity values and plurality of reflection intensity ratio values of diffusely reflected spectral data may be utilized for classification of skin type in response to generating the predetermined set of wavelengths. The process continues to block 10104, wherein normalization of reflection intensity values of spectral data may be done with respect to spectral source and spectral classification of skin type. The step of normalizing the reflection intensity values of diffusely reflected spectral data with respect to light source and detector spectral characteristics comprises a sub step of making diffusely reflected spectral data independent of measurement instrument. Finally at block 10105 skin photo type output may be generated by applying nonparametric regression analysis on diffusely reflected spectral data in response to normalizing the reflection intensity values of spectral data. The step of generating a skin photo type output by applying nonparametric regression analysis on measured spectral data comprises a sub step of using a plurality of intensity of reflection values, a plurality of differential reflection intensity (for example difference in reflection intensities: $I(400nm)-I(424nm)$, $I(474nm)-I(424nm)$, $I(512nm)-I(540nm)$, $I(512nm)-I(578nm)$,and ratios of reflection intensities: $I(400nm)/I(424nm)$, $I(474nm)/I(424nm)$, $I(512nm)/I(540nm)$, $I(512nm)/I(578nm)$) values and a plurality of ratios of reflection intensity values for deriving a skin photo type from regression tree previously generated by applying nonparametric regression analysis on measured spectral data.

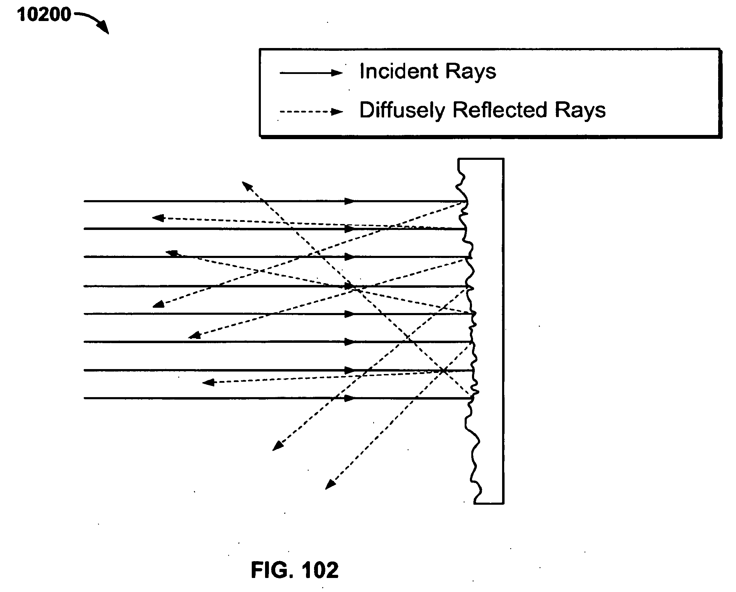

Referring to FIG. 102 a diagram depicting reflectance of spectral rays (diffusely reflected spectral rays) in all directions from the surface of the skin is depicted. In accordance with an example, when light is illuminated on the surface of the skin, spectral rays are reflected.

According to an exemplary embodiment of the present invention, the diffusely reflected spectral rays are analyzed for generation of skin photo type. Analysis of diffusely reflected spectral rays for determining skin photo type may be done by nonparametric classification of diffuse reflectance spectral data. The skin photo type may be of a human skin or a veterinary skin or the like. The diffuse reflectance measurements for determination of skin photo type may be performed in the Ultra - Violet spectral range (for example from 380 to 600nm or at the specific wavelengths (for example 400, 424, 474, 512, 540 and 578 nm). The nonparametric classification of diffuse reflectance spectral data is free from potential errors due to human interpretation. Further, the method for skin photo type determination by nonparametric classification of diffuse reflectance spectral data is machine autonomous and may be applicable to any diffused reflectance measurement system operating in the Ultraviolet- Visible Spectroscopy spectral range.

In accordance with an example of the present invention, skin photo type is determined by non-parametric classification of diffuse reflectance spectral data. The following steps are involved for generation of skin photo type. A predetermined set of wave lengths are generated for reflection intensity measurement of the spectral data. Generating a predetermined set of wavelengths for reflection intensity measurement of the spectral data comprises a sub step of generating a predetermined set of wavelengths for a plurality of incident spectral rays. The method for skin photo type determination by nonparametric classification of diffuse reflectance spectral data is machine autonomous and may be applicable to any diffused reflectance measurement system operating in the Ultraviolet- Visible Spectroscopy spectral range. According to an example, the nonparametric classification of diffuse reflectance spectral data is free from potential errors due to human interpretation.

According to an exemplary embodiment of the present invention, a plurality of reflection intensity values and a plurality of reflection intensity ratio values of the spectral data may be utilized for classification of a skin type response to generating the predetermined set of wavelengths. The step of utilizing a plurality of reflection intensity values and a plurality of reflection intensity ratio values of the spectral data for classification of a human skin type responsive to generating an original set of chosen wavelengths comprising a sub step of utilizing a plurality of differential reflection intensity values( for example difference in reflection intensities: I(400nm)-I(424nm), I(474nm)-I(424nm), I(512nm)-I(540nm), I(512nm)-I(578nm),and ratios of reflection intensities: I(400nm)/I(424nm), I(474nm)/I(424nm), I(512nm)/I(540nm), I(512nm)/I(578nm)).

In accordance with an example of the present, normalization of the reflection intensity values of spectral data may be done with respect to spectral source and spectral classification of the skin type. The step of normalizing the reflection intensity values of spectral data with respect to light source and detector spectral characteristics comprises a sub step of making spectral data independent of measurement instrument. Non parametric regression analysis may be applied on measured spectral data for generating the skin photo type in response to normalizing the reflection intensity values of spectral data. The step of generating a skin photo type output by applying nonparametric regression analysis on measured spectral data comprising a sub step of using a plurality of intensity of reflection values, a plurality of differential reflection intensity values and a plurality of ratios of reflection intensity values for deriving a skin photo type from regression tree previously generated by applying nonparametric regression analysis on measured spectral data.

In certain embodiments, methods, apparatuses and systems for management of overall health status of teeth has been disclosed. In certain such embodiments, design and implementation of methods for management of overall health status of teeth and systems and apparatuses thereof has been disclosed. Specifically, there is disclosed the design and implementation of methods for management of overall health status of teeth, such as determination of tooth enamel and other dermal structures thereof, determination of depth of enamel and predisposition of dental cavities and other dental problems, and systems and apparatuses thereof.

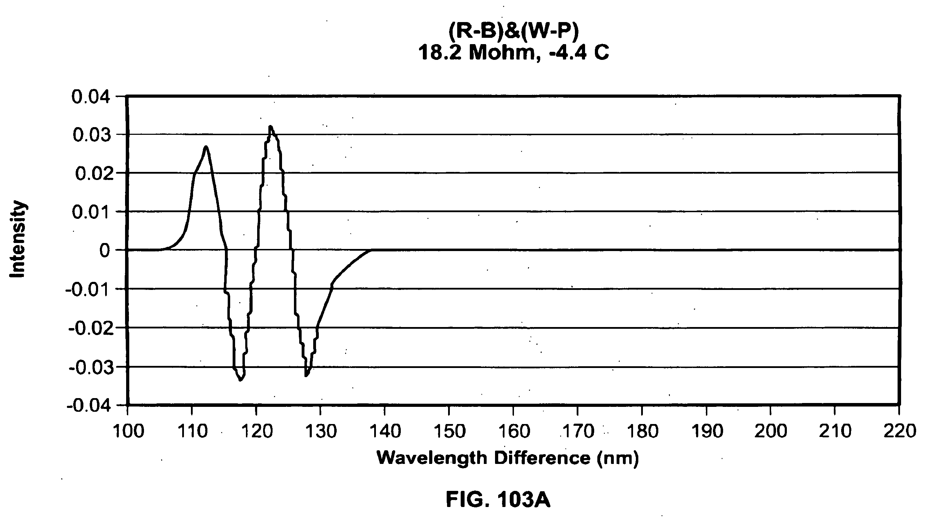

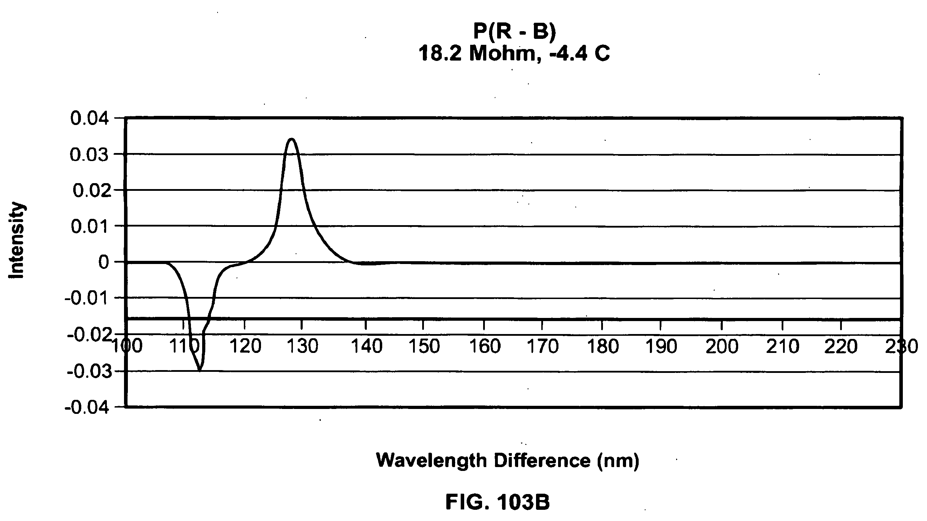

FIG. 103 depicts Opto-magnetic diagrams for 18.2 MW water at -4.4 °C. a) characteristics points for magnetic domain [(R-B)&(W-P)]: (105.16 nm, 0), (111.69 nm, + 0.0256), (114.95 nm, 0), (117.07 nm, -0.0323), (120.24 nm, 0), (121.99 nm, 0.0307), (125.49 nm, 0), (127.6 nm, -0.03063), (140.37, 0); b) Characteristics points for electrical domain [P(R-B)]: (104.01 nm, 0), (111.31 nm, -0.0237), (118.45 nm, 0), (127.88 nm, 0.0333), (137.61 nm, 0), in accordance with certain embodiments of the invention; and

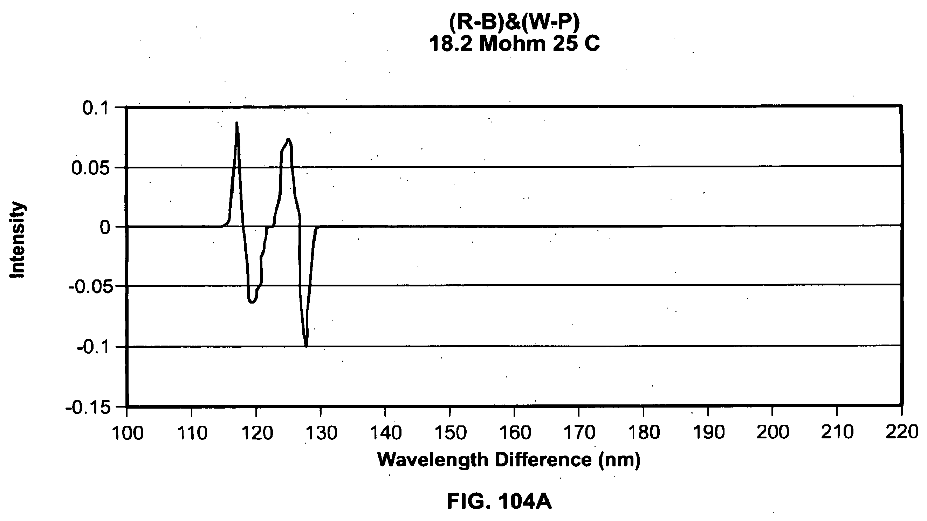

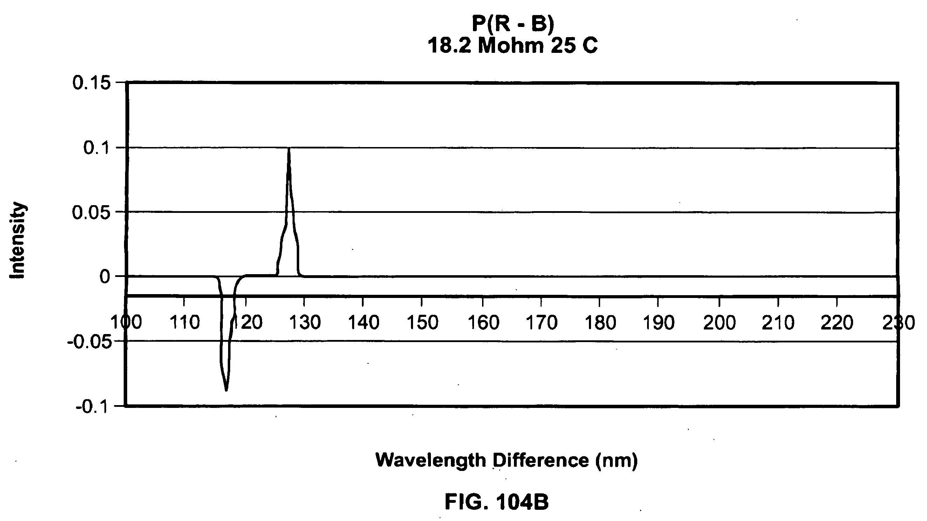

FIG. 104 depicts Opto-magnetic diagrams for 18.2 MW water at 25 °C. a) Characteristics points for magnetic domain [(R-B)&(W-P)]: (113.81 nm, 0), (116.69 nm, + 0.0781), (117.95 nm, 0), (118.92 nm, -0.0627), (121.7 nm, 0), (124.79 nm, 0.0722), (126.19 nm, 0), (127.3 nm, -0.0978), (130.73, 0) b)Characteristics points for electrical domain [P(R-B)]: (113.29 nm, 0), (116.67 nm, -0.0782), (118.71 nm, 0), (124.16 nm, 0), (127.33 nm, 0.1003), (129.07 nm, 0), in accordance with certain embodiments of the invention.

The methods and systems described herein may be deployed in part or in whole through a machine that executes computer software, program codes, and/or instructions on a processor. The processor may be part of a server, client, network infrastructure, mobile computing platform, stationary computing platform, or other computing platform. A processor may be any kind of computational or processing device capable of executing program instructions, codes, binary instructions and the like. The processor may be or include a signal processor, digital processor, embedded processor, microprocessor or any variant such as a co-processor (math co-processor, graphic co-processor, communication co-processor and the like) and the like that may directly or indirectly facilitate execution of program code or program instructions stored thereon. In addition, the processor may enable execution of multiple programs, threads, and codes. The threads may be executed simultaneously to enhance the performance of the processor and to facilitate simultaneous operations of the application. By way of implementation, methods, program codes, program instructions and the like described herein may be implemented in one or more thread. The thread may spawn other threads that may have assigned priorities associated with them; the processor may execute these threads based on priority or any other order based on instructions provided in the program code. The processor may include memory that stores methods, codes, instructions and programs as described herein and elsewhere. The processor may access a storage medium through an interface that may store methods, codes, and instructions as described herein and elsewhere. The storage medium associated with the processor for storing methods, programs, codes, program instructions or other type of instructions capable of being executed by the computing or processing device may include but may not be limited to one or more of a CD-ROM, DVD, memory, hard disk, flash drive, RAM, ROM, cache and the like.

A processor may include one or more cores that may enhance speed and performance of a multiprocessor. In embodiments, the process may be a dual core processor, quad core processors, other chip-level multiprocessor and the like that combine two or more independent cores (called a die).

The methods and systems described herein may be deployed in part or in whole through a machine that executes computer software on a server, client, firewall, gateway, hub, router, or other such computer and/or networking hardware. The software program may be associated with a server that may include a file server, print server, domain server, internet server, intranet server and other variants such as secondary server, host server, distributed server and the like. The server may include one or more of memories, processors, computer readable media, storage media, ports (physical and virtual), communication devices, and interfaces capable of accessing other servers, clients, machines, and devices through a wired or a wireless medium, and the like. The methods, programs or codes as described herein and elsewhere may be executed by the server. In addition, other devices required for execution of methods as described in this application may be considered as a part of the infrastructure associated with the server.

The server may provide an interface to other devices including, without limitation, clients, other servers, printers, database servers, print servers, file servers, communication servers, distributed servers and the like. Additionally, this coupling and/or connection may facilitate remote execution of program across the network. The networking of some or all of these devices may facilitate parallel processing of a program or method at one or more location without deviating from the scope of the invention. In addition, any of the devices attached to the server through an interface may include at least one storage medium capable of storing methods, programs, code and/or instructions. A central repository may provide program instructions to be executed on different devices. In this implementation, the remote repository may act as a storage medium for program code, instructions, and programs.

The software program may be associated with a client that may include a file client, print client, domain client, internet client, intranet client and other variants such as secondary client, host client, distributed client and the like. The client may include one or more of memories, processors, computer readable media, storage media, ports (physical and virtual), communication devices, and interfaces capable of accessing other clients, servers, machines, and devices through a wired or a wireless medium, and the like. The methods, programs or codes as described herein and elsewhere may be executed by the client. In addition, other devices required for execution of methods as described in this application may be considered as a part of the infrastructure associated with the client.

The client may provide an interface to other devices including, without limitation, servers, other clients, printers, database servers, print servers, file servers, communication servers, distributed servers and the like. Additionally, this coupling and/or connection may facilitate remote execution of program across the network. The networking of some or all of these devices may facilitate parallel processing of a program or method at one or more location without deviating from the scope of the invention. In addition, any of the devices attached to the client through an interface may include at least one storage medium capable of storing methods, programs, applications, code and/or instructions. A central repository may provide program instructions to be executed on different devices. In this implementation, the remote repository may act as a storage medium for program code, instructions, and programs.

The methods and systems described herein may be deployed in part or in whole through network infrastructures. The network infrastructure may include elements such as computing devices, servers, routers, hubs, firewalls, clients, personal computers, communication devices, routing devices and other active and passive devices, modules and/or components as known in the art. The computing and/or non-computing device(s) associated with the network infrastructure may include, apart from other components, a storage medium such as flash memory, buffer, stack, RAM, ROM and the like. The processes, methods, program codes, instructions described herein and elsewhere may be executed by one or more of the network infrastructural elements.

The methods, program codes, and instructions described herein and elsewhere may be implemented on a cellular network having multiple cells. The cellular network may either be frequency division multiple access (FDMA) network or code division multiple access (CDMA) network. The cellular network may include mobile devices, cell sites, base stations, repeaters, antennas, towers, and the like. The cell network may be a GSM, GPRS, 3G, EVDO, mesh, or other networks types.

The methods, programs codes, and instructions described herein and elsewhere may be implemented on or through mobile devices. The mobile devices may include navigation devices, cell phones, mobile phones, mobile personal digital assistants, laptops, palmtops, netbooks, pagers, electronic books readers, music players and the like. These devices may include, apart from other components, a storage medium such as a flash memory, buffer, RAM, ROM and one or more computing devices. The computing devices associated with mobile devices may be enabled to execute program codes, methods, and instructions stored thereon. Alternatively, the mobile devices may be configured to execute instructions in collaboration with other devices. The mobile devices may communicate with base stations interfaced with servers and configured to execute program codes. The mobile devices may communicate on a peer to peer network, mesh network, or other communications network. The program code may be stored on the storage medium associated with the server and executed by a computing device embedded within the server. The base station may include a computing device and a storage medium. The storage device may store program codes and instructions executed by the computing devices associated with the base station.

The computer software, program codes, and/or instructions may be stored and/or accessed on machine readable media that may include: computer components, devices, and recording media that retain digital data used for computing for some interval of time; semiconductor storage known as random access memory (RAM); mass storage typically for more permanent storage, such as optical discs, forms of magnetic storage like hard disks, tapes, drums, cards and other types; processor registers, cache memory, volatile memory, non-volatile memory; optical storage such as CD, DVD; removable media such as flash memory (e.g. USB sticks or keys), floppy disks, magnetic tape, paper tape, punch cards, standalone RAM disks, Zip drives, removable mass storage, off-line, and the like; other computer memory such as dynamic memory, static memory, read/write storage, mutable storage, read only, random access, sequential access, location addressable, file addressable, content addressable, network attached storage, storage area network, bar codes, magnetic ink, and the like.

The methods and systems described herein may transform physical and/or or intangible items from one state to another. The methods and systems described herein may also transform data representing physical and/or intangible items from one state to another.

The elements described and depicted herein, including in flow charts and block diagrams throughout the figures, imply logical boundaries between the elements. However, according to software or hardware engineering practices, the depicted elements and the functions thereof may be implemented on machines through computer executable media having a processor capable of executing program instructions stored thereon as a monolithic software structure, as standalone software modules, or as modules that employ external routines, code, services, and so forth, or any combination of these, and all such implementations may be within the scope of the present disclosure. Examples of such machines may include, but may not be limited to, personal digital assistants, laptops, personal computers, mobile phones, other handheld computing devices, medical equipment, wired or wireless communication devices, transducers, chips, calculators, satellites, tablet PCs, electronic books, gadgets, electronic devices, devices having artificial intelligence, computing devices, networking equipments, servers, routers and the like. Furthermore, the elements depicted in the flow chart and block diagrams or any other logical component may be implemented on a machine capable of executing program instructions. Thus, while the foregoing drawings and descriptions set forth functional aspects of the disclosed systems, no particular arrangement of software for implementing these functional aspects should be inferred from these descriptions unless explicitly stated or otherwise clear from the context. Similarly, it will be appreciated that the various steps identified and described above may be varied, and that the order of steps may be adapted to particular applications of the techniques disclosed herein. All such variations and modifications are intended to fall within the scope of this disclosure. As such, the depiction and/or description of an order for various steps should not be understood to require a particular order of execution for those steps, unless required by a particular application, or explicitly stated or otherwise clear from the context.

The methods and/or processes described above, and steps thereof, may be realized in hardware, software or any combination of hardware and software suitable for a particular application. The hardware may include a general purpose computer and/or dedicated computing device or specific computing device or particular aspect or component of a specific computing device. The processes may be realized in one or more microprocessors, microcontrollers, embedded microcontrollers, programmable digital signal processors or other programmable device, along with internal and/or external memory. The processes may also, or instead, be embodied in an application specific integrated circuit, a programmable gate array, programmable array logic, or any other device or combination of devices that may be configured to process electronic signals. It will further be appreciated that one or more of the processes may be realized as a computer executable code capable of being executed on a machine readable medium.

The computer executable code may be created using a structured programming language such as C, an object oriented programming language such as C++, or any other high-level or low-level programming language (including assembly languages, hardware description languages, and database programming languages and technologies) that may be stored, compiled or interpreted to run on one of the above devices, as well as heterogeneous combinations of processors, processor architectures, or combinations of different hardware and software, or any other machine capable of executing program instructions.

Thus, in one aspect, each method described above and combinations thereof may be embodied in computer executable code that, when executing on one or more computing devices, performs the steps thereof. In another aspect, the methods may be embodied in systems that perform the steps thereof, and may be distributed across devices in a number of ways, or all of the functionality may be integrated into a dedicated, standalone device or other hardware. In another aspect, the means for performing the steps associated with the processes described above may include any of the hardware and/or software described above. All such permutations and combinations are intended to fall within the scope of the present disclosure.

The invention is intended to cover all equivalent embodiments, and is limited only by the appended claims. Various other embodiments are possible within the scope of the invention, as defined by the appended claims. While the invention may be susceptible to various modifications and alternative forms, the specific embodiments have been shown by way of example in the drawings and have been described in detail herein. The aforementioned specific embodiments are meant to be for explanatory purposes only, and not intended to delimit the scope of the invention, as defined by the appended claims. Rather, the invention is to cover all modifications, equivalents, and alternatives falling within the scope of the invention as defined by the following appended claims.

**Claims**

1. A system, comprising:

an illuminator (10002) for illuminating a skin element;

an image capturing device (10004) coupled to the illuminator for imaging the portion of the surface of the skin;

an image processor (10006) coupled to the image capturing device for processing the imaged portion of the surface of the skin and isolating sebaceous parameters pertaining to acne formation, including changes in the sebaceous glands and pores, by employing filter techniques to process the imaged portion of the surface of the skin; and

an optical assessment unit (10008) coupled to both the image capturing device and the image processor, the optical assessment unit further comprising:

> a color Red Green Blue (RGB) analysis device including a standard RGB (sRGB) color unit;
>
> a white light polarization device coupled to the RGB analysis device for comparing extreme positions, comprising maxima and minima intensity positions, of at least two unique convolutions or spectral signatures, each convolution or spectral signature being generated by convoluting data of a first image captured using white light and a second image captured using white light or polarized light to create the unique convolution or spectral signature; and
>
> a digital color analysis device coupled to both the white light polarization device and the RGB analysis device for generating a combination of color systems and for determining a health status of the imaged portion of the surface on the skin, wherein the health status comprises a level of acne and a predisposition of a portion of the skin to acne based on anatomical-physiological factors.

2. The system of claim 1, wherein the incident light comprises unpolarised light and light reflected or re-emitted from the skin element may comprise polarized light.

3. The system of claim 1 or claim 2, wherein the two unique convolutions comprise incident light that is perpendicular to the skin and incident light that is at a non-perpendicular angle to the skin.

4. The system of any of claims 1 to 3, wherein the incident light that is at a non-perpendicular angle to the skin is at the Brewster angle.

5. The system of any one of the above claims, wherein the illuminator (10002) comprises one light source or more than one light source.

6. The system of any one of the above claims, wherein the light source comprises violet light in the general range of about 385 nm.

7. The system of any one of the above claims, wherein the light source is a light or a light emitting diode (LED) emitting radiation at a wavelength selected from the group consisting of about 280 nm, 340 nm, 360 nm, 385 nm, 395 nm, 400 nm, 405 nm and 480 nm.

8. The system of any one of the above claims, wherein the system is useful for determining a predisposition of sebaceous pores and skin structures around a sebaceous gland, a level of acne, and a predisposition of a portion of skin to improve and worsen the acne.

9. The system according to any one of the above claims, wherein the optical assessment unit (10008) further comprises an algorithm for generating a skin condition assessment.

10. The system according to any one of the above claims, wherein an output of the optical assessment unit (10008) includes characteristics of acne conditions.

11. The system according to claim 10, wherein the output comprises a plurality of acne improvement and worsening conditions with a number of acne status outcomes.

12. The system according to any one of the above claims, wherein the output of the optical assessment unit (10008) includes at least one of the following of an acne condition unit: closed, partially open and open for a sebaceous pore opening; full, partially full and empty for sebaceous pore contents; blocked, partially blocked and clear for a gland and hair connection; full, partially full and empty for sebaceous gland contents; active, partially active and inactive for sebaceous gland activity; and high, medium, low and none for inflammation.

**13.** The system according to any one of the above claims, wherein the output of the system includes a property of the skin, wherein the property is at least one of a structure, form, concentration, number, size, state, and stage of at least one of a: melanocyte, melanin, hemoglobin, porphyrin, keratin, carotene, collagen, elastin, sebum, sebaceous gland activity, pore (sweat and sebaceous), moisture level, elasticity, luminosity, firmness, fine line, wrinkle count and stage, pore size, percent of open pores, skin elasticity, skin tension line, spot, skin color, psoriasis, allergies, red area, general skin disorder or infection, tumor, sunburn, rash, scratch, pimple, acne, insect bite, itch, bleeding, injury, inflammation, photodamage, pigmentation, tone, tattoo, percent burn/ burn classification, mole (naevi, nevus), aspect of a skin lesion (structure, color, dimensions/asymmetry), melanoma, dermally observed disorder, cutaneous lesion, cellulite, boil, blistering disease, congenital dermal syndrome, (sub)cutaneous mycoses, melasma, vascular condition, rosacea, spider vein, texture, skin ulcer, wound healing, postoperative tracking, melanocytic lesion, non-melanocytic lesion, basal cell carcinoma, seborrhoic keratosis, sebum (oiliness), and nail and/or hair-related concern.

**14.** The system according to any one of the above claims, wherein the system is controlled by a computer and is programmed for:

  detecting a degree of polarization of light reflected from the skin; and
  determining a skin state based on an aspect of the polarization of the reflected light.

**15.** The system according to any one of the above claims, wherein the system is controlled by a computer and is programmed for:

  obtaining a baseline skin state assessment;
  recommending a monitoring interval based on at least one of a skin care goal, a product, and a regimen;
  obtaining a second skin state assessment;
  comparing the second assessment to the baseline assessment to determine progress towards the skin care goal; and
  optionally, optimizing the regimen or product in order to improve a skin state.

**16.** The system according to any one of the above claim, wherein the system is controlled by a computer and is programmed for:

  obtaining answers to a series of subjective questions regarding the skin;
  obtaining an objective skin analysis using a dermal imaging device; and
  combining the subjective and objective results algorithmically to obtain a skin state.

**17.** The system according to any one of the above claims wherein the image processor (10006) is configured for capturing an image of the skin illuminated with incident non-angled white light and an image of the skin illuminated with incident angled white light;
generating a normalized red and a normalized blue color channel histogram for at least one of a reflected and emitted light in each image;
correlating the normalized red and normalized blue color channel histograms to a wavelength scale to obtain red and blue color channel spectral plots; and
combining the red and blue color channel spectral plots by subtracting the spectral plot for angled light from the spectral plot for non-angled light for each color channel to generate red and blue normalized, composite color channel spectral plots of a specific wavelength scale and subtracting the normalized, composite blue channel spectral plot from the normalized, composite red channel spectral plot to generate a red-minus-blue (R-B) W spectral signature for the skin.

**18.** The system according to claim 17, wherein the (R-B)W spectral signature is further processed by:

  subtracting the spectral component due to angled white light from the (R-B) W spectral signature to generate a (R-B) W-P spectral signature; and
  comparing the intensity peaks from the (R-B)W spectral signature to the (R-B)W-P spectral signature.

**19.** The system according to any one of the above claims, wherein the image processor (10006) comprises a plurality of characteristic acne elements elimination unit for isolating sebaceous pore openings, sebaceous pore channel, sebaceous pore intersection, sebaceous gland intersection, blockage of sebaceous pore openings, contents of the sebaceous pore, determining age of sebum, whether the sebaceous gland is actively producing sebum, and a level of p-acne bacteria and isolating unhealthiness arising out of age of the sebaceous gland, inflammation around the gland, inflammation around the sebaceous pores, inflammation around the sebaceous gland, inflammation around hair follicles and level of p-acne bacteria.

**20.** The system according to any one of the above claims, wherein the characteristics of the skin are measured on at least one of: a discrete scale, and a continuous scale.

**21.** The system according to any one of the above claims, wherein the white light polarization device further comprises an output generator for determining a distance between minimum and maximum intensity positions in a convoluted red minus blue wavelength scale in the at least two unique convolutions to generate a numerical skin type output.

**22.** The system according to any one of the above claims, wherein the system comprises a marking unit for outlining and marking areas on the surface on the skin to thereby enable surgical excision of a skin structure.

**23.** A method, comprising:

providing an illuminator (10002) for illuminating a skin element;
providing an image capturing device (10004) and coupling the image capturing device to the illuminator;
providing an image processor (10006) and coupling the image processor to the image capturing device;
providing a color Red Green Blue (RGB) analysis device including a standard RGB (sRGB) color unit;
providing a white light polarization device and coupling the white light polarization device to the RGB analysis device;
providing an optical assessment unit (10008) and coupling the optical assessment unit to both the image capturing device and the image processor;
providing a digital color analysis device and coupling the digital color analysis device to both the white light polarization device and the RGB analysis device; and
imaging the portion of the surface of the skin;
processing the imaged portion of the surface of the skin and isolating sebaceous parameters pertaining to acne formation, including changes in the sebaceous glands and pores, by employing filter techniques to process the imaged portion of the surface of the skin;
generating at least two unique convolutions or spectral signatures by convoluting data of a first image captured using white light and a captured image captured using white light or polarized light to create the unique convolution or spectral signature
comparing extreme positions, comprising maxima and minima intensity positions, of the at least two unique convolutions or spectral signatures;;
generating a combination of color systems; and
determining a health status of the imaged portion of the surface on the skin, wherein
the health status comprises a level of acne and a predisposition of a portion of the skin to acne based on anatomical-physiological factors.

**24.** The method according to claim 23, further comprising:

providing a marking unit; and
marking areas on the surface on the skin.

**Patentansprüche**

**1.** System, umfassend:

einen Illuminator (10002) zum Beleuchten eines Hautelements;
eine Bilderfassungsvorrichtung (10004), die an den Illuminator gekoppelt ist, um den Abschnitt der Oberfläche der Haut abzubilden;
einen Bildprozessor (10006), der an die Bilderfassungsvorrichtung gekoppelt ist, um den abgebildeten Abschnitt der Oberfläche der Haut zu verarbeiten und Talgparameter, die Aknebildung betreffen, einschließlich Änderungen in den Talgdrüsen und Poren, zu isolieren, indem Filtertechniken zur Verarbeitung des abgebildeten Abschnitts der Oberfläche der Haut verwendet werden; und
eine optische Auswertungseinheit (10008), die sowohl an die Bilderfassungsvorrichtung als auch an den Bildprozessor gekoppelt ist, wobei die optische Auswertungseinheit ferner umfasst:

eine Farbanalysevorrichtung (rot, grün, blau (RGB)) einschließlich einer Standard-RGB (sRGB)-Farbeinheit;

eine Weißlicht-Polarisationsvornchtung, die an die RGB-Analysevorrichtung gekoppelt ist, um Extrempositionen, die Positionen mit maximaler und minimaler Intensität umfassen, von mindestens zwei eindeutigen Konvolutionen oder Spektralsignaturen zu vergleichen, wobei jede Konvolution oder Spektralsignatur generiert wird, indem Daten eines ersten Bildes, das unter Verwendung von Weißlicht erfasst wurde, und eines zweiten Bildes, das unter Verwendung von Weißlicht oder polarisiertem Licht erfasst wurde, gefaltet werden, um die eindeutige Konvolution oder Spektralsignatur zu erzeugen; und

eine Digitalfarbanalysevorrichtung, die sowohl an die Weißlicht-Polarisationsvorrichtung als auch die RGB-Analysevorrichtung gekoppelt ist, um eine Kombination von Farbsystemen zu generieren und um einen Gesundheitsstatus des abgebildeten Abschnitts der Oberfläche der Haut zu bestimmen, wobei der Gesundheitsstatus einen Schweregrad der Akne und eine Prädisposition eines Abschnitts der Haut für Akne basierend auf anatomisch-physiologischen Faktoren umfasst.

2. System nach Anspruch 1, wobei das einfallende Licht unpolarisiertes Licht umfasst, und Licht, das von dem Hautelement reflektiert oder erneut emittiert worden ist, polarisiertes Licht umfassen kann.

3. System nach Anspruch 1 oder Anspruch 2, wobei die beiden eindeutigen Konvolutionen einfallendes Licht, das senkrecht auf die Haut trifft, und einfallendes Licht, das in einem nicht-senkrechten Winkel auf die Haut trifft, umfassen.

4. System nach einem der Ansprüche 1 bis 3, wobei das einfallende Licht, das in einem nicht-senkrechten Winkel auf die Haut trifft, in dem Brewster-Winkel auftrifft.

5. System nach einem der obigen Ansprüche, wobei der Illuminator (10002) eine Lichtquelle oder mehr als eine Lichtquelle umfasst.

6. System nach einem der obigen Ansprüche, wobei die Lichtquelle Violettlicht im allgemeinen Bereich von etwa 385 nm umfasst.

7. System nach einem der obigen Ansprüche, wobei die Lichtquelle ein Licht oder eine Licht emittierende Diode (LED) ist, das bzw. die Strahlung mit einer Wellenlänge ausgewählt aus der Gruppe bestehend aus etwa 280 nm, 340 nm, 360 nm, 385 nm, 395 nm, 400 nm, 405 nm und 480 nm emittiert.

8. System nach einem der obigen Ansprüche, wobei das System brauchbar ist, um eine Prädisposition von Talgporen und Hautstrukturen um eine Talgdrüse herum, einen Schweregrad von Akne und eine Prädisposition eines Abschnitts der Haut, die Akne zu bessern oder zu verschlimmern, zu bestimmen.

9. System nach einem der obigen Ansprüche, wobei die optische Auswertungseinheit (10008) ferner einen Algorithmus zum Generieren einer Hautbedingungsauswertung umfasst.

10. System nach einem der obigen Ansprüche, wobei eine Ausgabe der optischen Auswertungseinheit (10008) Charakteristika der Aknebedingungen einschließt.

11. System nach Anspruch 10, wobei die Ausgabe eine Vielzahl von Aknebesserungs und -verschlimmerungsbedingungen mit einer Anzahl von Aknestatusergebnissen einschließt.

12. System nach einem der obigen Ansprüche, wobei die Ausgabe der optischen Auswertungseinheit (10008) mindestens eine der folgenden Aknebedingungseinheiten einschließt: geschlossen, teilweise geöffnet und geöffnet für die Talgporenöffnung; gefüllt, teilweise gefüllt und leer für Talgporeninhalte; blockiert, teilweise blockiert und durchgängig für eine Verbindung zwischen Drüse und Haar; gefüllt, teilweise gefüllt und leer für Talgdrüseninhalte; aktiv, teilweise aktiv und inaktiv für Talgdrüsenaktivität; und hoch, mittel, niedrig und keine für Entzündung.

13. System nach einem der obigen Ansprüche, wobei die Ausgabe des Systems eine Eigenschaft der Haut einschließt, wobei die Eigenschaft mindestens eine von Struktur, Form, Konzentration, Anzahl, Größe, Zustand und Stadium von mindestens einem der Folgenden ist: Melanozyt, Melanin, Hämoglobin, Porphyrin, Keratin, Carotin, Kollagen, Elastin, Hauttalg, Talgdrüsenaktivität, Poren (Schweiß und Talg), Feuchtigkeitsgrad, Elastizität, Leuchtkraft, Festigkeit, feine Linie, Fältchenzahl und -Stadium, Porengröße, Prozentsatz der offenen Poren, Hautelastizität, Hautspannungslinie, Fleck, Hautfarbe, Psoriasis, Allergien, roter Bereich, allgemeines Hautproblem oder allgemeine Hautinfektion, Tumor, Sonnenbrand, Ausschlag, Kratzer, Pickel, Akne, Insektenstich, Juckreiz, Blutung, Verletzung, Entzündung, Lichtschaden, Pigmentierung, Tonus, Tätowierung, prozentuale Verbrennung/Verbrennungsklassifizierung, Mal (Naevi, Naevus), Aspekt einer Hautläsion (Struktur, Farbe, Dimensionen/Asymmetrie), Melanom, dermal beobachtetes Problem, kutane Läsion, Cellulitis, Furunkel, blasenbildende Erkrankung, angeborenes Dermalsyndrom, (sub)kutane Mykosen, Melasma, Gefäßzustand, Rosacea, Teleangektasien, Textur, Hautulcer, Wundheilung, postoperative Nachverfolgung, Melanozytenläsion, Nichtmelanozytenläsion, Basalzellkarzinom, seborrhoische Keratose, Hauttalg (Öligkeit) und Besorgnis im Zusammenhang mit Nagel und/oder Haar.

14. System nach einem der obigen Ansprüche, wobei das System durch einen Computer gesteuert wird und auf Folgendes programmiert ist:

    Detektieren eines Polarisationsgrads des Lichts, das von der Haut reflektiert wird; und
    Bestimmen eines Hautzustands basierend auf einem Aspekt der Polarisation des reflektierten Lichtes.

15. System nach einem der obigen Ansprüche, wobei das System durch einen Computer gesteuert wird und auf Folgendes programmiert ist:

    Erhalten einer Baseline-Hautzustandsauswertung;
    Empfehlen eines Monitoring-Intervalls basierend auf mindestens einem von einem Hautpflegeziel, einem Produkt und einem Therapieschema;
    Erhalten einer zweiten Hautzustandsauswertung;
    Vergleichen der zweiten Auswertung mit der Baseline-Auswertung, um den Fortschritt in Richtung zu dem Hautpflegeziel zu bestimmen; und
    gegebenenfalls Optimieren des Therapieschemas oder Produkts, um einen Hautzustand zu verbessern.

16. System nach einem der obigen Ansprüche, wobei das System durch einen Computer gesteuert wird und auf Folgendes programmiert ist:

    Erhalten von Antworten auf eine Reihe von subjektiven Frage zu der Haut;
    Erhalten einer objektiven Hautanalyse unter Verwendung einer autbildgebungsvorrichtung
    algorithmisches Kombinieren der subjektiven und objektiven Ergebnisse, um einen Hautzustand zu erhalten.

17. System nach einem der obigen Ansprüche, wobei der Bildprozessor (10006) für Folgendes konfiguriert ist:

    Erfassen eines Bildes der Haut, die mit nicht im Winkel einfallendem Weißlicht beleuchtet wird, und eines Bildes der Haut, die mit in einem Winkel einfallendem Weißlicht beleuchtet wird;
    Generieren eines normalisierten roten und eines normalisierten blauen Farbkanalhistogramms für mindestens eines von einem reflektierten und emittierten Licht in jedem Bild;
    Korrelieren des normalisierten roten und des normalisierten blauen Farbkanalhistogramms mit einer Wellenlängenskala, um rote und blaue Farbkanalspektralauftragungen zu erhalten; und
    Kombinieren der roten und blauen Farbkanalspektralauftragungen, indem die Spektralauftragung für im Winkel einfallendes Licht von der Spektralauftragung für nicht im Winkel einfallendes Licht für jeden Farbkanal subtrahiert wird, um rote und blaue normalisierte zusammengesetzte Farbkanalspektralauftragungen einer spezifischen Wellenlängenskala zu erhalten, und Subtrahieren der normalisierten zusammengesetzten blauen Kanalspektralauftragung von der normalisierten zusammengesetzten roten Kanalspektralauftragung, um eine rot-minus-blau- (R-B)W-Spektralsignatur für die Haut zu generieren.

18. System nach Anspruch 17, wobei die (R-B)W-Spektralsignatur des Weiteren verarbeitet wird durch:

    Subtrahieren der durch im Winkel auftreffendes Weißlicht bedingten Spektralkomponente von der (R-B)W-Spektralsignatur, um eine (R-B)W-P-Spektralsignatur zu generieren; und
    Vergleichen der Intensitätspeaks von der (R-B)W-Spektralsignatur mit der (R-B)W-P-Spektralsignatur.

19. System nach einem der obigen Ansprüche, wobei der Bildprozessor (10006) eine Eliminierungseinheit für eine Vielzahl von charakteristischen Akneelementen zum Isolieren von Talgporenöffnungen, Talgporenkanal, Talgporenschnittpunkt, Talgdrüsenschnittpunkt, Blockade von Talgporenöffnungen, Inhalten der Talgpore, Ermitteln des Alters des Talgs, Ermitteln, ob die Talgdrüse aktiv Talg produziert, und einer Konzentration der p-Aknebakterien und zum Isolieren von Ungesundheit umfasst, die sich aus dem Alter der Talgdrüse, Entzündung um die Drüse herum, Entzündung um die Talgporen herum, Entzündung um die Talgdrüse herum, Entzündung um Haarfollikel herum und Konzentrationen der p-Aknebakterien ergibt.

20. System nach einem der obigen Ansprüche, wobei die Charakteristika der Haut auf mindestens einem von einer diskreten Skala und einer kontinuierlichen Skala gemessen werden.

21. System nach einem der obigen Ansprüche, wobei die Weißlicht-Polarisationsvorrichtung ferner einen Ausgabegenerator umfasst, um einen Abstand zwischen Positionen mit minimaler und maximaler Intensität in einer gefalteten rot-minus-blau-Wellenlängenskala in den mindestens zwei eindeutigen Konvolutionen zu ermitteln, um eine numerische Hauttypausgabe zu generieren.

22. System nach einem der obigen Ansprüche, wobei das System eine Markiereinheit zum Hervorheben und Markieren von Bereichen auf der Oberfläche der Haut umfasst, um dadurch chirurgische Exzision einer Hautstruktur zu ermöglichen.

23. Verfahren, umfassend:

Bereitstellen eines Illuminators (10002) zum Beleuchten eines Hautelements;
Bereitstellen einer Bilderfassungsvorrichtung (10004) und Koppeln der Bilderfassungsvorrichtung an den Illuminator;
Bereitstellen eines Bildprozessors (10006) und Koppeln des Bildprozessors an die Bilderfassungsvorrichtung;
Bereitstellen einer Farbanalysevorrichtung (rot, grün, blau (RGB)) einschließlich einer Standard-RGB-(sRGB)-Farbeinheit;
Bereitstellen einer Weißlicht-Polarisationsvorrichtung und Koppeln der Weißlicht-Polarisationsvorrichtung an die RGB-Analysevorrichtung;
Bereitstellen einer optischen Auswertungseinheit (10008) und Koppeln der optischen Auswertungseinheit sowohl an die Bilderfassungsvorrichtung als auch an den Bildprozessor;
Bereitstellen einer Digitalfarbanalysevorrichtung und Koppeln der Digitalfarbanalysevorrichtung an sowohl die Weißlicht-Polarisationsvorrichtung als auch an die RGB-Analysevorrichtung; und
Abbilden des Abschnitts der Oberfläche der Haut;
Verarbeiten des abgebildeten Abschnitts der Oberfläche der Haut und Isolieren von Talgparametern, die Aknebildung betreffen, einschließlich Änderungen in den Talgdrüsen und Poren, indem Filtertechniken zur Verarbeitung des abgebildeten Abschnitts der Oberfläche der Haut verwendet werden;
Generieren von mindestens zwei eindeutigen Konvolutionen oder Spektralsignaturen durch Falten von Daten eines ersten Bildes, das unter Verwendung von Weißlicht erfasst wurde, und eines erfassten Bildes, das unter Verwendung von Weißlicht oder polarisiertem Licht erfasst wurden, um die eindeutige Konvolution oder Spektralsignatur zu erzeugen,
Vergleichen von Extrempositionen, die Positionen mit maximaler und minimaler Intensität umfassen, von den mindestens zwei eindeutigen Konvolutionen oder Spektralsignaturen;
Generieren einer Kombination von Farbsystemen; und
Bestimmen eines Gesundheitsstatus des abgebildeten Abschnitts der Oberfläche der Haut, wobei
der Gesundheitsstatus einen Schweregrad der Akne und eine Prädisposition eines Abschnitts der Haut für Akne basierend auf anatomisch-physiologischen Faktoren umfasst.

24. Verfahren nach Anspruch 23, ferner umfassend:

Bereitstellen einer Markiereinheit; und
Markieren von Bereichen auf der Oberfläche der Haut.

**Revendications**

1. Système, comprenant:

un illuminateur (10002) permettant d'illuminer un élément cutané ;

un dispositif de capture d'image (10004) couplé à l'illuminateur afin de capturer en image la partie de la surface de la peau ;

un dispositif de traitement d'image (10006) couplé au dispositif de capture d'image afin de traiter la partie capturée en image de la surface de la peau et d'isoler des paramètres sébacés en lien avec une formation d'acné, y compris des modifications des glandes sébacées et des pores, en utilisant des techniques de filtrage pour traiter la partie capturée en image de la surface de la peau ; et

une unité d'évaluation optique (10008) couplée à la fois au dispositif de capture d'image et au dispositif de traitement d'image, l'unité d'évaluation optique comprenant en outre :

un dispositif d'analyse de couleur rouge, vert, bleu (RVB) comprenant une unité de couleur RVB normal (sRVB) ;

un dispositif de polarisation de lumière blanche couplé au dispositif d'analyse RVB afin de comparer des positions extrêmes, comprenant des positions d'intensité maximale et minimale, d'au moins deux convolutions ou signatures spectrales uniques, chaque convolution ou signature spectrale étant générée par des données de convolution d'une première image capturée en utilisant de la lumière blanche et d'une seconde image capturée en utilisant de la lumière blanche ou de la lumière polarisée pour créer la convolution ou signature spectrale unique ; et

un dispositif d'analyse de couleur numérique couplé à la fois au dispositif de polarisation de lumière blanche et au dispositif d'analyse RVB afin de générer une combinaison de systèmes de couleur et afin de déterminer un statut sanitaire de la partie capturée en image de la surface de la peau, dans lequel le statut sanitaire comprend un niveau d'acné et une prédisposition d'une partie de la peau à l'acné en se basant sur des facteurs anatomico-physiologiques.

2. Système selon la revendication 1, dans lequel la lumière incidente comprend une lumière non polarisée, et une lumière réfléchie ou réémise par l'élément cutané peut comprendre de la lumière polarisée.

3. Système selon la revendication 1 ou 2, dans lequel les deux convolutions uniques comprennent une lumière incidente qui est perpendiculaire à la peau et une lumière incidente formant un angle non perpendiculaire par rapport à la peau.

4. Système selon l'une quelconque des revendications 1 à 3, dans lequel la lumière incidente qui forme un angle non perpendiculaire par rapport à la peau forme l'angle de Brewster.

5. Système selon l'une quelconque des revendications précédentes, dans lequel l'illuminateur (10002) comprend une source lumineuse ou plus d'une source lumineuse.

6. Système selon l'une quelconque des revendications précédentes, dans lequel la source lumineuse comprend une lumière violette dans la plage générale d'environ 385 nm.

7. Système selon l'une quelconque des revendications précédentes, dans lequel la source lumineuse est une lumière ou une diode électroluminescente (DEL) émettant un rayonnement à une longueur d'onde sélectionnée parmi le groupe constitué d'environ 280 nm, 340 nm, 360 nm, 385 nm, 395 nm, 400 nm, 405 nm et 480 nm.

8. Système selon l'une quelconque des revendications précédentes, dans lequel le système est utile pour déterminer une prédisposition de pores sébacés et de structures cutanées situées autour d'une glande sébacée, un niveau d'acné, et une prédisposition d'une partie de peau à améliorer ou faire empirer l'acné.

9. Système selon l'une quelconque des revendications précédentes, dans lequel l'unité d'évaluation (10008) comprend en outre un algorithme permettant de générer une évaluation d'état de peau.

10. Système selon l'une quelconque des revendications précédentes, dans lequel une sortie de l'unité d'évaluation optique (10008) comprend des caractéristiques d'état acnéique.

11. Système selon la revendication 10, dans lequel ladite sortie comprend une pluralité d'états d'amélioration et de dégradation d'acné avec un certain nombre de résultats de statut d'acné.

12. Système selon l'une quelconque des revendications précédentes, dans lequel la sortie de l'unité d'évaluation optique (10008) comprend au moins un état parmi les suivants d'une unité d'état acnéique : fermé, partiellement ouvert et ouvert pour une ouverture de pore sébacé ; plein, partiellement plein et vide pour des contenus de pore sébacé ;

bloqué, partiellement bloqué et libre pour un raccordement glande et poil ; plein, partiellement plein et vide pour des contenus de glande sébacée ; actif, partiellement actif et inactif pour une activité de glande sébacée ; et élevé, intermédiaire, faible et nul pour une inflammation.

**13.** Système selon l'une quelconque des revendications précédentes, dans lequel la sortie du système comprend une propriété de la peau, dans lequel la propriété est au moins une parmi une structure, une forme, une concentration, un nombre, une taille, un état, et un stade d'au moins un parmi : mélanocyte, mélanine, hémoglobine, porphyrine, kératine, carotène, collagène, élastine, sébum, activité de glande sébacée, pore (sudoripare et sébacé), niveau d'humidité, élasticité, luminosité, fermeté, ridule, nombre et stade de ride, taille de pore, pourcentage de pores ouverts, élasticité de la peau, ligne de tension de la peau, tache, couleur de peau, psoriasis, allergie, couperose, trouble ou infection cutané(e) général(e), tumeur, coup de soleil, éruption cutanée, égratignure, bouton, acné, morsure d'insecte, démangeaison, saignement, blessure, inflammation, dommage lié à la lumière, pigmentation, teint, tatouage, pourcentage de brûlure/classe de brûlure, grain de beauté (naevi, naevus), aspect d'une lésion cutanée (structure, couleur, dimensions/asymétrie), mélanome, trouble dermique observé, lésion cutanée, cellulite, furoncle, phlyctène, syndrome dermique congénital, mycose (sous) cutanée, melasma, état vasculaire, acné rosacée, télangiectasie, texture, ulcère cutané, cicatrice, sonde postopératoire, lésion mélanocytique, lésion non mélanocytique, carcinome baso-cellulaire, verrue séborrhéique, sébum (aspect huileux), et problème ongulaire et/ou pileux.

**14.** Système selon l'une quelconque des revendications précédentes, dans lequel le système est commandé par un ordinateur et est programmé pour :

détecter un degré de polarisation de la lumière reflétée par la peau ; et
déterminer un état cutané en se basant sur un aspect de la polarisation de la lumière réfléchie.

**15.** Système selon l'une quelconque des revendications précédentes, dans lequel le système est commandé par un ordinateur et est programmé pour :

obtenir une évaluation de l'état cutané de référence ;
recommander un intervalle de surveillance en se basant sur au moins un parmi un objectif de soin cutané, un produit, et un régime ;
obtenir une deuxième évaluation d'état cutané ;
comparer la deuxième évaluation à l'évaluation de référence afin de déterminer un progrès en direction de l'objectif de soin cutané ; et
éventuellement, optimiser le régime ou le produit afin d'améliorer un état cutané.

**16.** Système selon l'une quelconque des revendications précédentes, dans lequel le système est commandé par un ordinateur et est programmé pour :

obtenir des réponses à une série de questions subjectives concernant la peau ;
obtenir une analyse cutanée objective en utilisant un dispositif d'imagerie dermique ; et
combiner les résultats subjectifs et objectifs de manière algorithmique afin d'obtenir un état cutané.

**17.** Système selon l'une quelconque des revendications précédentes, dans lequel le dispositif de traitement d'image (10006) est configuré pour
capturer une image de la peau illuminée par une lumière blanche incidente non inclinée et une image de la peau illuminée par une lumière blanche incidente inclinée ;
générer un histogramme de canal couleur rouge normalisé et un histogramme de canal couleur bleu normalisé pour au moins une parmi une lumière réfléchie et une lumière émise dans chaque image ;
corréler les histogrammes de canal couleur rouge normalisé et bleu normalisé avec une échelle de longueur d'onde afin d'obtenir des tracés spectraux de canal couleur rouge et bleu ; et
combiner les tracés spectraux de canal couleur rouge et bleu en soustrayant le tracé spectral pour une lumière inclinée du tracé spectral pour une lumière non inclinée pour chaque canal couleur afin de générer des tracés spectraux de canal couleur composites normalisés rouge et bleu pour une échelle de longueur d'onde spécifique et soustraire le tracé spectral de canal bleu composite normalisé du tracé spectral de canal rouge composite normalisé afin de générer une signature spectrale rouge moins bleu (R-B)W pour la peau.

**18.** Système selon la revendication 17, dans lequel la signature spectrale (R-B)W est en outre traitée par :

Soustraction, de la signature spectrale (R-B)W, de la composante spectrale due à la lumière blanche inclinée afin de générer une signature spectrale (R-B) W-P ; et

comparer les pics d'intensité issus de la signature spectrale (R-B)W à la signature spectrale (R-B)W-P.

**19.** Système selon l'une quelconque des revendications précédentes, dans lequel le dispositif de traitement d'image (10006) comprend une pluralité d'unités d'élimination d'éléments acnéiques caractéristiques permettant d'isoler des ouvertures de pore sébacé, un canal de pore sébacé, une intersection de pore sébacé, une intersection de glande sébacée, un blocage d'ouvertures de pore sébacé, des contenus du pore sébacé, une détermination de l'âge du sébum, le fait que la glande sébacée produit activement du sébum ou non, et un niveau de bactérie P. acnes, et d'isoler une insalubrité découlant de l'âge de la glande sébacée, une inflammation autour de la glande, une inflammation autour des pores sébacés, une inflammation autour de la glande sébacée, une inflammation autour des follicules pileux et un niveau de bactérie P. acnes.

**20.** Système selon l'une quelconque des revendications précédentes, dans lequel les caractéristiques de la peau sont mesurées sur au moins une parmi : une échelle discrète, et une échelle continue.

**21.** Système selon l'une quelconque des revendications précédentes, dans lequel le dispositif de polarisation de lumière blanche comprend en outre un générateur de sortie permettant de déterminer une distance entre des positions d'intensité minimale et maximale dans une échelle de longueur d'onde à convolution rouge moins bleu dans les au moins deux convolutions uniques pour générer une sortie numérique de type cutané.

**22.** Système selon l'une quelconque des revendications précédentes, dans lequel le système comprend une unité de marquage permettant de dessiner et marquer des zones sur la surface de peau afin de permettre ainsi une excision chirurgicale d'une structure cutanée.

**23.** Procédé, comprenant les étapes consistant à :

fournir un illuminateur (10002) permettant d'illuminer un élément cutané ;
fournir un dispositif de capture d'image (10004) et coupler le dispositif de capture d'image à l'illuminateur ;
fournir un dispositif de traitement d'image (10006) et coupler le dispositif traitement d'image au dispositif de capture d'image ;
fournir un dispositif d'analyse de couleur rouge vert bleu (RVB) comprenant une unité de couleur RVB normal (sRVB) ;
fournir un dispositif de polarisation de lumière blanche et coupler le dispositif de polarisation de lumière blanche au dispositif d'analyse RVB ;
fournir une unité d'évaluation optique (10008) et coupler l'unité d'évaluation optique à la fois au dispositif de capture d'image et au dispositif de traitement d'image ;
fournir un dispositif numérique d'analyse de couleur et coupler le dispositif numérique d'analyse de couleur à la fois au dispositif de polarisation de lumière blanche et au dispositif d'analyse RVB ; et
capturer en image la partie de la surface de la peau ;
traiter la partie capturée en image de la surface de la peau et isoler des paramètres sébacés en lien avec une formation d'acné, y compris des modifications des glandes sébacés et des pores, en utilisant des techniques de filtrage pour traiter la partie capturée en image de la surface de la peau ;
générer au moins deux convolutions ou signatures spectrales uniques par convolution de données d'une première image capturée en utilisant de la lumière blanche et d'une image capturée en utilisant de la lumière blanche ou de la lumière polarisée pour créer la convolution ou la signature spectrale unique ;
comparer des positions extrêmes, comprenant des positions d'intensité maximale et minimale, des au moins deux convolutions ou signatures spectrales uniques ;
générer une combinaison de systèmes de couleur ; et
déterminer un statut sanitaire de la partie capturée en image de la surface de la peau, dans lequel le statut sanitaire comprend un niveau d'acné et une prédisposition d'une partie de la peau à l'acné en se basant sur des facteurs anatomico-physiologiques.

**24.** Procédé selon la revendication 23, comprenant en outre les étapes consistant à :

fournir une unité de marquage ; et
marquer des zones sur la surface de la peau.

FIG. 1

FIG. 2

FIG. 3

EP 2 389 573 B1

FIG. 4B

400

FIG. 4A

400

98

**MySkinRecord**

Name: Sally Myers, 24 yo, F, New York
Skin Type: IV/OSTP

<u>Take</u>
<u>SkinTypeTest</u>

| MySkinProducts | MySkinHistory | | **Review** |

Body Part : Left Forearm
Photo: 5
First Photo:      7 July 2006
Latest Photo     20 July 2007

Next Photo:
10 August 2007

Add new Reminder

Rollover:        [Condition]
Date last Photo:  24 Feb 07
Next Photo:     18 Aug 07

4 of 4

3 of 4

2 of 4

Date: 7 July 2006        1 of 4

Category

7.7.06      7.7.06      7.7.06      7.7.06      10.8.06
①      ②      ③      ④      Ⓝ

Create Report

**FIG. 5**

EP 2 389 573 B1

# INTERACTIVE TOOLS TO MODEL IMPACT OF AGE, AND DIFFERENT COSMETIC REGIMENS

300

| MYSKIN PRODUCTS | MYSKIN HISTORY | COMPARE |

TODAY

10 YEARS (DROPDOWN)

| 20 | AGE | 85 |
| 0/IN | WRINKLES | 5/IN |
| 20% | MOISTURE | 15% |
| 30 | ELASTICITY | 15 |
| 10 | LUMINOSITY | 5 |

LEARN HOW THE SKIN IS CHANGING

Skin Cross Sectional Anatomy

FIG. 6

**700**

# RECOMMENDATIONS ON BEST PERSONALIZED PRODUCTS WILL BE PROVIDED

MYSKINPRODUCTS | MYSKINHISTORY

**COMPARE**

SKIN

MOISTURE

SKIN

NORMAL

DECLINE

MAX

RECOVERY

NORMAL

AGING

◇ NORMAL
□ Poor Skin Care
△ Positive Skin Care

POOR SKIN

CARE

MOISTURE

RECOMMENDED PRODUCTS FOR MOISTURE
RECOVERY
ABC CREAM
XYZ
123

**FIG. 7**

EP 2 389 573 B1

**800**

My Skin

Enabling Personalized Skin Care

[Register] [Sign In]

| Home | Products | Spas/Salons | Doctors | Skin Concern Analysis |

*Sample Report*

For me, a 27 year old, single, white women, living in New York, the most popular creams for moisturizing were:

| *Nivea:* | | 9/10 |
| *L'oreal:* | | 7/10 |
| *Olay:* | | 6/10 |

*Learn what works best for YOUR skin:*

*My sex is* O *Male*    O *Female*

*My date of birth is* [1994 ▼]

*My location is (enter ZIP code)* [          ]

*My skin color is (select the closest match, roll over for more info)* [Still unsure?]

O    O    O    O    O    O

*What is the best product to*

O *Moisturize* O *Protect* O *Cleanse* O *Beauty*

[Find]

| About MySkin Inc | Information | Careers | Disclaimer |

*"This is the best site!*
*ever seen"*

Kathy Morgan, San Francisco

**FIG. 8**

900

My Skin

Enabling Personalized Skin Care

Register | Sign In

| Home | Products | Spas/Salons | Doctors | Skin Concern Analysis |

For: 27 year old, Female, in NY Area, Skin Type V

*"This is the best site! ever seen"*

Kathy Morgan, San Francisco

| Type | Website | Brand | Product | Price | Popularity |
|------|---------|-------|---------|-------|------------|
| Moisturizer | www.skin caresupply company.com | L'oreal | L'oreal Day Cream | ****** | 400 links |
| Moisturizer | www.skin products.com | Nivea | Daily Adventures | $20-580 | 250 links |

**Recommend another product:**

Brand:     [ Item 1 ▼ ]     other:[                    ]

Product:   [ Item 1 ▼ ]     other:[                    ]

Goal:      [ Item 1 ▼ ]

Your Rating:    O Poor    O Fair    O Average    O Good    O Great

Comments:
(Optional)

[                                    ]

[ Submit ]          [ Forward to Your Friend ]

[ About MySkin Inc ] [ Information ] [ Careers ] [ Disclaimer ]

**FIG. 9**

**Myskin**      address your skin concern from the convenience and privacy of your home

| Welcome, Milena | Log out |
|---|---|

**MySkin Home**

You are Main User, and you have 2 Family Users.

**MySkin Info:**

| | |
|---|---|
| **Select User:** | Milena |
| **Relationship:** | Self |
| **First Name:** | Milena |
| **Second Name:** | Milovanovic |
| **Sex:** | Female |
| **Occupation:** | Student |
| **Birth Date:** | 08/08/1975 |
| **ID:** | 1234567 |

**Home Address:**

Kraljice Marije 16/5

Belgrade, Serbia

| **Phone Number:** | +381 11 1234567 |
|---|---|
| **Fax Number** | |
| **E-mail:** | milena@srd.com |

( Update MySkin Info )

( Regiser a New Family Member )

**Skin History**

| Lesion ID | Skin Area | Report Status | |
|---|---|---|---|
| 08-08-2006-001 | Upper Left Arm | Analysis Complete | 📄 |
| 08-08-2006-002 | Face | Analysis Complete | 📄 |
| 22-08-2006-003 | Upper Left Arm | Analysis Complete | 📄 |
| 01-09-2006-004 | Right Leg | In-Progress | |
| 01-09-2006-005 | Skin Health Test - Face | Week 4 to 6 | |

1 | 2 | Show All

Currently showing last 5 submitted lesions.

( Skin Health Test )

( Submit a New Skin Concern )

( Forward Analysis )

( Ask an Expert )

( Payment Info/History )

**FIG. 10**

EP 2 389 573 B1

1100 ⌐

**Myskin**  address your skin concern from the convenience and privacy of your home

**Welcome, Milena**  |  **Log out**

**Skin Health Test**  You are Main User, and you have 2 Family Users.

# Welcome to your Skin Health Test

Many products work differently for different people. Skin Types, Skin Color, Lifestyle all play a role in how effective a product meets your needs.

**Here, we can baseline your skin's health through 4 key areas:**

- Elasticity    Elasticity is based on...and is influenced by .......
- Wrinkles / Fine Line    ....
- Sun Damage    ....
- Glow / Luminosity    ....

Using the latest in skin technologies, we analyze and give you a personalized assessment of your skin regimen

[ Begin ]

[ Cancel ]

**FIG. 11**

EP 2 389 573 B1

**1200**

**Myskin** address your skin concern from the convenience and privacy of your home

**Welcome, Milena** | **Log out**

*Skin Health Test*

You are Main User, and you have 2 Family Users.

| Basic Info | Test Purpose | Current Usage |

**Select User:** Milena

Skin type:

Skin Analysis

**For your Skin Health Test, where do/will you use the product.**
- ☐ Face
- ☐ Hands
- ☐ Neck
- ☐ Legs
- ☐ Torso

**How long have been using your product?**
- ☐ Never - just purchased
- ☐ 0-2 weeks
- ☐ <1 month
- ☐ 1-4 months
- ☐ >6 months

**Why are you using your product? (Select all that apply)**
- ☐ To protect
- ☐ To repair

**How often do you apply your Product? (Check all theat apply)**
- ☐ Daily
- ☐ In the Morning
- ☐ During the day
- ☐ At Nighttime

Email Notification?
Email:
SMS Notification?
Mobile Phone:

**Why are /will you be using your product? (Select all that apply)**
- ☐ Reduce Wrinkles / Fine Lines
- ☐ Increase Shine / Luminosity
- ☐ Increase Softness / Elasticity

**When do you apply your product? (Check all that apply)**
- ☐ As needed
- ☐ In the Morning
- ☐ During the day
- ☐ At Nighttime
- ☐ After washing hands

Submit and Begin Scan

Cancel

**FIG. 12**

EP 2 389 573 B1

**FIG. 13**

**1400**

EP 2 389 573 B1

# Myskin
### address your skin concern from the convenience and privacy of your home

**Welcome, Milena** | **Log out**

**Skin Health Test**

You are Main User, and you have 2 Family Users.

| **Skin Category** | **Measure** | **Description** | **Completed 1 of 6 Samples** |
|---|---|---|---|

**Wrinkles**

12 wsx

Wrinkle Defination
...xalskjfblaksjdbfikajsbifkj
your wrinkle level is below average for
someone your age. Wrinkles are
caused x,y,z,...

[More Info]

Last Picture: Today

**Elasticity**

.7 esx

Elasticity Defination
...asdasjhdlkajshdlakjsdhlkajshd
your elasticity is above average for
your age

[More Info]

Next Picture: in 7 days

**Congratulations, you have
completed your first
baseline assessment!**

**Luminosity**

233 lsx

Description of how your skin level
compares to healthy skin at your age
level...

[More Info]

Continue to use your
product in according to
your regimen and rescan
your skin in the next 7
days

**Firmness**

1.5 fsx

Description of how your skin level
compares to healthy skin at your age
level...

[More Info]

**Tightness**

4 tsx

Description of how your skin level
compares to healthy skin at your age
level...

[More Info]

[Take Next Picture]
[MySkin Record]
[Cancel]

**FIG. 14**

**1500**

EP 2 389 573 B1

**Myskin**  address your skin concern from the convenience and privacy of your home

**Welcome, Milena** | **Log out**

*Skin Health Test*  You are Main User, and you have 2 Family Users.

| Skin Category | Trend | Description | Completed 3 of 6 Samples |
|---|---|---|---|

**Wrinkles** 1502  12 wsx ⬇  This shows an intermediate analysis on how the user is doing (is the trend okay?)  More Info

Last Picture: 3 days ago

**Elasticity**  .7 esx ⬆  This shows an intermediate analysis on how the user is doing (is the trend okay?)  More Info

Next Picture: in 4 days

**Luminosity**  233 lsx ↖  This shows an intermediate analysis on how the user is doing (is the trend okay?)  More Info

**Firmness**  1.5 fsx ➡  This shows an intermediate analysis on how the user is doing (is the trend okay?)  More Info

Take Next Picture

Return to MySkin Record

Cancel

**Tightness**  4 tsx ↗  This shows an intermediate analysis on how the user is doing (is the trend okay?)  More Info

**FIG. 15**

**1600**

**Myskin**        **address your skin concern from the convenience and privacy of your home**

**Welcome, Milena**        **Log out**

*Skin Health Test*        **You are Main User, and you have 2 Family Users.**

| Overall Analysis | Your Goal | Your Product's assessment |
|---|---|---|

SX

.Time

.XXXX

Your Product

Wrinkles    12 wsx

Elasticity    .7 esx     . Effective

Luminosity    233 lsx

Firmness    1.5 fsx

Tightness    4 tsx

Step-by-Step Analysis

Cancel

Print Full Report

**FIG. 16**

**Myskin**  address your skin concern from the convenience and privacy of your home

**Welcome, Milena** | **Log out**

*Skin Health Test* · | You are Main User, and you have 2 Family Users.

**Elasticity**

**Objective**

· Congratulations, your regimen has improved elasticity

**Other products to Consider**

· you want a product that can ..XXXX.Xxx.xxx.

· With the following ingredients ..XXXX.Xxx.xxx.

**Top Products used by other users for this objective**

· Giselle by L'Oreal
· 2069 users

· Clarins Day Cream
· 2069 users

More Products

**Your Product / Regimen**

· Congratulations, your regimen has improved elasticity

B 1 2 3 4 F

**Next Indicator**

**Return to Summary**

EP 2 389 573 B1

**FIG. 17**

1800

**HOME PAGE
FUN/SERIOUS**

**HOME PAGE
SERIOUS**

**INFO FOR ME**

**HOME PAGE
FUN/REVIEW**

**REPORT: WHAT IS THE
BEST PRODUCT FOR
ME?**

RATE AND
GO TO NEXT

**HOME PAGE
CONTRIBUTE**

**BLOGS BY PRODUCT
OR SKIN TYPE**

NO
REGISTRATI
ON
REQUIRED

**ALL PRODUCTS/PAGES
CAN LINK THROUGH
MYBEAUTYSHELF**

PARTIAL
INFO IF NOT
REGISTERED

**REPORT: WHAT DID
PEOPLE THINK?**

REQUIRES
REGISTRATION

**FIG. 18**

EP 2 389 573 B1

**MySkin**

Your unbiased Skin care adviser.

| **REVIEW** | MY EXPERIENCE | WHAT WORK | INFO FOR ME | CHECKOUT |

## FOR FUN

What I'm using to reduce redness

LayD*
age: 32
gender: female
location: San Diego CA
skin type: dry / sensitive
skin color: white
goal: reduce redness

Brand
Clinique
Product
Redness Solution:
Daily Relief Cream

LayD' comments:

I brought this because it's targeted at my specific problem. It works, but sometimes I feel mild stinging since I started using it. Is this ok? Is there anything better I can use?

What do you think?

☆ ☆ ☆ ☆ ☆

Comment:
Terrible!

Recommend a better product
Brand (Lancome ▽)
Product (Aqua Fusion SPF 15 ▽)

(SUBMIT)

## FOR ME

Help me select the best skin care product for me...

Age:[    ]  Gender: (Female ▽)

Skin Type:

Oily ⌐ T T T O Dry

Sensitive O T T T ⌐ Normal

Skin Color: (automatically detected)

Goal: (Anti-Aging/wrinkles/tightness ▽)

Current Brand and product:

(Clinique ▽) (Redness Solution ▽)

Detailed Skin Assessment
(for more accurate results)...

(SUBMIT)

My BeautyShelf

"I feel so drawn to this site. There are many products around and that used to make me very insecure. Everything's changed with this site!"
Queen, Victoria

3

**1900**

**FIG. 19**

EP 2 389 573 B1

**MySkin** — Find the best product for my skin !

Register Login

| REVIEW | MY EXPERIENCE | WHAT WORK | INFO FOR ME | CHECKOUT |

Ask Others...

**LayD***

age: 32
gender: female
location: San Diego CA
skin type: dry / sensitive
skin color: white
goal: reduce redness

What I'm using to reduce redness

Brand
Clinique
Product
Redness Solution
Daily Relief Cream

SKIP ▷

What do you think?

☆1 ☆2 ☆3 ☆4 ☆5

Comment:
Terrible!

Recommend a better product

Brand
Lancome ▽

Product
Aqua Fusion SPF 15 ▽

SUBMIT

My BeautyShelf

"I feel so drawn to this site. There are many products around and that used to make me very insecure. Everything's changed with this site!"
Queen, Victoria

3

2000

EP 2 389 573 B1

FIG. 20

FIG. 21

EP 2 389 573 B1

**FIG. 22**

EP 2 389 573 B1

2300

## FIG. 23

**MySkin**

Find the best product for my skin!

Register Login

| REVIEW | MY EXPERIENCE | WHAT WORKS | **INFO FOR ME** | CHECKOUT |

WHAT WORKS FOR PEOPLE LIKE ME IN.... [Reduce Redness ▾]

Brand
Clinique

Product
Redness Solution:
Daily Relief Cream

[NEXT △]

TIPS FOR TOMORROW, 06 OCTOBER 2007 FOR LayD*

Location: San Diego, CA, USA
Weather: (89° F) sunny throughout the day
UV Rating: 8

Make sure you use plenty of moisturiser with high SPF. Avoid exposure to the sun between 10am and 3 pm.

Comment:

[SEND TO COMMUNITY]
[SEND TO FRIENDS]

My BeautyShelf

NEW PRODUCTS COMING SOON FOR.... [Reduce Redness ▾]

| Product | | Claim | Price | Buzz? |
|---|---|---|---|---|
| | Brand Aveda — Product Tourmaline charged hydrating cream | [GO] | $25.00 | [YES] |
| | Brand Lancome — Product Aqua Fusion SPE 15 | [GO] | $22.50 | [YES] |
| | Brand The Body Shop — Product Wise Women day regenerating cream | [GO] | $19.99 | [YES] |
| | Brand Elizabeth Arden — Product Millennium night renewal cream | [GO] | $17.25 | [YES] |

2400

| My BeautyShelf | | | | | |
|---|---|---|---|---|---|
| | Morning | Evening | Sun + Fun | Just in Case | |
| Moisturize | | | CLARINS | | |
| Anti-Aging | LAMER | MySkin | | | |
| UV Protection | | LAMER | | | |

Pop-Up (For each item)

Clarins Day Cream 50 ml

Effectiveness for you: [1-5]
Ingredients:
SuggesstedUsage
Expiration Date: **Nov 2008**
<Click> to Blog This

**FIG. 24**

FIG. 25

**MySkin**
Your Trusted Skincare Advisor

EP 2 389 573 B1

Enabling Personalized Skin Care

| Register | Sign In |

| Spas | Salons | Doctors | My samples |

Tag bar --- shows your personalization info is (Age, Skin Type, SkinTone, etc)- constant over pages once you've logged in or given initial information

Ads

**Your Skin Care Priorites**

1. Moisturized
2. Reduce Wrinkles
3. Acne reduction
4. Glow
5. Firmness

Organize by: Routine      Goal

| | | Clean (Cleanse, Tone, Exfoliate) | | Protect (Moisturize, Sun, AntiAge, Firmness) | | Repair (Wrinkles, Cellulite, Blemishes) | |
|---|---|---|---|---|---|---|---|
| Your Cabinet | | | Name | | Name | | Name |
| | | | O | | O | | O |
| | | | Name | | Name | | Name |
| | | | O | | O | | O |
| | | | Name | | Name | | Name |
| | | | O | | O | | O |
| Your Added Favorites | | | Name | | Name | | Name |
| | | | O | | O | | O |
| Friends say, try this... | | | Name | | Name | | Name |
| | | | O | | O | | |

| | Clean | Protect | Repair |
|---|---|---|---|
| Receive Price Alerts | ☑ | ☑ | ☑ |
| New Product Launches | ☑ | ☑ | ☑ |
| New User Comment Alert | ☑ | ☑ | ☑ |

**FIG. 26**

| Page ID | 1.6 | Page Title | Registration |
|---------|-----|-----------|--------------|
| Date | 8 Aug 07 | Version | 1.0 |

**MySkin**
Your Trusted Skincare Advisor

Enabling Personalized Skin Care

| Register | | Sign In |

Spas          Salons          Doctors          My samples

**Your Info**
Email Address:
Password:
Screen name:
Age: [Prefilled]
Sex [ Prefilled]
Zip: [Prefilled]

**Security Code to register**

☐ Opt-In to MySkin Alerts
☐ Notify Me of New Samples for Products that benefit me
☐ Add to Facebook
☐ Add to Yelp
☐ Add to RSS

**Your Goals**
(Check all that apply)

**Your Skin concerns**
(Check all that apply)

**Your Skin Type**

(Link: Take Questionn aire)

**Free Sample Registration**

Address Info

Notify of new samples for
- Skin Type
- Product Launches
- Free offers

FIG. 27

| Page ID | 1.7 | Page Title | Product Analytics for My Skin Type |
|---|---|---|---|
| Date | 8 Aug 07 | Version | 1.0 |

**MySkin**
Your Trusted Skincare Advisor

Enabling Personalized Skin Care

Register    Sign In

| Spas | Salons | Doctors | My samples |
|---|---|---|---|

Tag bar---shows what your personalization info is (Age, SkinType,SkinTone, etc) - constant over pages once you've logged in or given initial information

MyBeautyShelf (1.5)

**People in your Category**

| Sex: | Women: | 1,203,200 |
|---|---|---|
| Age group | 20 - 25]: | 500,302 |
| Skin Type | [Oily, Complex] 100,000 | |
| Skin Concerns | [Acne, Blemish] 50,000 | |

| Your Goals | Most Popular | Most new Buzz | Blogs |
|---|---|---|---|
| Moisturize | L'oreal etecetc | | |
| Sunscreen | L'oreal etcetc | | |
| Protect | L'oreal etc | | |
| Glow | L'oreal etc | | |

**FIG. 28**

## Mobile Content Map

2900

**FIG. 29**

**3000**

| MySkinMobl - Home Page |
|---|
| Scan a Product... |
| Help me find something... |
| SunCheck |
| **[Incoming Alert]** |
| [Options]          Return |

MySkin - How good is the product?

Put product barcode in picture

*Live Photo*

[Enter Manually]
<Return>          <Scan>

MySkin - Product Info

Name: L'oreal Day Cream
MySkin Rating: 5/10 [Details]
            Wrinkles: 5

Safety: 8 Green [Details]
Friend Rating: yay! 9.5 [more]
Tell your friends
Rate it
Online Best Price: $100 [BUY]
Local Retailers

MySkin - Product Info

Name: L'oreal Day Cream
MySkin Rating: 5/10 [Details]
            Wrinkles: 5

MyGoal1: wrinkle Reduction
Wrinkles: 5
Alternatives...

MySkin - Select Product

Manufacturer: [Drop Down]

Name: [Drop Down]

MySkin - Purchase Online

Top Retailers
Ebay.com, Price: $100
Qty[1]: Price:[$100] + Ship
Rating: *** [Buy]
Creams are Us.com, Price: $95
Qty[1]: Price:[$100] + Ship
Rating: *** [Buy]

**FIG. 30**

**3100**

**MySkinMobl - Home Page**

Scan a Product...

Help me find something...

SunCheck

**[Incoming Alert]**

| Options | Return |

---

**MySkin**

Gifts

Self

Update my Pick List
(go to options)

---

**MySkin  -Gifts**

Select your Friend:

Susie

Jane

Marija

Vesna

[Add new
Friend/Goal]

---

**MySkin – Gifts-
Occasion**

Birthday

Skincare

Makeup

Foundation

Other

---

**MySkin -  Self**

Product 1: L'oreal
day cream

Benefit:; 6/10 works
great  [ locate]

Product 2: Chivon
Night Cr

Benefit: 5/10

Product 3:

Product 4:

---

**MySkin -  Self**

Find something for

[Goal] (Dropdown)   –
Wrinkle/Moisture/Loo
ks/etc)

How does this new
product compare?

EP 2 389 573 B1

**FIG. 31**

3200

**MySkinMobl - Home Page**

Scan a Product...
Help me find something...
SunCheck

[Incoming Alert]

| Options | Return |

---

**MySkin – SunCheck**

Location: **Budva, CG**

Current UV Rating: **4/10**

MySkin Sun Impact: **HIGH**

Advice: Stay covered
**[More]**

Max Exposure:

Current time in sun: 10:00
(Start/Stop/Clear)

---

**MySkin – UV advice**

Best is to stay covered
during this weather. Based
on your skin type, you have
a high propensity to burn.

Max recommended time is:
10 minutes

**Dr. Sun Doctor says...**

---

**MySkin – Dr Sun Doctor
says...**

Video....

EP 2 389 573 B1

**FIG. 32**

ALERT – ADVICE NEEDED

**3300** ⟍

MySkinMobl - Home Page

Scan a Product...
Help me find something...
SunCheck

[Incoming Alert]

| Options | Return |

**MySkin - Alert**

[Julie] wants you to rate:
Product: L'oreal Skin Balm

Cool / Not Cool / Chat

**FIG. 33**

EP 2 389 573 B1

**3400**

**3402**

MySkinMobl - Home Page

Scan a Product...
Help me find something...
SunCheck

[Incoming Alert]

| Options | Return |

---

**3404**

MySkin

MyFriend List [DropDowm]

MyPick List [Show List]

MyAlerts [Add Alerts]

Address Setup

---

**Options**

**3408**

MySkin - MyFriend

{Pict} Susie -
Online!
Likes ---

{Pict} mary -
Online!
Likes ---

[Add]

---

**3410**

MySkin - MyAlerts

Notify my Friends when I
buy something new [On/Off]

Notify me when my Friends
buy something [On/Off]

Alert new products when
good for me [On/Off]

---

**3412**

• Drop down
  • Wrinkles
  • Moisture
  • Acne
  • [All]
(prioritized by their needs)

---

MySkin - MyPick List

Category [DropDown]

---

**3414**

MySkin - Address/Pmt
Setup

Current Shipping address

Current Billing Info

Allow [one - click] to buy

---

**FIG. 34**

3500

3502 — Take Digital Image with Non-angled Light

3508 — Generate RGB Histograms for Red and Blue Color Channels and Normalize to Intensity of Light

3512 — Correlate Histogram with Wavelength Scale to Generate Spectral Plot

3504 — Take Digital Image with Angled Light

3510 — Generate RGB Histograms for Red and Blue Color Channels and Normalize to Intensity of Light

3514 — Correlate Histogram with Wavelength Scale to Generate Spectral Plot

3518 — Subtract Spectral Plot for Angled Light from Spectral Plot for Non-angled Light for Each Color Channel

3520 — Subtract Blue Channel Composite Spectral Plot from Red Channel Composite Spectral Plot

FIG. 35

**FIG. 36**

FIG. 37

**FIG. 38**

EP 2 389 573 B1

FIG. 39

BLUE - WHITE and POLARIZED

4002

4004

Wavelength (nm)

FIG. 40A

Intensity

Normalized Spectral Intensity (between 0 to 1)

4000

465 470 475 480 485 490 495 500

-0.02 0 0.02 0.04 0.06 0.08 0.1 0.12 0.14

134

EP 2 389 573 B1

**RED - WHITE and POLARIZED**

4000

**FIG. 40B**

FIG. 41A

EP 2 389 573 B1

4100

**RED - WHITE minus POLARIZED**

**FIG. 41B**

FIG. 42

EP 2 389 573 B1

FIG. 43

4300

139

(R-B)&(W-P)
126

4400A

FIG. 44A

Wavelength Difference (nm)

Intensity

EP 2 389 573 B1

4400B

FIG. 44B

**4500A** ➘

(R-B)&(W-P)
Aluminium

FIG. 45A

FIG. 45B

EP 2 389 573 B1

FIG. 45C

FIG. 46A

EP 2 389 573 B1

FIG. 46B

**4700**

Skin Care Device

**4702** Radiation Source

**4704** Radiation Detector

**4708** Skin Condition Analysis Module

**4710** Skin Care Regiment Recommendation Module

**4712** Skin Care Regiment Effectiveness Module

**4714** User Interface

**FIG. 47**

EP 2 389 573 B1

EP 2 389 573 B1

**4800**

4808

4810

4804

4812

4802

**FIG. 48**

FIG. 49

FIG. 50

**Description of Positive and Negative Intensities of Waveforms**

FIG. 51

EP 2 389 573 B1

FIG. 52A

FIG. 52B

FIG. 53A

EP 2 389 573 B1

FIG. 53B

**WhiteBlue (WB) and PolarizedWhiteBlue (PB) of Skin Sample (S) from Light Source (O)**

**Normal Non-pigmented Skin**

**WB (S - O)**
**FOREHEAD normal**

5400A

5401

**FIG. 54A**

### WhiteBlue (WB) and PolarizedWhiteBlue (PB) of Skin Sample (S) from Light Source (O)

#### Normal Non-pigmented Skin

5400B

PB (S - O)
FOREHEAD normal

5402

**FIG. 54B**

**WhiteBlue (WB) and PolarizedWhiteBlue (PB) of Skin Sample (S) from Light Source (O)**

Normal Benign Pigmented Skin Lesion

5400C

WB (S - O)

5403

2534

FIG. 54C

# WhiteBlue (WB) and PolarizedWhiteBlue (PB) of Skin Sample (S) from Light Source (O)

## Normal Benign Pigmented Skin Lesion

5400D

5404

PB (S - O)
2534

**FIG. 54D**

EP 2 389 573 B1

FIG. 54E

**WhiteBlue (WB) and PolarizedWhiteBlue (PB) of Skin Sample (S) from Light Source (O)**

Malignant Pigmented Skin Lesion

PB (S - O)
2746

5400F

5406

**FIG. 54F**

**5500**

# Automated tracking system

Frame by frame comparison to determine direction of camera movement and placement

FIG. 55

EP 2 389 573 B1

Image of area to be excised

5602

5604

5610

5612

5608

**FIG. 56**

EP 2 389 573 B1

**5700** ⌐⟍

Image of area to be excised

5702 ⌐⟍

Analysis of Image

5704 ⌐⟍

Manually select border area

5708 ⌐⟍

Automatic Border area selection

5710 ⌐⟍

Draw border area

**FIG. 57**

EP 2 389 573 B1

5800

## Flowchart Illustrating a Process for, in Accordance with an Aspect of the Present Technique.

5810
| Capturing the Image |
| --- |

5820
| Image Analysis in Pixel by Pixel Manner |
| --- |

5830
| Conversion of Obtained Colors from Device Dependent RBG to Device Independent sRGB Color System |
| --- |

5840
| Storing Standard R, G, and B Values in their Range of Values between 0 and 225 |
| --- |

5850
| Approximate of Color Using Gaussian Normal Distributions |
| --- |

5860
| Generation of Estimated Values of Mathematical Expectation and Standard Deviation |
| --- |

5870
| Above Parameters are Equated to Fitzpatrick Notation Using the Decision Tree for Determination of Skin Phototype |
| --- |

## FIG. 58

EP 2 389 573 B1

## Diagram depicting pixel view of an acquired digital of a part of person's skin

**FIG. 59**

**6000**

**Diagram depicting pixel view of an acquired digital of a part of person's skin after quantization**

**FIG. 60**

Diagram depicting a Histogram / Distribution of R, G and B colors on one of the taken photographs of a patient
Whose skin phototype is classified as type III by Fitzpatrick, and their Gaussian normal approximation / hull.

6100

Legend:
----- Red
·········· Green
-··-··- Blue
——— Purple
– – – Cyan
-···-···- Chartreuse

**FIG. 61**

EP 2 389 573 B1

Diagram depicting a Histogram / Distribution of R, G and B colors on one of the patient's photographs whose skin phototype is classified as type VI by Fitzpatrick, and their Gaussian normal approximation / hull.

FIG. 62

**Flowchart Illustrating an Algorithm for Determining the Skin Phototype According to the Estimated Values of Mathematical Expectation for Standard R and B colour in sRGB Colour System.**

6300

6310 — Taking a Photograph w/digital Camera

6320 — Analyzing the Photograph Using Quantization

6330 — Calculating Expected Values $\mu_{R,G,B}$ and st. dev. in sRGB Colour System

6340

$\mu_R \leq M_R^{1,u}$
$\mu_B \leq M_B^{1,u}$ — Yes → Type 1

No

$M_R^{2,l} \leq \mu_R \leq M_R^{2,u}$
$M_B^{2,l} \leq \mu_B \leq M_B^{2,u}$ — Yes → Type 2

No

$M_R^{3,l} \leq \mu_R \leq M_R^{3,u}$
$M_B^{3,l} \leq \mu_B \leq M_B^{3,u}$ — Yes → Type 3

No

$M_R^{4,l} \leq \mu_R \leq M_R^{4,u}$
$M_B^{4,l} \leq \mu_B \leq M_B^{4,u}$ — Yes → Type 4

No

$M_R^{5,l} \leq \mu_R \leq M_R^{5,u}$
$M_B^{5,l} \leq \mu_B \leq M_B^{5,u}$ — Yes → Type 5

$M_R^{6,l} \leq \mu_R$
$\mu_B \leq M_B^{6,u}$ — Yes → Type 6

No

$M_R^{1/2,l} \leq \mu_R \leq M_R^{1/2,u}$
$M_B^{1/2,l} \leq \mu_B \leq M_B^{1/2,u}$ — Yes → Type 1/2

No

$M_R^{2/3,l} \leq \mu_R \leq M_R^{2/3,u}$
$M_B^{2/3,l} \leq \mu_B \leq M_B^{2/3,u}$ — Yes → Type 2/3

No

6340

$M_R^{3/4,l} \leq \mu_R \leq M_R^{3/4,u}$
$M_B^{3/4,l} \leq \mu_B \leq M_B^{3/4,u}$ — Yes → Type 3/4

No

$M_R^{4/5,l} \leq \mu_R \leq M_R^{4/5,u}$
$M_B^{4/5,l} \leq \mu_B \leq M_B^{4/5,u}$ — Yes → Type 4/5

No

$M_R^{5/6,l} \leq \mu_R \leq M_R^{5/6,u}$
$M_B^{5/6,l} \leq \mu_B \leq M_B^{5/6,u}$ — Yes → Type 5/6

No

6350 — Undecisive

**FIG. 63**

EP 2 389 573 B1

FIG. 64

**6500** ⟶

## Continue Reading...

### Ullamcorper velit eu nulla dolor eum, at sed vero

Dolore detenit exerci dolor, odio veniam ecc umsan. feugait ut nisl blandit. laoreet aliq uam duis ut magna. Ullamcorper velit eu nulla dolor eum, at sed vero eu nisl ut. Dolore autem at, suscipit vel enim. nulla duis blande niam acc umsan. feugait ut nisi blandit, laoreet aliq uam duis ut magna.

Ullamcorper velit eu nulla dolor emu. at sed vero eu nisl ut. Dolore autem at, suscipit vel enimit. te odio accumsan. Dolore delenit exerci dolor, odio veniam accumsan. feugait ut nisl blandit. laor eet. Dolore delenit exerci dolor. odio veniam acc umsa n. feugait ut risl blandit. laoreet aliquam duis ut magna. Ullamcorper velit eu nulla dolor eum at.

Continue Reading...

### Quis aliquip delenit commodo tation. Consectetuerdolore consequatvel

Dolore delenit exerci dolor odio veniam ecc umsan. feugait ut nisl blandit. laoreet aliq uam duis ut magna Ullamcorper velit eu nulla dolor eum, ai sed vero eu nisl ul. Dolore autem at, suscipit vel enim. nulla duis blande niam acc umsan. feugait ut nisi blandit. laoreet aliq uam duis ut magna.

Ullamcorper velit eu nulla dolor emu. at sed vero eu nisl ut. Dolore autem at, susciplit vel enimit. te odio accumsan. Dolore delenit exerci dolor, odio veniam accumsan. feugait ut nisl blandit. laor eet. Dolore delenit exerci dolor. odio veniam acc umsan. n, feugait ut risl blandit. laoreet aliquam duis ut magna. Ullamcorper velit eu nulla dolor eum, at sed vero eu nisl ut.

Dolre delenit exerci dolor, odio veniam accumsan, feugait ut nisl blandit, laoreet aliquam duis ut manga. Ullamcorper velit eu nulla dolor eum, at sed vero eu nisl ut. Dolore autem at, suscipit vel enim, nulla duis.

Continue Reading...

### Categories

\* Odio lusto consectetuer ea augue. Nostrud qui eratpraesent augue iriure.

\* Ea quis augue. et lincidunt blandit vulputate molestie euismod nostrud

\* Praesent ut ea hendrerit enim tincidunt.

### Archives

\* 2008

   \*October (23)

   \*November (84)

   \*December (13)

**6502**

**FIG. 65**

EP 2 389 573 B1

6600

**Continue Reading...**

## Ullamcorper velit eu nulla dolor eum, at sed vero

Dolore detenit exerci dolor, odio veniam ecc umsan. feugait ut nisl blandit. laoreet aliq uam duis ut magna. Ullamcorper velit eu nulla dolor eum, at sed vero eu nisl ut. Dolore autem at, suscipit vel enim. nulla duis blande niam acc umsan. feugait ut nisi blandit, laoreet aliq uam duis ut magna.

Ullamcorper velit eu nulla dolor emu. at sed vero eu nisl ut. Dolore autem at, susciplit vel enimit. te odio accumsan. Dolore delenit exerci dolor, odio veniam accumsan. feugait ut nisl blandit. laor eet. Dolore delenit exerci dolor. odio veniam acc umsa n. feugait ut risl blandit. laoreet aliquam duis ut magna. Ullamcorper velit eu nulla dolor eum at.

Continue Reading...

### Quis aliquip delenit commodo tation. Consectetuerdolore consequatvel
~6602

Dolore detenit exerci dolor odio veniam ecc umsan. feugait ut nisl blandit. laoreet aliq uam duis ut magna Ullamcorper velit eu nulla dolor eum, ai sed vero eu nisl ul. Dolore autem at, suscipit vel enim. nulla duis blande niam acc umsan. feugait ut nisi blandit. laoreet aliq uam duis ut magna.

Ullamcorper velit eu nulla dolor emu. at sed vero eu nisl ut. Dolore autem at, susciplit vel enimit. te odio accumsan. Dolore delenit exerci dolor, odio veniam accumsan. feugait ut nisl blandit. laor eet. Dolore delenit exerci dolor. odio veniam acc umsan. n, feugait ut risl blandit. laoreet aliquam duis ut magna. Ullamcorper velit eu nulla dolor eum, at sed vero eu nisl ut.

Dolre delenit exerci dolor, odio veniam accumsan, feugait ut nisl blandit, laoreet aliquam duis ut manga. Ullamcorper velit eu nulla dolor eum, at sed vero eu nisl ut. Dolore autem at, suscipit vel enim, nulla duis.

Continue Reading...

◄ Previous / Next ►

**Categories**
* Odio Iusto consectetuer ea augue. Nostrud qul eratpraesent augue irlure.

* Ea quis augue. et lincidunt blandit vulputate molestie euismod nostrud

* Praesent ut ea hendrerit enim tincidunt.

**Archives**
* 2008
  * October (23)
  * November (84)
  * December (13)

**FIG. 66**

EP 2 389 573 B1

**FIG. 67**

6800

EP 2 389 573 B1

**mySkin**

What is your Concern ?

🔍 Acne, Dark Spots, Moisturizers, Dry Skin...    | Show Me Products

Welcome Back
Karen
Edit

🏠 myDashboard

📖 myShelf

♡ Products I Want

myShelf Products Adder

Blog

Forums

Your Skin Profile

70% Complete

**mySkin Blog**

## Ullamcorper velit eu nulla dolor eum

Dolore detenit exerci dolor odio veniam ecc umsan. feugait ut nisl blandit. laoreet aliq uam duis ut magna Ullamcorper velit eu nulla dolor eum, al sed vero eu nisl ul. Dolore autem at, suscipit vel enim. nulla duis blande niam acc umsan. feugait ut nisi blandit. laoreet aliq uam duis ut magna.

Ullamcorper velit eu nulla dolor emu. at sed vero eu nisl ut. Dolore autem at, suscipit vel enimit. te odio accumsan. Dolore delenit exerci dolor, odio veniam accumsan. feugait ut nisl blandit. laor eet. Dolore delenit exerci dolor. odio veniam acc umsa n. feugait ut risl blandit. laoreet aliquam duis ut magna. Ullamcorper velit eu nulla dolor eum at.

Continue Reading...

## Ullamcorper velit eu nulla dolor eum, at sed vero

Dolore detenit exerci dolor odio veniam ecc umsan. feugait ut nisl blandit. laoreet aliq uam duis ut magna Ullamcorper velit eu nulla dolor eum, ai sed vero eu nisl ul. Dolore autem at, suscipit vel enim. nulla duis blande niam acc umsan. feugait ut nisl blandit. laoreet aliq uam duis ut magna.

Ullamcorper velit eu nulla dolor emu. at sed vero eu nisl ut. Dolore autem at, suscipit vel enimit. te odio accumsan. Dolore delenit exerci dolor, odio veniam accumsan. feugait ut nisl blandit. laor eet. Dolore delenit exerci dolor. odio veniam acc umsa n. feugait ut risl blandit. laoreet aliquam duis ut magna. Ullamcorper velit eu nulla dolor eum at.

Continue Reading...

### About the mySkin Blog

Luptatum. nisl dolore odio facilisi vulputate enim accumsan. Nisl nulla dolor. exerci eu duis present qui ut et sed enim et. volutpat in nisl vero. At euismod nibh wisi delenit amet esse amet nostrud, diam ut aliquip nulla delenit. At nostrud et hendrent facilisi.

Wrinkles    12 WSX

### Categories
* Odio lusto consectetuer ea augue. Nostrud qui eratpraesent augue irlure.
* Ea quis augue. et lincidunt blandit vulputate molestie euismod nostrud
* Praesent ut ea hendrerit enim tincidunt.

### Archives
* 2008
  * October (23)
  * November (84)
  * December (13)

**FIG. 68**

175

6900

Comments (12)

Jacksonfive (July 20, 2008 at 11:13 PM)

Dolore delenit exerci dolor, odio veniam accumsan, feugait ut nisl blandit, laoreet ailquam duis ut magna. Ullamcorper velit eu nulla dolor eum, at sed vero eu nist ut.

jacksonfive (July 20, 2008 at 11:13 PM)

Quis aliquip delenit common tation. Consectetuer dolore consequatvel, eu duis aliquip qui, lobortis nonummy qui volutpat ut nostrud vulputate. Nibh commodo ut consectetuer tation nostrud, dolore dignissim vulputate minim augue. Nulla veniam ex, esse illum, feugait tincidunt quis tation minim, enim nostrud in. Exerci eros aliquam tation esse et delenit ut odio molestie te enim vel eros consequat esse aliquip. Hendrerit feugait qui isuto lorem veniam vel, feugait sciurus?

Wrinkles

12 wsx

Add Comment

Submit Comment

**FIG. 69**

7000

**Comments (12)**

jacksonfive (July 20, 2008 at 11:13 PM)

Dolore delenit exerci dolor, odio veniam accumsan, feugait ut nisl blandit, laoreet aliquam duis ut magna. Ullamcorper velit eu nulla dolor eum, at sed vero eu nist ut.

jacksonfive (July 20, 2008 at 11:13 PM)

Quis aliquip delenit common tation. Consectetuer dolore consequatvel, eu duis aliquip qui, lobortis nonummy qui volutpat ut nostrud vulputate. Nibh commodo ut consectetuer tation nostrud, dolore dignissim vulputate minim augue. Nulla veniam ex, esse illum, feugait tincidunt quis tation minim, enim nostrud in. Exerci eros aliquam tation esse et delenit ut odio molestie te enim vel eros consequat esse aliquip. Hendrerit feugait qui isuto lorem veniam vel, feugait sciurus?

**Add Comment**

Wrinkles

12 WSX

I'd like to comment about how effective this product was for my skin...

Submit Comment

**FIG. 70**

EP 2 389 573 B1

FIG. 71

EP 2 389 573 B1

7200

7202

7208 | 7212

7210

7204

7214

7216  7218  7220

7206

**FIG. 72**

EP 2 389 573 B1

7300

7302 ←→ 7304

7306

FIG. 73

EP 2 389 573 B1

**FIG. 74**

7500

7502
( Start )

7504
Convolute Data Associated with First Set of Images of Blood Plasma Sample Captured Using White Light (or Unangled White Light)

7506
Convolute Data Associated with Second Set of Images of Blood Plasma Sample Captured Using White Light (or Polarized White Light)

7508
Compare Extrema (i.e. Maxima and Minima) (or Extreme) Positions of Pair of Unique Convolutions Generated by Convolution of Data from the First and Second Set of Images

7510
Determine Distance Between Minimum and Maximum (or Extremum) Intensity Positions in Convoluted Red (R) Minus Blue (B) Spectral Plots from the pair of Unique Convolutions to Generate Numerical (or Quantitative) Blood Plasma Type

7512
( End )

**FIG. 75**

FIG. 76A

FIG. 76B

FIG. 77A

**FIG. 77B**

FIG. 78A

FIG. 78B

FIG. 79A

**FIG. 79B**

FIG. 80A

**FIG. 80B**

EP 2 389 573 B1

8100

8102

8104

8106

**FIG. 81**

**FIG. 82**

8300

8302
( Start )

8304
Convolute Data Associated with First Set of Images of Pap test Plasma Sample Captured Using White Light (or Unangled White Light)

8306
Convolute Data Associated with Second Set of Images of Pap test Plasma Sample Captured Using White Light (or Polarized White Light)

8308
Compare Extrema (i.e. Maxima and Minima) (or Extreme) Positions of Pair of Unique Convolutions Generated by Convolution of Data from the First and Second Set of Images

8310
Determine Distance Between Minimum and Maximum (or Extremum) Intensity Positions in Convoluted Red (R) Minus Blue (B) Spectral Plots from the pair of Unique Convolutions to Generate Numerical (or Quantitative) Pap test Sample Type

8312
( End )

**FIG. 83**

**FIG. 84A**

**FIG. 84B**

FIG. 84C

FIG. 85A

FIG. 85B

FIG. 85C

EP 2 389 573 B1

**FIG. 86A**

**FIG. 86B**

FIG. 86C

EP 2 389 573 B1

FIG. 87

Group - III        Patient - 16

FIG. 88

EP 2 389 573 B1

Group - IV    Patient - 4

FIG. 89

Group - V          Patient - 7

FIG. 90

**FIG. 91**

EP 2 389 573 B1

**FIG. 92**

EP 2 389 573 B1

9300

```
                    ┌─────────────┐
                    │    Start    │
                    └──────┬──────┘
                           │
                           ▼
    ┌──────────────────────────────────────────────┐
    │   First Imaging Sensor Captures Image of a    │
    │ Material Illuminated with Non-angled White Light │
    └──────────────────────┬───────────────────────┘
                           │
                           ▼
    ┌──────────────────────────────────────────────┐
    │  First Imaging Sensor Captures Image of the   │
    │  Material Illuminated with Angled White Light  │
    └──────────────────────┬───────────────────────┘
                           │
                           ▼
    ┌──────────────────────────────────────────────┐
    │  Second Imaging Sensor Captures Image of the  │
    │ Material Illuminated with Non-angled White Light │
    └──────────────────────┬───────────────────────┘
                           │
                           ▼
    ┌──────────────────────────────────────────────┐
    │   Second Imaging Sensor Captures Image of a   │
    │  Material Illuminated with Angled White Light  │
    └──────────────────────┬───────────────────────┘
                           │
                           ▼
    ┌──────────────────────────────────────────────┐
    │   Third Imaging Sensor Captures Image of the  │
    │ Material Illuminated with Non-angled White Light │
    └──────────────────────┬───────────────────────┘
                           │
                           ▼
    ┌──────────────────────────────────────────────┐
    │   Third Imaging Sensor Captures Image of the  │
    │  Material Illuminated with Angled White Light  │
    └──────────────────────┬───────────────────────┘
                           │
                           ▼
    ┌──────────────────────────────────────────────┐
    │  Fourth Imaging Sensor Captures Image of the  │
    │ Material Illuminated with Non-angled White Light │
    └──────────────────────┬───────────────────────┘
                           │
                           ▼
    ┌──────────────────────────────────────────────┐
    │  Fourth Imaging Sensor Captures Image of the  │
    │  Material Illuminated with Angled White Light  │
    └──────────────────────┬───────────────────────┘
                           │
                           ▼
                    ┌─────────────┐
                    │     End     │
                    └─────────────┘
```

**FIG. 93**

**FIG. 94**

9500

9504

9514

9506

9512

9508

9510

9502

**FIG. 95**

9600

FIG. 96

**9700**

Image of Area to be Excised

**9702**

Visible Melanoma or Suspect Tissue

**9704**

Normal Looking(Visible) Skin, but Containing Unhealthy / Diseased Tissue that must be Excised

**9706**

Healthy Tissue that should remain intact

**9708**

Border between Healthy and Non Healthy Tissue

**9710**

Outline Area where Surgeon Cut Tissue

**FIG. 97**

EP 2 389 573 B1

**9800**

Image of Area to be Excised

9802 — Analysis Image

9804 — Manually Select Border Area

9806 — Automatic Border Area Selection

9808 — Draw Border Area

**FIG. 98**

EP 2 389 573 B1

**9900** ⬅

EP 2 389 573 B1

FIG. 99

FIG. 100

**10100**

**10102**
Illuminating Surface of the Skin

**10104**
Generating a Predetermined Set of wavelengths for a Plurality of Incident Spectral Rays

**10106**
Utilizing Plurality of Reflection Intensity Values for Generating Skin Photo Type

**10108**
Normalizing Plurality of Reflection Intensity Values with Respect to Spectral Source and Spectral Classification Skintype

**10110**
Generate Skin Phototype Output

**FIG. 101**

10200

Incident Rays

Diffusely Reflected Rays

FIG. 102

**(R-B)&(W-P)**
**18.2 Mohm, -4.4 C**

**FIG. 103A**

**P(R - B)**
**18.2 Mohm, -4.4 C**

Wavelength Difference (nm)

**FIG. 103B**

EP 2 389 573 B1

**(R-B)&(W-P)**
**18.2 Mohm 25 C**

**FIG. 104A**

EP 2 389 573 B1

**P(R - B)**
**18.2 Mohm 25 C**

Intensity

Wavelength Difference (nm)

**FIG. 104B**

EP 2 389 573 B1

**EP 2 389 573 B1**

## REFERENCES CITED IN THE DESCRIPTION

**Patent documents cited in the description**

- WO 2009089292 A
- US 2008050438 W
- WO 2008086311 A